# EUROPEAN PATENT APPLICATION

(11) **EP 3 778 556 A1**
(43) Date of publication of application: **17.02.2021**
(21) Application number: 19777908.5
(22) Date of filing: 28.03.2019
(51) Int. Cl.: C07C 219/00, C07C 229/00, C07C 233/00, C07F 9/09, A61K 48/00

(54) **APPLICATION OF COMPOUND OR TRADITIONAL CHINESE MEDICINE EXTRACT IN PREPARATION OF NUCLEIC ACID DELIVERY AGENT, AND RELATED PRODUCTS**

(30) Priority: 29.03.2018 WO PCT/CN2018/081155
(71) Applicant: Institute of Basic Medical Sciences, Chinese Academy of Medical Sciences, Beijing 100005 (CN)
(72) Inventor: JIANG, Chengyu, Beijing 100005 (CN); LI, Xiaoyun, Beijing 100005 (CN); DU, Jianchao, Beijing 100005 (CN); LIANG, Zhu, Beijing 100005 (CN); WANG, Zhiqing, Beijing 100005 (CN); WANG, Chenxuan, Beijing 100005 (CN)
(74) Representative: Lahrtz, Fritz
(86) International application number: PCT/CN2019/080175
(87) International publication number: WO 2019/184991

(57) **Abstract**

The present invention relates to extracting multiple types of compounds or synthetic compounds capable of promoting nucleic acid delivery from a traditional Chinese medicine, promoting the absorption of a nucleic acid such as sRNA and the entry thereof into a target cell by using the extracted compounds or multiple combinations, and promoting the entry of the nucleic acid into a target site in an object body having such a need. The traditional Chinese medicine is selected from traditional Chinese medicine decoction pieces derived from Rhodiola rosea, Herba Taraxaci, Herba andrographitis, and Flos Lonicerae. The nucleic acid is used for treating diseases, such as heart, spleen, kidney, stomach, lung and/or bowel diseases.

## Description

The present application claims the priority of PCT application No. PCT/CN2018/081155 entitled "Application of compound or traditional Chinese medicine extract in the preparation of a nucleic delivery reagent and related products thereof', filed on March 29, 2018, the entirety of which is hereby incorporated by reference.

### Technical Field

The present application relates to various compounds that are extracted from traditional Chinese medicines or are synthetic and that are capable of promoting nucleic acid delivery, and use of the extracted compounds or various combinations thereof to promote the absorption and entry of nucleic acids, such as sRNA, into target cells, and to promote entry into target sites in vivo in a subject in need thereof.

### Background

In the past few decades, the concept of using nucleic acid molecules, including RNA molecules, as therapeutic drugs has moved from concept to clinical reality. In fact, nucleic acid molecules have many properties that make it a therapeutic drug. They can fold to form complex conformations that allow them to bind to proteins, small molecules or other nucleic acids, and some can even form catalytic centers. Small interfering RNA (siRNA), as an effector molecule of RNAi, has an increasingly broad prospect as a therapeutic drug. At present, a variety of siRNA drugs have entered clinical trials, indicating a good development prospect. Generally, siRNA, miRNA and other non-coding small RNA are indiscriminately referred to as small nucleic acids or small RNA (sRNA). However, since nucleic acid molecules are easily degraded and have a relatively short half-life *in vivo,* they are generally considered to be a poor choice as therapeutic drugs.

Therefore, how to efficiently deliver nucleic acid molecules, including small RNA, to a target organ and a target cell *in vivo* in order to achieve their biological activity and therapeutic or preventive effects is a problem to be considered by those skilled in the art.

### Summary of Invention

After extensive tests, the inventor has unexpectedly discovered some lipid components in some traditional Chinese medicines (including *Rhodiola crenulata*, *Taraxacum mongolicum*, *Andrographis paniculata* and *Lonicera japonica*), and these lipids derived from the traditional Chinese medicines can promote absorption/entry of nucleic acids, such as small RNA into cells and/or target parts in a subject in need thereof. In the present invention, the lipid component is synthetic.

Specifically, in one aspect, the present application relates to a compound having the following structure extracted from a traditional Chinese medicine, and use of the compound for the manufacture of a reagent for nucleic acid delivery: 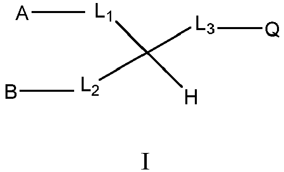
wherein, L₁, L₂ or L₃ is absent, or L₁, L₂ and L₃ are each independently selected from the group consisting of -C(O)O-CH₂-, -CH(OH)-, -C(O)-NH-CH₂-, -CH₂-O-C(O)-, -CH₂-NH-C(O)-, -C(O)O-, -C(O)NH-, -OC(O)-, -NH-C(O)-, -CH₂-, 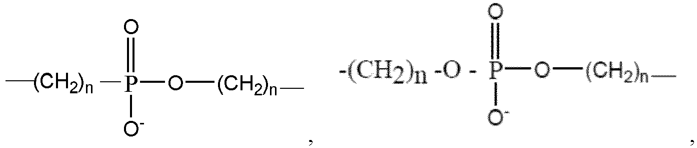 and 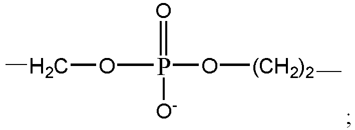 with the proviso that at most two of L1, L₂, and L₃ are absent;
with respect to the divalent groups L₁, L₂, the dash "-" on the left side is linked to the groups A and B, respectively, and the dash "-" on the right side is linked to the central carbon atom;
with respect to the divalent group L₃, the dash "-" on the left side is linked to the central carbon atom, and the dash "-" on the right side is linked to the group Q;
A, B and Q are each independently selected from the group consisting of H, -OH, C₁₋₂₀ alkyl, C₁₋₂₀ alkenyl, C₁₋₂₀ heteroalkyl, C₁₋₂₀ heteroalkenyl, -NH₂, and -NR₃⁺, R is H or C₁₋₆ alkyl; and
n is an integer 0, 1, 2, 3 or 4;

In one embodiment, in saiduse, in the structure of the compound:
L₁ is absent, or L₁ is selected from -C(O)O-CH₂- and -CH(OH)-,
L₂ is absent, or L₂ is selected from -C(O)O- and -C(O)NH-,
L₃ is absent, or L₃ is selected from -C(O)O-, -CH₂-OC(O)-, -CH₂- and 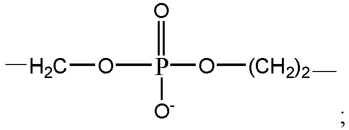
A is selected from the group consisting of H, C₁₋₂₀ alkyl and C₁₋₂₀ alkenyl;
B is selected from the group consisting of H, -NH₂, C₁₋₂₀ alkyl and C₁₋₂₀ alkenyl;
Q is selected from the group consisting of H, -OH, C₁₋₂₀ alkyl and C₁₋₂₀ alkenyl, and -NR₃⁺, wherein R is H or C₁₋₆ alkyl.

In one embodiment, the compound has the following formula:

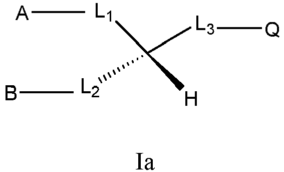

In one embodiment, in the structure of the compound:
A is selected from the group consisting of H, C₁₀₋₂₀ alkyl and C₁₀₋₂₀ alkenyl;
B is selected from the group consisting of H, -NH₂, C₁₀₋₂₀ alkyl and C₁₀₋₂₀ alkenyl;
Q is selected from the group consisting of H, -OH, C₁₀₋₂₀ alkyl and C₁₀₋₂₀ alkenyl, and -NR₃⁺, wherein R is H or C₁₋₄ alkyl.

In one embodiment, in the structure of the compound:
A is selected from the group consisting of H, a straight-chain C₁₅₋₁₈ alkyl group and a straight-chain C₁₅₋₁₈ alkenyl group;
B is selected from the group consisting of H, -NH₂, a straight-chain C₁₅₋₁₈ alkyl group and a straight-chain C₁₅₋₁₈ alkenyl group;
Q is selected from the group consisting of H, -OH, a straight-chain C₁₅₋₁₈ alkyl group and a straight-chain C₁₅₋₁₈ alkenyl group, and -NR₃⁺ wherein R is H or a C₁₋₄ alkyl group; and
the alkenyl group in the A, B, Q has 1-5 double bonds.

In one embodiment, in the A, B, Q of the structure, the alkenyl group has 1-3 double bonds and is in a Z configuration.

In one embodiment, the said compound is selected from the following formulas: 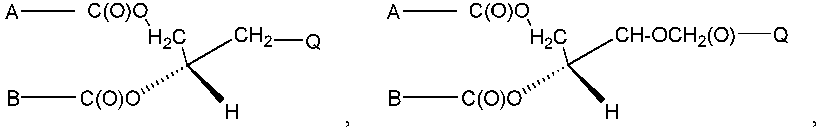 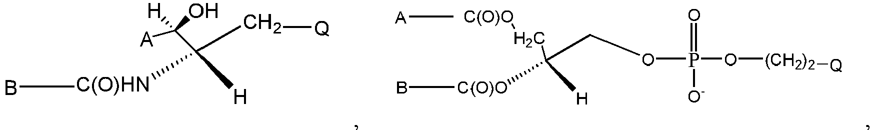 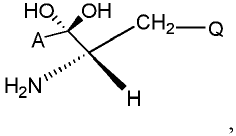 and A-L₃-Q,
wherein
A is selected from a straight-chain C₁₅₋₁₈ alkyl group and a straight-chain C₁₅₋₁₈ alkenyl group;
B is selected from a straight-chain C₁₅₋₁₈ alkyl group and a straight-chain C₁₅₋₁₈ alkenyl group;
Q is selected from the group consisting of H, -OH, a straight-chain C₁₅₋₁₈ alkyl group and a straight-chain C₁₅₋₁₈ alkenyl group, and -NR₃⁺ wherein R is H or methyl; and
L₃ is -C(O)O-.

In one embodiment, the compound is lysolecithin, ceramide, diglyceride, phosphatidylethanolamine, phosphatidylcholine, triglyceride, monogalactosyl diglyceride, sphingosine, phosphatidyl ethanol, monoacylglycerol, fatty acid, platelet activating factor, or dimethyl phosphatidyl ethanolamine.

In one embodiment, the compound is a lipid shown in Table 1.

In one embodiment, the compound is a lipid shown in Table 1 as No. 11, No. 12, No. 41, No. 71, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, or No. 70.

In a second aspect, the present application relates to use of a combination comprising any one or more of the above compounds. Preferably any one or more of the lipids selected Table 1, for the manufacture of a nucleic acid delivery reagent. Preferably, the combination comprises any one of the lipids in Table 1 as No. 11, No. 12, No. 41, No. 71, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, or No. 70, or combination thereof with any one or more of the other lipids in Table 1.

In a third aspect, the present application relates to use of a traditional Chinese medicine for the manufacture of a nucleic acid delivery reagent.

In one embodiment, the traditional Chinese medicine is selected from *Rhodiola crenulata*, *Taraxacum mongolicum*, *Andrographis paniculata* and *Lonicera japonica* Chinese medicine decoction pieces.

In one embodiment, the reagent comprises a compound extracted from a traditional Chinese medicine. Preferably, the reagent comprises any one or more of the above compounds, preferably any one or more lipids selected from Table 1. Preferably, the reagent comprises any one of the lipids shown in Table 1 as No. 11, No. 12, No. 41, No. 71, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, or No. 70, or its combination with any one or more of the other lipids shown in Table 1.

In one embodiment, the compound is extracted by decoction of a traditional Chinese medicine. In another embodiment, the compound is extracted by soaking the traditional Chinese medicine pieces in water, followed by performing intense heating and slow heating sequentially, and then the heated Chinese medicine soup is concentrated, and then is sequentially added with chloroform-methanol, chloroform and water for stirring, and the chloroform layer is obtained.

In one embodiment, the compound has the structure shown in any one of the preceding embodiments.

In one embodiment, the compound is selected from lysolecithin, ceramide, diglyceride, phosphatidylethanolamine, phosphatidylcholine, triglyceride, monogalactosyldiglyceride, (neural) sphingosine, phosphatidyl ethanol, monoacylglycerol, fatty acid, platelet activating factor, or dimethyl phosphatidyl ethanolamine.

In one embodiment, wherein the compound is selected from Table 1.

In one embodiment, wherein the compound is the lipid shown in Table 1 as No. 11, No. 12, No. 41, No. 71, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, or No. 70.

In one embodiment, wherein the delivery comprises *in vitro* cell delivery, or *in vivo* gastrointestinal delivery.

In one embodiment, the use includes the manufacture of lipid nucleic acid mixture.

In one embodiment, the lipid nucleic acid mixture is manufactured by a boiling method, or by a reverse evaporation method, or by direct mixing.

In one embodiment, temperature in said boiling method is from about 25 °C to about 100 °C, preferably from about 80 °C to about 100 °C; temperature in the reverse evaporation method is from about 25 °C to about 70 °C, preferably about 55 °C.

In a fourth aspect, the present application relates to a pharmaceutical composition comprising a compound of the structure of any one of the preceding embodiments and a nucleic acid. Preferably, the said pharmaceutical composition comprises any one or more of the above compounds, preferably one or more lipids selected from Table 1. Preferably, the pharmaceutical composition comprises any one of the lipids shown in Table 1 as No. 11, No. 12, No. 41, No. 71, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No.52, No. 53, No. 54, No. 55, No. 56, No. 57, No. 58, No. 59, No. 61, No. 65, No. 66, No. 67, No. 68, No. 69, or No. 70, or its combination with any one or more of the other lipids shown in Table 1, or its combination with any one or more lipids and other related chemicals.

In one embodiment, in said pharmaceutical composition, the lipid and nucleic acid are at least partially or wholly existed in the form of lipid nucleic acid mixture.

In one embodiment, in said pharmaceutical composition, the lipid nucleic acid mixture is manufactured by a boiling method, or by a reverse evaporation method, or by direct mixing.

In one embodiment, in said pharmaceutical composition, temperature in the boiling method is from 25 °C to about 100 °C, preferably from about 80 °C to 100 °C. temperature in the reverse evaporation method is from about 25 °C to about 70 °C, preferably about 55 °C.

In a fifth aspect, the present application relates to a kit comprising the lipid and the nucleic acid of the preceding embodiments, wherein the lipid and nucleic acid are each independently provided in a first container and a second container, the first container and the second container are the same or different. Preferably, the kit comprises any one or more of the above compounds, preferably any one or more lipids selected from Table 1. Preferably, the kit comprises any one of the lipids shown in Table 1 as No. 11, No. 12, No. 41, No. 71, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No.52, No. 53, No. 54, No. 55, No. 56, No. 57, No. 58, No. 59, No. 61, No. 65, No. 66, No. 67, No. 68, No. 69, or No. 70, or its combination with any one or more of the other lipids shown in Table 1, or its combination with any one or more lipids and other related chemicals.

In one embodiment, in said pharmaceutical composition, the lipid and nucleic acid are at least partially or wholly manufactured into lipid nucleic acid mixture immediately prior to use.

In one embodiment, in said pharmaceutical composition, the lipid nucleic acid mixture is manufactured by a boiling method, or by a reverse evaporation method, or by direct mixing.

In one embodiment, in said pharmaceutical composition, temperature in the boiling method is from 25 °C to about 100 °C, preferably about 100 °C, temperature in the reverse evaporation method is from about 25 °C to about 70 °C, preferably about 55 °C.

In a sixth aspect, the present application relates to a method of delivering a nucleic acid into a target cell, comprising providing the nucleic acid in a form of the pharmaceutical composition or the kit of any one of the preceding embodiments.

In a seventh aspect, the present application relates to a method of delivering a nucleic acid into a subject *in vivo* in need thereof, comprising providing the nucleic acid in a form of the pharmaceutical composition or the kit of any one of the preceding.

In one embodiment, in the above method, the subject is a human or an animal, such as a mammal.

In one embodiment, in the above method, the nucleic acid is delivered to blood circulation or a target tissue/cell in a subject *in vivo.*

In one embodiment, the above methodcomprises directly delivering the pharmaceutical composition or the kit of any one of the preceding embodiments to a subject in need thereof by digestive tract.

In any one of the preceding aspects or embodiments, for example in the pharmaceutical composition or the kit, the nucleic acid and the lipid are manufactured for topical administration and/or injection.

In any one of the preceding aspects or embodiments, for example in the pharmaceutical composition or the kit, wherein the nucleic acid and the lipid are manufactured for digestive administration, respiratory administration and/or injection.

In any one of the preceding aspects or embodiments, for example in the pharmaceutical composition or the kit, wherein the nucleic acid and the lipid are manufactured for oral administration, inhalation administration and/or injection.

In any one of the preceding aspects or embodiments, for example in the pharmaceutical composition or the kit, wherein the nucleic acid is a small RNA.

In any one of the preceding aspects or embodiments, for example in the pharmaceutical composition or the kit, wherein the nucleic acid has a stem-loop structure.

In any one of the preceding aspects or embodiments, for example in the pharmaceutical composition or the kit, wherein the small RNA has a length of 14-32 bp or 18-24 bp, for example, a length of 14, 15, 16, 17, 18, 19, 20, 21, 22, 23, 24, 25, 26, 27, 28, 29, 30, 3 1 and 32 bp.

In any one of the preceding aspects or embodiments, the pharmaceutical composition, the kit or the compound can be orally administered.

In any one of the preceding aspects or embodiments, the nucleic acid can be used for treating a disease, for example heart, spleen, kidney, gastric and/or intestinal diseases, such as cancer, e.g., gastric cancer or lung cancer.

In any one of the preceding aspects or embodiments, a lipid combination can be used, and the lipid combination is any one of the following: a lipid combination of No. 8: No. 41=6:1; a lipid combination of No. 38:No. 41=6:1; a lipid combination of No. 39:No. 41=6:1; a lipid combination of No. 40:No. 41=6:1; a lipid combination of No. 38:No. 12:No. 41:No. 29=1:2:1:1; a lipid combination of No. 40:No. 12:No. 41=2:4:3; a lipid combination of No. 12:No. 41=1:6; a lipid combination of No. 12:No. 41=1:1; a lipid combination of No. 12:No. 41=6:1; a lipid combination of No. 40:No. 12:No. 41=2:2:2; a lipid combination of No. 4:No. 12:No. 41=1:1:1; DG combination of No. 1:No.2:No.3:No. 19:No.35=1: 1:1:1:1; TG combination of No. 6:No. 9:No. 10:No. 13:No. 15:No. 16:No. 18:No. 20:No. 21:No. 22:No. 23:No. 24:No. 25:No. 26:No. 27:No. 28:No. 32:No. 33 =1:1:1:1:1:1:1:1:1:1:1:1:1:1:1:1:1:1; LPC combination of No. 36:No. 37=1:1; PC combination of No. 11:No. 12=1:1; PE combination of No. 8:No. 38=1:1; Cer combination of No. 4:No. 14=1:1; So combination of No. 17:No. 30:No. 31=1:1:1; an equal volume combination of No. 1-36 without No. 5, No. 7; an equal volume combination of No. 1-36 without No. 5, No. 7, No. 34; an equal volume combination of No. 1-36 without No. 5, No. 7, No. 1, No. 2, No. 3, No. 19, No. 35; an equal volume combination of No. 1-36 without No. 5, No. 7, No. 6, No. 9, No. 10, No. 13, No. 15, No. 16, No. 18, No. 20, No. 21, No. 22, No. 23, No. 24, No. 25, No. 26, No. 27, No. 28, No. 32, No. 33; an equal volume combination of No. 1-36 without No. 5, No. 7, No. 36, No. 37; an equal volume combination of No. 1-36 without No. 5, No. 7, No. 11, No. 12; an equal volume combination of No. 1-36 without No. 5, No. 7, No. 8 ; an equal volume combination of No. 1-36 without No. 5, No. 7, No. 4, No. 14; an equal volume combination of No. 1-36 without No. 5, No. 7, No. 29; a lipid combination of No. 1:No. 34=2:1; a lipid combination of No. 1: said DG composition=2:1; a lipid combination of No. 1: said TG composition=2:1; a lipid combination of No. 1: said LPC composition=2:1; a lipid combination of No. 1:No. 8=2:1; a lipid combination of No. 1:No. 12=2:1; a lipid combination of No. 1: said Cer composition=2:1; a lipid combination of No. 1: said So composition=2:1; a lipid combination of No. 1:No. 29=2:1; a lipid combination of No. 1:No. 8:No. 12=1:1:1; a lipid combination of No. 8:No. 34=2:1; a lipid combination of No. 8: said DG composition=2:1; a lipid combination of No. 8: said TG composition=2:1; a lipid combination of No. 8: said LPC composition=2:1; a lipid combination of No. 8:No. 37=4:1; a lipid combination of No. 8:No. 12=2:1; a lipid combination of No. 8: said Cer composition=2: 1 ; a lipid combination of No. 8: said So composition=2:1; a lipid combination of No. 8:No. 31=6:1; a lipid combination of No. 8:No. 29=2:1; a lipid combination of No. 12:No. 34=2:1; a lipid combination of No. 12: said DG composition=2:1; a lipid combination of No. 12: said TG composition=2:1; a lipid combination of No. 12: said LPC composition=2:1; a lipid combination of No. 12:No. 8=2:1; a lipid combination of No. 12: said Cer composition=2:1; a lipid combination of No. 12: said So composition=2:1; a lipid combination of No. 12:No. 29=2:1; a lipid combination of No. 12:No. 8:No. 1&2=2:1:1; a lipid combination of No. 12:No. 8:No. 15=2:1:1; a lipid combination of No. 12:No. 8:No. 36&37=2:1:1; a lipid combination of No. 12:No. 8:No. 11=2:1:1; a lipid combination of No. 12:No. 8:No. 12=2:1:1; a lipid combination of No. 12:No. 8:No. 4=2:1:1; a lipid combination of No. 12:No. 8:No. 31=2:1:1; a lipid combination of No. 12:No. 8:No. 29=2:1:1; a lipid combination of No. 12:No. 8:No. 34=3:2:1; a lipid combination of No. 12:No. 8:No. 34=4:2:3; a lipid combination of No. 12:No. 8:No. 2=4:2:3; a lipid combination of No. 12:No. 8:No. 2=16:8:3; a lipid combination of No. 12:No. 8:No. 32=4:2:3; a lipid combination of No. 12:No. 8:No. 37=4:2:3; a lipid combination of No. 12:No. 8:No. 11=4:2:3; a lipid combination of No. 12:No. 8:No. 38=4:2:3; a lipid combination of No. 12:No. 8:No. 4=4:2:3; a lipid combination of No. 12:No. 8:No. 31=4:2:3; a lipid combination of No. 12:No. 8:No. 29=4:2:3; a lipid combination of No. 12:No. 8:No. 29:No. 31=2:1:1:1; a lipid combination of No. 12:No. 8:No. 29:No. 31:No. 34=4:2:2:2:5; a lipid combination of No. 12:No. 8:No. 29:No. 31:No. 2=4:2:2:2:5; a lipid combination of No. 12:No. 8:No. 29:No. 31:No. 32=4:2:2:2:5; a lipid combination of No. 12:No. 8:No. 29:No. 31:No. 11=4:2:2:2:5; a lipid combination of No. 12:No. 8:No. 29:No. 31:No. 37=4:2:2:2:5; a lipid combination of No. 12:No. 8:No. 29:No. 31:No. 38=4:2:2:2:5; a lipid combination of No. 12:No. 8:No. 29:No. 31:No. 4=4:2:2:2:5; a lipid combination of No. 12:No. 8:No. 29:No. 31:No. 4:No. 1:No. 16=2:1:1:3:2:2:3; a lipid combination of No. 1:No. 8:No. 12:No. 1&2=2:2:2:3; a lipid combination of No. 1:No. 8:No. 12:No. 15=2:2:2:3; a lipid combination of No. 1:No. 8:No. 12:No. 36&37=2:2:2:3; a lipid combination of No. 1:No. 8:No. 12:No. 12=2:2:2:3; a lipid combination of No. 1:No. 8:No. 12:No. 4=2:2:2:3; a lipid combination of No. 1:No. 8:No. 12:No. 31=2:2:2:3; a lipid combination of No. 1:No. 8:No. 12:No. 29=2:2:2:3; a lipid combination of No. 8:No. 34:No. 1&2=2:1:1; a lipid combination of No. 8:No. 34:No. 15=2:1:1; a lipid combination of No. 8:No. 34:No. 36&37=2:1:1; a lipid combination of No. 8:No. 34:No. 12=2:1:1; a lipid combination of No. 8:No. 34:No. 4=2:1:1; a lipid combination of No. 8:No. 34:No. 31=2:1:1; a lipid combination of No. 8:No. 34:No. 29=2:1:1; a lipid combination of No. 8:No. 31:No. 34=12:3:5; a lipid combination of No. 8:No. 31:No. 2=12:3:5; a lipid combination of No. 8:No. 31:No. 37=12:3:5; a lipid combination of No. 8:No. 31:No. 11=12:3:5; a lipid combination of No. 8:No. 31:No. 12=12:3:5; a lipid combination of No. 8:No. 31:No. 4=12:3:5; a lipid combination of No. 8:No. 31:No. 29=12:3:5; a lipid combination of No. 8:No. 31:No. 32=12:3:5; a lipid combination of No. 8:No. 4:No. 34=12:3:5; a lipid combination of No. 8:No. 4:No. 2=12:3:5; a lipid combination of No .8:No. 4:No. 37=12:3:5; a lipid combination of No. 8:No. 4:No. 12=12:3:5; a lipid combination of No. 8:No. 4:No. 31=12:3:5; a lipid combination of No. 8:No. 4:No. 29=12:3:5; a lipid combination of No. 8:No. 4:No. 32=12:3:5; a lipid combination of No. 38:No. 34=2:1; a lipid combination of No. 38:No. 1=2:1; a lipid combination of No. 38:No. 2=2:1; a lipid combination of No. 38:No. 1&2=2:1; a lipid combination of No. 38:No. 15=2:1; a lipid combination of No. 38:No. 32=2:1; a lipid combination of No. 38:No. 37=2:1; a lipid combination of No. 38:No. 37=4:1; a lipid combination of No. 38:No. 11=2:1; a lipid combination of No. 38:No. 12=2:1; a lipid combination of No. 38:No. 11&12=2:1; a lipid combination of No. 38:No. 12=4:1; a lipid combination of No. 38:No. 8=2:1; a lipid combination of No. 38:No. 4=2:1; a lipid combination of No.38: So (30)=2:1; a lipid combination of No. 38:No. 31=2:1; a lipid combination of No. 38:No. 29=2:1; a lipid combination of No. 1:No. 38:No. 12:No. 34=2:2:2:3; a lipid combination of No. 1:No. 38:No. 12:No. 15=2:2:2:3; a lipid combination of No. 1:No. 38:No. 12:No. 37=2:2:2:3; a lipid combination of No. 1:No. 38:No. 12:No. 8=2:2:2:3; a lipid combination of No. 1:No. 38:No. 12:No. 4=2:2:2:3; a lipid combination of No. 1:No. 38:No. 12:No. 31=2:2:2:3; a lipid combination of No. 1:No. 38:No. 12:No. 29=2:2:2:3; a lipid combination of No. 38:No. 34:No. 1=2:1:3; a lipid combination of No. 38:No. 34:No. 15=2:1:3; a lipid combination of No. 38:No. 34:No. 37=2:1:3; a lipid combination of No. 38:No. 34:No. 12=2:1:3; a lipid combination of No. 38:No. 34:No. 8=2:1:3; a lipid combination of No. 38:No. 34:No. 4=2:1:3; a lipid combination of No. 38:No. 34:No. 31=2:1:3; a lipid combination of No. 38:No. 34:No. 29=2:1:3; a lipid combination of No. 38:No. 12:No. 1=2:1:3; a lipid combination of No. 38:No. 12:No. 2=4:1:3; a lipid combination of No. 38:No. 12:No. 15=2:1:3; a lipid combination of No. 38:No. 12:No. 37=2:1:3; a lipid combination of No. 38:No. 12:No. 8=2:1:3; a lipid combination of No. 38:No. 12:No. 4=2:1:3; a lipid combination of No. 38:No. 12:No. 31=2:1:3; a lipid combination of No. 38:No. 12:No. 29=2:1:3; a lipid combination of No. 38:No. 12:No. 1:No. 15:No. 34=22:22:22:33:36; a lipid combination of No. 38:No. 12:No. 1:No. 15:No. 37=22:22:22:33:36; a lipid combination of No. 38:No. 12:No. 1:No. 15:No. 4=22:22:22:33:36; a lipid combination of No. 38:No. 12:No. 1:No. 15:No. 31=22:22:22:33:36; a lipid combination of No. 38:No. 12:No. 1:No. 15:No. 29=22:22:22:33:36; a lipid combination of No. 38:No. 34:No. 37:No. 1=44:22:33:36; a lipid combination of No. 38:No. 34:No. 37:No. 15=44:22:33:36; a lipid combination of No. 38:No. 34:No. 37:No. 12=44:22:33:36; a lipid combination of No. 38:No. 34:No. 37:No. 4=44:22:33:36; a lipid combination of No. 38:No. 34:No. 37:No. 31=44:22:33:36; a lipid combination of No. 38:No. 12:No. 4:No. 34=44:22:33:36; a lipid combination of No. 38:No. 12:No. 4:No. 1=44:22:33:36; a lipid combination of No. 38:No. 12:No. 4:No. 15=44:22:33:36; a lipid combination of No. 38:No. 12:No. 4:No. 37=44:22:33:36; a lipid combination of No. 38:No. 12:No. 4:No. 37=8:2:5:3; a lipid combination of No. 38:No. 12:No. 4:No. 31=44:22:33:36; a lipid combination of No. 38:No. 12:No. 4:No. 29=44:22:33:36; a lipid combination of No. 38:No. 12:No. 4:No. 29:No. 34=88:44:66:72:135; a lipid combination of No. 38:No. 12:No. 4:No. 29:No. 1=88:44:66:72:135; a lipid combination of No. 38:No. 12:No. 4:No. 29:No. 15=88:44:66:72:135; a lipid combination of No. 38:No. 12:No. 4:No. 29:No. 37=88:44:66:72:135; a lipid combination of No. 38:No. 12:No. 4:No. 29:No. 31=88:44:66:72:135; a lipid combination of No. 38:No. 12:No. 4:No. 2=20:10:15:9; a lipid combination of No. 38:No. 12:No. 4:No. 6=20:10:15:9; a lipid combination of No. 38:No. 12:No. 4:No. 17=20:10:15:9; a lipid combination of No. 38:No. 12:No. 4:No. 29=20:10:15:9; a lipid combination of No. 38:No. 12:No. 4:No. 34=20:10:15:9; a lipid combination of No. 38:No. 12:No. 4:No. 37=20:10:15:9; a lipid combination of No. 38:No. 12:No. 31:No. 34=2:1:3:3; a lipid combination of No. 38:No. 12:No. 31:No. 1=2:1:3:3; a lipid combination of No. 38:No. 12:No. 31:No. 15=2:1:3:3; a lipid combination of No. 38:No. 12:No. 31:No. 37=2:1:3:3; a lipid combination of No. 38:No. 12:No. 31:No. 4=2:1:3:3; a lipid combination of No. 38:No. 12:No. 31:No. 29=2:1:3:3; a lipid combination of No. 38:No. 34:No. 37:No. 31:No. 1=88:44:66:72:135; a lipid combination of No. 38:No. 34:No. 37:No. 31:No. 15=88:44:66:72:135; a lipid combination of No. 38:No. 34:No. 37:No. 31:No. 12=88:44:66:72:135; a lipid combination of No. 38:No. 34:No. 37:No. 31:No. 4=88:44:66:72:135; a lipid combination of No. 38:No. 34:No. 37:No. 31:No. 29=88:44:66:72:135; a lipid combination of No. 38:No. 37:No. 34=4:2:3; a lipid combination of No. 38:No. 37:No. 1=4:2:3; a lipid combination of No. 38:No. 37:No. 2=4:2:3; a lipid combination of No. 38:No. 37:No. 1&2=4:2:3; a lipid combination of No. 38:No. 37:No. 2=32:8:5; a lipid combination of No. 38:No. 37:No. 32=32:8:5; a lipid combination of No. 38:No. 37:No. 15=4:2:3; a lipid combination of No. 38:No. 37:No. 32=4:2:3; a lipid combination of No. 38:No. 37:No. 8=4:2:3; a lipid combination of No. 38:No. 37:No. 11=4:2:3; a lipid combination of No. 38:No. 37:No. 12=4:2:3; a lipid combination of No. 38:No. 37:No. 11&12=4:2:3; a lipid combination of No. 38:No. 37:No. 12=4:1:1; a lipid combination of No. 38:No. 37:No. 4=4:2:3; a lipid combination of No. 38:No. 37:No. 30=4:2:3; a lipid combination of No. 38:No. 37:No. 31=4:2:3; a lipid combination of No. 38:No. 37:No. 29=4:2:3; a lipid combination of No. 8:No. 37:No. 32=4:1:2; a lipid combination of No. 8:No. 37:No. 2=4:1:2; a lipid combination of No. 38:No. 37:No. 15:No. 34=64:16:10:45; a lipid combination of No. 38:No. 37:No. 15:No. 1=64:16:10:45; a lipid combination of No. 38:No. 37:No. 15:No. 12=64:16:10:45; a lipid combination of No. 38:No. 37:No. 15:No. 4=64:16:10:45; a lipid combination of No. 38:No. 37:No. 15:No. 31=64:16:10:45; a lipid combination of No. 38:No. 37:No. 15:No. 29=64:16:10:45; a lipid combination of No. 38:No. 2:No. 37=4:2:3; a lipid combination of No. 38:No. 2:No. 31=4:2:3; a lipid combination of No. 38:No. 2:No. 29=4:2:3; a lipid combination of No. 38:No. 2:No. 34=4:2:3; a lipid combination of No. 38:No. 2:No. 32=4:2:3; a lipid combination of No. 38:No. 2:No. 12=4:2:3; a lipid combination of No. 38:No. 2:No. 12=4:5: 1; a lipid combination of No. 38:No. 2:No. 4=4:2:3. In one embodiment, lipids No. 1&2, No. 11&12 or No. 36&37 can represent lipids No. 1 and No. 2 in any ratio, lipids No. 11 or No. 12 in any ratio, lipids No. 36 and No. 37 in any ratio, respectively, wherein the working concentrations of the lipids are shown in Table 1 and Examples.

The present application also provides a compound having a structure of the following formula, a combination or a composition comprising the compound, and a method of using the compound, combination or composition for nucleic acid delivery, and use of the compound, combination or composition for the manufacture of a nucleic acid delivery reagent: 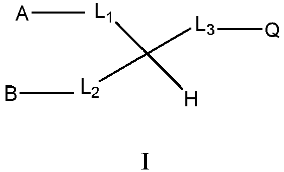 wherein
L₁, L₂ or L₃ is absent, or L₁, L₂ and L₃ are each independently selected from the group consisting of -C(O)O-CH₂-, -CH(OH)-, -CH₂-OC(O), -C(O)O-, -C(O)NH-;
with the proviso that at most two of L₁, L₂ and L₃ are absent;
with respect to the divalent groups L₁, L₂, the dash "-" on the left side is linked to the groups A and B, respectively, and the dash "-" on the right side is linked to the central carbon atom;
with respect to the divalent group L₃, the dash "-" on the left side is linked to the central carbon atom, and the dash "-" on the right side is linked to the group Q;
A, B and Q is independently selected from the group consisting of H, -OH, C₁₋₂₀ alkyl, C₁₋₂₀ alkenyl, -NH₂, and -NR₃⁺, R is H or C₁₋₆ alkyl.

In one embodiment, the compound can have the following structure: 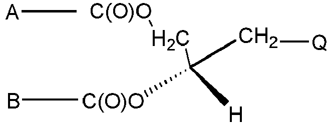 wherein
A is selected from the group consisting of a straight-chain C₁₀₋₂₀ alkyl group and a straight-chain C₁₀₋₂₀ alkenyl group;
B is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₅₋₂₀ alkyl group and a straight-chain C₁₅₋₂₀ alkenyl group;
B is selected from the group consisting of a straight-chain C₁₅₋₂₀ alkyl group and a straight-chain C₁₅₋₂₀ alkenyl group;
Q is -OH;
preferably,
A is selected from the group consisting of a straight-chain C₁₅₋₁₈ alkyl group and a straight-chain C₁₅₋₁₈ alkenyl group;
B is selected from the group consisting of a straight-chain C₁₅₋₁₈ alkyl group and a straight-chain C₁₅₋₁₈ alkenyl group;
Q is -OH.

In another embodiment, the said compound can have the following structure: 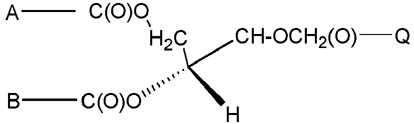 wherein
A is selected from the group consisting of a straight-chain C₁₀₋₂₀ alkyl group and a straight-chain C₁₀₋₂₂ alkenyl group;
B is selected from the group consisting of a straight-chain C₁₀₋₂₀ alkyl group and a straight-chain C₁₀₋₂₂ alkenyl group;
Q is selected from the group consisting of a straight-chain C₁₀₋₂₀ alkyl group and a straight-chain C₁₀₋₂₂ alkenyl group;
preferably,
A is selected from the group consisting of a straight-chain C₁₅₋₁₈ alkyl group and a straight-chain C₁₅₋₂₂ alkenyl group;
B is selected from the group consisting of a straight-chain C₁₅₋₁₈ alkyl group and a straight-chain C₁₅₋₂₂ alkenyl group;
Q is selected from the group consisting of a straight-chain C₁₅₋₁₈ alkyl group and a straight-chain C₁₅₋₂₂ alkenyl group;
preferably,
A is selected from the group consisting of a straight-chain C₁₅₋₁₈ alkyl group and a straight-chain C₁₅₋₂₀ alkenyl group;
B is selected from the group consisting of a straight-chain C₁₅₋₁₈ alkyl group and a straight-chain C₁₅₋₂₀ alkenyl group;
Q is selected from the group consisting of a straight-chain C₁₅₋₁₈ alkyl group and a straight-chain C₁₅₋₂₀ alkenyl group.

In another embodiment, the compound can have the following structure: 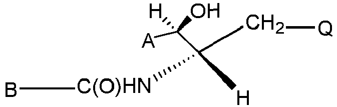 wherein
A is selected from the group consisting of a straight-chain C₁₀₋₂₀ alkyl group and a straight-chain C₁₀₋₂₀ alkenyl group;
B is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₅₋₂₀ alkyl group and a straight-chain C₁₅₋₁₈ alkenyl group;
B is selected from the group consisting of a straight-chain C₁₅₋₁₈ alkyl group and a straight-chain C₁₅₋₁₈ alkenyl group;
Q is -OH;
preferably,
A is a straight-chain C₁₅₋₂₀ alkyl group;
B is a straight-chain C₁₅₋₁₈ alkyl group;
Q is -OH.

In another embodiment, the compound can have the following structure: 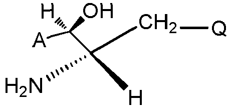 wherein
A is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₀₋₂₀ alkyl group and a straight-chain C₁₅₋₁₈ alkenyl group;
Q is -OH;
preferably,
A is a straight-chain C₁₅₋₂₀ alkyl group;
Q is -OH.

In any aspects or embodiments of the present application, the compound can be a compound as described above.

In any aspects or embodiments of the present application, the compound, the extract or the composition can be derived synthetically, naturally or extracted from a traditional Chinese medicine.

In any aspects or embodiments of the present application, delivery of nucleic acid is delivery of nucleic acid to a cell or an organ. In each embodiment, the organ is selected from the group consisting of heart, spleen, kidney, stomach, lung and intestine.

The above technical solutions provided by the present application can significantly improve the high-efficiency targeted delivery of a nucleic acid, and overcome the shortcomings in the prior art of nucleic acid liposome, including low encapsulation rate, poor safety, poor stability, complicated manufacture process, heterogeneity in product, low reproducibility, and the to-be-improved targeting.

**Table 1-1 List of 69 lipids derived from traditional Chinese medicine**

| **No.** | **Manufacturer** | **Catalogue #** | **Abbreviation** | **Working Concentration (mg/mL)** |
|---|---|---|---|---|
| 1 | Avanti | 110882 | DG(18:0/18:0/0:0) | 5 |
| 2 | Avanti | 110883 | DG(18:0/16:0/0:0) | 5 |
| 3 | Avanti | 800816C | DG(16:0/16:0/0:0) | 10 |
| 4 | Avanti | 860627P | C18 Dihydroceramide (d18:0/18:0) | 10 |
| 6 | Avanti | 110613 | TG(18:1/18:1/18:1) | 1 |
| 8 | Avanti | 850756C | PE(16:0/18:2) | 10 |
| 9 | Avanti | 110521 | TG(16:0/16:0/18: 1) | 5 |
| 10 | Avanti | 111000 | TG(16:0/16:0/16:0) | 10 |
| 11 | Avanti | 850468 | PC(18:0/18:2) | 10 |
| 12 | Avanti | 850458C | PC(16:0/18:2) | 10 |
| 13 | Avanti | 111002 | TG(18:2/18:2/18:2) | 10 |
| 14 | Avanti | 860634P | C16 Dihydroceramide (d18:0/16:0) | 5 |
| 15 | Sigma | P8577 | TG(16:0/18:1/18:2) | 1 |
| 16 | Nu-chek | T-160 | TG(18:0/18:0/18:0) | 1 |
| 17 | Matreya | 1326 | So(d16:0) | 1 |
| 18 | Sigma | D1782 | TG(16:0/18:1/18:1) | 5 |
| 19 | Larodan | 32-1656-7 | DG(16:0/18:2) | 5 |
| 20 | Larodan | 34-1603-7 | TG(16:0/16:0/18:2) | 5 |
| 21 | Larodan | 34-1862-7 | TG(16:0/18:2/18:2) | 5 |
| 22 | Larodan | 34-3003-7 | TG(18:0/16:0/18:1) | 5 |
| 23 | Larodan | 34-1822-7 | TG(18:0/18:1/18:1) | 5 |
| 24 | Larodan | 34-3007-7 | TG(18:0/18:1/18:2) | 5 |
| 25 | Larodan | 34-1827-7 | TG(18:1/18:1/18:2) | 5 |
| 26 | Larodan | 34-1828-7 | TG(18:1/18:1/18:3) | 5 |
| 27 | Larodan | 34-1866-7 | TG(18:1/18:2/18:2) | 5 |
| 28 | Larodan | 34-1855-7 | TG(18:3/18:2/18:2) | 5 |
| 29 | Larodan | 10-1840-4 | FA(18:4) | 5 |
| 30 | Avanti | 110748 | Sphinganine (d18:0) | 5 |
| 31 | Avanti | 110749 | Sphinganine (d20:0) | 1 |
| 32 | Avanti | 110520 | TG(18:0/16:0/16:0) | 5 |
| 33 | Larodan | 34-1810-7 | TG(18:0/16:0/18:0) | 10 |
| 34 | Larodan | 31-1820-7 | MG(18:2p) | 10 |
| 35 | nu-chek | D-251 | DG(18:2/18:2) | 10 |
| 36 | Larodan | 38-1802-0 | LPC(18:2) | 10 |
| 37 | avanti | 791251 | LPC(18:3) | 10 |
| 38 | avanti | 791016 | PE(16:0/16: 1) | 10 |
| 39 | avanti | 792077C | 16:1-18:1PE | 10 |
| 40 | avanti | 792078C | 16:0-22:1 PE | 10 |
| 41 | avanti | 792079P | Sphinganine(d22:0) | 10 |
| 42 | Larodan | 31-2220 | MG(22:2) | 10 |
| 43 | Larodan | 32-1658 | DG(16:0/18:3) | 10 |
| 44 | Larodan | 34-1289 | TG(18:1/18:1/20:4) | 10 |
| 45 | Larodan | 34-1870 | DG(18:3/18:2) | 10 |
| 46 | Larodan | 32-1871 | DG(20:5/18:2) | 10 |
| 47 | Larodan | 34-1880 | TG(18:3/18:2/18:3) | 10 |
| 48 | Larodan | 34-2230 | TG(18:1/22:1/22:1) | 10 |
| 49 | Larodan | 34-3031 | TG(16:0/16:1/18:1) | 10 |
| 50 | Larodan | 34-3032 | TG(16:0/18:1/18:3) | 10 |
| 51 | Larodan | 34-3033 | TG(16:0/18:1/20:4) | 10 |
| 52 | Larodan | 34-3034 | TG(18:3/18:2/20:5) | 10 |
| 53 | Avanti | 792143 | Cer(d16:0/16:0) | 10 |
| 54 | Avanti | 792144 | Cer(d20:0/18:0) | 10 |
| 55 | Avanti | 792145 | Cer(d22:0/18:0) | 10 |
| 56 | Avanti | 792146 | TG(16:0/18:2/18:3) | 10 |
| 57 | Avanti | 792147 | TG(18:1/18:2/18:3) | 10 |
| 58 | Avanti | 792150 | PEt(16:1/16:1) | 10 |
| 59 | Avanti | 792151 | dMePE(16:1/14:0) | 10 |
| 60 | Avanti | 792152 | dMePE(16:1/16:1) | 10 |
| 61 | Avanti | 792153 | dMePE(18:1/14:0) | 10 |
| 62 | Avanti | 792154 | dMePE(16:1/18:1) | 10 |
| 63 | Avanti | 792156 | PC(18:0/18:3(6Z,9Z,12Z )) | 10 |
| 64 | Avanti | 792155 | PE(15:0/24:1(15Z)) | 10 |
| 65 | Avanti | 792157 | PC(20:0/14:1(9Z)) | 10 |
| 66 | Avanti | 792160 | TG(18:0/18:2/18:3) | 10 |
| 67 | Avanti | 792148 | TG(18:1/18:2/20:5) | 10 |
| 68 | Avanti | 792149 | TG(20:5/18:2/18:2) | 10 |
| 69 | Avanti | 792158 | PC(18:1(11Z)-1 6:1(9Z)) | 10 |
| 70 | Larodan | 32-1830-7 | DG(18:3/18:3) | 25 |
| 71 | Larodan | 37-1620-7 | PE(16:0/16:0) | 25 |

**Table 1-2 Description of lipids 1-32**

| **No.** | **Abbreviation** | **IUPAC name** | **Structure** |
|---|---|---|---|
| 1 | DG(18:0/18:0/0:0) | (2S)-1-hydroxy-3-(octadecanoyloxy)prop an-2-yl octadecanoate | 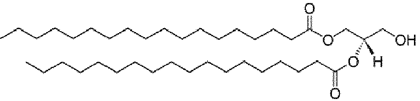 |
| 2 | DG(18:0/16:0/0:0) | (2S)-2-(hexadecanoyloxy)-3-hydroxypropyl octadecanoate | 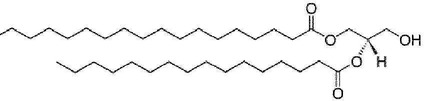 |
| 3 | DG(16:0/16:0/0:0) | (2S)-1-(hexadecanoyloxy)-3-hydroxypropan-2-yl hexadecanoate | 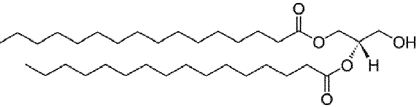 |
| 4 | C18 Dihydroceramide (d18:0/18:0) | N-[(2S,3R)-1,3-dihydroxyoctadecan-2-yl]octadecanamide | 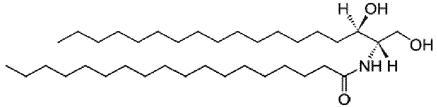 |
| 6 | TG(18:1/18:1/18:1) | 1,3-bis[(9Z)-octadec-9-enoyloxy]propan-2-yl (9Z)-octadec-9-enoate | 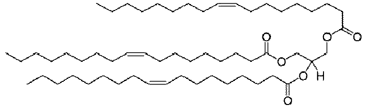 |
| 8 | PE(16:0/18:2) | (2-aminoethoxy) [(2R)-3 - (hexadecanoyloxy)-2-[(9Z,12Z)-octadeca-9,12-dienoyloxy]propoxy]ph osphinic acid | 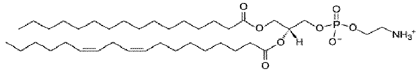 |
| 9 | TG(16:0/16:0/18:1) | (2R)-2,3-bis(hexadecanoyloxy)p ropyl (9Z)-octadec-9-enoate | 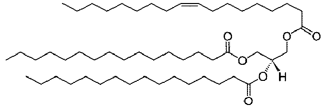 |
| 10 | TG(16:0/16:0 /16:0) | 1,3-bis(hexadecanoyloxy)p ropan-2-yl hexadecanoate | 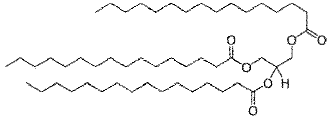 |
| 11 | PC(18:0/18:2) | trimethyl(2-{[(2R)-2-[(9Z,12Z)-octadeca-9,12-dienoyloxy]-3-(octadecanoyloxy)propyl phosphonato]oxy}ethyl)azanium | 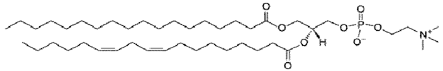 |
| 12 | PC(16:0/18:2) | (2-{[(2R)-3-(hexadecanoyloxy)-2-[(9Z,12Z)-octadeca-9, 12-dienoyloxy]propyl phosphonato]oxy}ethyl )trimethylazanium | 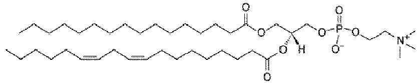 |
| 13 | TG(18:2/18:2/18:2) | 1,3-bis[(9Z,12Z)-octadeca-9,12-dienoyloxy]propan-2-yl (9Z,12Z)-octadeca-9,12-dienoate | 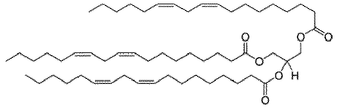 |
| 14 | C16 Dihydroceramide (d18:0/16:0) | N-[(2S,3R)-1,3-dihydroxyoctadecan-2-yl]hexadecanamide | 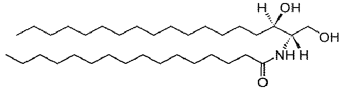 |
| 15 | TG(16:0/18:1/18:2) | 1 -Palmitoyl-2-oleoyl-3 -linoleoyl-rac-glycerol | 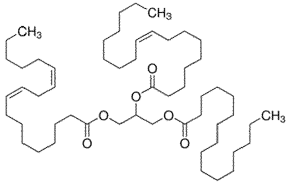 |
| 16 | TG(18:0/18:0/18:0) | 1,3-bis(octadecanoyloxy)pr opan-2-yl octadecanoate | 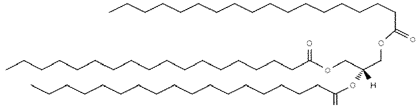 |
| 17 | So(d16:0) | D,L-2-Aminohexadecane-1,3-diol | 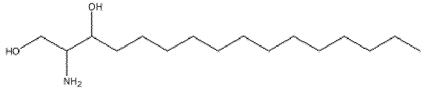 |
| 18 | TG(16:0/18:1/18:1) | 1,2-Di(cis-9-octadecenoyl)-3-hexadecanoyl-rac-glycerol | 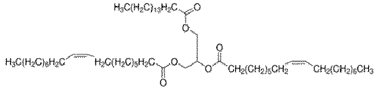 |
| 19 | DG(16:0/18:2) | 1-Palmitoyl-3-Linoleoyl-sn-glycerol | 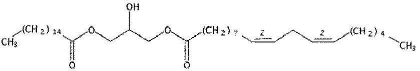 |
| 20 | TG(16:0/16:0/18:2) | 1,2-Palmitoyl-3-Linoleoyl-sn-glycerol | 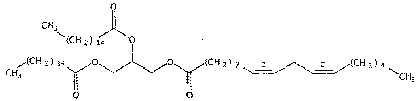 |
| 21 | TG(16:0/18:2/18:2) | 1,2-Linoleoyl-3-Palmitoyl-sn-glycerol | 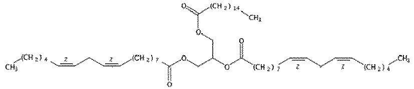 |
| 22 | TG(18:0/16:0/18:1) | 1-Stearoyl-2-Palmitoyl-3 -Oleoyl-sn-glycerol | 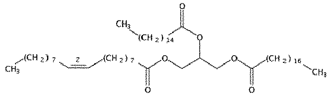 |
| 23 | TG(18:0/18:1/18:1) | 1,2-olein-3-stearin | 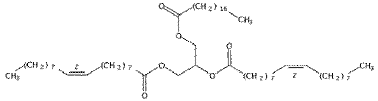 |
| 24 | TG(18:0/18:1/18:2) | 1-Stearoyl-2-Oleoyl-3 -Linoleoyl-sn-glycerol | 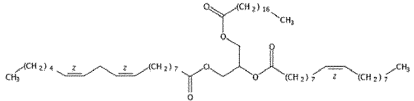 |
| 25 | TG(18:1/18:1/18:2) | 1,2-Oleoyl-3-Linoleoyl-sn-glycerol | 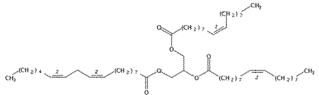 |
| 26 | TG(18:1/18:1/18:3) | 1,2-Oleoyl-3-Linolenoyl-sn-glycerol | 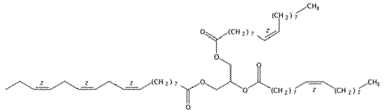 |
| 27 | TG(18:1/18:2/18:2) | 1,2-Linoleoyl-3-Oleoyl-sn-glycerol | 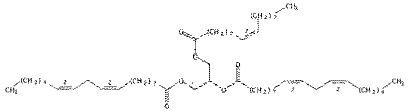 |
| 28 | TG(18:3/18:2/18:2) | 1,2-Linoleoyl-3-Linolenoyl-sn-glycerol | 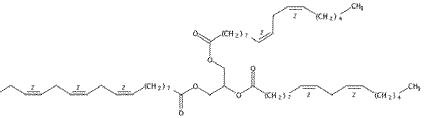 |
| 29 | FA(18:4) | 6c,9c,12c,15c-Octadecatetraenoic Acid | 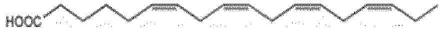 |
| 30 | Sphinganine (d18:0) | (2S,3R)-2-aminooctadecane-1,3 - diol | 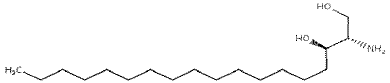 |
| 31 | Sphinganine (d20:0) | (2S,3R)-2-amino-1,3-eicosanediol | 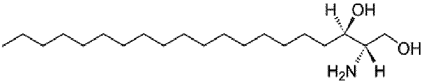 |
| 32 | TG(18:0/16:0/16:0) | (2R)-2,3-bis(hexadecanoyloxy)p ropyl octadecanoate | 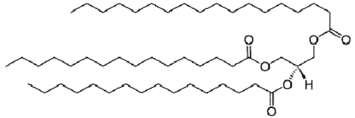 |

**Table 1-2 Description of lipids 33-71**

| No. | Abbreviation | IUPAC Name | Structure |
|---|---|---|---|
| 33 | TG(18:0/16:0/ 18:0) | 1,3-Stearin-2-Palmitin | 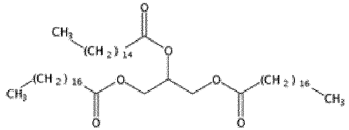 |
| | | 1,3-Octadecanoyl-2-Palmitoyl-glycerol | |
| 34 | MG(18:2p) | (9Z,12Z)-Octadeca-9,12-dienoic acid, monoester with glycerol | 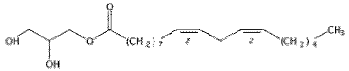 |
| | | 9,12-Octadecadienoic acid | |
| | | (9Z,12Z)-, monoester with 1,2,3-propanetriol | 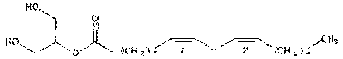 |
| | | 9,12-Octadecadienoic acid, (Z,Z)-, monoester with 1,2,3-propanetriol | |
| 35 | DG(18:2/18:2) | (2S)-1-hydroxy-3-[(9Z,12Z)-octadeca-9, 12-dienoyloxy]propan-2-yl | 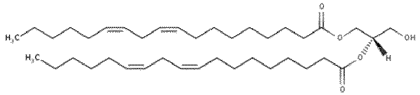 |
| | | (9Z, 12Z)-octadeca-9,12-dienoate , | |
| 36 | LPC(18:2) | 1-Linoleoyl-2-Hydroxy-sn-Glycero-3-Phosphatidylcholine | 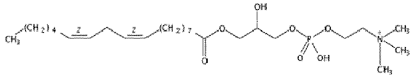 |
| 37 | LPC(18:3) | (2-{ [(2R)-2-hydroxy-3-[(9Z,12Z,15Z)-octadeca-9,12,15-trienoyloxy]propyl phosphonato]oxy}ethyl)tri methylazanium | 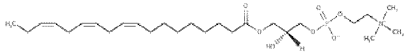 |
| 38 | PE(16:0/16:1) | (2-aminoethoxy)[(2R)-2-[(9Z)-hexadec-9-enoyloxy]-3-(hexadecanoyloxy)propoxy ]phosphinic acid | 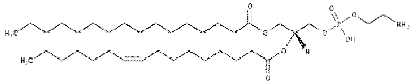 |
| 39 | 16:1-18:1PE | (2-aminoethoxy)[(2R)-3-[(9Z)-hexadec-9-enoyloxy]-2-[(11Z)-octadec-11-enoyloxy]propoxy]phosphi nic acid | 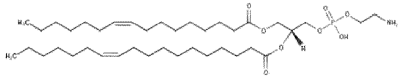 |
| 40 | 16:0-22:1 PE | (2-aminoethoxy)[(2R)-2-[(13Z)-docos-13-enoyloxy]-3-(hexadecanoyloxy)propoxy ]phosphinic acid | 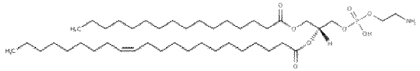 |
| 41 | Sphinganine(d22:0) | | 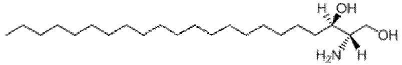 |
| 42 | MG(22:2) | Monodocosadienoin | 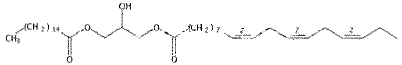 |
| 43 | DG(16:0/18:3) | 1-Palmitin-3-Linolenin | 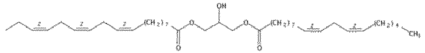 |
| 44 | TG(18:1/18:1/20:4) | 1,2-Olein-3-Arachidonin(5Z,8Z,11Z,14 Z) | 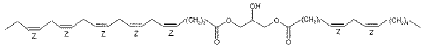 |
| 45 | DG(18:3/18:2) | 1 -Linolein-3-Linolenin | 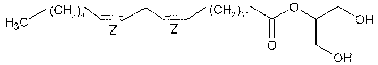 |
| | | | 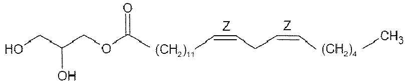 |
| 46 | DG(20:5/18:2) | 1 -EPA-3 -Linolein | 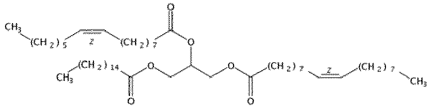 |
| 47 | TG(18:3/18:2/18:3) | 1,3-Linolenin-2-Linolein | 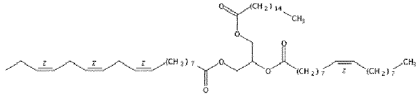 |
| 48 | TG(18:1/22:1/22:1) | 1,2-Eucin(13Z)-3-Olein | 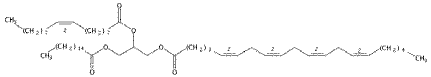 |
| 49 | TG(16:0/16:1/18:1) | 1-Palmitin-2-Palmitolein-3-Olein | 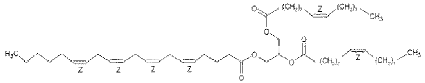 |
| 50 | TG(16:0/18:1/18:3) | 1-Palmitin-2-Olein-3-Linolenin | 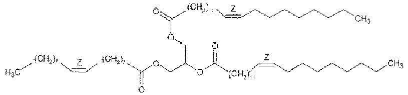 |
| 51 | TG(16:0/18:1/20:4) | 1-Palmitin-2-Olein-3-Arachidonin(5Z,8Z,11Z,14 Z) | 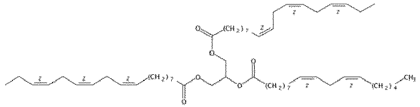 |
| 52 | TG(18:3/18:2/20:5) | 1-Linolenin-2-Linolein-3-EPA | 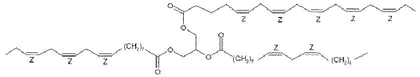 |
| 53 | Cer(d16:0/16:0) | C16 dihydroceramide (d16:0/16:0) | 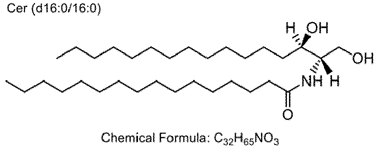 |
| 54 | Cer(d20:0/18:0) | C18 dihydroceramide (d20:0/18:0) | 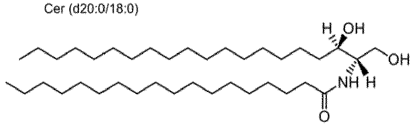 |
| 55 | Cer(d22:0/18:0) | C18 dihydroceramide (d22:0/18:0) | 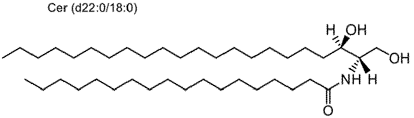 |
| 56 | TG(16:0/18:2/18:3) | Triglyceride(16:0/18:2/18:3 ) | 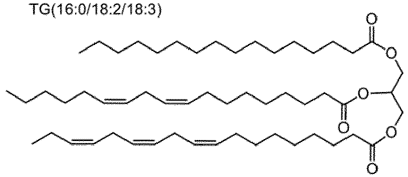 |
| 57 | TG(18:1/18:2/18:3) | Triglyceride(18:1/18:2/18:3 ) | 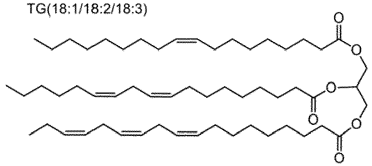 |
| 58 | PEt(16:1/16:1) | 1,2-dipalmitoleoyl-sn-glycero-3 -phosphoethanol | 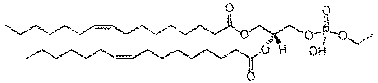 |
| 59 | dMePE(16: 1/14:0) | 1-palmitoleoyl-2-myristoyl-sn-glycero-3- phosphoethanolamine-N,N-dimethyl | 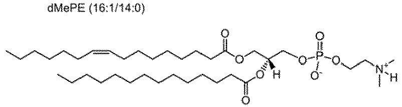 |
| 60 | dMePE(16:1/16:1) | 1,2-dipalmitoleoyl-sn-glycero-3-phosphoethanolamine-N,N-dimethyl | 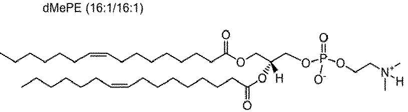 |
| 61 | dMePE(18:1/14:0) | 1-oleoyl-2-myristoyl-sn-glycero-3-phosphoethanolamine-N,N-dimethyl | 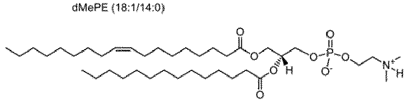 |
| 62 | dMePE(16:1/18:1) | 1-palmitoleoyl-2-oleoyl-sn-glycero-3-phosphoethanolamine-N,N-dimethyl | 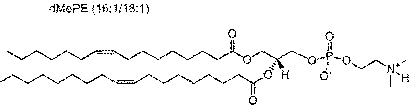 |
| 63 | PC(18:0/18:3(6Z,9Z,12Z)) | 1-stearoyl-2-linolenoyl(gamma)-sn-glycero-3 -phosphocholine | 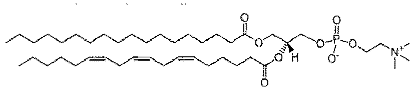 |
| 64 | PE(15:0/24:1(15Z)) | 1 -pentadecanoyl-2-nervonoyl- sn-glycero-3- phosphoethanolamine | 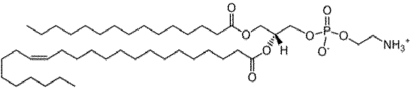 |
| 65 | PC(20:0/14:1(9Z)) | 1-eicosanoyl-2-myristoleoyl-sn-glycero-3-phosphocholine | 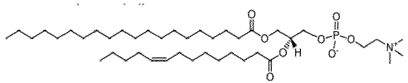 |
| 66 | TG(18:0/18:2/18:3) | Tnglyceride(18:0/18:2/18:3 ) | 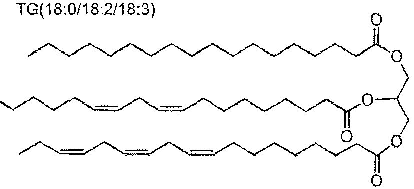 |
| 67 | TG(18: 1/18:2/20:5) | Triglyceride(18:1/18:2/20:5 ) | 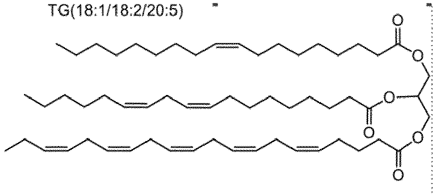 |
| 68 | TG(20:5/18:2/18:2) | Triglyceride(20:5/18:2/18:2 ) | 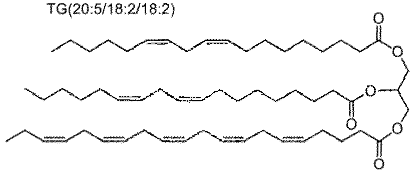 |
| 69 | PC(18:1(11Z)-16:1(9Z)) | 1-vaccenoyl-2-palmitoleoyl- sn-glycero-3 - phosphocholine | 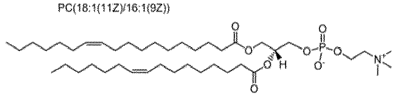 |
| 70 | DG(18:3/18:3) | Dilinolenin | CCC=CCC=CCC=CCCCCCCCC(=O)OCC(O)COC(=O)CCCCCCCCC=CCC=CCC=CCC |
| 71 | PE(16:0/16:0) | 1,2-Dipalmitoyl-sn-Glycero-3-Phosphatidylethanolamine | 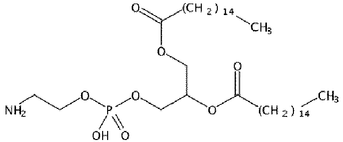 |

### Definition of terms

The term as used herein may have a single dash "-" (or horizontal line) or a double dash "=" in front of and/or behind it to indicate the bond level of the bond between the mentioned substituent and its parent moiety; a single dash "-" (or horizontal line) refers to a single bond, and a double dash refers to a double bond; in the absence of single or double dash, it is understood that a single bond is formed between the substituent and its parent moiety; in addition, the substituent is to be construed "from left to right" unless the dash indicatesotherwise; for example, a C1-C6 alkoxycarbonyloxy group and an -OC(O)OC1-C6 alkyl group refer to the same functional group.

The term "alkyl" as used herein refers to a straight or branched saturated hydrocarbon chain. As described herein, an alkyl group has 1 to 20 carbon atoms (i.e., C1-20 alkyl), 1 to 8 carbon atoms (i.e., C1-8 alkyl), 1 to 6 carbon atoms (i.e., C1-6 alkyl), or 1 to 4 carbon atoms (i.e., C1-4 alkyl). In one embodiment, the alkyl group is a C10-20 alkyl group. In one embodiment, the alkyl group is a C15-20 alkyl group. In one embodiment, the alkyl group is a C15-18 alkyl group, i.e., a C15, C16, C17, C18 alkyl group.

The term "alkenyl" as used herein refers to an aliphatic group containing at least one carbon-carbon double bond and having 2 to 20 carbon atoms (i.e., C2-20 alkenyl), 2 to 8 carbon atoms (i.e., C2-8 alkenyl) , 2 to 6 carbon atoms (i.e., C2-6 alkenyl) or 2 to 4 carbon atoms (i.e., C2-4 alkenyl). In one embodiment, the alkenyl group is a C10-20 alkenyl group. In one embodiment, the alkenyl group is a C15-20 alkenyl group. In one embodiment, the alkenyl group is a C15-18 alkenyl group, i.e. a C15, C16, C17, C18 alkenyl group.

The term "heteroalkyl" and "heteroalkenyl" as used herein refer to alkyl and alkenyl as defined above, respectively, wherein one or more carbon atoms are each independently substituted by the same or different heteroatom groups. For example, 1, 2 or 3 carbon atoms may be independently substituted by the same or different heteroatom groups. Heteroatom groups include, but are not limited to, -NR1-, -O-, - S-, -S(O)-, -S(O)2-, and the like, wherein R1 is H, alkyl. Examples of heteroalkyl groups include -OCH3, -CH2OCH3, -SCH3, -CH2SCH3, -NR1CH3 and - CH2NR1CH3, wherein R1 is hydrogen, alkyl.

The term reverse evaporation method as described herein refers to adding an aqueous solution of nucleic acid to an organic solvent solution of lipid, ultrasonicating, evaporating to remove the organic solvent, and then hydrating to obtain a lipid nucleic acid mixture.

The term "boiling method" (also refers to "heating method") as described herein refers to adding an organic solvent solution of lipid to an aqueous solution of nucleic acid and boiling at about 100°C for 30 minutes to obtain a lipid nucleic acid mixture. The method is not limited to heating by boiling, and other means of heating or raising temperature known in the art can also be used.

Reverse evaporation method and boiling method are carried out under controlled temperature and mixing conditions. Suitable processing times, and temperatures can be readily determined by a person skilled in the art. For example, the temperature of reverse evaporation method is ranged preferably from about 25 °C to about 70 °C, more preferably from about 30°C to about 65 °C, and more preferably from about 40°C to about 60 °C, especially about 55 °C. The temperature of boiling method is ranged preferably from about 25 °C to about 100 °C, more preferably from about 50°C to about 100 °C, and more preferably from about 95°C to about 100 °C, especially preferably from about 80 °C to 100 °C.

The nucleic acid as described herein comprises DNA and RNA, preferably small RNA, for example, the small RNA having a length of 14-32 bp, 16-28 bp, 18-24 bp, and particularly, a length of 14, 15, 16, 17, 18, 19, 20, 21, 22, 23, 24, 25, 26, 27, 28, 29, 30, 31, 32 bp.

### Brief Descriptions of the Drawings

Fig.1: Effect of 12 lipids on nucleic acid (HJT-sRNA-m7) absorption and entry into cells (human gastric cancer cell line NCI-N87) (reverse evaporation method).
Fig.2: 27 single lipidspromote nucleic acid entry into MRC-5 cell line (reverse evaporation method).
Fig.3: 23 single lipids promote nucleic acid entry into MRC-5 cell line (boiling method).
Fig.4: 23 single lipids promote nucleic acid entry into A549 cell line (boiling method).
Fig.5: Lipid combination can promote nucleic acid entry into MRC-5 cell line (reverse evaporation method).
Fig.6: Lipid combination can promote nucleic acid entry into A549 cell line (reverse evaporation method).
Fig.7: Lipid combination can promote nucleic acid entry into MRC-5 cell line (boiling method).
Fig.8: Lipid combination can promote nucleic acid entry into A549 cell line (boiling method).
Fig.9: Different types of lipid combinations promote nucleic acid entry into Caco-2 cell line (reverse evaporation method).
Fig.10: Different types of lipid combinations promote nucleic acid entry into Caco-2 cell line (boiling method).
Fig.11A-C: Single lipids (No. 11 and No. 12) promote nucleic acids having different sequences entry into different cells.
Fig.12: Fluorescence in situ hybridization experiment indicates that the nucleic acids enter into the cytoplasm with the aid of single lipid.
Fig.13: Single lipids (No. 11 and No. 12) promote nucleic acid entry into cells, targeting the gene 3'UTR region.
Fig.14: Single lipids (No. 11 and No. 12) promote nucleic acid entry into blood and lung by digestive tract.
Fig.15: Lipid combinations prepared by reverse evaporation method and boiling method facilitate nucleic acid entry into blood and lung by digestive tract.
Fig.16: Different types of lipid combinations deliver single-stranded nucleic acid into MRC-5.
Fig.17A-F: Lipid combinations deliver single-stranded nucleic acid into MRC-5 or Caco-2 cells.
Fig.18A-C: Lipid combinations deliver single-stranded nucleic acid into cells.
Fig.19A-C: Lipid combinations deliver single-stranded nucleic acid into cells.
Fig.20A-C: Lipid combinations deliver single-stranded nucleic acid into cells.
Fig.21: Lipid combinations deliver single-stranded nucleic acid into A549 cell.
Fig.22: Lipid combinations deliver single-stranded nucleic acid into A549 cell.
Fig.23: Lipid combinations deliver single-stranded nucleic acid into A549 cell.
Fig.24: Lipid combinations deliver single-stranded nucleic acid into A549 cell.
Fig.25: Lipid combinations deliver single-stranded nucleic acid into A549 cell.
Fig.26: Lipid combinations deliver single-stranded nucleic acid into A549 cell.
Fig.27: Lipid combinations deliver single-stranded nucleic acid into A549 cell.
Fig.28: Lipid combinations deliver single-stranded nucleic acid into A549 cell.
Fig.29: Lipid combinations deliver single-stranded nucleic acid into A549 cell.
Fig.30: Lipid combinations deliver single-stranded nucleic acid into A549 cell.
Fig.31: Lipid combinations deliver single-stranded nucleic acid into A549 cell.
Fig.32: Lipid combinations deliver single-stranded nucleic acid into A549 cell.
Fig.33: Lipid combinations deliver double-stranded nucleic acid into MRC-5 cell.
Fig.34: Lipid combinations deliver double-stranded nucleic acid into MRC-5 cell.
Fig.35: Lipid combinations deliver double-stranded nucleic acid into A549 cell.
Fig.36: Lipid combinations deliver double-stranded nucleic acid into A549 cell.
Fig.37: Lipid combinations deliver double-stranded nucleic acid into A549 cell.
Fig.38: Lipid combinations deliver double-stranded nucleic acid into A549 cell.
Fig.39: Lipid combinations deliver double-stranded nucleic acid into A549 cell.
Fig.40: Lipid combinations deliver double-stranded nucleic acid into A549 cell.
Fig.41: Lipid combinations deliver double-stranded nucleic acid into A549 cell.
Fig.42: Lipid combinations deliver double-stranded nucleic acid into A549 cell.
Fig.43: Lipid combinations deliver double-stranded nucleic acid into MRC-5 cell.
Fig.44: Lipid combinations deliver double-stranded nucleic acid into MRC-5 cell.
Fig.45: Lipid combinations deliver double-stranded nucleic acid into MRC-5 cell.
Fig.46: Lipid combinations deliver double-stranded nucleic acid into MRC-5 cell.
Fig.47: Lipid combinations deliver double-stranded nucleic acid into MRC-5 cell.
Fig.48: Lipid combinations deliver double-stranded nucleic acid into MRC-5 cell.
Fig.49: Lipid combinations deliver double-stranded nucleic acid into MRC-5 cell.
Fig.50: Lipid combinations promote nucleic acid entry into lung via digestive tract.
Fig.51: No. 8(PE):No. 12(PC) (v:v=1:2) mediates anti-fibrotic HJT-sRNA-m7 entry into MRC-5 cell.
Fig.52: No. 8(PE):No. 12(PC) (v:v=1:2) mediates siRNA entry into A549 cell.
Fig.53: No. 8(PE):No. 12(PC) (v:v=1:2) mediates siRNA entry into A549 cell.
Fig.54: No. 8(PE):No. 12(PC) (v:v=1:2) mediates siRNA entry into THP-1 cell.
Fig.55: No. 8(PE):No. 12(PC):No. 2(DG) (v:v:v=2:4:3) mediates anti-fibrotic HJT-sRNA-m7 entry into MRC-5 cell.
Fig.56: No. 8(PE):No. 12(PC):No. 2(DG) (v:v:v=2:4:3) lipid mixture mediates XRN2 siRNA entry into A549 cell to inhibit gene expression.
Fig.57: No. 8(PE):No. 12(PC):No. 4(Cer) (v:v:v=1:2:1) lipid mixture mediates anti-fibrotic HJT-sRNA-m7 entry into MRC-5 cell (boiling method).
Fig.58: No. 8(PE):No. 12(PC):No. 4(Cer) (v:v:v=1:2:1) lipid mixture mediates NFκB siRNA entry into THP-1 cell to inhibit gene expression (boiling method).
Fig.59: No. 8(PE):No. 12(PC):No. PC(11) (v:v:v=1:2:1) lipid mixture mediates XRN2 siRNA entry into A549 cell to inhibit gene expression.
Fig.60: No. 8(PE):No. 12(PC):No. LPC(37) (v:v:v=1:2:1) lipid mixture mediates XRN2 siRNA entry into A549 cell to inhibit gene expression.
Fig.61: No. 8(PE):No. 12(PC):No. MG(34) (v:v:v=2:3:1) lipid mixture mediates CPSF4 siRNA entry into A549 cell to inhibit gene expression.
Fig.62: No. 38(PE):No. 37(LPC):No. 32(TG) (v:v:v=32:8:5) lipid mixture mediates anti-fibrotic HJT-sRNA-m7 entry into MRC-5 cell (boiling method).
Fig.63: No. 38(PE):No. 37(LPC):No. 32(TG) (v:v:v=32:8:5) lipid mixture mediates XRN2 siRNA entry into A549 to inhibit gene expression.
Fig.64: No. 1(DG):No. 8(PE):No. 12(PC):No. 4(Cer):No. 31(So):No. 29(FA):No. 16(TG) (v:v:v:v:v:v:v=2:1:2:2:3:1:3) mediates anti-fibrotic HJT-sRNA-m7 entry into MRC-5 cell (boiling method).
Fig.65: No. 1(DG):No. 8(PE):No. 12(PC):No. 4(Cer):No. 31(So):No. 29(FA):No. 16(TG) (v:v:v:v:v:v:v=2:1:2:2:3:1:3) lipid mixture mediates XRN2 siRNA entry into A549 to inhibit gene expression (boiling method).
Fig.66: No. 8(PE):No. 12(PC):No. 31(So):No. 29(FA):No. 4(Cer) (v:v:v:v:v=2:4:2:2:2:5) mediates anti-fibrotic HJT small RNA HJT-sRNA-3, HJT-sRNA-a2, HJT-sRNA-h3, HJT-sRNA-m7 entry into MRC-5 cell (boiling method).
Fig.67: No. 8(PE):No. 12(PC):No. 31(So):No. 29(FA):No. 4(Cer) (v:v:v:v:v=2:4:2:2:5) lipid mixture can effectively deliver nucleic acid into cell.
Fig.68: No. 38(PE):No. 37(LPC) (v:v=4:1) mediates anti-fibrotic HJT small RNA HJT-sRNA-3, HJT-sRNA-a2, HJT-sRNA-h3, HJT-sRNA-m7 entry into MRC-5 cell (boiling method).
Fig.69: No. 38(PE):No. 37(LPC) (v:v=4:1) lipid mixture mediates XRN2 siRNA entry into A549 cell to inhibit gene expression (boiling method).
Fig.70: No. 38(PE):No. 12(PC):No. 2(DG) (v:v:v=4:1:3) lipid mixture mediates XRN2 siRNA entry into A549 cell to inhibit gene expression.
Fig.71: No. 38(PE):No. 37(LPC):No. 12(PC) (v:v:v=4:1:1) lipid mixture mediates XRN2 siRNA entry into A549 cell to inhibit gene expression (reverse evaporation method).
Fig.72: No. 4(Cer):No. 12(PC):No. 38(PE):No. 37(LPC) (v:v:v:v=5:2:8:3) lipid mixture mediates anti-fibrotic small RNA HJT-sRNA-3, HJT-sRNA-a2, HJT-sRNA-h3, and HJT-sRNA-m7 entry into MRC-5 cell (boiling method).
Fig.73: No. 4(Cer):No. 12(PC):No. 38(PE):No. 37(LPC) (v:v:v:v=5:2:8:3) lipid mixture mediates XRN2 siRNA entry into A549 cell to inhibit gene expression (boiling method).
Fig.74: No. 38(PE):No. 2(DG):No. 31(So) (v:v:v=4:2:3) lipid mixture mediates anti-fibrotic small RNA HJT-sRNA-3, HJT-sRNA-a2, HJT-sRNA-h3, HJT-sRNA-m7 entry into MRC-5 cell (boiling method).
Fig.75: No. 38(PE):No. 2(DG):No. 31(So) (v:v:v=4:2:3) lipid mixture mediates XRN2 siRNA entry into A549 cell to inhibit gene expression (boiling method).
Fig.76: Lipid No. 41 delivers double-stranded RNA into A549 cell by different preparation methods (boiling or reverse evaporation method).
Fig.77: Lipid No. 41 delivers double-stranded RNA into MRC-5 cell by different preparation methods (boiling or reverse evaporation method).
Fig.78: Lipid No. 41 delivers single-stranded RNA into A549 and MRC-5 cells by boiling method.
Fig.79: Digital PCR (ddPCR) technology determines the efficiency of nucleic acid delivery by lipid.
Fig.80: Flow cytometry technology determined the efficiency of nucleic acid delivery by lipid.
Fig.81: Confocal fluorescence microscopy observes the localization of nucleic acid delivered by lipid in cell.
Fig.82: Western Blotting assay determined the efficiency of nucleic acid delivery by lipid.
Fig.83: Single lipid No. 41 mediates anti-fibrotic HJT-sRNA-m7 entry into MRC-5 cell (boiling method).
Fig.84: Effects of lipid combination 1 (No. 8+No. 41=6:1) and lipid combination 2 (No. 38+No. 41=6:1) in nucleic acid delivery.
Fig.85: Effects of lipid combination 3 (No. 39+No. 41=6:1) and lipid combination 4 (No. 40+No. 41=6:1) in nucleic acid delivery.
Fig.86: Effects of lipid combination 5 (38+12+41+29=1:2:1:1) in nucleic acid delivery.
Fig.87: Effects of lipid combination 6 (40(PE)+12(PC)+41(So)=2:4:3) in nucleic acid delivery.
Fig.88: Effects of lipid combination 7 (12(PC)+41(So)=1:6) and lipid combination 8 (12(PC)+41(So)=1:1) in nucleic acid delivery.
Fig.89: Effects of lipid combination 9 (12(PC)+41(So)=6: 1) and lipid combination 10 (40(PE)+12(PC)+41(So)=2:2:2) in nucleic acid delivery.
Fig.90: Effects of lipid combination 11 (4(Cer)+12(PC)+41 (So)=1: 1: 1) in nucleic acid delivery.
Fig.91: Lipid No. 38 delivers double-stranded RNA into A549 and MRC-5 cells by boiling method.
Fig.92: Lipid No. 38 delivers single-stranded RNA into A549 cells and MRC-5 cells by boiling method.
Fig.93: Digital PCR (ddPCR) technology determined the efficiency of nucleic acid delivery by lipid.
Fig.94: Flow cytometry technology determined the efficiency of nucleic acid delivery by lipid.
Fig.95: Confocal fluorescence microscopy observes the location of nucleic acid delivered by lipid in cell.
Fig.96: Lipid No. 64 delivers double-stranded RNA into A549 cell by different prepration methods (boiling or reverse evaporation method).
Fig.97: The efficiency of nucleic acid delivery by lipid as determined by flow cytometry technology.
Fig.98: The localization of nucleic acid delivered by lipid in cell as observed by confocal fluorescence microscopy.
Fig.99: Lipid No. 40 delivers dsRNA into A549 cell by different preparation methods (boiling or reverse evaporation method).
Fig.100: The efficiency of nucleic acid delivery by lipid as determined by Digital PCR (ddPCR).
Fig.101: The location of nucleic acid delivered by lipid in cell as observed by confocal fluorescence microscopy.
Fig.102: The efficiency of nucleic acid delivery by lipid as determined by Western Blotting assay.
Fig.103: Lipid No. 37 delivers single-stranded RNA into A549 cell and MRC-5 cells by boiling method.
Fig.104: Lipid No. 39 delivers double-stranded RNA into A549 cell by different preparation methods (boiling or reverse evaporation method).
Fig.105: The efficiency of nucleic acid delivery by lipid determined by Digital PCR (ddPCR).
Fig.106: Lipid No. 60 delivers double-stranded RNA into A549 cell by different preparation methods (boiling or reverse evaporation method).
Fig.107: Lipid No. 62 delivers double-stranded RNA into A549 cell by different preparation methods (boiling or reverse evaporation method).
Fig.108: Lipid No. 41 promotes small RNA entry into blood and protects it from degradation in the blood.
Fig.109: Lipid No. 41 promotes small RNA entry into stomach cell and protects it from degradation in the stomach.
Fig.110: Lipid No. 41 promotes small RNA entry into small intestine cell and protects it from degradation in the small intestine.
Fig.111: Lipid No. 41 promotes small RNA entry into liver and protects it from degradation in the liver.
Fig.112: Single PE (No. 38) effectively delivers single-stranded sRNA nucleic acid into mouse blood by oral administration.
Fig.113: Single PE (No. 40) effectively delivers single-stranded sRNA nucleic acid into mouse blood by oral administration.
Fig.114: Single PE (No. 64) effectively delivers single-stranded sRNA nucleic acid into mouse blood by oral administration.
Fig.115: Single PE (No. 71) effectively delivers single-stranded sRNA nucleic acid into mouse blood by oral administration.
Fig.116: Lipids effectively deliver single-stranded nucleic acid into MRC-5 cell at different temperature gradients.
Fig.117a-c: Lipid No. 33 delivers small RNA into blood, heart and kidney via oral administration.
Fig.118a-b: Lipid No. 35 delivers small RNA into heart and kidney via oral administration.
Fig.119: Lipid No. 37 delivers small RNA into intestine via oral administration.
Fig.120a-b: Lipid No. 42 delivers small RNA into spleen and kidney via oral administration.
Fig.121a-b: Lipid No. 43 delivers small RNA into heart and intestine via oral administration.
Fig.122a-b: Lipid No. 44 delivers small RNA into spleen and intestine via oral administration.
Fig.123a-d: Lipid No. 45 delivers small RNA into heart, spleen, kidney and intestine via oral administration.
Fig.124a-d: Lipid No. 46 delivers small RNA into blood, heart, kidney and intestine via oral administration.
Fig.125a-c: Lipid No. 47 delivers small RNA into blood, heart and intestine via oral administration.
Fig.126a-c: Lipid No. 48 delivers small RNA into blood, heart, and kidney via oral administration.
Fig.127a-c: Lipid No. 49 delivers small RNA into blood, heart, and spleen via oral administration.
Fig.128a-c: Lipid No. 50 delivers small RNA into blood, heart, and spleen via oral administration.
Fig. 129a-b: Lipid No. 51 delivers small RNA into blood and heart via oral administration.
Fig. 130a-b: Lipid No. 52 delivers small RNA into blood and heart via oral administration.
Fig.131a-c: Lipid No. 53 delivers small RNA into blood, heart, and kidney via oral administration.
Fig. 132a-b: Lipid No. 54 delivers small RNA into blood and kidney via oral administration.
Fig.133a-c: Lipid No. 55 delivers small RNA into blood, heart, and kidney via oral administration.
Fig. 134a-b: Lipid No. 56 delivers small RNA into blood and heart via oral administration.
Fig.135: Lipid No. 57 delivers small RNA into kidney via oral administration.
Fig. 136a-b: Lipid No. 58 delivers small RNA into heart and intestine via oral administration.
Fig. 137: Lipid No. 59 delivers small RNA into intestine via oral administration.
Fig.138: Lipid No. 60 delivers small RNA into intestine via oral administration.
Fig.139a-c: Lipid No. 61 delivers small RNA into heart, kidney and intestine via oral administration.
Fig.140: Lipid No. 62 delivers small RNA into intestine via oral administration.
Fig.141a-b: Lipid No. 65 delivers small RNA into kidney and intestine via oral administration.
Fig.142a-c: Lipid No. 66 delivers small RNA into blood, heart and intestine via oral administration.
Fig.143a-c: Lipid No. 67 delivers small RNA into heart, spleen and intestine via oral administration.
Fig.144a-c: Lipid No. 68 delivers small RNA into heart, spleen and intestine via oral administration.
Fig. 145a-b: Lipid No. 69 delivers small RNA into spleen and kidney via oral administration.
Fig. 146a-b: Lipid No. 70 delivers small RNA into heart and spleen via oral administration.

### Detailed Description of the Invention:

The following is a further description of the present application, but is not intended to limit the invention in any way, and any changes made based on the teachings of the present application fall within the scope of protection of the present application.

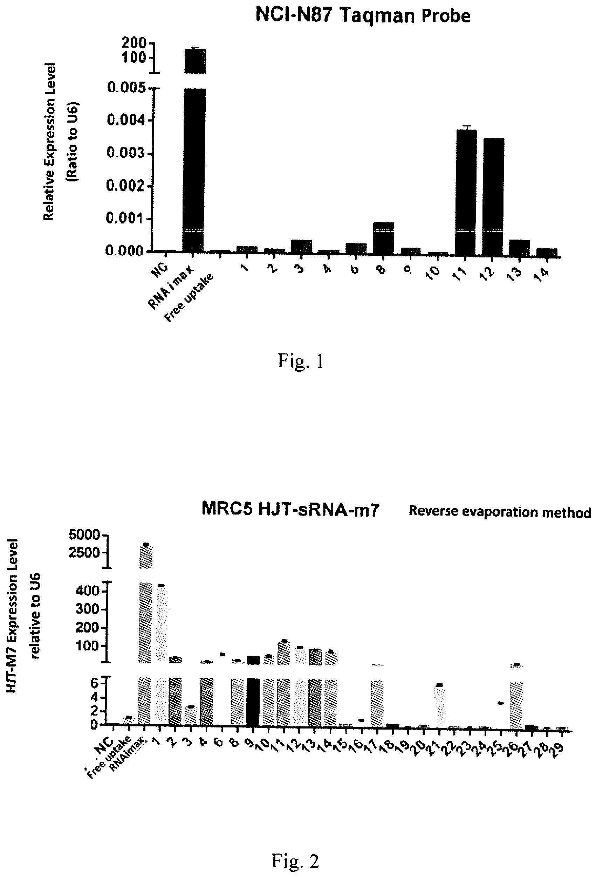

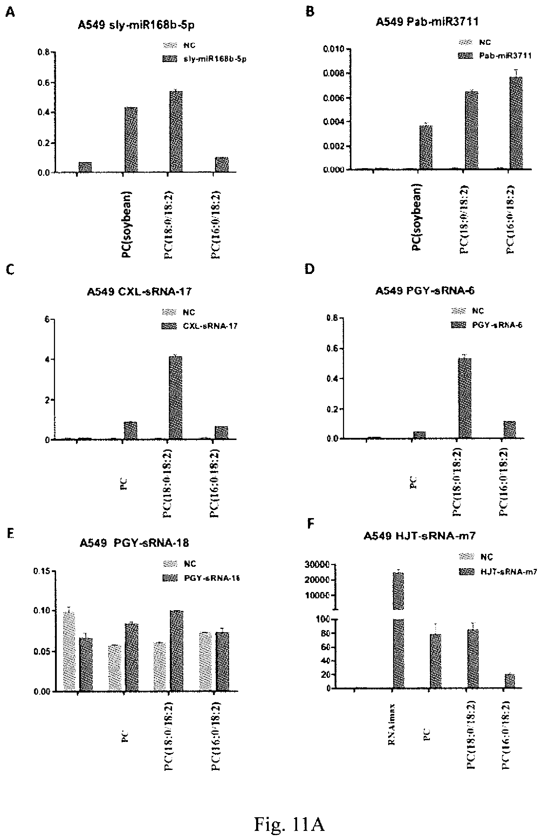

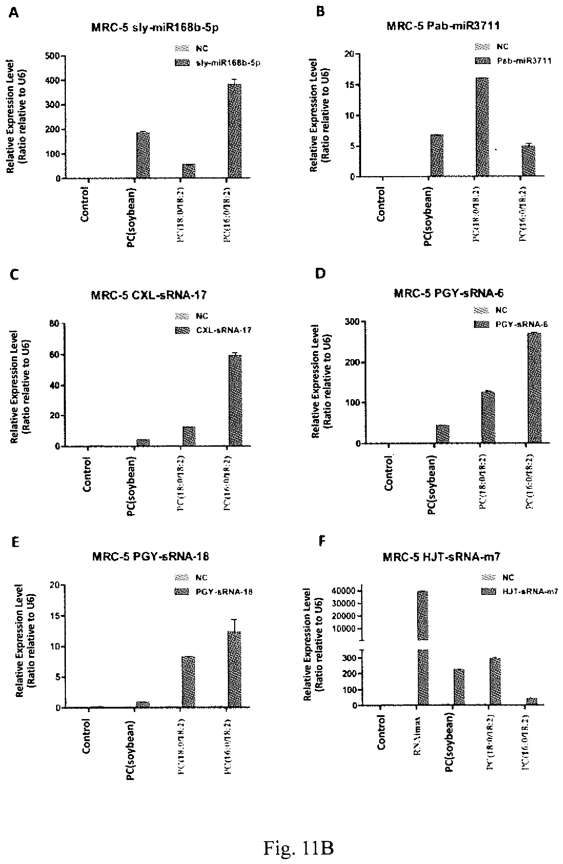

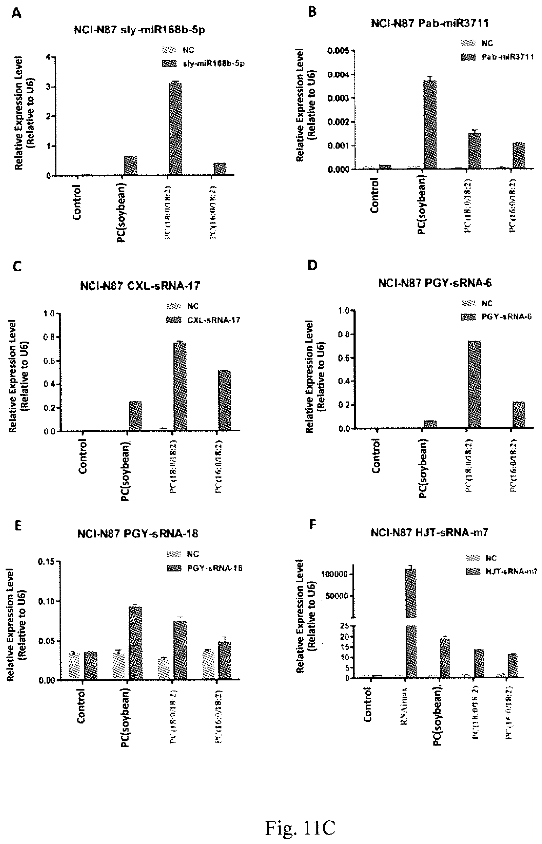

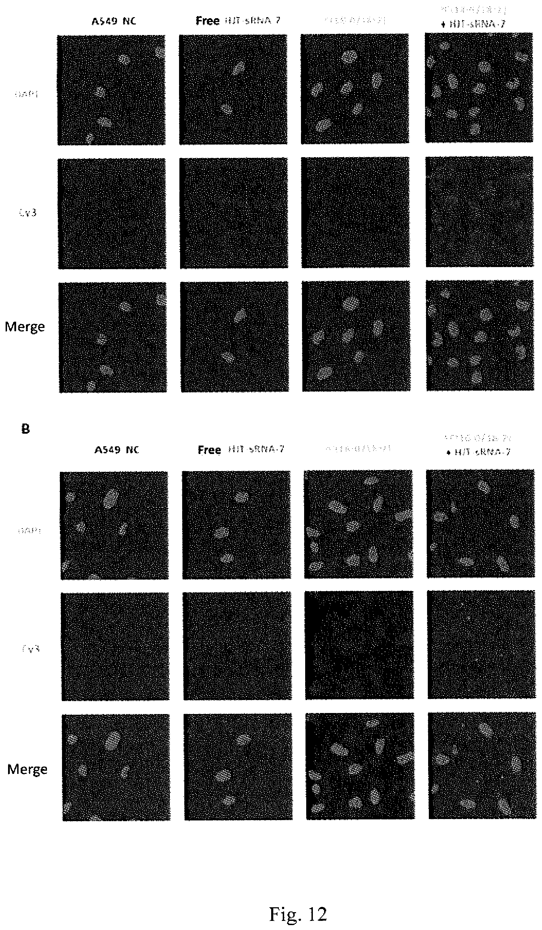

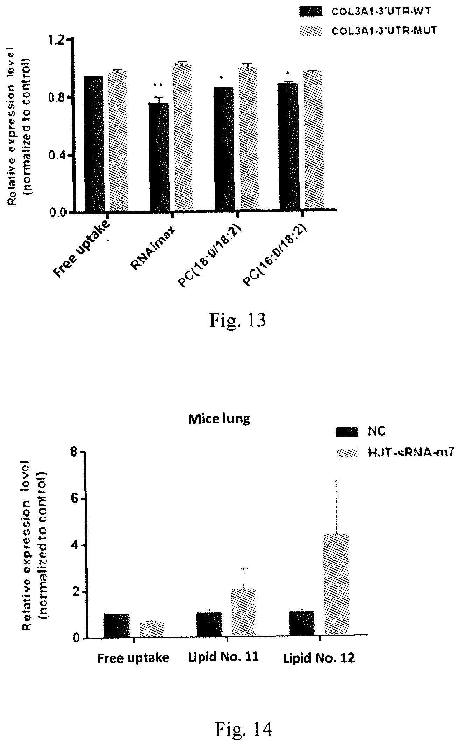

In the present application, lipid-soluble components were extracted from traditional Chinese medicines (including *Rhodiola crenulata*, *Taraxacum mongolicum*, *Andrographis paniculata* and *Lonicera japonica*) by the Bligh&Dyer method, and the lipid components were identified by HPLC-MS/MS (a total of 138 lipid components were identified, 125 in positive mode, 13 in negative mode). 71 of them (see Table 1-1 to Table 1-3) were used for the preparation of the lipid nucleic acid mixtures, and observed for whether they could promote cellular absorption and entry of exogenous nucleic acids. It should be noted that the lipids used in the present application were commercially purchased or commercially synthesized, and were not directly extracted from traditional Chinese medicines. The inventor has surprisingly found that various lipids can form lipid nucleic acid mixtures that effectively promote cellular absorption and entry of nucleic acid (see Figures 1-116), having the potential of increasing the efficiency of the nucleic acid drug delivery in clinical settings. Further studies have shown that the lipid nucleic acid mixture of the present application promotes the efficiency of nucleic acid absorption and entry in different cell lines, but differences were observed in different cell lines (see Figures 1-10), which opens up the possibility of targeted drug delivery. Moreover, nucleic acid delivery by such lipid nucleic acid mixture is not sequence specific, capable of delivering nucleic acid fragments having different sequences and a size corresponding to that of small RNA (e.g. about 20bp) (see Fig.11). In addition, fluorescence in situ hybridization (FISH) confirmed that the lipid nucleic acid mixture formed by lipids derived from the decoction can effectively promote the entry of exogenous nucleic acids into cytoplasm (see Fig.12). The inventor has unexpectedly discovered that lipid nucleic acid mixtures prepared by boiling or reverse evaporation method can facilitate entry of nucleic acids, such as sRNA, into blood circulation and target tissue via non-invasive routes (e.g. via digestive tract, respiratory tract and topical administration). (See Figures 14-15). The inventor has also surprisingly discovered that lipids of the present application are capable of promoting entry of nucleic acids, such as sRNA, into cells and modulating (e.g., inhibiting) the expression of their target sequences, while not exhibiting such regulatory effects on non-target sequences, suggesting a target-specific regulation, which can be used as a means for the delivery of nucleic acid drug (see Figure 13).

Based on the above unexpected discoveries, the inventors have arrived at the present application.

In one aspect, the present application provides compounds extracted from traditional Chinese medicines for facilitating nucleic acid delivery, wherein the said compounds are selected from the group consisting of lysolecithin, ceramide, diglyceride, phosphatidylethanolamine, phosphatidylcholine, triglyceride, monogalactosyl diglycerides, sphingosine, phosphatidyl ethanol, monoacylglycerol, fatty acid, platelet activating factor, or dimethyl phosphatidyl ethanolamine, preferably selected from the lipids shown in Table 1. In one embodiment, the lipid is non-natural, e.g. synthetic, or manufactured from fermentation.

In one embodiment, the lipid is used to deliver a nucleic acid into a target cell. In another embodiment, the lipid is used to deliver a nucleic acid into a subject in need thereof and into its blood circulation and/or a target site/cell.

In a preferred embodiment, the lipid is selected from phosphatidylcholine, e.g., 1-stearoyl-2-oleoyl-sn-glycerol-3-phosphocholine (PC(18:0/18:2), i.e., lipid No. 11 in Table 1), and 1-palmitoyl-2-oleoyl-sn-glycerol-3-phosphocholine (PC(16:0/18:2), i.e., lipid No. 12 in Table 1). These two phosphocholine (PC) are capable of efficiently encapsulating nucleic acids or promoting entry of nucleic acids into cells. In one embodiment, the lipid may be lipid No. 41 in Table 1 , i.e. sphinganine(d22:0), which is capable of efficiently encapsulating nucleic acids or promoting entry of nucleic acids into cells.

In another aspect, the present application provides pharmaceutical compositions comprising the above lipids and nucleic acids. Preferably the nucleic acid is small RNA.

In one embodiment, the pharmaceutical composition of the present application can be prepared for administration via non-invasive routes (e.g., topical administration) and/or injection, e.g., administration via digestive tract, respiratory tract, and/or injection, e.g., oral administration, inhalation and/or injection. In some cases, invasive routes are preferred (e.g., injection, including intramuscular, subcutaneous, intravenous, intraarterial, intraperitoneal, and injection into a target tissue); in other cases, non-invasive routes are preferred.

In another embodiment, in the pharmaceutical composition of the present application, at least part of or all of the lipids and nucleic acids can be prepared into the form of lipid nucleic acid mixture. Various methods for the manufacture of lipid nucleic acid mixtures have been widely disclosed, and the suitable method for the manufacture of lipid nucleic acid mixture can be selected according to actual needs.

In a third aspect, the present application provides kits comprising the lipids and nucleic acids described herein, wherein the lipids and the nucleic acids are each independently provided in a first container and a second container. The first container and the second container may be the same or different. In some embodiments, at least part of or all of the lipids and the nucleic acids are prepared into lipid nucleic acid mixtures immediately prior to use.

In a fourth aspect, the present application provides methods of delivering a nucleic acid into a target tissue/cell, wherein the nucleic acid is provided in a form of the pharmaceutical composition or the kit as described herein.

In a fifth aspect, the present application provides methods of delivering a nucleic acid into a subject *in vivo* in need thereof, wherein the nucleic acid is provided in a form of the pharmaceutical composition or the kit as described herein, e.g., delivering the nucleic acid into blood circulation or a target tissue/cell of the subject *in vivo,* e.g., wherein the lipid and the nucleic acid are administrated by non-invasive routes (e.g., topical administration) and/or injection, e.g., by digestive tract, respiratory tract and/or injection, e.g., by oral administration, inhalation and/or injection.

In a sixth aspect, the present application provides methods of preventing and/or treating a disease/disorder that can be prevented and/or treated with a nucleic acid, the methods comprising providing the pharmaceutical composition or the kit described herein to a subject in need thereof, e.g., wherein the lipid and the nucleic acid are administered by non-invasive routes (e.g., topical administration) and/or by injection, e.g., by digestive tract, respiratory tract and/or injection, e.g., by oral administration, inhalation and/or injection. Surprisingly, the non-invasive routes of administration (e.g., by digestive tract, respiratory tract, including oral administration, gavage, inhalation and the like) can significantly promote the entry and efficacy of nucleic acids.

In a seventh aspect, the present application provides methods for the manufacture of the pharmaceutical composition or the kit, and use of the pharmaceutical composition and/or the kit in the methods described in the above aspects. Besides, also provided arelipids, pharmaceutical compositions and/or kits for use in the various methods described above.

In various embodiments of the present application, the nucleic acid may be a small RNA, for example, the small RNA may have a length of 14-32 bp, 16-28 bp, 18-24 bp, in particular, a length of 14, 15, 16, 17, 18, 19, 20, 21, 22, 23, 24, 25, 26, 27, 28, 29, 30, 31, 32 bp. In addition, the small RNA may be single-stranded, e.g., having a stem-loop structure, or double-stranded. For example, the nucleic acid may be HJT-sRNA-m7 having the following sequence: ugagguagua gguugugugg uuguaagc.

In one embodiment, the pharmaceutical compositions or the kits or the compounds of the present application can be used for treating a disease, for example heart, spleen, kidney, gastric, lung and/or intestinal diseases, such as cancer, e.g., gastric cancer, lung cancer, and the like.

In one embodiment, the pharmaceutical compositions or the kits or the compounds of the present application can be used for treating *in vitro* or *in vivo,* e.g., to inhibit the growth of NCI-N87 cell (gastric cancer cell), MRC-5 cell (lung fibroblast) and A549 cell (lung cancer cell).

In various embodiments of the present application, the lipid nucleic acid mixture can be obtained by a variety of methods, e.g., reverse evaporation method or boiling method. In the reverse evaporation method, an aqueous solution of nucleic acid is added to an organic solvent solution of lipid, ultrasonicated, evaporated to remove the organic solvent, and then hydrated to obtain a lipid nucleic acid mixture. The boiling method described in the present application refers to adding an organic solvent solution of lipid to an aqueous solution of nucleic acid and boiling at about 100 °C for 30 minutes to obtain a lipid nucleic acid mixture. The reverse evaporation method and the boiling method are carried out under controlled temperature and mixing conditions. Suitable processing times and temperatures can be readily determined by a person skilled in the art. For example, the temperature of reverse evaporation method can range preferably from about 25 °C to about 70 °C, more preferably from about 30 °C to about 65 °C, more preferably from about 40 °C to about 60 °C, especially preferably about 55 °C. The temperature of the boiling method (also referred to as heating) can range preferably from about 25 °C to about 100 °C, more preferably from about 50 °C to about 100 °C, more preferably from about 95 °C to about 100 °C, especially preferably about 100 °C.

### Examples

The following examples are merely illustrative of the invention disclosed herein, and are not to be construed as limiting the scope of the appended claims.

**Table 2. Small RNA and their sequences used in the examples**

| **No** | **siRNA** | **Sequence** | **Length** |
|---|---|---|---|
| 1 | sly-miR168b-5p | TCGCTTGGTGCAGGTCGGGAC | 21 |
| 2 | Pab-miR3711 | GGCCCTCCTTCTAGCGCCA | 19 |
| 3 | CXL-sRNA-17 | CAGAGTCGCGCAGCGGAA | 18 |
| 4 | PGY-sRNA-6 | GTTCAGAGTTCTACAGTCCGA | 21 |
| 5 | PGY-sRNA-18 | CGGGGCTACGCCTGTCTGAGCGTCGC | 26 |
| 6 | HJT-sRNA-m7 | TGAGGTAGTAGGTTGTGTGGTTGTAAGC | 28 |
| 7 | HJT-sRNA-3 | CAGCCAAGGATGACTTGCCGG | 21 |
| 8 | HJT-sRNA-a2 | TAGCACCATCCGAAATCGGTA | 21 |
| 9 | HJT-sRNA-h3 | TGGGGCTACGCCTGTCTGAGCGTCGCT | 27 |
| 10 | si-XRN2 | GAGUACAGAUGAUCAUGUUGAGTACAGATGATCATGTT | 19 |
| 11 | si-Ssu72 | GACUCACGUGAAGCUUCCAGACTCACGTGAAGCTTCCA | 19 |
| 12 | si-CPSF4 | GAGUCAUCUGUGUGAAUUAGAGTCATCTGTGTGAATTA | 19 |
| 13 | si-LAMP1 | CAAUGCGAGCUCCAAAGAA | 19 |
| 14 | si-LAMP2 | GCGGUCUUAUGCAUUGGAA | 19 |
| 15 | si-NFκB | AGUACCCUGAAGCUAUAUUUU | 21 |
| 16 | si-TNFα | CACAACCAACUAGUGGUGCUU | 21 |
| 17 | PGY-sRNA-23 | CCCTCCGCGGCCAGCTTCT | 19 |
| 18 | PGY-sRNA-26 | TCCGGAATGATTGGGCGTAAAGCGT | 25 |
| 19 | PGY-sRNA-32 | CCGGCCCCGAACCCGTCGGC | 20 |

| | | | |
|---|---|---|---|
| Note: Double-stranded sRNA is indicated by the "si-" prefix. | | | |

### Examples for lipids shown in Table 1 as No. 1-32

### 1. Extraction of lipids from traditional Chinese medicine

### 1.1 Decoction of traditional Chinese medicine

1) 100 g Chinese medicine decoction pieces (*Rhodiola crenulata*, purchased from Ningbo Haishu Qiancao Biotechnology Co., Ltd.; *Taraxacum mongolicum*, *Lonicera japonica*, *Andrographis paniculata*, purchased from Beijing Tongrentang pharmacy), was added into 1000 mL ddH2O and soaked for 30 min.
2) The Chinese medicine decoction pot was boiled for 15 min with intense heating, and for 20 min with slow heating.
3) 400 mL of the heated Chinese medicine soup was added to a rotary evaporator at 60 °C, 60 rpm, and concentrated to 100 mL.

### 1.2 Lipid extraction

1) Chloroform-methanol mixture (chloroform: methanol=1:2, v/v) 600 ml was added to the Chinese medicine soup obtained from the above step 1.1(centrated by rotary evaporator), to make chloroform:methanol:water=1:2:0.8, and stirred for 10-15 min to mix.
2) 200 mL chloroform was added to an Erlenmeyer flask, and stirred for 10 min to mix.
3) 200 ml ddH₂O was added to the Erlenmeyer flask to make chloroform:methanol:water=2:2:1.8, and stirred for 10 min to mix.
4) The liquid of upper layer and the insoluble substances from intermediate layer were removed, and the lower chloroform layer were obtained. Storage at -40 °C.

### 1.3 HPLC-MS/MS identification of lipid components

### Instrument setup

### 1) Chromatography setup:

Instrument: Ultimate 3000; column: Kinetex C18 (100 × 2.1 mm, 1.9 µm); column temperature: 45 °C; mobile phase A: acetonitrile: water (V/V, 60:40), the solution containing 10 mmol/L ammonium formate, mobile phase B: acetonitrile: isopropanol (10:90, V/V), the solution containing 10 mmol/L ammonium formate and 0.1% formic acid. Flow rate: 0.4 mL/min; injection volume: 4µl.

### 2) Mass spectrometry parameters:

a) Positive mode: Heater Temp 300 °C, Sheath Gas Flow rate, 45 arb, Aux Gas Flow Rate, 15 arb, Sweep Gas Flow Rate, 1 arb, spray volt age, 3.0 KV, Capillary Temp, 350 °C, S -Lens RF Level, 30%. Scan ranges: 200-1500.
b) Negative mode: Heater Temp 300 °C, Sheath Gas Flow rate, 45 arb, Aux Gas Flow Rate, 15 arb, Sweep Gas Flow Rate, 1 arb, spray voltage, 2.5KV, Capillary Temp, 350 °C, S- Lens RF Level, 60%. Scan ranges: 200-1500.

### 1 .4 Identification of the lipids derived from the Chinese medicines

The lipid components were identified by HPLC-MS/MS, and a total of 138 lipid components derived from traditional Chinese medicines were identified, among which 125 were identified in positive mode and 13 in negative mode. The following experiments were performed on compounds No. 1-32 as shown in Table 1.

It should be noted that the lipids tested below were all commercially purchased or commercially synthesized, and used as described in Table 1-1.

### 2. Manufacture of lipid nucleic acid mixture

### 2.1 Reverse evaporation method:

600 µl lipid in diethyl ether solution was prepared, and grouped according to the lipid number shown in Table 1, wherein the diethyl ether solution had a concentration of 0.017857 mg/mL for the lipid group No. 1/2/4/9/14/18/19/20/21/22/23/24/25/26/27/28/29/30/32, 0.035714 mg/mL for the lipid group No. 3/8/10/11/12/13, and 0.0035714 mg/mL for the lipid group No. 6/15/16/17/31; the lipid solution was added to 120 µl HJT-sRNA-m7 single-stranded RNA in DEPC-treated aqueous solution (15 nmol) in a volume ratio of 5:1, and sonitcated for 3 min. Diethyl ether was removed by evaporation at 55 °C, and then 600 µl DEPC water was added for hydration to give HJT-sRNA-m7 lipid mixture.

### 2.2 boiling method:

60 µl lipid in chloroform solution was prepared, and grouped according to the lipid numbers shown in Table 1, wherein the chloroform solution had a concentration of 5 mg/mL for the lipid group No. 1/2/4/9/14/18/19/20/21/22/23/24/25/26/27/28/29/30/32 , 10 mg/mL for the lipid group No. 3/8/10/11/12/13 , 1 mg/mL for the lipid groupNo. 6/15/16/17/32 ; the above lipid chloroform solution was mixed with 600 µl HJT-sRNA-m7 single-stranded RNA in DEPC-treated aqueous solution (15 nmol) and heated at 100 °C for 30 min to give HJT-sRNA-m7 lipid mixture.

### 3. In vitro delivery experiment of lipid nucleic acid mixture

**3.1** NCI-N87 cell (gastric cancer cell), MRC-5 cell (lung fibroblast), A549 cell (lung cancer cell) were cultured to logarithmic growth phase, and then plated to a six-well plate at cell density of 1× 10⁶/2 mL medium/well; MRC-5 cell was cultured in Eagle's MEM medium (MEM, Gibco); A549 cell was cultured in Ham's F-12 medium (HyClone); NCI-N87 cell was cultured in RPMI-1640 medium (HyClone); followed by incubation overnight at 37 °C, and the follow-up experiments were performed after the cells were attached to the walls.

### 3.2 Experimental groups as follows:

1) NC group: referred to untreated cells; this group served as a negative control group.
2) RNAimax treatment group: 2 µl RNAimax transfection reagent and HJT-sRNA-m7 solution were diluted in 100 µl opti-MEM medium respectively and then the two were mixed, allowed to stand for 15 min, added into cells and then mixed. The final concentration of HJT-sRNA-m7 was 200 nM; this group served as a positive control group.
3) Free uptake group: HJT-sRNA-m7 solution was directly added (the final concentration was 200 nM), and the group served as a negative control group.
4) Lipid nucleic acid mixture: the mixture of lipid and HJT-sRNA-m7 prepared from the step 2 were added into cells and mixed, and the final concentration of RNA was kept at 200 nM.

**3.3** After co-incubation with the small RNA for 3 hours, the cells were washed 2-3 times with PBS. The cells were harvested with TRIzol lysis buffer, and the total RNA was extracted. The abundance of small RNA that entered cells was detected by RT-qPCR, and the localization of RNA was detected by fluorescence in situ hybridization; protocols of each detection method were as follows:
3.3.1 RT-qPCR detection of small RNA (Taqman probe method)
   1) The sRNA was reverse transcribed to cDNA: Reverse Transcription Kit (TaqMan® MicroRNA Reverse Transcription Kit, cat. No. 4366597) was used to reverse transcribe sRNA into cDNA.The reverse transcription system was as follows: 100 mM dNTPs (with dTTP) 0.15 µl, MultiScribe™ reverse transcriptase 50 U/µl 1.00 µl, 10X RT buffer 1.5 µl, RNase inhibitor (20 U/µl) 0.19µl, nuclease-free H₂O 4.6 µl, 5 µl RNA template (200 ng/µl) was added after mixing, 3 µl 5 x Taqman probe primer was added after mixing, brief centrifuging after mixing, and then kept on ice for 5 min before loading into a PCR reactor. The reaction condition was as follows: (1) 16 °C, 30 min; (2) 42 °C, 30 min; (3) 85 °C, 5 min; (4) 4 °C, termination of reaction. 10 µl RNase-free ddH₂O was added to make up the final volume to 25 µl after the reaction. The Taqman probe primer used in the reverse transcription process was synthesized by Invitrogen (U6: 4440887, HJT-sRNA-m7: 4398987).
   2) Quantitative PCR amplification reaction: qPCR reaction system had a total volume of 10 µl, containing: 5 µl 2 × TaqMan® Universal Master Mix II, with UNG, 0.5 µl 20xTaqman Primer, 1µl cDNA by reverse transcription, 3.5 µl RNase-free dH₂O. LightCycler 480 fluorescence quantitative PCR instrument was used, and the PCR reaction conditions were: 50 °C for 2 min, 95 °C for 10 min for pre-denaturation, followed by PCR amplification cycle: (1) 95 °C, 15 s; (2) 60 °C, 60s; (3) 60 °C, 60 s; a total of 40 cycles; 40 °C for 10 s in the end to cool down. The Taqman probe for the amplification reaction was designed and synthesized by Invitrogen (U6: 4440887, HJT-sRNA-m7: 4398987).
   3) The relative expression level was calculated by 2-ΔCt method.
3.3.2 RT-qPCR detection of small RNA (SYBR Green dye method)
   1) The sRNA was reverse transcribed to cDNA: Reverse Transcription Kit (High-Capacity cDNA Reverse Transcription Kits, Applied Biosystems, cat. No. 4368813) was used to reverse transcribe sRNA into cDNA by stem-loop method, and the reverse transcription system was as follows: RNA template (150 ng/µl) 10 µl, 10X RT buffer, 2.0 µl, 25X dNTP Mix (100 mM) 0.8 µl, U6 RT stem-loop primer 2.0 µl, HJT-sRNA-RT-m7 stem-loop primer 2.0 µl, MultiScribe ™ reverse transcriptase 1.0 µl, RNase inhibitor 1.0 µl, nuclease-free H₂O 1.2 µl, loaded into a PCR reactor after brief centrifugation, the reaction conditions were as follows: (1) 25 °C, 10 min; (2) 37 °C, 120 min; (3) 85 °C, 5 min; (4) 4 °C, termination of the reaction. 20 µl RNase-free ddH₂O was added to make up the final volume to 40 µl after the reaction. The stem-loop primer used in the reverse transcription process was synthesized by Beijing Tsingke Biotechnology Co., Ltd. (U6 RT primer: GTCGTATCCAGTGCAGGGTCCGAGGTATTCGCACTGGATACGACAAAAAT ATG; HJT-sRNA-m7 RT stem-loop primer: 
GTCGTATCCAGTGCACGCTCCGAGGTATTCGCACTGGATACGACGCTTAC
AA).
   2) Quantitative PCR amplification reaction: the qPCR reaction system has a total volume of 10 µl, containing: 5 µL 2 × SYBR Green Master Mix, 0.5 µl forward primer (10 µM), 0.5 µl reverse primer (10 µM), 1 µl cDNA by reverse transcription, 3 µl RNase-free dH₂O. LightCycler 480 fluorescence quantitative PCR instrument was used, and the PCR reaction conditions were: 95 °C for 5 min for pre-denaturation, followed by PCR amplification cycle: (1) 95 °C, 10 s; (2) 55 °C, 10 s; (3) 72 °C , 20 s; a total of 40 cycles; 40 °C for 10 s in the end to cool down. Both the forward and reverse primers of the amplification reaction were designed and synthesized by Beijing Tsingke Biotechnology Co., Ltd. (U6 F Primer: GCGCGTCGTGAAGCGTTC, U6 R Primer: GTGCAGGGTCCGAGGT, HJT-sRNA-m7 F Primer: TCGCGCTGAGGTAGTAGGTT, HJT-sRNA-m7 R Primer: GTGCACGCTCCGAGGT).
   3) The relative expression level was calculated by 2-ΔCt method.
3.3.3 Fluorescence in situ hybridization (FISH) of small RNA
   1) The medium was removed and washed 3 times with PBS (500 µl/well).
   2) Fix in 4% paraformaldehyde (500 µl/well, prepared in PBS buffer) at room temperature for 20 min.
   3) Wash with 1 × PBS (500 µ1/well) and soak for 5 min in fresh 1 × PBS (500 µl/well).
   4) PBS was removed, and cells were permeabilized with PK (proteinase K) buffer at room temperature for 10 min.
   5) Wash with 1 × PBS (500 µl/well), and fix in 4% paraformaldehyde (500 µl/well, prepared in PBS buffer) at room temperature for 10 min.
   6) Wash with 1 × PBS, and soak for 5min in fresh 1 × PBS (500 µl/well).
   7) Cells were treated with 0.1 M TEA at room temperature for 10 min.
   8) Wash with 1 × PBS (500 µl/well) and soak for 5 min in fresh 1 × PBS (500 µl/well).
   9) The culture plate was placed in a hybridization cassette in hybridization buffer (50% formamide, 5 × SSC, 5 × Denharts, 250 µg/mL yeast RNA, 500 µg/mL herring sperm DNA) and pre-incubated at room temperature for 1 hour.
   10) Add RNA probes (HJT-sRNA-m7 probe: 5'-GCTTACAACCACACAACCTACTACCTCA-3', Scrambled probe: 5'-CAGTACTTTTGTGTAGTACAA-3', U6 probe: 5'-TTTGCGTGTCATCCTTGCG-3') to the hybridization buffer (the concentration of RNA probes was 0.1-0.2 ng/µl), denature at 85 °C for 5 min, and quickly place it on ice.
   11) Remove the pre-hybridization buffer from step 9, and replace it with the hybridization buffer containing the RNA probe from step 10, then place the plate in the hybridization cassette, and incubate overnight (12-16 hours) at 65 °C.
   12) Pre-heat the 0.2 × SSC solution to 65 °C and wash three times with 0.2 × SSC (1 mL/well) for 20 min each time.
   13) Add 0.2 × SSC solution (1 mL/well) at room temperature and stand for 5 min.
   14) Aspirate 0.2 × SSC, add Buffer B1 (0.1 M Tris-HCl (pH 7.4~7.5), 150 mM NaCl), and wash twice at room temperature, 5 min each time.
   15) Wash three times with PBS, 5 min each time.
   16) Observe under confocal microscopy.

### 3.4 Effects of traditional Chinese medicine extracts on absorption and entry of nucleic acids into cells

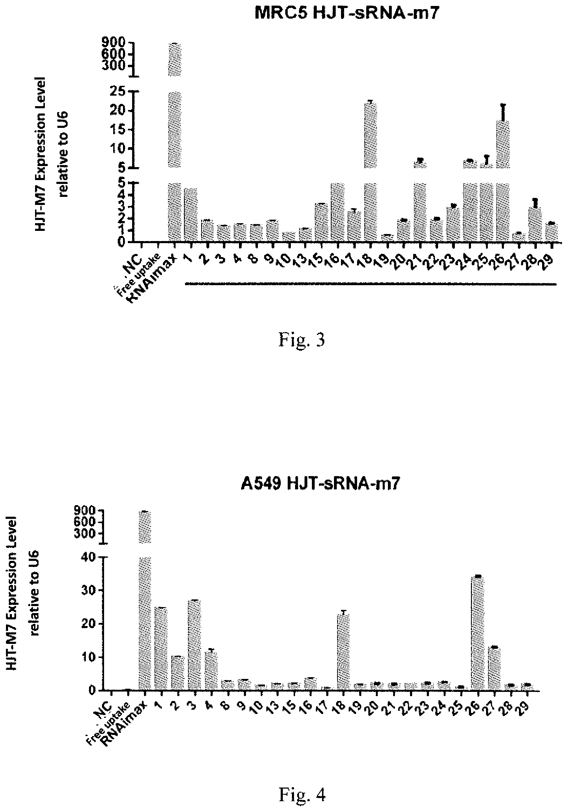

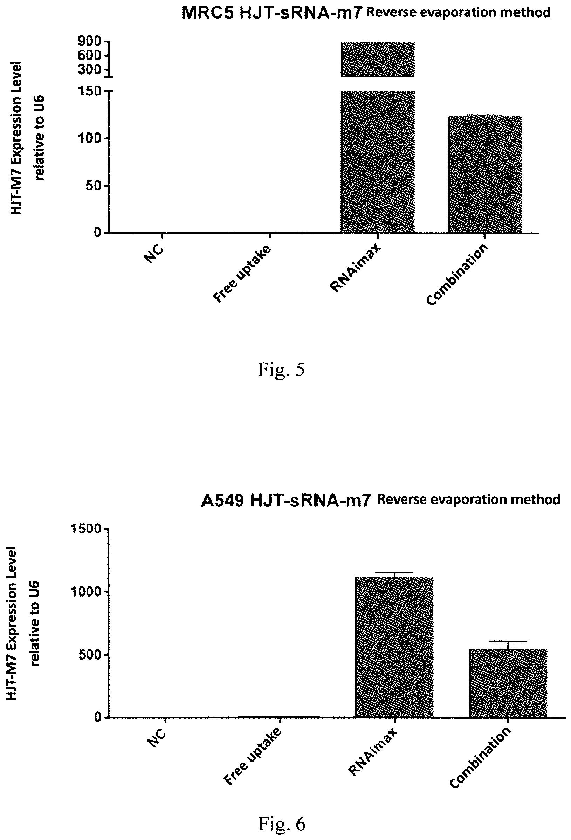

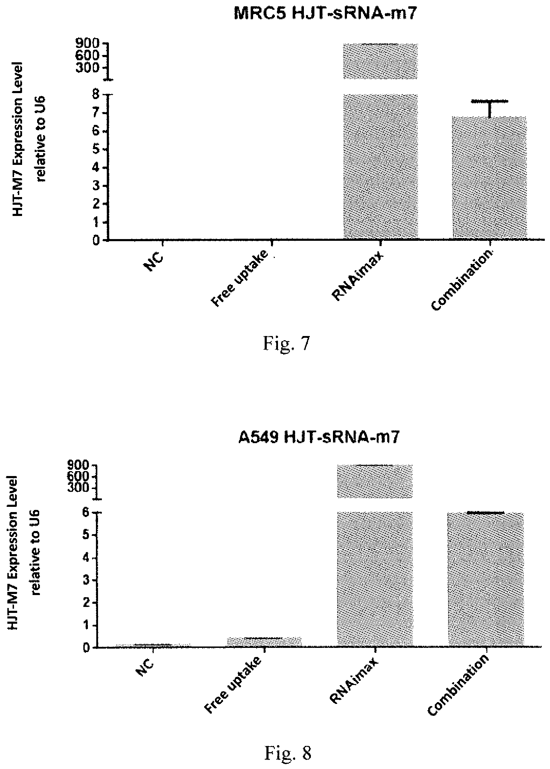

1) 30 lipids shown in Table 1 were selected for the experiments, and experimental groups were numbered according to the lipid numbers shown in Table 1. Lipid nucleic acid mixtures were prepared according to the reverse evaporation method and the boiling method described in Step 2. The *in vitro* delivery experiment was carried out using the lipid nucleic acid mixtures according to steps 3.1-3.3, and the abundance of intracellular RNA was determined.
   The experimental results were shown in Figures 1-4. Figures 1-2 indicated that the lipid nucleic acid mixtures prepared by the reverse evaporation method could successfully deliver nucleic acids to NCI-N87 and MRC-5 cells; Figures 3-4 showed that the lipid nucleic acid mixtures prepared by the boiling method could successfully deliver nucleic acids to MRC-5 and A549 cells.
2) Further, various lipids of Table 1 were combined. 200 µl lipid combination in diethyl ether solution (having a concentration of 0.00326 mg/mL for the lipid combination of No. 1/2/4/9/18/19/20/21/22/23/24/25/26/27/28/29, 0.00652 mg/mL for the lipid combination of No. 3/8/10/13, 0.000652 mg/mL for the lipid combination of No. 15/16/17) and 3 µl lipid combination in chloroform solution (having a concentration of 5 mg/mL for lipid combination of No. 1/2/4/9/18/19/20/21/22/23/24/25/26/27/28/29, 10 mg/mL for the lipid combination of No. 3/8/10/13, 1 mg/mL for the lipid combination of No. 5/16/17) were prepared. The above lipids were mixed in equal volume to obtain the mixed lipids and to prepare the lipid nucleic acid mixtures by the reverse evaporation method and the boiling method, respectively, as described below. The *in vitro* delivery experiment was carried out using the lipid nucleic acid mixtures according to steps 3.1-3.3, and the abundance of intracellular RNA was determined.
   Preparation of mixture of lipid combination and nucleic acid by reverse evaporation method:
   200 µl lipid combination in diethyl ether solution was added to 40 µl HJT-sRNA-m7 aqueous solution (5 µM) at a volume ratio of 5:1 between the lipid solution and the RNA, and sonicated for 3min; diethyl ether was removed by evaporation at 55 °C, and then 200 µl DEPC water was added for hydration to obtain lipid nucleic acid mixture.
   Manufacture of mixture of lipid combination and nucleic acid by boiling method:
   3 µl lipid combination in chloroform solution was mixed with 100 µl HJT-sRNA-m7 aqueous solution (2 µM), and heated at 100 °C for 30 min.
   The experimental results were shown in Figures 5-8. Figures 5-6 demonstrated the mixture of lipid combination and nucleic acid prepared by the reverse evaporation method could successfully facilitate nucleic acid entry into a target cell; Figures 7-8 demonstrated the mixture of lipid combination and nucleic acid by the boiling method could successfully facilitate nucleic acid entry into a target cell.
3) The different types of lipids shown in Table 1, e.g., TG mixture, DG mixture and the like, were combined, and used for the preparation of lipid nucleic acid mixture by the reverse evaporation method and the boiling method, respectively. The *in vitro* delivery experiment was carried out using the lipid nucleic acid mixtures according to steps 3.1-3.3, and the abundance, intracellular localization and targeted regions of intracellular RNA were determined.
   Different types of lipids were combined as follows:
   Combination 1: combination of lipids No. 1-32, No. 1/2/3/4/6/8/9/10/13-32 without lipids No. 5, 7, 11, and 12;
   Combination 2: combination 1 without lipid No. 29;
   Combination 3: combination 1 without lipids No. 1, 2, 3, 19;
   Combination 4: combination 1 without lipids No. 4, 14;
   Combination 5: combination 1 without lipids No. 6, 9, 10, 13, 15, 16, 18, 20-28, 32;
   Combination 6: combination 1 without lipid No. 8;
   Combination 7: combination 1 without lipids No. 17, 30, 31;
   FA: lipid No. 29;
   DG combination: combination of lipids No. 1, 2, 3, 19;
   Cer combination: combination of lipids No. 4, 14;
   TG combination: combination of lipids No. 6, 9, 10, 13, 15, 16, 18, 20-28, 32;
   PE combination: lipid No. 8;
   So combination: combination of lipids No. 17, 30, 31.

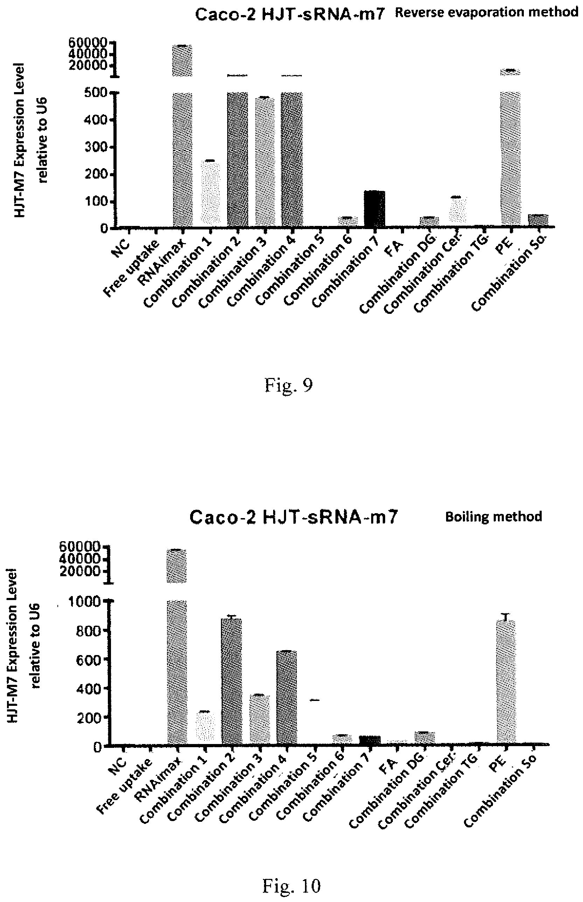

   The experimental results were shown in Figures 9-10. The results showed that different types of lipid combination (e.g., mixture of TG, mixture of DG, etc.) by different methods (boiling or reverse evaporation) could promote nucleic acid entry into a target cell.
4) Further, lipids No. 11 and No. 12 were selected for experiments to investigate the efficiency of the lipids to deliver nucleic acid fragments having different sequences, as well as the localization and the targeted gene regions of the nucleic acids. The protocols were as follows:
   Mixtures of soybean PC, lipid No. 11 (18:0/18:2) and lipid No. 12 (16:0/18:2), and various small RNA(see below Table 3) were prepared by the reverse evaporation method and then added into A549 cell line (the final concentration of sRNA was 200 nM); the negative control group (control) was directly added with the same concentration of sRNA; the positive control group (RNAimax) was transfected with Lipofectamine RNAimax (transfection reagent 6 µl/well). The abundance of sRNA in the cells was detected by Taqman probe after 3 hours, and the relative expression level of sRNA was calculated by 2-ΔCt method.

The experimental results were shown in Figures 11-13. Figures 11A-C showed that compared with the control, the two lipids (lipid No. 11 (18:0/18:2) and lipid No. 12 (16:0/18:2)) could effectively facilitate nucleic acid molecules of different sequences entry into various cells; Fig. 12 showed that nucleic acids that were delivered by lipid No. 11(18:0/18:2) and lipid No. 12 (16:0/18:2) entered into cytoplasm and were primarily localized in cytoplasm. In addition, with reference to Figure 13, the inventor unexpectedly found that both lipids No. 11 and No. 12 promoted the entry of small fragments of nucleic acids, which acted on the wild-type 3'UTRs of their target genes and reduced the relative expression level of the Luciferase with the wild-type 3'UTR in the target gene, while did not act on the mutated 3'UTR of their target genes. It can be used as a means for the delivery of nucleic acid drug.

### 4. In vivo delivery experiments of lipid nucleic acid mixture

### 4.1 Experimental steps

1. Preparation of lipid nucleic acid mixture: the mixture of lipid No. 11 or No. 12 with nucleic acid, and the mixture of the lipid combination of No. 1/2/4/9/14/18/19/20/21/22/23/24/25/26/27/28/29/30/32, No. 3/8/10/11/12/13, and No. 6/15/16/17/31 with nucleic acid were prepared by reverse evaporation and boiling method (refer to steps 2.1-2.2).
2. Gavage were performed on 6-8 weeks old male C57 mice : 200 µl/aminal, grouped as follows:
   (1) Control group (free uptake group): no treatment or HJT-sRNA-m7 was given by gavage;
   (2) Lipid No. 11 (18:0/18:2) group: lipid No. 11 (18:0/18:2) or a mixture of lipid No. 11 (18:0/18:2) and HJT-sRNA-m7 were given by gavage;
   (3) Lipid No. 12 (16:0/18:2) group: lipid No. 12 (16:0/18:2) or a mixture of lipid No. 12 (16:0/18:2) and HJT-sRNA-m7 were given by gavage;
3. Sample collection: 6 hours after gavage, mouse whole blood (500 µl) and lung (110 mg) were collected by 1.5 mL TRIzol-LS or 3 mL TRIzol, respectively, homogenized and then frozen under -80 °C for storage.
4. Total RNA extraction: (1) Add TRIzol or TRIzol-LS lysis buffer(Sigma Corporation) to the cells, which were then left at room temperature for 5 min to be fully lysed (for mouse lung tissue, to 100 mg tissue was added 1.0 mL TRIzol lysis buffer, and the solution was ground with a homogenizer, centrifuged at 12,000 rpm, 4 °C for 10 min to remove the tissue precipitate which was not homogenized; for the mouse whole blood, to 500 µl of whole blood was added 1.5 mL TRIzol-LS lysis buffer centrifuged at 12,000 rpm, 4 °C for 10min to remove the precipitate that has not been fully cleaved); (2) 12,000 rpm, 4 °C, centrifuge for 5 min, and discard the precipitate; (3) add chloroform at a ratio of 200 µl/mL TRIzol, vortex to mix, allow to stand at room temperature for 15 min. (4) 12,000 rpm, 4 °C, centrifuge for 15 min, pipette the upper aqueous phase to another centrifuge tube; (5) Repeat step 4, add equal amount of chloroform into the upper aqueous phase, mix well, and allow to stay for 10 min at room temperature, 12,000 rpm, 4 °C, centrifuge for 15min; (6) Draw the upper aqueous phase to a fresh EP tube, add isopropanol at a ratio of 0.5 ml/mL TRIzol, mix, and allow to stay at room temperature for 5-10min; (7) 12,000 rpm, 4 °C, centrifuge for 10 min, discard the supernatant; (8) add 1 mL 75% ethanol, gently shake the centrifuge tube, suspend the precipitate; (9) 8000 g, 4 °C, centrifuge for 5 min, discard the supernatant as much as possible; and (10) dry at room temperature for 5-10 min and dissolve the RNA sample with 50 µl DEPC-treated H₂O.
5. RT-qPCR detection: see the method described in above Sections 3.3.1 and 3.3.2.

### 4.2 Experimental results

With reference to Fig.14, the inventor unexpectedly discovered that lipid No. 11 (18:0/18:2) and lipid No. 12 (16:0/18:2) could promote entry of small fragments of nucleic acids into the blood and lung by (non-invasive) gavage, which can be used as a means for the delivery of nucleic acid drug. Surprisingly, the lipid nucleic acid mixture obtained by direct boiling method achieved a significant delivery effect.

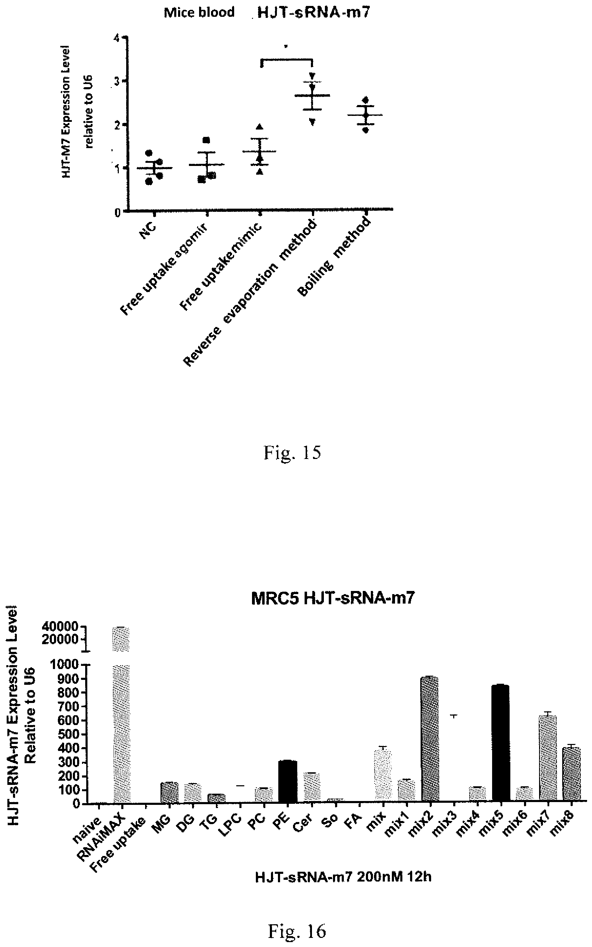

With reference to Fig.15, the inventor surprisingly found that, the mixture of 28 lipids could facilitate entry of small fragments of nucleic acids into the blood by the (non-invasive) gavage, which can be used as a means for the delivery of nucleic acid drug. Surprisingly, the mixture of lipid combination with nucleic acid obtained by direct boiling method achieved a significant delivery effect.

### Examples for lipids shown in Table 1 as No. 1-71

### Method

### 1. Extraction of lipids from traditional Chinese medicine

### 1.1 Decoction of Chinese medicine

1) 100g Chinese medicine decoction pieces (*Rhodiola crenulata, Taraxacum mongolicum, Lonicera japonica* and *Andrographis paniculata,* purchased from Beijing Tongrentang pharmacy) was added to 1000 mL ddH₂O and soaked for 30 min.
2) The Chinese medicine decoction pot was boiled for 15 min with intense heating, and for 20 min with slow heating.
3) 400 mL of the heated Chinese medicine soup was added to a rotary evaporator, and was concentrated to 100 mL at 60 °C, 60 rpm, 30 min.

### 1.2 Lipid extraction

1) To the 160 mL Chinese medicine soup (concentrated by rotary evaporator) was added chloroform-methanol mixture (chloroform: methanol=1:2, v/v) 600 mL to make chloroform:methanol:water=1:2:0.8, and stirred for 10-15 min to mix.
2) 200 mL chloroform was add to the Erlenmeyer flask and stirred for 10 min to mix.
3) 200 ml ddH₂O was added to the Erlenmeyer flask to make chloroform:methanol:water=2:2:1.8, stirred for 10 min to mix .
4) The liquid of upper layer and the insoluble substances of intermediate layer was removed, and the chloroform layer of lower layer was taken out and stored at - 40°C.

### 1.3 HPLC-MS/MS identification of lipid components

### Instrument setup

### 1) Chromatographic setup:

Instrument: Ultimate 3000; column: Kinetex C18 (100 × 2.1 mm, 1.9 µm); column temperature: 45 °C; mobile phase A: acetonitrile: water (v/v, 60:40), the solution containing 10 mmol/L ammonium formate, mobile phase B: acetonitrile: isopropanol (10:90, v/v), the solution containing 10 mmol/L ammonium formate and 0.1 % formic acid. Flow rate: 0.4 mL/min; injection volume: 4µl.

### 2) Mass spectrometry parameters:

a) Positive mode: Heater Temp 300 °C, Sheath Gas Flow rate, 45 arb, Aux Gas Flow Rate, 15 arb, Sweep Gas Flow Rate, 1 arb, spray volt age, 3.0 KV, Capillary Temp, 350 °C, S -Lens RF Level, 30%. Scan ranges: 200-1500.
b) Negative mode: Heater Temp 300 °C, Sheath Gas Flow rate, 45 arb, Aux Gas Flow Rate, 15 arb, Sweep Gas Flow Rate, 1 arb, spray voltage, 2.5KV, Capillary Temp, 350 °C, S- Lens RF Level, 60%. Scan ranges: 200-1500.

### 1.4 Identification of the lipids derived from Chinese medicine

The lipid components were identified by HPLC-MS/MS, and a total of 138 lipid components derived from traditional Chinese medicine were identified, among which 125 were identified in positive mode and 13 in negative mode. The following experiments was performed on the compounds 1-69 shown in Table 1. It should be noted that the lipids tested below were all commercially purchased or commercially synthesized, and used as described in Table 1-1.

### 2. Manufacture of lipid nucleic acid mixture

### 2.1 Reverse evaporation method:

100 µl lipid in diethyl ether solution was prepared, and grouped according to the lipid numbers shown in Table 1 (the lipid concentrations are shown in the table below). To the lipid solution was added 20 µl nucleic acid solution (HJT sRNA or siRNA) at the volume ratio of 5:1, and sonicated for after 3 min. The diethyl ether was removed by evaporation at 55 °C, and then 100 µl DEPC water was added for hydration to give nucleic acid lipid mixture.

**Table 3**

| **Figure** | **Single lipid or lipid combination** | **Concentration/(mg/mL)** | |
|---|---|---|---|
| 51 | 8 + 12=1:2 | No. 8 | 0.0833 |
| | | No. 12 | 0.1667 |
| 71 | 38+12+37=4:1:1 | No.38 | 0.2 |
| | | No. 12 | 0.05 |
| | | No. 37 | 0.05 |
| 76/77/79/82 | No. 41 | No. 41 | 0.25 |
| 87 | 40+12+41=2:4:3 | No. 40 | 0.0667 |
| | | No. 12 | 0.1333 |
| | | No. 41 | 0.1 |
| 88 left | 12+41 =1:6 | No. 12 | 0.0428 |
| | | No. 41 | 0.2571 |
| 88 left | 12+41=1:1 | No. 12 | 0.15 |
| | | No. 41 | 0.15 |
| 89 left | 12+41=6:1 | No. 12 | 0.2571 |
| | | No. 41 | 0.0428 |
| 89 right | 4+12+41=1:1:1 | No. 4 | 0.1 |
| | | No. 12 | 0.1 |
| | | No. 41 | 0.1 |
| 90 | 4+12+41=1:1:1 | No. 4 | 0.1 |
| | | No. 12 | 0.1 |
| | | No. 41 | 0.1 |
| 93 | No. 38 | No. 38 | 0.25 |
| 99/100/102 | No. 40 | No. 40 | 0.25 |
| 104/105 | No. 39 | No. 39 | 0.25 |
| 106 | No. 60 | No. 60 | 0.25 |
| 107 | No. 62 | No. 62 | 0.25 |

### 2.2 boiling method:

100 µL of the nucleic acid solution (HJT sRNA or siRNA) was added to 2-5 µL of the lipid in chloroform (the concentration was shown in Table 1), mixed, and heated at 80-100 °C for 15-30 min to give nucleic acid lipid mixture.

### 3. In vitro delivery experiment of lipid nucleic acid mixture

### 3.1 Real-time quantitative PCR (RT-qPCR) detection of intracellular expression of nucleic acids delivered by lipid.

3.1.1 MRC-5 cell (pulmonary embryonic fibroblast), A549 cell (human lung adenocarcinoma cell), Caco-2 cell (human colon adenocarcinoma cell) (purchased from the Cell Resource Center of the Institute of Basic Medical Sciences, Chinese Academy of Medical Sciences) were cultured to logarithmic growth phase, then plated into 12-well plates at a cell density of 6×10⁵/1 mL medium/well; MRC-5 and Caco-2 cells were cultured in Eagle's MEM medium (MEM, Gibco); A549 cells were cultured in Ham's F-12 medium (HyClone); followed by incubation overnight at 37 °C, and the follow-up experiments were performed after the cells were attached to the walls.

### 3.1.2 Experimental groups were as follows:

1) naive group: it referred to untreated cells, and this group served as a blank control group.
2) RNAimax treatment group: 2 µl Lipofectamine™RNAimax transfection reagent (full name of Lipofectamine RNAiMAX, Invitrogen, Thermo Fisher Scientific) and HJT-sRNA-m7 solution were diluted in 100 µl opti-MEM medium (purchased from Invitrogen, Thermo Fisher Scientific) respectively and then the two were mixed, allowed to stand for 15 min, added into cells and then mixed. The final concentration of HJT-sRNA-m7 was 100 nM; this group served as a positive control group.
3) Free uptake group: HJT-sRNA-m7 solution was directly added (the final concentration was 100 nM), and the group served as a negative control group.
4) Lipid nucleic acid mixture: the mixture of lipid and HJT-sRNA-m7 prepared from the step 2 were added into cells and mixed, and the final concentration of HJT-sRNA-m7 was 100 nM.

3.1.3 After co-incubation with cells for 12-24 hours, the cells were washed twice with PBS. The cells were harvested with TRIzol lysis buffer (purchased from Sigma-Aldrich), and total RNA was extracted. The abundance of HJT-sRNA-m7 that entered the cells was detected by RT-qPCR; the protocols were as follows:
1) Extraction of total cellular RNA:
   A. To the cells cultured in a 12-well plate (about 1×10⁶ cells/well) was added 1 mL TRIzol lysis buffer in each well, and then placed on ice. After to all the samples was added TRIzol, they were allowed to stand at room temperature for 5 min to allow them fully lysed.
   B. Centrifuge at 4 °C, 12,000 rpm for 5 min, discard the pellet and transfer TRIzol to a fresh centrifuge tube;
   C. Add chloroform at a ratio of 200 µL chloroform/mL TRIzol, shake well, mix and allow to stand for 5 min at room temperature;
   D. Centrifuge at 4 °C, 12,000 rpm for 15 min;
   E. Pipette the upper aqueous phase into another centrifuge tube, add isopropanol at a ratio of 0.5 mL isopropanol/mL TRIzol and allow to stand at room temperature for 5-10 min;
   F. Centrifuge at 4 °C, 12,000 rpm for 15 min, discard the supernatant, and allow the RNA to precipitate to the bottom of the tube;
   G. Add 1 mL 75% ethanol, gently shake the tube to suspend the precipitate;
   H. Centrifuge at 4 °C, 12,000 rpm for 10 min, discard the supernatant, add 1 mL 75% ethanol, gently shake the centrifuge tube to suspend the precipitate;
   I. Centrifuge at 4 °C, 12,000 rpm for 10 min, discard the supernatant, dry at room temperature, dissolve the RNA sample with 50 µL RNase-free H₂O, and quantify the RNA concentration by the measurement of OD value.
2) Total RNA was reverse transcribed to cDNA: Reverse Transcription Kit (High-Capacity cDNA Reverse Transcription Kits, Applied Biosystems, cat. no. 4368813) was used to reverse transcribe sRNA to cDNA by stem-loop method (see, e.g. Real-time quantification of microRNAs by stem-loop RT-PCR, Nucleic Acids Res. 2005 Nov 27; 33(20):e179, incorporated by reference herein). The reverse transcription system was as follows: template RNA (150 ng/µL) 10 µL, 10X RT buffer 2.0 µL, 25X dNTP Mix (100 mM) 0.8 µL, U6 RT stem-loop primer 2.0 µL, HJT-sRNA-m7 RT stem-Loop primer 2.0 µL, MultiScribe ™ reverse transcriptase 1.0 µL, RNase inhibitor 1.0 µL, nuclease-free H₂O 1.2 µL, loaded into a PCR reactor after brief centrifugation. The reaction conditions were as follows: (1) 25 °C, 10 min; (2) 37 °C, 120 min; (3) 85 °C, 5 min; (4) 4 °C, termination of reaction. 20 µl RNase-free ddH₂O was added to make up the final volume to 40 µl after the reaction. The stem-loop primer used in the reverse transcription process was synthesized by Beijing Tsingke Biotechnology Co., Ltd. (U6 RT primer, because the quantification of small RNA by RT-qPCR reaction can only be relative, so U6 was used as a standard reference gene for calculating relative expression level): GTCGTATCCAGTGCAGGGTCCGAGGTATTCGCACTGGATACGACAAAAAT ATG; HJT-sRNA-m7 RT stem-loop primer: 
GTCGTATCCAGTGCACGCTCCGAGGTATTCGCACTGGATACGACGCTTAC
AA).
3) Quantitative PCR amplification reaction: the qPCR reaction system had a total volume of 10 µl, containing: 5 µL 2 × SYBR Green Master Mix, 0.5 µl forward primer (10 µM), 0.5 µl reverse primer (10 µM), 1 µl cDNA by reverse transcription, 3 µl RNase-free dH₂O. LightCycler 480 fluorescence quantitative PCR instrument was used, and the PCR reaction conditions were: 95 °C, pre-denaturation for 5 min, followed by PCR amplification cycle: (1) 95 °C, 10 s; (2) 55 °C, 10 s; (3) 72 °C , 20 s; a total of 40 cycles; 40 °C for 10 s in the end to cool down. Both the forward and reverse primers of the amplification reaction were designed and synthesized by Beijing Tsingke Biotechnology Co., Ltd. (U6 forward primer: GCGCGTCGTGAAGCGTTC, U6 reverse primer: GTGCAGGGTCCGAGGT, HJT-sRNA-m7 forward primer: TCGCGCTGAGGTAGTAGGTT, HJT-sRNA-m7 reverse primer: GTGCACGCTCCGAGGT).
4) 2-ΔCt method (relative gene expression level= 2-(Ct target gene-Ct internal reference gene)) was used to calculate the relative amount of entry (single or double stranded RNA).

### 3.2 Real-time quantitative PCR (RT-qPCR) detection of mRNA expression levels

3.2.1 THP-1 cell (human monocyte) was cultured to logarithmic growth phase, then plated into 12-well plates at a cell density of 6×10⁵/1 mL medium/well; THP-1 cells were cultured in RPMI-1640 medium (HyClone); the cells were incubated overnight at 37 °C, and the follow-up experiments were performed after the cells were attached to the walls.

### 3.2.2 Exeperimental groups were as follows:

1) naive group: referred to untreated THP-1 cells, and this group served as a blank control group.
2) RNAiMAX treatment group: 2 µl Lipofectamine™RNAimax transfection reagent (Invitrogen, Thermo Fisher Scientific) and nucleic acid solution (TNFα siRNA) were diluted in 100 µl opti-MEM medium (Invitrogen, Thermo Fisher Scientific) respectively and then the two were mixed, allowed to stand for 15 min, added into cells, and then mixed. The final concentration of nucleic acid was 400 nM; this group served as a positive control group.
3) Free uptake group: nucleic acid solution (TNFα siRNA) was directly added (the final concentration was 400 nM), the group served as a negative control group.
4) Lipid nucleic acid mixture: the mixture of lipid and nucleic acid prepared from the step 2 were added into cells and mixed, and the final concentration of nucleic acid was to 400 nM.

3.2.3 After 24 hours of treatment, the cells were stimulated with 1 µg/mL E. coli LPS (Lipopolysaccharide, LPS, *Escherichia coli* 0111:B4, L4391, Sigma-Aldrich), and harvested using TRIzol lysis buffer after 9 hours to extract total RNA. The mRNA expression level of TNF- α (the target genes of the subsequent examples varied case by case and were indicated in the Figures) was determined by RT-qPCR (SYBR Green dye method), and the protocols were as follows:
1) Extraction of the total RNA from cells: the procedures were the same as the method of extracting total RNA in Section 3.1.3.
2) Total RNA was reverse transcribed to cDNA: Reverse Transcription Kit (High-Capacity cDNA Reverse Transcription Kits, Applied Biosystems, cat. no. 4368813) was used to reverse transcribe the total RNA to cDNA. The reverse transcription system was as follows: template RNA (150 ng/µL) 10 µL, 10X RT buffer 2.0 µL, 25X dNTP Mix (100 mM) 0.8 µL, random primers 2.0 µL, MultiScribe ™ reverse transcriptase 1.0 µL, RNase inhibitor 1.0 µL, nuclease-free H₂O 3.2 µL, loaded into a PCR reactor after brief centrifugation. The reaction conditions were as follows: (1) 25 °C, 10 min; (2) 37 °C, 120 min; (3) 85 °C, 5 min; (4) 4 °C, termination of reaction. 20 µl RNase-free dd H₂O was added to make up the final volume to 40 µl after the reaction.
3) Quantitative PCR amplification reaction: the total volume of qPCR reaction system was 10 µl, containing: 5 µL 2 × SYBR Green Master Mix, 0.5 µl forward primer (10 µM), 0.5 µl reverse primer (10 µM), 1 µl cDNA by reverse transcription, 3 µl RNase-free dH₂O. LightCycler 480 fluorescence quantitative PCR instrument was used, the PCR reaction conditions were: 95 °C, pre-denaturation for 5 min, followed by PCR amplification cycle: (1) 95 °C, 10 s; (2) 55 °C, 10 s; (3) 72 °C , 20 s; a total of 40 cycles; 40 °C for 10 s in the end to cool down. Both the forward and reverse primers of the amplification reaction were designed and synthesized by Beijing Qingke Biotechnology Co., Ltd.. The primer sequences were as follows: forward primer for internal reference gene UBC: CTGGAAGATGGTCGTACCCTG, reverse primer for internal reference gene UBC: GGTCTTGCCAGTGAGTGTCT; forward primer for target gene TNF-α: CTGCCCCAATCCCTTTATT: reverse primer for target gene TNF-α: CCCAATTCTCTTTTTGAGCC.
4) The relative expression level was calculated 2-ΔCt method as described above.

### 3.3 Western blot detection of protein expression levels

3.3.1 MRC-5 cell (pulmonary embryonic fibroblast), and A549 cell (human lung adenocarcinoma cell) were cultured to logarithmic growth phase, and then plated into 12-well plates at a cell density of 6×10⁵/1 mL medium/well; MRC-5 cells were cultured in Eagle's MEM medium (MEM, Gibco); A549 cells were cultured in Ham's F-12 medium (HyClone); followed by incubation overnight at 37 °C, and the follow-up experiments were performed after the cells were attached to the walls.

### 3.3.2 Exeperimental groups were as follows:

1) Naive group: it referred to the untreated cells, and this group served as a blank control group.
2) RNAiMAX treatment group: 2 µl Lipofectamine™RNAimax transfection reagent (Invitrogen, Thermo Fisher Scientific) and nucleic acid solution were diluted in 100 µl opti-MEM medium (Invitrogen, Thermo Fisher Scientific) respectively and then the two were mixed, allowed to stand for 15 min, added into cells, and then mixed. The final concentration of nucleic acid was 400 nM; this group served as a positive control group.
3) Free uptake group: the nucleic acid solution was directly added (the final concentration was 400 nM), and the group served as a negative control group.
4) Lipid nucleic acid mixture: the mixture of lipid and nucleic acid prepared from the step 2 were added into cells and mixed, and the final concentration of nucleic acid was 400 nM.

3.3.3 After 24 hours of treatment, the cells were stimulated with the stimulant (1 µg/mL poly (I:C) (P1530, Sigma-Aldrich) as double-stranded RNA viruses mimetics) or 3 ng/mL transforming growth factor TGFβ1 (Pepro Tech)). The cells were harvested using strong RIPA lysis buffer, and after incubation for some time, Western blot was used to detect the protein expression level of the related genes (the types of the related gene varied case by case and were indicated in the corresponding Figures) (the protein expression level of REL-A was detected 24 hours after the A549 cells were stimulated by poly(I:C) with β-actin as the internal reference protein; the protein expression levels of fibronectin and α-SMA were detected 72 hours after MRC-5 cells were stimulated with TGF-β1 with GAPDH as the internal reference protein; the protein expression of the corresponding knockdown genes was detected in the siRNA delivery assay with β-actin as the internal reference protein). The protocols were as follows:
1) Collection of protein samples and determination of the concentration by BCA method.
A. Discard the medium, add 1 mL PBS buffer into each well of the 12-well plate to wash the cells once, add 100 µL precooled strong RIPA lysis buffer into each cell, scrap off the cells with a pipette tip and transfer to a centrifuge tube, place and keep on ice for 20 min for lysis;
B. Centrifuge at 4 °C, 12,000 rpm for 10 min, transfer the supernatant to a frech centrifuge tube;
C. Mix BCA reagent A and B (50:1, v/v) thoroughly to prepare a BCA working solution;
D. Add 25 µL of the freshly prepared BSA standard solution and the samples to be tested to a 96-well plate, add 200 µL BCA working solution to each well and mix well; incubate at 37 °C for 30 min;
E. Measure the absorbance at 562 nm using an ultraviolet spectrophotometer (Synergy 4 multi-function microplate reader), and calculate the protein concentration in the samples according to the standard curve;
F. Adjust the concentration of the samples with RIPA lysis buffer and loading buffer so that the concentration of each sample was the same;
G. Denaturation at 95 °C for 10 min.

2) Western blot
A. Gel preparation: a resolving gel (lower layer gel) with a concentration of 10% and stacking gel (upper layer gel) with a concentration of 5 % were used. The lanes were made with a 15-well comb, and equal amounts of protein were loaded in each lane;
B. Protein electrophoresis: add electrophoresis buffer and use an initial voltage of 80V for electrophoresis; when the bromophenol blue dye reach the resolving gel, increase the voltage to 120V and continue electrophoresis until the bromophenol blue dye reach the bottom or completely out of the resolving gel;
C. Wet transfer: make the assembly in the following order: transfer pad (anode) - sponge-filter paper-gel-PVDF membrane-filter paper-sponge-transfer pad(cathode); install the assembly and put the whole transfer device at 4 °C cold chamber; set constant current at 300 mA for a 120 min transfer;
D. Blocking: place the membrane in a 3% BSA blocking solution after the transfer and block at room temperature for 1 hour;
E. Primary antibody incubation: transfer the blocked PVDF membrane to the hybridization bag, add 3% BSA blocking solution containing the corresponding primary antibody (the primary antibody informations were as follows), remove the bubbles in the bag, and incubate overnight at 4 °C.

**Table 4**

| **Primary Antibody** | **Company** | **Cat. No.** | **Dilution ratio of primary antibody** | **Secondary Antibody** |
|---|---|---|---|---|
| fibronectin | Sigma Aldrich | F7387 | 1: 2000 | M |
| α-SMA | Abcam | ab7817 | 1:1000 | M |
| GAPDH | Protein Tech | 60004-1-lg | 1:5000 | M |
| LAMP1 | Santa Cruze | sc-20011 | 1:1000 | M |
| LAMP2 | Santa Cruze | sc-18822 | 1:1000 | M |
| XRN2 | Santa Cruze | sc-365258 | 1:2000 | M |
| CPSF4 | Protein Tech | 15023-1-AP | 1:1000 | R |
| Ssu72 | CST | 12816s | 1:1000 | R |
| NF-κB | CST | 4764S | 1:2000 | R |
| β-actin | Sigma Aldrich | A5441 | 1:5000 | M |

F. Membrane wash: take out the PVDF membrane and wash the membrane 3 times with TBST for 10 min each time;
G. Secondary antibody incubation: discard TBST, add 3% BSA blocking solution containing goat anti-rabbit or goat anti-mouse secondary antibody with horseradish peroxidase (HRP) (purchased from Hangzhou Lianke Biotechnology Co., Ltd.) (dilution ratio of secondary antibody was 1:5000), incubate for 1 hour at room temperature;
H. Membrane wash: wash the membrane 3 times with TBST for 10 min each time;
I. Developing: prepare Western developing solution (1:1, V/V, Merck Millipore, ECL chemiluminescence developing solution purchased from Millipore), and add the prepared developing solution evenly to the side the membrane that is bound to the proteins; carefully wrap the film with plastic wrap and observe after developing;
J. Analysis: analysis was performed using Image J software.

### 4. In vivo delivery exeperiments of lipid nucleic acid mixture

### 4.1 Experimental steps:

1) Preparation of lipid nucleic acid mixture: boiling method was used. To 400 µL HJT-sRNA-m7 (5 nmol) single-stranded RNA in DEPC-treated solution was added 9 µL or 18 µL lipid combinations (lipid PE (No. 38) & LPC (No. 37) & TG (No. 32), 4:2:3, V/V/V) respectively, mixed and heated at 100 °C for 30 min.
2) Intragastric administration of RNA in 6-8 weeks old male C57BL/6J wild type mice: HJT-sRNA-m7 aqueous solution or the mixture solution of lipid and HJT-sRNA-m7 were administered using a gavage needle, 400 µL/animal (HJT)-sRNA-m7, 5 nmol/animal). The groups were as follows:
   A. Control group (naive group): mice that did not receive any treatment;
   B. Negative control group (lipid group): intragastric administration of 9 µL lipid combinations (lipid PE (No. 38) & LPC (No. 37) & TG (No. 32), 4:2:3, V/V/V);
   C. Free uptake group: direct intragastric administration of HJT-sRNA-m7 single-stranded RNA solution;
   D. Lipid and nucleic acid mixture group: intragastric administration of the mixture of lipid combination and HJT-sRNA-m7 single-stranded RNA.
3) Sample collection: 3 hours after intragastric administration, the mouse whole lung was lysed with 3 mL TRIzol, homogenized and frozen at -80 °C.
4) Total RNA extraction:
   A. Add 3.0 mL TRIzol lysis buffer to mouse lung tissue, grind with a homogenizer, centrifuge at 12,000 rpm, 4 °C, for 10 min, remove the tissue precipitate that failed to homogenize;
   B. Add chloroform at a ratio of 200 µl/mL TRIzol, shake well to mix, and keep at room temperature for 15 min.
   C. centrifuge at 12,000 rpm, 4 °C, for 15 min, pipette the upper aqueous phase to another centrifuge tube;
   D. Repeat the above step, add equal amount of chloroform to the upper aqueous phase, mix well, and keepfor 10 min at room temperature;
   E. 12,000 rpm, 4 °C, centrifuge for 15min;
   F. Draw the upper aqueous phase to a fresh EP tube, add isopropanol a ratio of 0.5 ml/mL TRIzol, mix and keep at room temperature for 5-10min;
   G. 12,000 rpm, 4 °C, centrifuge for 15 min, discard the supernatant;
   H. Add 1 mL 75% ethanol, gently shake the centrifuge tube, and suspend the precipitate;
   I. 12,000 rpm, 4 °C, centrifuge for 10 min, discard the supernatant as much as possible;
   J. Dry at room temperature for 5-10 min and dissolve the RNA sample with 50 µl DEPC-treated H₂O.
5) Detection of the abundance of HJT-sRNA-m7 by RT-qPCR (SYBR Green universal dye method).

Unless otherwise indicated, the single stranded HJT-sRNA-m7 solution refers to single-stranded HJT-sRNA-m7 in DEPC-treated aqueous solution. The double-stranded HJT-sRNA-m7 solution refers to adouble-stranded HJT-sRNA-m7 in DEPC-treated aqueous solution.

### Example 1-1: Delivery of single-stranded nucleic acids into MRC-5 cell by different types of lipid combination

### 1. Experimental groups:

1) Naive group: untreated MRC-5 cell;
2) RNAiMAX treatment group: 2 µl RNAiMAX transfection reagent and single-stranded HJT-sRNA-m7 in DEPC-treated aqueous solution were diluted in 100 µl opti-MEM medium, respectively, and then the two were mixed, allowed to stand for 15 min, added into cells, and then mixed. The final concentration of single-stranded HJT-sRNA-m7 was 200 nM;
3) Free uptake group: single-stranded HJT-sRNA-m7 solution was directly added (the final concentration was 200 nM);
4) Lipid nucleic acid mixture: mixtures of 3 µL single lipid or lipid combination and HJT-sRNA-m7 single-stranded nucleic acid solution treated by boiling method were added to the cells and mixed. The final concentration of RNA was 200 nM.

### 2. Experimental procedures

1) Boiling method conditions: to 100 µL single-stranded HJT-sRNA-m7 solution was added 3 µL single lipid or lipid combination in chloroform solution (lipid No. 1/2/4/9/14/18/19/20/21/22/23/24/25/26/27/28/29/30/32 in chloroform solution having a concentration of 5 mg/mL, lipid No. 3/8/10/11/12/13/33/34/35/36 in chloroform solution having a concentration of 10 mg/mL, lipid No. 6/15/16/17/31 in chloroform solution having a concentration of 1 mg/mL), and heated at 100 °C for 30 min;
   a) Lipid combination:
   b) MG (monoglyceride): 3 µL lipid No. 34;
   c) DG (diglyceride): 3 µL mixture of equal volume of lipids No. 1/2/3/19/35 in chloroform solution;
   d) TG (triglyceride): 3 µL mixture of equal volume of lipids No. 6/9/10/13/15/16/18/20/21/22/23/24/25/26/27/28/32/33 in chloroform solution;
   e) LPC (Lysophosphatidylcholine): 3 µL mixture of equal volume of lipids No. 36/37 in chloroform solution;
   f) PC (phosphatidylcholine): 3 µL mixture of equal volume of lipids No. 11/12 in chloroform solution;
   g) PE (phosphatidylethanolamine): 3 µL mixture of equal volume of lipids No. 8/38 in chloroform solution;
   h) Cer (Ceramides): 3 µL mixture of equal volume of lipids No. 4/14 in chloroform solution;
   i) So (Sphingoshine): 3 µL mixture of equal volume of lipids No. 17/30/31 in chloroform solution;
   j) FA (fatty acid): 3µL lipid No. 29;
   k) Mixture: 3 µL mixture of equal volume of lipids No. 1-36 (without No. 5/7) in chloroform solution;
   l) Mixture 1: 3 µL mixture of equal volume of lipids No. 1-36 (without No. 5/7/34) in chloroform solution;
   m) Mixture 2: 3 µL mixture of equal volume of lipids No. 1-36 (without No. 5/7/1/2/3/19/35) in chloroform solution;
   n) Mixture 3: 3 µL mixture of equal volume of lipids No. 1-36 (without No. 5/7/6/9/10/13/15/16/18/20/21/22/23/24/25/26/27/28/32/33) in chloroform solution;
   o) Mixture 4: 3 µL mixture of equal volume of lipids No. 1-36 (without No. 5/7/36/37) in chloroform solution;
   p) Mixture 5: 3 µL mixture of equal volume of lipids No. 1-36 (without No. 5/7/11/12) in chloroform solution;
   q) Mixture 6: 3 µL mixture of equal volume of lipids No. 1-36 (without No. 5/7/8) in chloroform solution;
   r) Mixture 7: 3 µL mixture of equal volume of lipids No. 1-36 (without No. 5/7/4/14) in chloroform solution;
   s) Mixture 8: 3 µL mixture of equal volume of lipids No. 1-36 (without No. 5/7/29) in chloroform solution;
2) Experimental conditions: the final concentration of HJT-sRNA-m7 was 200 nM. 12 hours after being added to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipid". The experiments were all performed in triplicates.

**Conclusions:** The results showed that the above lipid combinations were all effective in delivering nucleic acids into cells as compared to the free uptake group (see Fig.16), having the potential of improving the efficiency of the delivery of nucleic acid drug in clinical settings. Nucleic acids that were mediated by the mixture 2, mixture 3, mixture 5, mixture 7, mixture 8 entered into MRC-5 cells in higher amounts..

### Example 1-2: Delivery of single-stranded nucleic acids into MRC-5 cell and Caco-2 cell by lipid combination

### 1. Experimental groups:

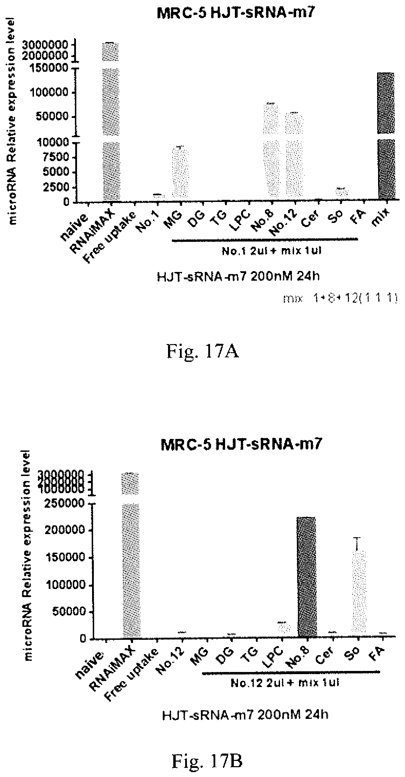

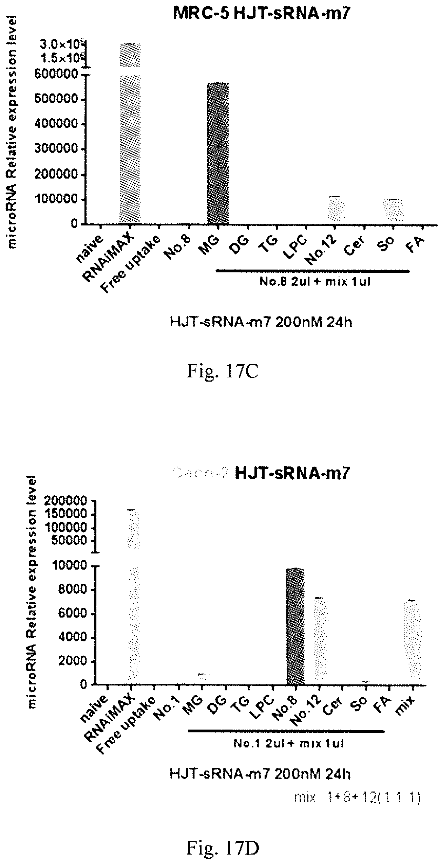

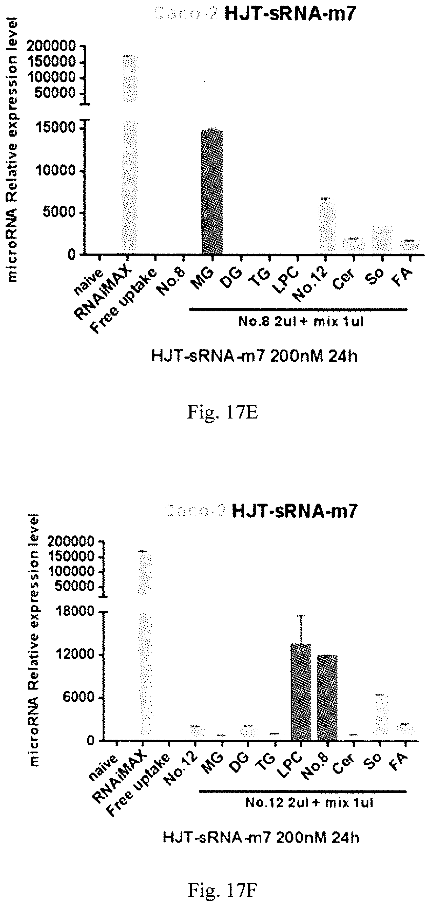

Cells to be tested were MRC-5 cell and Caco-2 cell.
1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and the two were mixed, allowed to stand for 15 min, added into cells, and then mixed. The final concentration of single-stranded HJT-sRNA-m7 was 200 nM;
3) Free uptake group: single-stranded HJT-sRNA-m7 solution was directly added (the final concentration was 200 nM);
4) Treatment group with single lipid and nucleic acid: a mixture of 3 µL single lipid (No.1 or 8 or 12) and the HJT-sRNA-m7 single-stranded nucleic acid solution that was treated by boiling method was added to the cells and mixed, and the final concentration of RNA was 200 nM;
5) Treatment group with lipid combination mixture and nucleic acid mixture: a mixture of 3 µL lipid combination (No. 1/8/12 mixed in equal volumes) and HJT-sRNA-m7 single-stranded nucleic acid solution treated by boiling method was added to the cells and mixed, and the final concentration of RNA was 200 nM;
6) Treatment group with lipid combination and nucleic acid mixture: a mixture of 3µL lipid combination (a mixture of 2 µL single lipid No.1 or No.8 or No. 12 and 1 µL of the following types of lipids (MG, DG, TG, LPC, Cer, So, or FA)) and HJT-sRNA-m7 single-stranded nucleic acid solution that were treated by boiling method was added to the cells and mixed, and the final concentration of RNA was 200 nM. In Figures 17A-F, the treatment groups were collectively represented as No.1 2 µL+mix 1 µL, No.8 2 µL+mix 1 µL, and No. 122 µL+mix 1 µL, wherein, within the horizontal line, MG represented 2 µL single lipid of No. 1 or No. 8 or No. 12+1 µL MG, DG represented 2µL single lipid of No. 1 or No. 8 or No. 12+1 µL DG, TG represented 2µL single lipid of No. 1 or No. 8 or No. 12+1 µL TG, LPC represented 2µL single lipid of No. 1 or No. 8 or No. 12+1 µL LPC, Cer represented 2µL single lipid of No. 1 or No. 8 or No. 12+1 µL Cer, So represented 2µL single lipid of No. 1 or No. 8 or No. 12+1 µL So, FA represented 2 µL single lipid of No. 1 or No. 8 or No. 12+1 µL FA.

### 2. Experimental procedures

1) Conditions of the boiling method: to 100 µL single-stranded HJT-sRNA-m7 solution was added 3 µL single lipid (lipid No. 1 in chloroform solution having a concentration of 5 mg/mL, lipids No. 8/12 in chloroform solution having a concentration of 10 mg/mL) or lipid combination, and heated at 100 °C for 30 min;
   MG (monoglyceride): 2 µL lipid No. 34;
   DG (diglyceride): 2 µL mixture of equal volume of lipids No. 1/2/3/19/35 in chloroform solution;
   TG (triglyceride): 2 µL mixture of equal volume of lipids No. 6/9/10/13/15/16/18/20/21/22/23/24/25/26/27/28/32/33 in chloroform solution;
   LPC (Lysophosphatidylcholine): 2 µL mixture of equal volume of lipids No. 36/37 in chloroform solution;
   Cer (Ceramides): 2 µL mixture of equal volume of lipids No. 4/14 in chloroform solution;
   So (Sphingoshine): 2 µL mixture of equal volume of lipids No. 17/30/31 in chloroform solution;
   FA (fatty acid): 2 µL lipid No. 29;
2) Experimental conditions: the final concentration of HJT-sRNA-m7 was 200 nM. 24 hours after being added to the cells, the amount of HJT-sRNA-m7 that entered into the cells was detected by RT-qPCR method (SYBR Green Universal dye method). For the protocols, see "Real-time quantitative PCR detection of intracellular expression of nucleic acids delivered by lipid". All experiments were performed in triplicates.

**Conclusion:** The results showed that for MRC-5 cells, the mixture (No. 1/8/12 mixed in equal volume), No. 1 2 µL+No. 8 1 µL, No. 1 2 µL+No. 12 1 µL, No.1 2 µL+MG 1 µL, No. 8 2 µL+MG 1 µL, No. 12 2 µL+No. 8 1 µL, and No. 122 µL+So 1 µL, delivered nucleic acid more efficiently.

For Caco-2 cells, the mixtures (No. 1/8/12 in equal volume), No.1 2 µL+No. 8 1 µL, No.1 2 µL+No. 12 1 µL, No.1 2 µL+MG 1 µL, No. 8 2 µL+MG 1 µL, No. 12 2 µL+No. 8 1 µL, No. 12 2 µL+EPC 1 µL and No. 122 µL+So 1 µL, delivered nucleic acid more efficiently.

### Example 1-3: Delivery of single-stranded nucleic acid into cell by lipid combination

Cell types: A549, MRC-5 and Caco-2 cells.

### 1. Experimental groups:

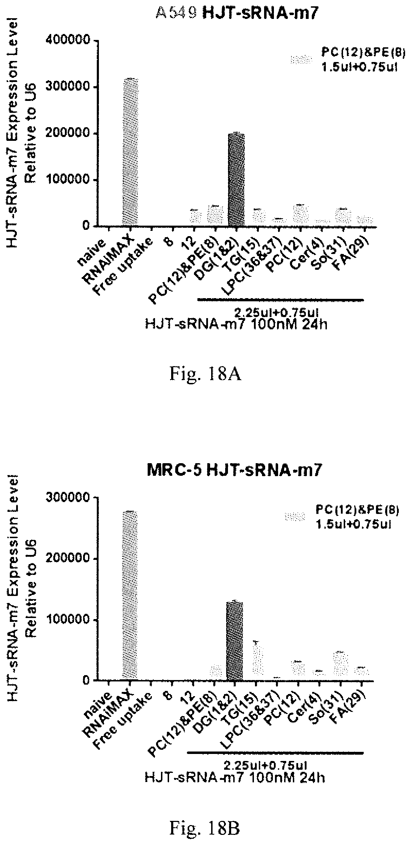

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group by single lipid and nucleic acid: a mixture of 3 µL single lipid (No. 8 or No. 12) and the HJT-sRNA-m7 single-stranded nucleic acid solution that was treated by boiling method was added to the cells and mixed, and the final concentration of RNA was 100 nM;
5) Treatment group by lipid combination PC (No. 12) & PE (No. 8) and nucleic acid mixture: a mixture of 2.25 µL lipid combination (PC (No. 12) & PE (No. 8), 2:1, V/V) and the HJT-sRNA-m7 single-stranded nucleic acid solution that was treated by boiling method was added to the cells and mixed, and the final concentration of RNA was 100 nM;
6) Treatment group by lipid combination and nucleic acid mixture: a mixture of 3 µL lipid combination (mixture of 2.25 µL lipid combination PC (No. 12) & PE (No. 8) and 0.75 µL of the following types of lipid, DG, TG, LPC, PC, Cer, So or FA) and the HJT-sRNA-m7 single-stranded nucleic acid solution that were treated by boiling method was added to the cells and mixed, and the final concentration of RNA was 100 nM. In Fig. 18A-C, the mixture treatment group corresponds to the treatment groups within the horizontal line above "2.25 µL+0.75 µL".

### 2. Experimental procedures

1) Boiling method conditions: to 100 µL single-stranded HJT-sRNA-m7 solution was added single lipid (lipids No. 8/12 in chloroform solution having a concentration of 10 mg/mL) or lipid combination, and heated at 100 °C for 30 min;
   DG (diglyceride): 0.75 µL mixture of equal volume of lipids No. 1/2 in chloroform solution;
   TG (triglyceride): 0.75 µL lipid No. 15 in chloroform solution;
   LPC (Lysophosphatidylcholine): 0.75 µL mixture of equal volume of lipids No. 36/37 in chloroform solution;
   PC (Lysophosphatidylcholine): 0.75 µL lipid No. 12 in chloroform solution;
   Cer (Ceramides): 0.75 µL lipid No. 4 in chloroform solution;
   So (Sphingoshine): 0.75 µL lipid No. 31 in chloroform solution;
   FA (fatty acid): 0.75 µL lipid No. 29;
2) Experimental conditions: the final concentration of HJT-sRNA-m7 was 100 nM. 24 hours after being added to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

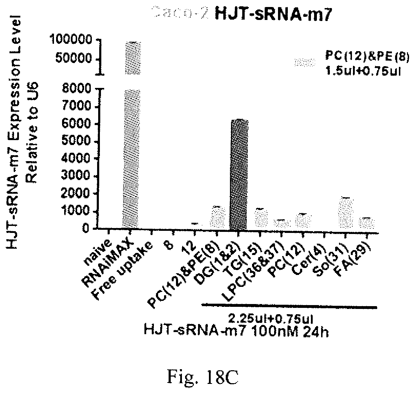

**Conclusions:** The results indicated that the above single lipids and lipid combinations were effective in delivering nucleic acids into cells as compared to the free uptake group (see Fig.18), having the potential of improving the efficiency of the delivery of nucleic acid drug in clinical settings.

For A549, MRC-5 and Caco-2 cells, 2.25 µL PC (No. 12) & PE (No. 8)+0.75 µL DG (mixture of equal volume of lipids No. 1/2 in chloroform solutions) achieved the best efficiency of delivery.

### Example 1-4: Delivery of single-stranded nucleic acid into cells by lipid combination

Cell types: A549, MRC-5 and Caco-2 cells.

### 1. Experimental groups:

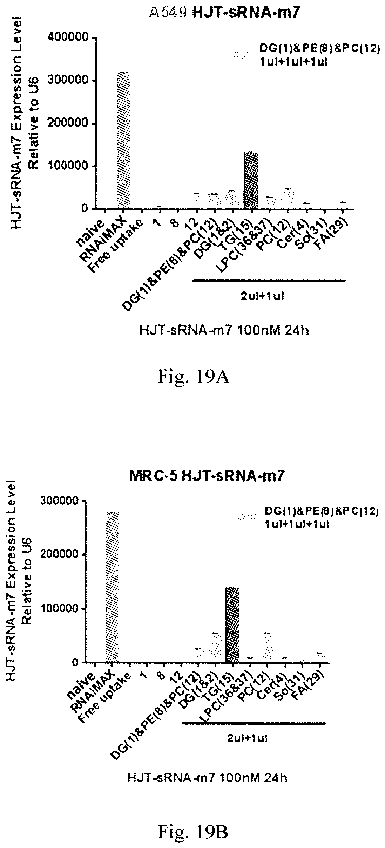

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of single lipid and nucleic acid: a mixture of 3 µL single lipid (No. 8 or No. 12) and the HJT-sRNA-m7 single-stranded nucleic acid solution that was treated by boiling method was added to the cells and mixed, and the final concentration of RNA was 100 nM;
5) Treatment group of lipid combination DG (No. 1) & PE (No. 8) & PC (No. 12) and nucleic acid mixture: a mixture of 3 µL lipid combination (DG (No. 1) & PE (No. 8) & PC (No. 12), 1:1:1, V/V/V) and the HJT-sRNA-m7 single-stranded nucleic acid solution that was treated by boiling method was added to the cells and mixed, and the final concentration of RNA was 100 nM;
6) Treatment group of lipid combination and nucleic acid mixture: a mixture of 3 µL lipid combination (mixture of 2 µL lipid combination DG (No. 1) & PE (No. 8) & PC (No. 12) and 1 µL of the following types of lipids, DG, TG, LPC, PC, Cer, So or FA) and the HJT-sRNA-m7 single-stranded nucleic acid solution that was treated by boiling method was added to the cells and mixed, and the final concentration of RNA was 100 nM. In Fig.19A-C, the mixture treatment groups correspond to the treatment groups within the horizontal line above 2 µL lipid combination DG (No. 1) & PE (No. 8) & PC (No. 12)) +1 µL.

### 2. Experimental procedures

1) Boiling method conditions: to 100 µL single-stranded HJT-sRNA-m7 solution was added 3 µL single lipid (lipid No. 1 in chloroform solution having a concentration of 5 mg/mL, lipids No. 8/12 in chloroform solution having a concentration of 10 mg/mL) or lipid combination, and heated at 100 °C for 30 min;
   DG (diglyceride): 1 µL mixture of equal volume of lipids No. 1/2 in chloroform solution;
   TG (triglyceride): 1 µL lipid No. 15 in chloroform solution;
   LPC (Lysophosphatidylcholine): 1 µL mixture of equal volume of lipids No. 36/37 in chloroform solution;
   PC (Lysophosphatidylcholine): 1 µL lipid No. 12 in chloroform solution;
   Cer (Ceramides): 1 µL lipid No. 4 in chloroform solution;
   So (Sphingoshine): 1 µL lipid No. 31 in chloroform solution;
   FA (fatty acid): 1 µL lipid No. 29;
2) Experimental conditions: the final concentration of HJT-sRNA-m7 was 100 nM. 24 hours after being added to the cells, the amount of HJT-sRNA-m7 was detected by RT-qPCR method (SYBR Green Universal dye method). For the protocols, see "Real-time quantitative PCR detection of intracellular expression of nucleic acids delivered by lipids". All experiments were performed in triplicates.

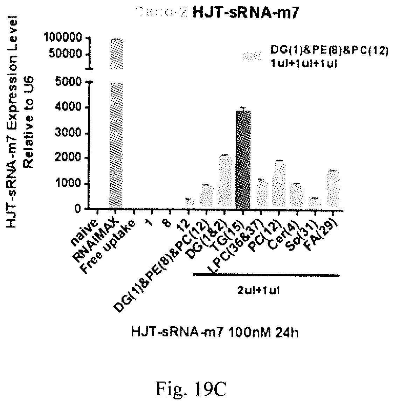

**Conclusions:** The results indicated that the above lipid combinations were effective in delivering nucleic acids into cells as compared to the free uptake group (see Fig. 19), having the potential of improving the efficiency of the delivery of nucleic acid drug in clinical settings.

For A549, MRC-5 and Caco-2 cells, 2 µL DG (No. 1) & PE (No. 8) & PC (No. 12)+1 µL TG (No. 15) achieved the best efficiency of delivery.

### Example 1-5: Delivery of single-stranded nucleic acid into cell by lipid combination

Cell types: A549, MRC-5 and Caco-2 cells.

### 1. Experimental groups:

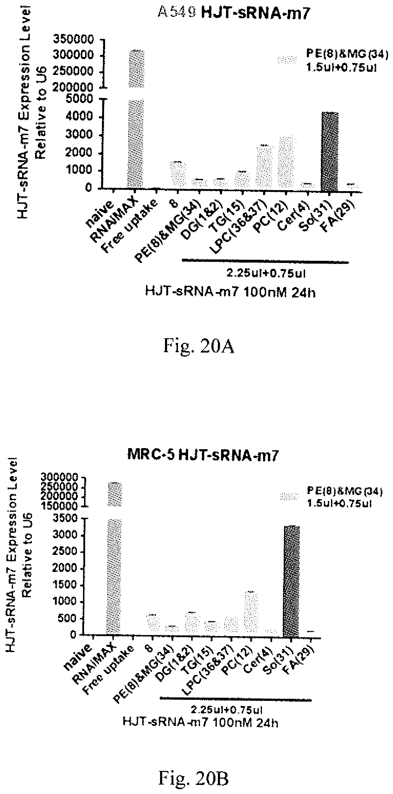

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of single lipid and nucleic acid: a mixture of 3 µL single lipid of No. 8 and the HJT-sRNA-m7 single-stranded nucleic acid solution that was treated by boiling method was added to the cells and mixed, and the final concentration of RNA was 100 nM;
5) Treatment group of lipid combination PE (No. 8) & MG (No. 34) and nucleic acid mixture: a mixture of 2.25 µL lipid combination (PE (No. 8) & MG (No. 34), 2: 1, V/V) and the HJT-sRNA-m7 single-stranded nucleic acid solution that was treated by boiling method was added to the cells and mixed, and the final concentration of RNA was 100 nM;
6) Treatment group of lipid combination and nucleic acid mixture: a mixture of 3 µL lipid combination (mixture of 2.25 µL lipid combination PE (No. 8) & MG (No. 34) and 0.75 µL of the following types of lipid, DG, TG, LPC, PC, Cer, So or FA) and the HJT-sRNA-m7 single-stranded nucleic acid solution that was treated by boiling method was added to the cells and mixed, and the final concentration of RNA was 100 nM. In Fig.20A-C, the mixture treatment group corresponds to the treatment groups within the horizontal line above "2.25 µL [lipid combination PE (No. 8) & MG (No. 34)]+0.75 µL".

### 2. Experimental procedures

1) Boiling method conditions: to 100 µL single-stranded HJT-sRNA-m7 solution was added single lipid (lipid No. 8 in chloroform solution having a concentration of 10 mg/mL) or lipid combination, and heated at 100 °C for 30 min;
   DG (diglyceride): 0.75 µL mixture of equal volume of lipids No. 1/2 in chloroform solution;
   TG (triglyceride): 0.75 µL lipid No. 15 in chloroform solution;
   LPC (Lysophosphatidylcholine): 0.75 µL mixture of equal volume of lipids No. 36/37 in chloroform solution;
   PC (Lysophosphatidylcholine): 0.75 µL lipid No. 12 in chloroform solution;
   Cer (Ceramides): 0.75 µL lipid No. 4 in chloroform solution;
   So (Sphingoshine): 0.75 µL lipid No. 31 in chloroform solution;
   FA (fatty acid): 0.75 µL lipid No. 29;
2) Experimental conditions: the final concentration of HJT-sRNA-m7 was 100 nM. 24 hours after being added to the cells, the amount of HJT-sRNA-m7 that entered in to cells was detected by RT-qPCR method (SYBR Green Universal dye method). For the protocols, see "Real-time quantitative PCR detection of intracellular expression of nucleic acids delivered by lipids". All experiments were performed in triplicates.

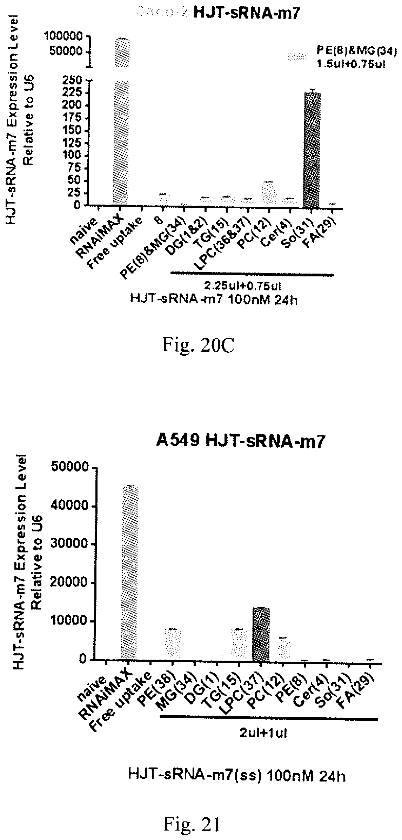

**Conclusions:** The results indicated that the above single lipid and lipid combinations were effective in delivering nucleic acids into cells as compared to the free uptake group (see Fig.20), having the potential of improving the efficiency of the delivery of nucleic acid drug in clinical settings.

For A549, MRC-5 and Caco-2 cells, 2.25 µL PE (No. 8) & MG (No. 34)+0.75 µL So (No. 31) achieved the best efficiency of delivery.

### Example 1-6: Delivery of single-stranded nucleic acid into A549 cells by lipid combination

### 1. Experimental groups:

1) Naive group: untreated A549 cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of single lipid and nucleic acid: a mixture of 3 µL single lipid No. 38 and the HJT-sRNA-m7 single-stranded nucleic acid solution that was treated by boiling method was added to the cell, and mixed, and the final concentration of RNA was 100 nM;
5) Treatment group of lipid combination and nucleic acid mixture: a mixture of 3 µL lipid combination (mixture of 2 µL single lipid No. 38 and 1 µL of the following types of lipid, MG, DG, TG, LPC, PC, PE, Cer, So or FA) and the HJT-sRNA-m7 single-stranded nucleic acid solution that was treated by boiling method was added to the cells and mixed, and the final concentration of RNA was 100 nM;

### 2. Experimental procedures

1) Boiling method conditions: to 100 µL single-stranded HJT-sRNA-m7 solution was added 3 µL single lipid (lipid No. 38 in chloroform solution having a concentration of 10 mg/mL) or lipid combination, and heated at 100 °C for 30 min;
   MG (monoglyceride): 1 µL lipid No. 34;
   DG (diglyceride): 1 µL lipid No. 1 in chloroform solution;
   TG (triglyceride): 1 µL lipid No. 15 in chloroform solution;
   LPC (Lysophosphatidylcholine): 1 µL lipid No. 37 in chloroform solution;
   PC (Lysophosphatidylcholine): 1 µL lipid No. 12 in chloroform solution;
   PE (phosphatidylethanolamine): 1 µL lipid No. 8 in chloroform solution;
   Cer (Ceramides): 1 µL lipid No. 4 in chloroform solution;
   So (Sphingoshine): 1 µL lipid No. 31 in chloroform solution;
   FA (fatty acid): 1 µL lipid No. 29 in chloroform solution;
2) Experimental conditions: the final concentration of HJT-sRNA-m7 was 100 nM. 24 hours after being added to the cell, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

Conclusions: The results indicated that for A549 cells, the above 2 µL single lipid No. 38 and 1 µL LPC (No. 37), TG (No. 15), PC (No. 12), DG (No. 1) were effective in delivering nucleic acids into cells as compared to the free uptake group (see Fig.21).

### Example 1-7: Delivery of single-stranded nucleic acid into A549 cells by lipid combination

### 1. Experimental groups:

1) Naive group: untreated A549 cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination DG (No. 1) & PE (No. 38) & PC (No. 12) and nucleic acid mixture: a mixture of 3 µL lipid combination (DG (No. 1) & PE (No. 38) & PC (No. 12), 1:1:1, V/V/V) and the HJT-sRNA-m7 single-stranded nucleic acid solution that was treated by boiling method was added to the cells and mixed, and the final concentration of RNA was 100 nM;
5) Treatment group of lipid combination and nucleic acid mixture: a mixture of 3 µL lipid combination (mixture of 2 µL lipid combination DG (No. 1) & PE (No. 38) & PC (No. 12) and 1 µL of the following types of lipid, MG, TG, LPC, PE, Cer, So or FA) and the HJT-sRNA-m7 single-stranded nucleic acid solution that was treated by boiling method was added to the cells and mixed, and the final concentration of RNA was 100 nM.

### 2. Experimental procedures

1) Boiling method conditions: to 100 µL single-stranded HJT-sRNA-m7 solution was added 3 µL lipid combination, and heated at 100 °C for 30 min;
   MG (monoglyceride): 1 µL lipid No. 34;
   TG (triglyceride): 1 µL lipid No. 15 in chloroform solution;
   LPC (Lysophosphatidylcholine): 1 µL lipid No. 37 in chloroform solution;
   PE (phosphatidylethanolamine): 1 µL lipid No. 8 in chloroform solution;
   Cer (Ceramides): 1 µL lipid No. 4 in chloroform solution;
   So (Sphingoshine): 1 µL lipid No. 31 in chloroform solution;
   FA (fatty acid): 1 µL lipid No. 29 in chloroform solution;
2) Experimental conditions: the final concentration of HJT-sRNA-m7 was 100 nM. 24 hours after being added to the cells, the amount of HJT-sRNA-m7 was detected by RT-qPCR method (SYBR Green Universal dye method). For the protocols, see "Real-time quantitative PCR detection of intracellular expression of nucleic acids delivered by lipids". All experiments were performed in triplicates.

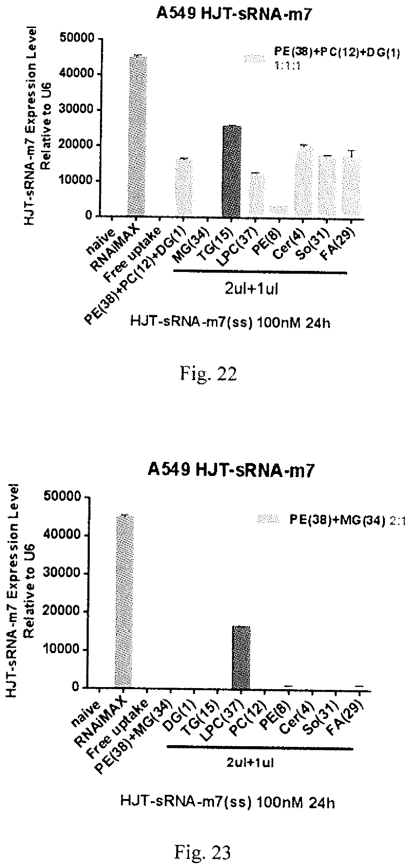

**Conclusions:** The results indicated that the above 2 µL lipid combination DG (No. 1) & PE (No. 38) & PC (No. 12) and 1 µL TG (No. 15), Cer (No. 4), So (No. 31), FA (No. 29), LPC (No. 37), PE (No. 8) were all effective in delivering nucleic acids into A549 cells as compared to the free uptake group (see Fig.22), having the potential of improving the efficiency of the delivery of nucleic acid drug in clinical settings.

### Example 1-8: Delivery of single-stranded nucleic acid into A549 cells by lipid combination

### 1. Experimental groups:

1) Naive group: untreated A549 cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & MG (No. 34) and nucleic acid mixture: a mixture of 3 µL lipid combination (PE (No. 38) & MG (No. 34), 2: 1, V/V) and the HJT-sRNA-m7 single-stranded nucleic acid solution that was treated by boiling method was added to the cells and mixed, and the final concentration of RNA was 100 nM;
5) Treatment group of lipid combination and nucleic acid mixture: a mixture of 3 µL lipid combination (mixture of 2 µL lipid combination PE (No. 38) & MG (No. 34) and 1 µL of the following types of lipid, DG, TG, LPC, PC, PE, Cer, So or FA) and the HJT-sRNA-m7 single-stranded nucleic acid solution that was treated by boiling method was added to the cells and mixed, and the final concentration of RNA was 100 nM;

### 2. Experimental procedures

1) Boiling method conditions: to 100 µL single-stranded HJT-sRNA-m7 solution was added 3 µL lipid combination, and heated at 100 °C for 30 min;
   DG (diglyceride): 1 µL lipid No. 1 in chloroform solution;
   TG (triglyceride): 1 µL lipid No. 15 in chloroform solution;
   LPC (Lysophosphatidylcholine): 1 µL lipid No. 37 in chloroform solution;
   PC (phosphatidylcholine): µL lipid No. 12 in chloroform solution;
   PE (phosphatidylethanolamine): 1 µL lipid No. 8 in chloroform solution;
   Cer (Ceramides): 1 µL lipid No. 4 in chloroform solution;
   So (Sphingoshine): 1 µL lipid No. 31 in chloroform solution;
   FA (fatty acid): 1 µL lipid No. 29 in chloroform solution;
2) Experimental conditions: the final concentration of HJT-sRNA-m7 was 100 nM. 24 hours after being added to the cells, the amount of HJT-sRNA-m7 was detected by RT-qPCR method (SYBR Green Universal dye method). For the protocols, see "Real-time quantitative PCR detection of intracellular expression of nucleic acids delivered by lipids". All experiments were performed in triplicates.

**Conclusions:** The results indicated that the above lipid combinations were all effective in delivering nucleic acids into cells (see Fig.23), having the potential of improving the efficiency of the delivery of nucleic acid drug in clinical settings, wherein 2 µL lipid combination PE (No. 38) & MG (No. 34) and 1 µL LPC (No. 37) achieved the best delivery effect.

### Example 1-9: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & PC (No. 12) and nucleic acid mixture: a mixture of 3 µL lipid combination (PE (No. 38) & PC (No. 12), 2: 1, V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2 µL lipid combination PE (No. 38) & PC (No. 12) and 1 µL of the following types of lipid, MG, DG, TG, LPC, PE, Cer, So or FA) and the HJT-sRNA-m7 single-stranded nucleic acid solution that was treated by boiling method was added to the cells, and mixed, and the final concentration of RNA was 100 nM;

### 2. Experimental procedures

1) Boiling method conditions: to 100 µL single-stranded HJT-sRNA-m7 solution was added 3 µL lipid combination, and heated at 100 °C for 30 min;
   MG (monoglyceride): 1 µL lipid No. 34;
   DG (diglyceride): 1 µL lipid No. 1 in chloroform solution;
   TG (triglyceride): 1 µL lipid No. 15 in chloroform solution;
   LPC (Lysophosphatidylcholine): 1 µL lipid No. 37 in chloroform solution;
   PE (phosphatidylethanolamine): 1 µL lipid No. 8 in chloroform solution;
   Cer (Ceramides): 1 µL lipid No. 4 in chloroform solution;
   So (Sphingoshine): 1 µL lipid No. 31 in chloroform solution;
   FA (fatty acid): 1 µL lipid No. 29;
2) Experimental conditions: the final concentration of HJT-sRNA-m7 was 100 nM, 24 hours after being added to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

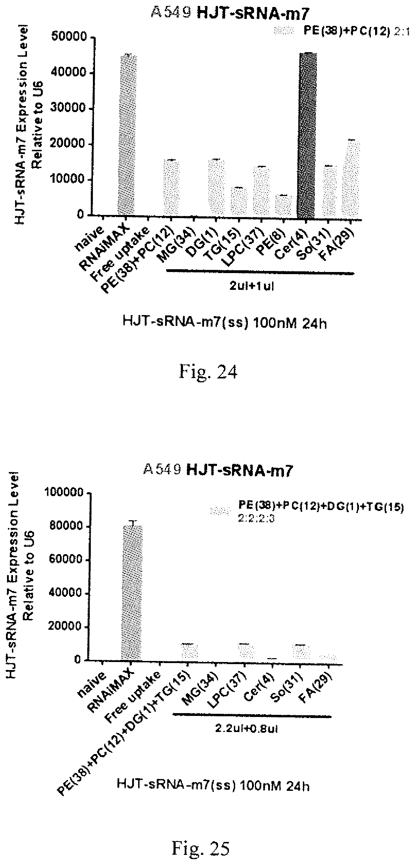

**Conclusions:** The results indicated that the above lipid combinations were effective in delivering nucleic acids into cells (see Fig.24), having the potential of improving the efficiency of the delivery of nucleic acid drug in clinical settings, wherein 2 µL lipid combination PE (No. 38) & PC (No. 12) and 1 µL Cer (No. 4) achieved the best effect.

### Example 1-10: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & PC (No. 12) & DG (No. 1) & TG (No. 15) and nucleic acid mixture: a mixture of 3 µL lipid combination (PE (No. 38) & PC (No. 12) & DG (No. 1) & TG (No. 15), 2:2:2:3, V/V/V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2.2 µL lipid combination PE (No. 38) & PC (No. 12) & DG (No. 1) & TG (No. 15) and 0.8 µL of the following types of lipid, MG, LPC, Cer, So or FA) and the HJT-sRNA-m7 single-stranded nucleic acid solution that was treated by boiling method was added to the cells, and mixed, and the final concentration of RNA was 100 nM.

### 2. Experimental procedures

1) Boiling method conditions: to 100 µL single-stranded HJT-sRNA-m7 solution was added 3 µL lipid combination, and heated at 100 °C for 30 min;
   MG (monoglyceride): 0.8 µL lipid No. 34;
   LPC (Lysophosphatidylcholine): 0.8 µL lipid No. 37 in chloroform solution;
   Cer (Ceramides): 0.8 µL lipid No. 4 in chloroform solution;
   So (Sphingoshine): 0.8 µL lipid No. 31 in chloroform solution;
   FA (fatty acid): 0.8 µL lipid No. 29;
2) Experimental conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

**Conclusions:** The results indicated that the above lipid combinations were effective in delivering nucleic acids into cells (see Fig.25), wherein 2.2 µL lipid combination PE (No. 38) & PC (No. 12) & DG (No. 1) & TG (No. 15), 2.2 µL lipid combination PE (No. 38) & PC (No. 12) & DG (No. 1) & TG (No. 15) and 0.8 µL LPC (No. 37) or So (No. 31) achieved relative better efficiency of delivery.

### Example 1-11: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & MG (No. 34) & LPC (No. 37) and nucleic acid mixture: a mixture of 3 µL lipid combination (PE (No. 38) & MG (No. 34) & LPC (No. 37), 4:2:3, V/V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2.2 µL lipid combination PE (No. 38) & MG (No. 34) & LPC (No. 37) and 0.8 µL of the following types of lipid, DG, TG, PC, Cer, or So) and the HJT-sRNA-m7 single-stranded nucleic acid solution that was treated by boiling method was added to the cells, and mixed, and the final concentration of RNA was 100 nM;

### 2. Experimental procedures

1) Boiling method conditions: to 100 µL single-stranded HJT-sRNA-m7 solution was added 3 µL lipid combination, and heated at 100 °C for 30 min;
   DG (diglyceride): 0.8 µL lipid No. 1 in chloroform solution;
   TG (triglyceride): 0.8 µL lipid No. 15 in chloroform solution;
   PC (phosphatidylcholine): 0.8 µL lipid No. 12 in chloroform solution;
   Cer (Ceramides): 0.8 µL lipid No. 4 in chloroform solution;
   So (Sphingoshine): 0.8 µL lipid No. 31 in chloroform solution;
2) Experimental conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

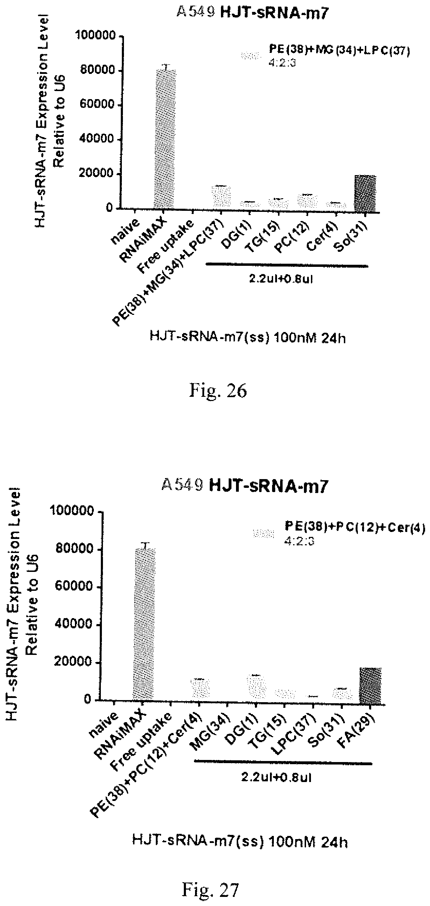

**Conclusions:** The results indicated that the above lipid combinations were effective in delivering nucleic acids into cells (see Fig.26), having the potential of improving the efficiency of the delivery of nucleic acid drug in clinical settings.

### Example 1-12: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & PC (No. 12) & Cer (No. 4) and nucleic acid mixture: a mixture of 3 µL lipid combination (PE (No. 38) & PC (No. 12) & Cer (No. 4), 4:2:3, V/V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2.2 µL lipid combination PE (No. 38) & PC (No. 12) & Cer (No. 4) and 0.8 µL of the following types of lipid, MG, DG, TG, LPC, So or FA) and the HJT-sRNA-m7 single-stranded nucleic acid solution that was treated by boiling method was added to the cells, and mixed, and the final concentration of RNA was 100 nM.

### 2. Experimental procedures

1) Boiling method conditions: to 100 µL single-stranded HJT-sRNA-m7 solution was added 3 µL lipid combination, and heated at 100 °C for 30 min;
   MG (monoglyceride): 0.8 µL lipid No. 34;
   DG (diglyceride): 0.8 µL lipid No. 1 in chloroform solution;
   TG (triglyceride): 0.8 µL lipid No. 15 in chloroform solution;
   LPC (lysophosphatidylcholine): 0.8 µL lipid No. 37 in chloroform solution;
   So (Sphingoshine): 0.8 µL lipid No. 31 in chloroform solution;
   FA (fatty acid): 0.8 µL lipid No. 29 in chloroform solution;
2) Experimental conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

**Conclusions:** The results indicated that the above lipid combinations were effective in delivering nucleic acids into cells (see Fig.27), wherein 2.2 µL lipid combination PE (No. 38) & PC (No. 12) & Cer (No. 4) and 0.8 µL FA (No. 29) achieved the best efficiency of delivery.

### Example 1-13: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & PC (No. 12) & Cer (No. 4) & FA (No. 29) and nucleic acid mixture: a mixture of 3 µL lipid combination (PE (No. 38) & PC (No. 12) & Cer (No. 4) & FA (No. 29), 44:22:33:36, V/V/V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of PE (No. 38) & PC (No. 12) & Cer (No. 4) & FA (No. 29) and 1 µL of the following types of lipid) and the HJT-sRNA-m7 single-stranded nucleic acid solution that was treated by boiling method was added to the cells, and mixed, and the final concentration of RNA was 100 nM.

### 2. Experimental procedures

1) Boiling method conditions: to 100 µL single-stranded HJT-sRNA-m7 solution was added 3 µL lipid combination, and heated at 100 °C for 30 min;
   MG (monoglyceride): 1 µL lipid No. 34;
   DG (diglyceride): 1 µL lipid No. 1 in chloroform solution;
   TG (triglyceride): 1 µL lipid No. 15 in chloroform solution;
   LPC (lysophosphatidylcholine): 1 µL lipid No. 37 in chloroform solution;
   So (Sphingoshine): 1 µL lipid No. 31 in chloroform solution;
2) Experimental conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

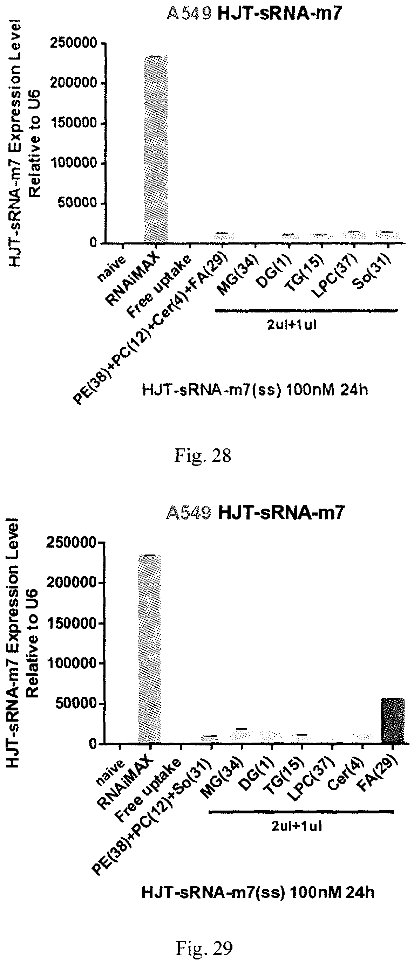

**Conclusions:** The results indicated that the above lipid combinations were effective in delivering nucleic acids into cells (see Fig.28), having the potential of improving the efficiency of the delivery of nucleic acid drug in clinical settings.

### Example 1-14: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & PC (No. 12) & So (No. 31) and nucleic acid mixture: a mixture of 3 µL lipid combination (PE (No. 38) & PC (No. 12) & So (No. 31), 2:1:3, V/V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2 µL PE (No. 38) & PC (No. 12) & So (No. 31) and 1 µL of the following types of lipid, MG, DG, TG, LPC, Cer or FA) and the HJT-sRNA-m7 single-stranded nucleic acid solution that was treated by boiling method was added to the cells, and mixed, and the final concentration of RNA was 100 nM.

### 2. Experimental procedures

1) Boiling method conditions: to 100 µL Single-stranded HJT-sRNA-m7 solution was added 3 µL lipid combination, and heated at 100 °C for 30 min;
   DG (diglyceride): 1 µL lipid No. 1 in chloroform solution;
   TG (triglyceride): 1 µL lipid No. 15 in chloroform solution;
   PC (phosphatidylcholine): 1 µL lipid No. 12 in chloroform solution;
   Cer (Ceramides): 1 µL lipid No. 4 in chloroform solution;
   FA (fatty acid): 1 µL lipid No. 29 in chloroform solution;
2) Experimental conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

**Conclusions:** The results indicated that the above lipid combinations were effective in delivering nucleic acids into cells (see Fig.29), wherein 2 µL lipid combination PE (No. 38) & PC (No. 12) & So (No. 31) and 1 µL FA (No. 29) achieved the best delivery effect.

### Example 1-15: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & MG (No. 34) & LPC (No. 37) & So (No. 31) and nucleic acid mixture: a mixture of 3 µL lipid combination (PE (No. 38) & MG (No. 34) & LPC (No. 37) & So (No. 31), 44:22:33:36, V/V/V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2 µL PE (No. 38) & MG (No. 34) & LPC (No. 37) & So (No. 31) and 1 µL of the following types of lipid, DG, TG, PC, Cer or FA) and the HJT-sRNA-m7 single-stranded nucleic acid solution that was treated by boiling method was added to the cells, and mixed, and the final concentration of RNA was 100 nM.

### 2. Experimental procedures

1) Boiling method conditions: to 100 µL single-stranded HJT-sRNA-m7 solution was added 3 µL lipid combination, and heated at 100 °C for 30 min;
   DG (diglyceride): 1 µL lipid No. 1 in chloroform solution;
   TG (triglyceride): 1 µL lipid No. 15 in chloroform solution;
   PC (phosphatidylcholine): 1 µL lipid No. 12 in chloroform solution;
   Cer (Ceramides): 1 µL lipid No. 4 in chloroform solution;
   FA (fatty acid): 1 µL lipid No. 29 in chloroform solution;
2) Experiment conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

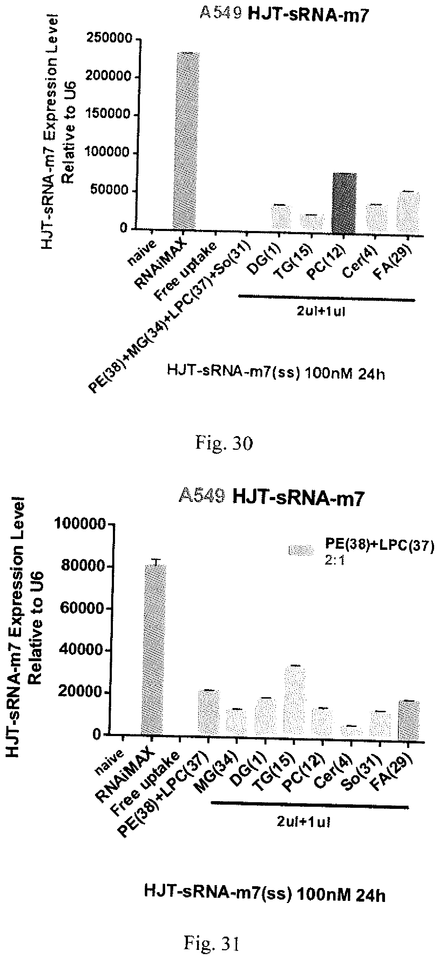

**Conclusions:** The results indicated that as compared to the free uptake group, the addition of 1 µL DG (No. 1), TG (No. 15), PC (No. 12), Cer (No. 4) or FA (No. 29) to 2 µLPE (No. 38) & MG (No. 34) & LPC (No. 37) & So (No. 31), could efficiently deliver nucleic acids into cells (see Fig.30), having the potential of improving the efficiency of the delivery of nucleic acid drug in clinical settings. The addition of 1 µL PC (No. 12) achieved the best efficiency in nucleic acid delivery and could enhance the efficiency of delivery.

### Example 1-16: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & LPC (No. 37) and nucleic acid mixture: a mixture of 3 µL lipid combination (PE (No. 38) & LPC (No. 37), 2:1, V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2 µL PE (No. 38) & LPC (No. 37) and 1 µL of the following types of lipid, MG, DG, TG, PC, Cer, So or FA) and the HJT-sRNA-m7 single-stranded nucleic acid solution that was treated by boiling method was added to the cells, and mixed, and the final concentration of RNA was 100 nM;

### 2. Experimental procedures

1) Boiling method conditions: to 100 µL single-stranded HJT-sRNA-m7 solution was added 3 µL lipid combination, and heated at 100 °C for 30 min;
   MG (monoglyceride): 1 µL lipid No. 34;
   DG (diglyceride): 1 µL lipid No. 1 in chloroform solution;
   TG (triglyceride): 1 µL lipid No. 15 in chloroform solution;
   PC (phosphatidylcholine): 1 µL lipid No. 12 in chloroform solution;
   Cer (Ceramides): 1 µL lipid No. 4 in chloroform solution;
   So (Sphingoshine): 1 µL lipid No. 31 in chloroform solution;
   FA (fatty acid): 1 µL lipid No. 29 in chloroform solution;
2) Experiment conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

**Conclusions:** The results indicated that as compared to the free uptake group, the above lipid combinations were effective in delivering nucleic acids into cells (see Fig.31), having the potential of improving the efficiency of the delivery of nucleic acid drug in clinical settings. The addition of 1 µL TG (No. 15) to 2 µL lipid combination PE (No. 38) & LPC (No. 37) achieved the best effect in nucleic acid delivery.

### Example 1-17: Delivery of single-stranded nucleic acid into A549 cells by lipid combination

### 1. Experimental groups:

1) Naive group: untreated cell;
2) RNAiMAX treatment group: 2 µl RNAiMAX transfection reagent and single-stranded HJT-sRNA-m7 solution were diluted in 100 µl opti-MEM medium, respectively, and then the two were mixed, allowed to stand for 15 min, added into cells, and then mixed. The final concentration of single-stranded HJT-sRNA-m7 was 100 nM;
3) Free uptake group: single-stranded HJT-sRNA-m7 solution was directly added (the final concentration was 100 nM);
4) Treatment group of lipid combination PE (No. 38) & LPC (No. 37) & TG (No. 15) and nucleic acid mixture: a mixture of 3 µL lipid combination (PE (No. 38) & LPC (No. 37) & TG(No. 15), 32:8:5, V/V/V) and the HJT-sRNA-m7 single-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2 µL PE (No. 38) & LPC (No. 37) & TG (No. 15) and 1 µL of the following types of lipid, MG, DG, PC, Cer, So or FA) and the HJT-sRNA-m7 single-stranded nucleic acid solution that was treated by boiling method was added to the cells, and mixed, and the final concentration of RNA was 100 nM;

### 2. Experimental procedures

1) Boiling method conditions: to 100 µL single-stranded HJT-sRNA-m7 solution was added 3 µL lipid combination, and heated at 100 °C for 30 min;
   MG (monoglyceride): 1 µL lipid No. 34;
   DG (diglyceride): 1 µL lipid No. 1 in chloroform solution;
   PC (phosphatidylcholine): 1 µL lipid No. 12 in chloroform solution;
   Cer (Ceramides): 1 µL lipid No. 4 in chloroform solution;
   So (Sphingoshine): 1 µL lipid No. 31 in chloroform solution;
   FA (fatty acid): 1 µL lipid No. 29 in chloroform solution;
2) Experiment conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

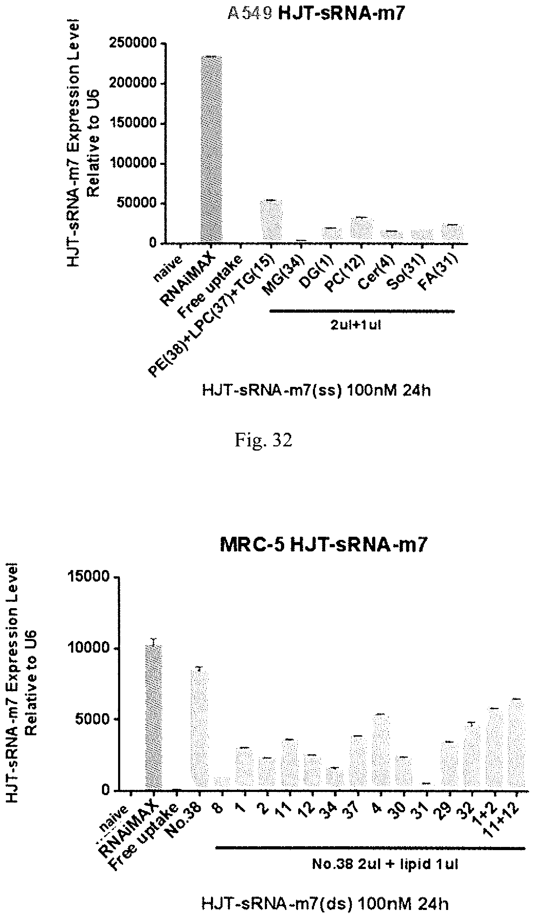

**Conclusions:** The results indicated that the above lipid combinations were effective in delivering nucleic acids into cells (see Fig.32), having the potential of improving the efficiency of the delivery of nucleic acid drug in clinical settings. The lipid combination PE (No. 38) & LPC (No. 37) & TG (No. 15) efficiently delivered nucleic acids into cells. Further addition of other types of lipid to the lipid combination PE (No. 38) & LPC (No. 37) & TG (No. 15) did not enhance this effect.

### Example 2-1: Delivery of double-stranded nucleic acid into MRC-5 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) Treatment group of single lipid and nucleic acid: a mixture of 3 µL single lipid No. 38 and the HJT-sRNA-m7 double-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2 µL single lipid No. 38 and 1 µL lipid No. 8, 1, 2, 11, 12, 34, 37, 4, 30, 31, 29, 32, 1+2 (mixed in equal volume) or 11+12 (mixed in equal volume) in chloroform solution) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 3 µL single lipid (lipid No. 38 in chloroform solution having a concentration of 10 mg/mL) or lipid combination, and heated at 100 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

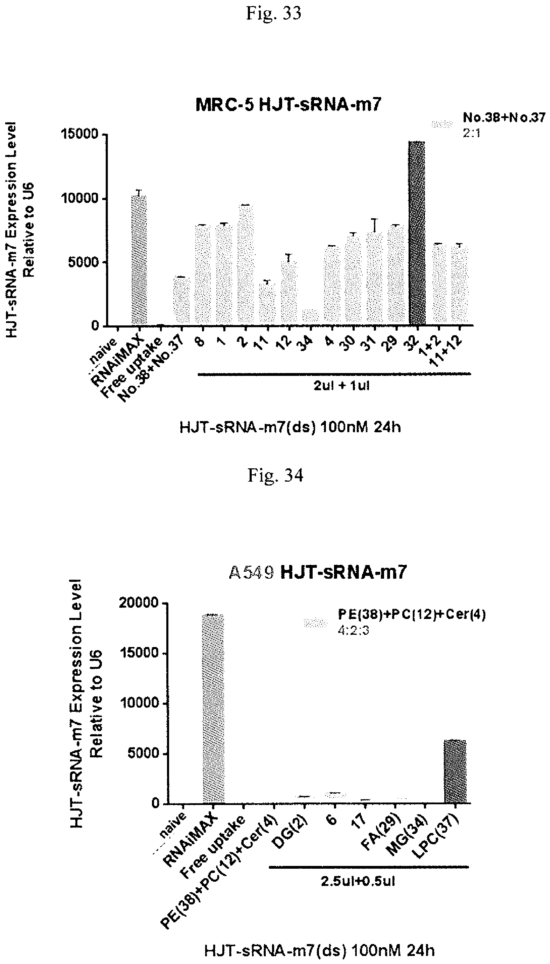

**Conclusions:** The results indicated that the above single lipids and lipid combinations were effective in delivering nucleic acids into cells (see Fig.33), having the potential of improving the efficiency of the delivery of nucleic acid drug in clinical settings. The single lipid No. 38 effectively delivered nucleic acids into MRC-5 cells, showing an effect close to the transfection reagent RNAiMAX. The addition of other lipids to it did not further enhance the effect.

### Example 2-2: Delivery of double-stranded nucleic acid into MRC-5 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) Treatment group of lipid combination (No. 38 & No. 37, 2: 1, V/V) and nucleic acid: a mixture of 3 µL lipid combination and the HJT-sRNA-m7 double-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2 µL lipid combination No. 38 & No. 37 and 1 µL lipid No. 8, 1, 2, 11, 12, 34, 37, 4, 30, 31, 29, 32, 1+2 (mixed in equal volume) or 11+12 (mixed in equal volume) in chloroform solution) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added to 3 µL lipid combination, and heated at 100 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

**Conclusions:** The results indicated that the above single lipids and lipid combinations were effective in delivering nucleic acids into cells (see Fig.34), having the potential of improving the efficiency of the delivery of nucleic acid drug in clinical settings. Lipid No. 38 & No. 37 mixture efficiently delivered nucleic acids into MRC-5 cells. To addition of 1 µL lipids, except No. 11 and 34, to 2 µL No. 38 & No. 37 mixture could enhance this effect. In addition, unexpectedly, the addition of 1 µL lipid No. 32 to 2 µL No. 38 & No37 mixture achieved the best effect, even better than the effect of RNAiMAX.

### Example 2-3: Delivery of double-stranded nucleic acid into A549 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) Treatment group of lipid combination (PE (No. 38) & PC (No. 12) & Cer (No. 4)) and nucleic acid: a mixture of 3 µL lipid combination (PE (No. 38) & PC (No. 12) & Cer (No. 4), 4:2:3, V/V/V) and the HJT-sRNA-m7 double-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2.5 µL PE (No. 38) & PC (No. 12) & Cer (No. 4) and 0.5 µL lipids (DG (No. 2), TG (No. 6), So (No. 17), FA (No. 29), MG (No. 34) and LPC (No. 37)) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL HJT-sRNA-m7 double-stranded solution was added 3 µL lipid combination, and heated at 100 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

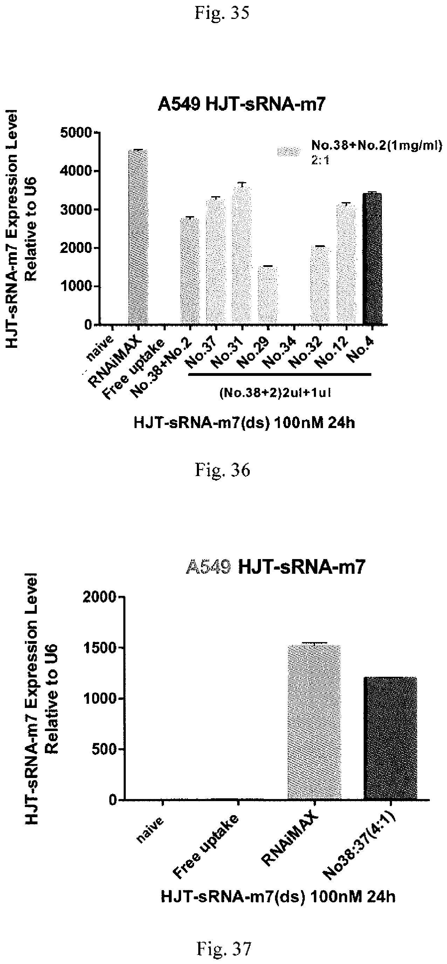

**Conclusions:** The results indicated that the above single lipids and lipid combinations were effective in delivering nucleic acids into cells (see Fig.35), having the potential of improving the efficiency of the delivery of nucleic acid drug in clinical settings. The addition of 1/5 LPC (No. 37) to PE (No. 38) & PC (No. 12) & Cer (No. 4) mixture could significantly enhance the effect in delivery of the nucleic acid. In addition, the addition of DG (No. 2) and TG (No. 16) could also further enhance the effect in delivery.

### Example 2-4: Delivery of double-stranded nucleic acid into A549 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) Treatment group of lipid combination (PE (No. 38) & DG (No. 2)) and nucleic acid: a mixture of 3 µL lipid combination (PE (No. 38) & DG (No. 2), 2:1, V/V) and the HJT-sRNA-m7 double-stranded nucleic acid solution that was treated by boiling method was added to the cells, and mixed, and the final concentration of RNA was 100 nM;
5) Treatment group of lipid combination and nucleic acid mixture: a mixture of 3 µL lipid combination (mixture of 2 µL PE (No. 38) & DG (No. 2) mixture and 1 µL other lipid of No. 37, 31, 29, 34, 12 or 4) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 3 µL lipid combination, and heated at 100 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method (SYBR Green Universal dye method). For the protocols, see "Real-time quantitative PCR detection of intracellular expression of nucleic acids delivered by lipids". All experiments were performed in triplicates.

**Conclusions:** The results indicated that the above single lipids and lipid combinations were effective in delivering nucleic acids into cells (see Fig.36), having the potential of improving the efficiency of the delivery of nucleic acid drug in clinical settings. Lipid combination (2 µL PE (No. 38) & DG (No. 2) mixture) could effectively deliver the double stranded nucleic acid into the A549 cells. As compared with this lipid combination, the lipid combination of 2µL PE (No. 38) & DG (No. 2) and No. 37, 31, 12 or 4 mixed at a ratio of 2:1 could enhance the efficiency of delivery.

### Example 2-5: Delivery of double-stranded nucleic acid into A549 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination (PE (No. 38) & LPC (No. 37), 4:1, V/V) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 3 µL lipid combination, and heated at 70 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

**Conclusions:** The results indicated that the above lipid combinations were effective in delivering nucleic acids into cells (see Fig.37), having the potential of improving the efficiency of the delivery of nucleic acid drug in clinical settings, with an effect close to the transfection reagent RNAiMAX.

### Example 2-6: Delivery of double-stranded nucleic acid into A549 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination (PE (No. 38) & PC (No. 12), 4:1, V/V) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 3 µL lipid combination, and heated at 70 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

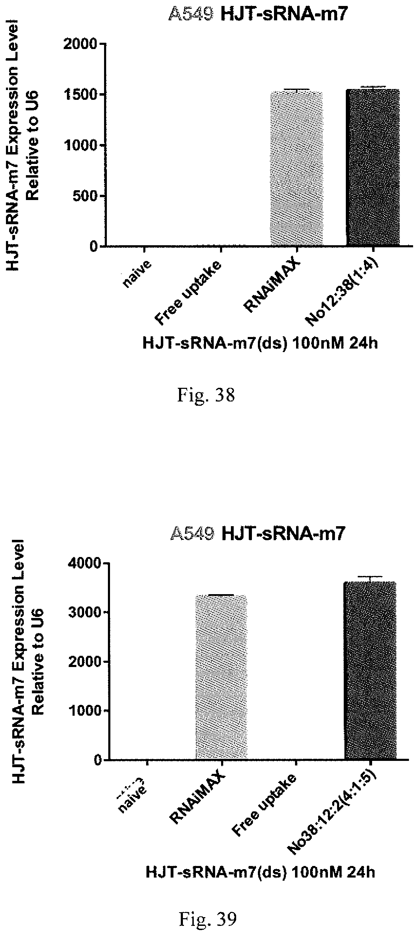

**Conclusions:** The results indicated that the above lipid combinations were effective in delivering nucleic acids into cells (see Fig.38), having the potential of improving the efficiency of the delivery of nucleic acid drug in clinical settings. The effect is better than or the same as that of RNAiMAX.

### Example 2-7: Delivery of double-stranded nucleic acid into A549 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination (PE (No. 38) & PC (No. 12) & DG (No. 2), 4:1:5, V/V/V) and the double-stranded HJT-sRNA-m7 nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 2 µL lipid combination, and heated at 80 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

**Conclusions:** The results indicated that the above lipid combinations were effective in delivering nucleic acids into cells (see Fig.39), having the potential of improving the efficiency of the delivery of nucleic acid drug in clinical settings. Lipid combination (PE (No. 38) & PC (No. 12) & DG (No. 2), 4:1:5, V/V/V) showed better effect in the delivery of double-stranded nucleic acid into A549 cells than RNAiMAX.

### Example 2-8: Delivery of double-stranded nucleic acid into A549 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination (PE (No. 38) & LPC (No. 37) & DG (No. 2), 32:8:5, V/V/V) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 2 µL lipid combination, and heated at 80 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

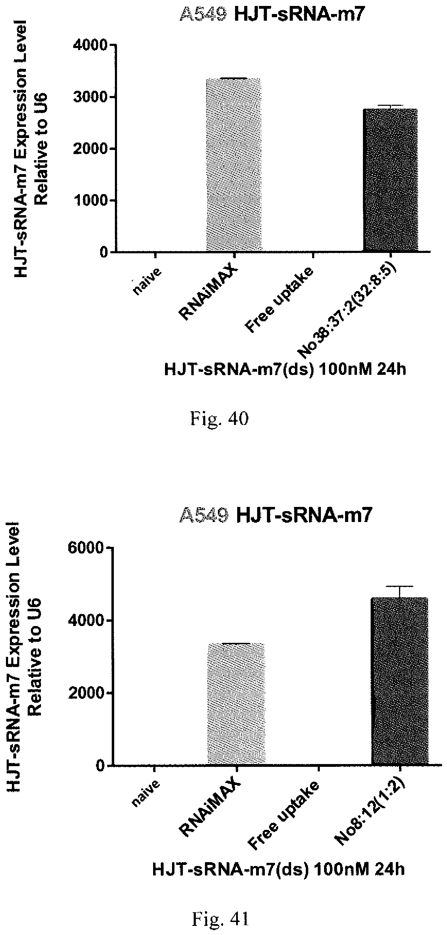

**Conclusions:** The results indicated that the above lipid combinations were effective in delivering nucleic acids into cells (see Fig.40), having the potential of improving the efficiency of the delivery of nucleic acid drug in clinical settings. The effect was similar to that of RNAiMAX.

### Example 2-9: Delivery of double-stranded nucleic acid into A549 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination (PE (No. 8) & PC (No. 12), 1:2, V/V) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added to 2 µL lipid combination, and heated at 80 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

**Conclusions:** The results indicated that the above lipid combinations were effective in delivering nucleic acids into cells (see Fig.41), having the potential of improving the efficiency of the delivery of nucleic acid drug in clinical settings. Lipid combination (PE (No. 8) & PC (No. 12), 1:2, V/V) showed better effect in the delivery of double-stranded nucleic acid into A549 cells than RNAiMAX.

### Example 2-10: Delivery of double-stranded nucleic acid into A549 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination (PE (No. 8) & LPC (No. 37), 4:1, V/V) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 2 µL lipid combination, and heated at 80 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

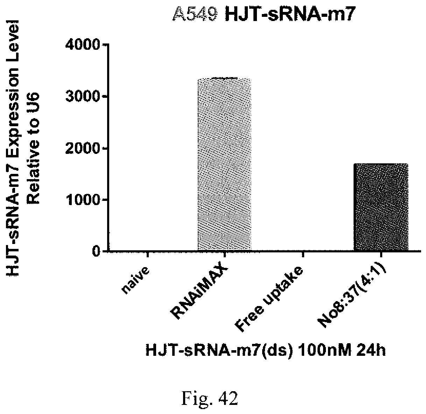

**Conclusions:** The results indicated that the above lipid combinations were effective in delivering nucleic acids into cells (see Fig.42), having the potential of improving the efficiency of the delivery of nucleic acid drug in clinical settings.

### Example 2-11: Delivery of double-stranded nucleic acid into MRC-5 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of Lipid combination (PE (No. 8) & PC (No. 12), 1:2, V/V) and the HJT-sRNA-m7 double-stranded nucleic acid solution that was treated by boiling method was added to the cells, and mixed, and the final concentration of RNA was 100 nM;
5) Treatment group of lipid combination and double-stranded HJT-sRNA-m7 mixture: a mixture of 3 µL lipid combination (mixture of 2 µL PE (No. 8) & PC (No. 12) and 1 µL other type of lipids(MG (No.34), DG (No. 2), TG (No. 32), LPC (No. 37), PC (No. 11), PE (No. 38), Cer (No. 4), So (No. 31) or FA (No. 29)) and the double-stranded HJT-sRNA-m7 nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added to 3 µL lipid combination, and heated at 80 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

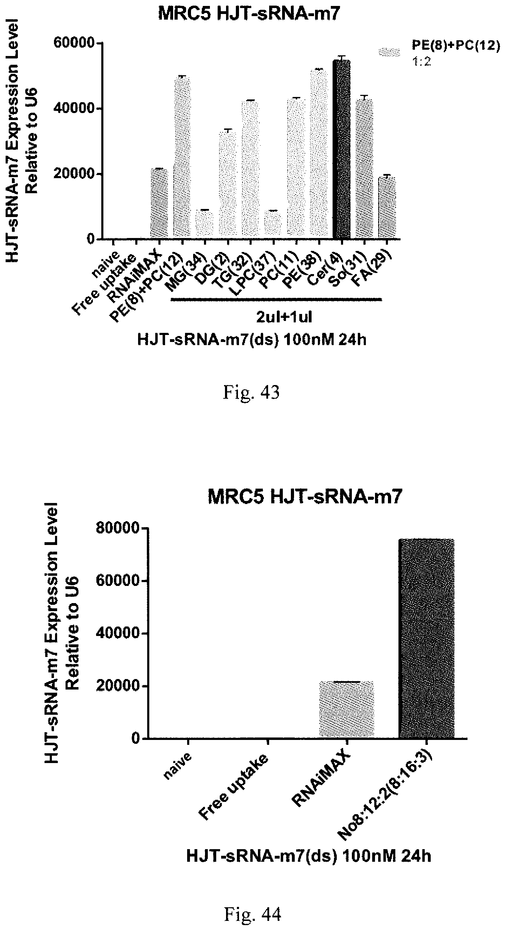

**Conclusions:** The results indicated that the above lipid combinations were effective in delivering nucleic acids into cells (see Fig.43), having the potential of improving the efficiency of the delivery of nucleic acid drug in clinical settings. PE (No. 8) & PC (No. 12) could effectively deliver nucleic acids into cells with significantly better effect than RNAiMAX. Compared to PE (No. 8) & PC (No. 12), a mixture of PE (No. 8) & PC (No. 12) and Cer (No. 4) or PE (No. 38) at a ratio of 2: 1 could enhance this effect.

### Example 2-12: Delivery of double-stranded nucleic acid into MRC-5 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination (PE (No. 8) & PC (No. 12) &DG (No. 2), 8:16:3, V/V/V) and the HJT-sRNA-m7 double-stranded nucleic acid solution that was treated by boiling method was added to the cells, and mixed, and the final concentration of RNA was 100 nM;

### 5). Experimental procedures

1) Boiling method conditions: to 100 µL double-stranded HJT-sRNA-m7 solution was added 2 µL lipid combination, and heated at 80 °C for 30 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

**Conclusions:** The results indicated that as compared with the free uptake group and RNAiMAX group, the lipid combination (PE (No. 8) & PC (No. 12) &DG (No. 2), 8:16:3, V/V/V) showed better effect in delivery than RNAiMAX (see Fig.44), having the potential of improving the efficiency of the delivery of nucleic acid drug in clinical settings.

### Example 2-13: Delivery of double-stranded nucleic acid into MRC-5 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination and the HJT-sRNA-m7 double-stranded nucleic acid solution that was treated by boiling method was added to the cells, and mixed, and the final concentration of RNA was 100 nM;
   Mixture 1: PE (No. 8):LPC (No. 37):TG (No. 32)-4:1:2
   Mixture 2: PE (No. 8):LPC(No. 37):DG (No. 2)-4:1:2
   Mixture 3: PE (No. 8):PC (No. 12):So (No. 31):FA (No. 29)-1:2:1:1

### 5). Experimental procedures

1) Boiling method conditions: to 100 µL double-stranded HJT-sRNA-m7 solution was added 2.5 µL lipid combination, and heated at 90 °C for 15 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

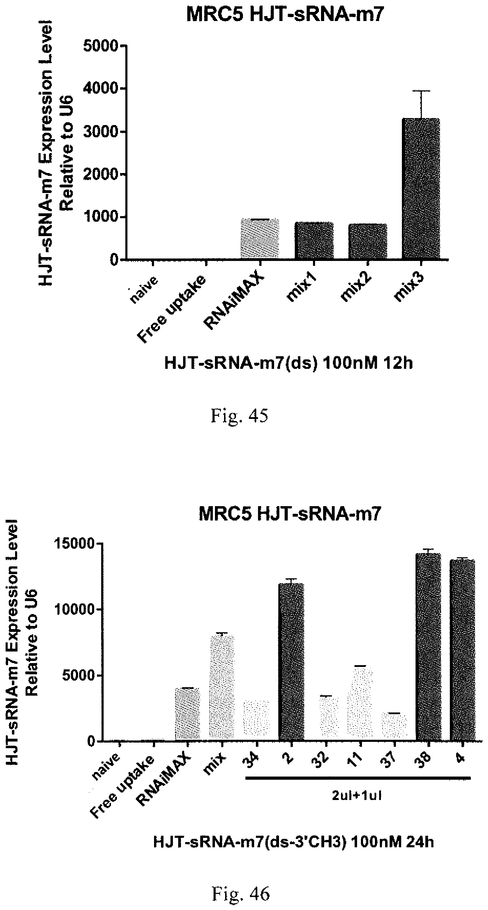

**Conclusions:** The results indicated that the above lipid combinations were effective in delivering nucleic acids into cells (see Fig.45), having the potential of improving the efficiency of the delivery of nucleic acid drug in clinical settings. As compared with RNAiMAX group, mixture 1: PE (No. 8):LPC (No. 37):TG (No. 32)-4:1:2, and mixture 2: PE (No. 8):LPC(No. 37):DG (No. 2)-4:1:2 showed comparable effect in delivery, whereas mixture 3: PE (No. 8):PC (No. 12):So (No. 31):FA (No. 29)-1:2:1:1 showed better effect.

### Example 2-14: Delivery of double-stranded nucleic acid into MRC-5 cells by lipid combination

### 1. Experimental groups:

1) Naive group: referred to untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) Treatment group of lipid combination mixture and double-stranded HJT-sRNA-m7 mixture: a mixture of 3 µL lipid combination (PE (No. 8):PC (No. 12):So (No. 31):FA (No. 29)-1:2:1: 1) and the HJT-sRNA-m7 double-stranded nucleic acid solution that was treated by boiling method was added to the cells, and mixed, and the final concentration of RNA was 100 nM;
5) Treatment group of lipid combination and double-stranded HJT-sRNA-m7 mixture: a mixture of 3 µL lipid combination (mixture of 2 µL lipid combination mix and 1 µL other type of lipid shown in Fig.46, i.e. lipids No. 34, 2, 32, 11, 37, 38 or4) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 3 µL lipid combination, and heated at 90 °C for 15 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

**Conclusions:** The results indicated that the above lipid combinations were effective in delivering nucleic acids into cells (see Fig.46), having the potential of improving the efficiency of the delivery of nucleic acid drug in clinical settings. The mixture (PE (No. 8):PC (No. 12):So (No. 31):FA (No. 29)-1:2:1:1) showed better effect in delivery than RNAiMAX. Compared to mixture (PE (No. 8):PC (No. 12):So (No. 31):FA (No. 29)-1:2:1: 1), the addition of mixture PE (No. 8):PC (No. 12):So (No. 31):FA (No. 29)-1:2:1:1) to lipids No. 2, 38 or 4 at a ratio of 2:1 could enhance the delivery effect.

### Example 2-15: Delivery of double-stranded nucleic acid into MRC-5 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) A mixture of lipid combination (PE (No. 8) & So (No. 31), 6:1, V/V) and the HJT-sRNA-m7 double-stranded nucleic acid solution that was treated by boiling method was added to the cells, and mixed, and the final concentration of RNA was 100 nM;

### 5). Experimental procedures

1) Boiling method conditions: to 100 µL double-stranded HJT-sRNA-m7 solution was added 2 µL lipid combination, and heated at 90 °C for 15 min;
2) Experiment conditions: the final concentration of HJT-sRNA-m7 was 100 nM, 24 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

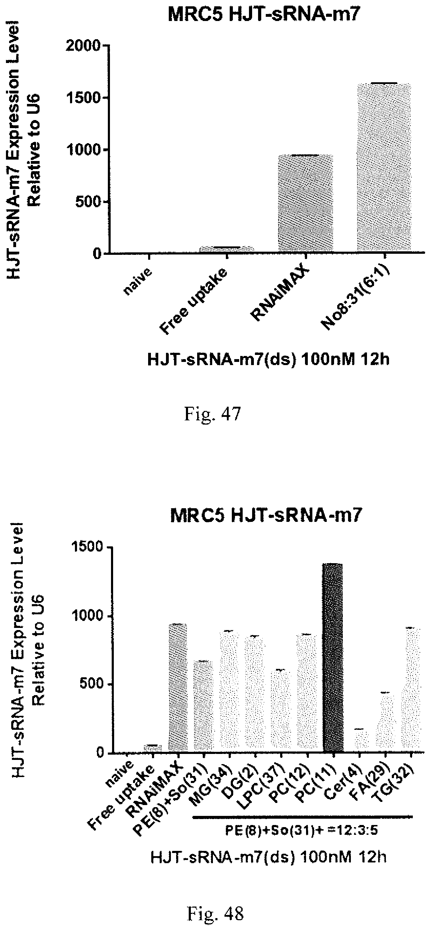

**Conclusions:** The results indicated that the lipid combination (PE (No. 8) & So (No. 31), 6:1, V/V) showed better effect in delivery than RNAiMAX (see Fig.47), having the potential of improving the efficiency of the delivery of nucleic acid drug in clinical settings.

### Example 2-16: Delivery of double-stranded nucleic acid into MRC-5 cells by lipid combination

### 1. Experimental groups:

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) Treatment group of lipid combination (PE (No. 8) & So (No. 31), 4: 1, V/V) and the HJT-sRNA-m7 mixture: a mixture of 2 µL the lipid combination and the HJT-sRNA-m7 double-stranded nucleic acid solution that was treated by boiling method was added to the cells, and mixed, and the final concentration of RNA was 100 nM;
5) Treatment group of lipid combination and double-stranded HJT-sRNA-m7 mixture: a mixture of lipid combination (a mixture of PE (No. 8) & So (No. 31), 4: 1, V/V) and other types of lipid (MG (No. 34), DG (No. 2), LPC (No. 37), PC (No. 12), PC (No. 11), Cer (No. 4), FA (No. 29) or TG (No. 32), 12:3:5, V/V, Fig.48) and the HJT-sRNA-m7 double-stranded nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 2 µL lipid combination, and heated at 90 °C for 15 min;
2) Experiment conditions: the final concentration of HJT-sRNA-m7 was 100 nM, 12 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

**Conclusions:** The results indicated that the above lipid combinations were effective for delivering nucleic acids into cells (see Fig.48), having the potential of improving the efficiency of the delivery of nucleic acid drug in clinical settings. PE (No. 8):So (No. 31) (4:1, V/V) could effectively deliver nucleic acids into cells with an effect close to RNAiMAX. Compared to PE (No. 8):So (No. 31), the mixture of PE (No. 8):So (No. 31) and MG (No. 34), DG (No. 2), PC (No. 12), PC (No. 11), or TG (No. 32) at a ratio of 12:3:5 could enhance the effect in delivery of nucleic acid, and PE (No. 8):So (No. 31):PC (No. 11) showed the best effect, significantly better than RNAiMAX.

### Example 2-17: Delivery of double-stranded nucleic acid into MRC-5 cells by lipid combination

### 1. Experimental groups:

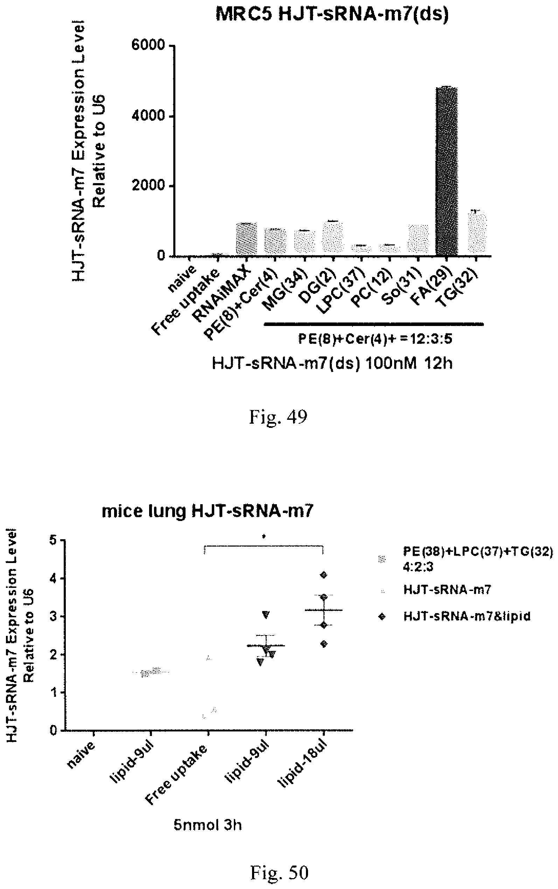

1) Naive group: untreated cell;
2) RNAiMAX treatment group: 2 µl RNAiMAX transfection reagent and double-stranded HJT-sRNA-m7 solution were diluted in 100 µl opti-MEM medium, respectively, and then the two were mixed, allowed to stand for 15 min, added into cells, and then mixed. The final concentration of double-stranded HJT-sRNA-m7 was 100 nM;
3) Free uptake group: double-stranded HJT-sRNA-m7 solution was directly added (the final concentration was 100 nM);
4) Treatment group of lipid combination (PE (No. 8):Cer (No. 4), 4:1, V/V) and the HJT-sRNA-m7 mixture: a mixture of 2 µL lipid combination and the HJT-sRNA-m7 double-stranded nucleic acid solution that was treated by boiling method was added to the cells, and mixed, and the final concentration of RNA was 100 nM;
5) Treatment group of lipid combination and double-stranded HJT-sRNA-m7 mixture: a mixture of lipid combination (mixture of PE (No. 8):Cer (No. 4) and other types of lipids MG (No. 34), DG (No. 2), LPC (No. 37), PC (No. 12), PC (No. 31), FA (No. 29) or TG (No. 32), 12:3:5, V/V, Fig.49) and the double-stranded HJT-sRNA-m7 nucleic acid solution that was treated by boiling method was added to the cells, and mixed, and the final concentration of RNA was 100 nM;

### 2. Experimental procedures

1) Boiling method conditions: to 100 µL double-stranded HJT-sRNA-m7 solution was added 2 µL lipid combination, and heated at 90 °C for 15 min;
2) Experiment conditions: the final concentration of HJT-sRNA-m7 was 100 nM, 12 hours after the addition to the cells, the amount of HJT-sRNA-m7 that entered the cells was detected by RT-qPCR method. For the protocols, see "Real-time quantitative PCR detection of intracellular expression of nucleic acids delivered by lipids". All experiments were performed in triplicates.

**Conclusions:** The results indicated that the above lipid combinations were effective for delivering nucleic acids into cells (see Fig.49), having the potential of improving the efficiency of the delivery of nucleic acid drug in clinical settings. Lipid combination PE (No. 8): Cer (No. 4) could effectively deliver nucleic acids into cells with an effect close to RNAiMAX. Compared to PE (No. 8): So (No. 31), the mixture of PE (No. 8): So (No. 31) and DG (No. 2), FA (No. 29) or TG (No. 32) at a ratio of 12:3:5 could enhance the effect in delivery of nucleic acid, and FA (No. 29) could significantly improve the effect (significantly better than RNAiMAX) of PE (No. 8): So (No. 31) in delivery.

### Example 3: Lipid combination promotes nucleic acid entry into the lung through digestive tract

The lipid combination was as follow:
Lipids PE (No. 38) & LPC (No. 37) & TG (No. 32), 4:2:3, V/V/V

### 1. Preparation of lipid nucleic acid mixture:

### Method: boiling method

To 400 µL of HJT-sRNA-m7 (5 nmol) single-stranded RNA in DEPC-treated aqueous solution was added 9 µL or 18 µL lipid combination (lipid PE (No. 38) & LPC (No. 37) & TG (No. 32), 4:2:3, V/V/V), mixed and heated at 100 °C for 30 min.

### 2. Delivery experiment of nucleic acid via digestive tract

RNA was administered via gavage to 6-8 weeks old male C57 mice: HJT-sRNA-m7 in aqueous solution or a mixture solution of lipid and HJT-sRNA-m7 was administered via gavage needle, 400 µL/animal (HJT-sRNA-m7, 5 nmol/animal). Groups were as follows:
(1) Control group (naive group): mice that did not receive any treatment;
(2) Negative control group (lipid group): administration of 9 µL lipid combinations (lipid PE (No. 38) & LPC (No. 37) & TG (No. 32), 4:2:3, V/V/V) via gavage;
(3) Free uptake group: direct administration of single-stranded HJT-sRNA-m7 RNA via gavage;
(4) Lipid and nucleic acid mixture group: administration of a mixture of lipid combination and single-stranded HJT-sRNA-m7 RNA via gavage.

3 hours after administration via gavage, the mouse whole lung was lysed with 3 mL TRIzol, the total RNA was extracted and the abundance of HJT-sRNA-m7 was detected by RT-qPCR.

**Conclusion:** As shown in Fig.50, 9 µL or 18 µL lipid combination (lipid PE (No. 38) & LPC (No. 37) & TG (No. 32), 4:2:3, V/V/V) significantly promoted the entry of small fragments of nucleic acids into lung tissue (* indicating a P value of less than 0.05) as compared to the free uptake group. With this (non-invasive) administration via gavage, the lipid combination (lipid PE (No. 38) & LPC (No. 37) & TG (No. 32), 4:2:3, V/V/V) could promote small fragments of nucleic acids entering the lung tissue, which could be used as a means of nucleic acid drug delivery.

### Example 4: Function experiments of delivery of double-stranded nucleic acid into cells mediated by Chinese traditional medicine-derived lipid mixture

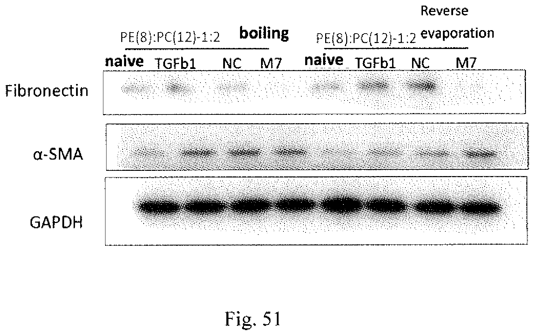

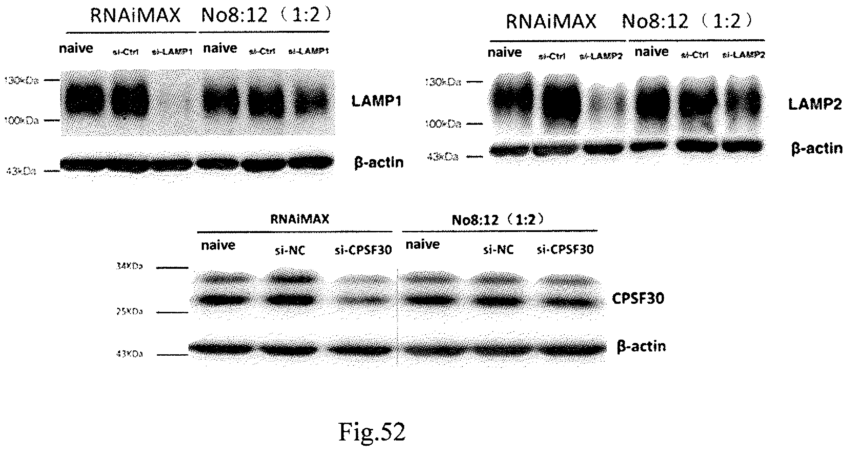

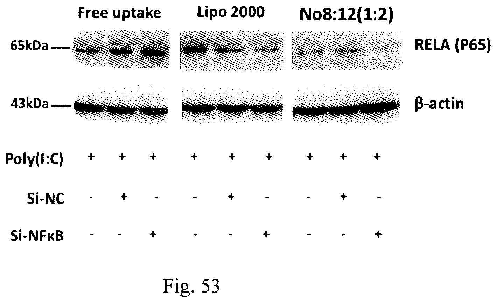

1. No. 8 (PE):No. 12 (PC) (v:v=1:2) lipid mixture mediated the entry of nucleic acids into cells to function
   Experimental method: Western blot, see above "Western blot detection of protein expression level".
   1) No. 8 (PE):No. 12 (PC) (v:v=1:2) lipid mixture mediated anti-fibrotic double-stranded HJT-sRNA-m7 entry into MRC-5 cells.
      As shown in Fig.51, by boiling method and reverse evaporation method, No. 8 (PE):No. 12 (PC) (V:V=1:2) lipid mixture could effectively deliver nucleic acid into cells to function.
      Naive group: untreated MRC-5 cells, i.e., a blank control group;
      TGF β G1 group: MRC-5 cells were stimulated with TGF β 1 protein (final concentration of 3 ng/mL), and the samples were collected after 72 hours.
      NC group: the mixture of lipid combination of No. 8 (PE):No. 12 (PC) (V:V=1:2) and double-stranded NC mimics was added to the MRC-5 cells and mixed well, and the final concentration of nucleic acid was 200 nM. After 24 hours, the cells were stimulated with TGFβ1 protein (final concentration of 3 ng/mL), and samples were collected 72 hours after the stimulation with TGFβ1.
      M7 group: the mixture of lipid combination of No. 8 (PE):No. 12 (PC) (V:V=1:2) with double-stranded HJT-sRNA-m7 was added to the MRC-5 cells and mixed, and the final concentration of nucleic acid was 200 nM. After 24 hours, the cells were stimulated with TGFβ1 protein (final concentration of 3 ng/mL), and samples were collected after 72 hours.
   2) No. 8 (PE):No. 12 (PC) (v:v=1:2) lipid mixture mediated siRNA entry into A549 cells.
      As shown in Figures 52 and 53, by the boiling method, lipid No. 8 (PE):No. 12 (PC) (v:v=1:2) lipid mixture could effectively deliver nucleic acid into cells to knockdown protein expression.
      The naive group in Fig.52: untreated cells, i.e., a blank control group;
      si-NC: the mixture of lipid combination of No.8 (PE):No. 12 (PC) (v:v=1:2) and si-NC (synthesized by Guangzhou Ribobio Co., Ltd., unknown sequences) was added to A549 cells and mixed, and the final concentration was 400 nM; the cells were harvested after 48 hours, and lysed by RIPA strong lysis buffer to collect protein samples.
      si-CPSF30: the mixture of lipid combination of No.8 (PE):No. 12 (PC) (v:v=1:2) and si-CPSF30 was added to A549 cells and mixed, and the final concentration was 400 nM; the cells were harvested after 48 hours, and lysed by RIPA strong lysis buffer to collect protein samples.
      si-LAMP1: the mixture of lipid combination of No.8 (PE):No. 12 (PC) (v:v=1:2) and si-LAMP1 was added to A549 cells and mixed, the final concentration was 400 nM; the cells were harvested after 48 hours, and lysed by RIPA strong lysis buffer to collect protein samples.
      si-LAMP2: the mixture of lipid combination of No.8 (PE):No. 12 (PC) (v:v=1:2) and si-LAMP2 was added to A549 cells and mixed, and the final concentration was 400 nM; the cells were harvested after 48 hours, and lysed by RIPA strong lysis buffer to collect protein samples.

      Free uptake group as shown in Fig.53: the nucleic acid solution was added directly.
      Lipo 2000 group: 2 µL Lipofectamine™ 2000 transfection reagent (Invitrogen, Thermo Fisher Scientific) and si-NF-κB solution were diluted in 100 µL opti-MEM medium, respectively, and the two were mixed, allowed to stay for 15 min, added to the cells and mixed, and the final concentration of nucleic acid solution was 400 nM; after 24 hours, the cells were stimulated with polyI:C (the concentration was 1 µg/mL), and the protein samples were collected after 6 hours.
      No. 8 (PE):No. 12 (PC) (1:2): No. 8 (PE):No. 12 (PC) (1:2) was mixed with the si-NF-κB solution by heating method, then added to the cells, and the final concentration of the nucleic acid solution was 400 nM; after 24 hours, the cells were stimulated with polyI:C (the concentration was 1 µg/mL), and the protein samples were collected after 6 hours.
      See Table 2 for the types and sequences of the above nucleic acids.
   3) No. 8 (PE):No. 12 (PC) (v:v=1:2) lipid mixture mediated siRNA entry into THP-1 cells.

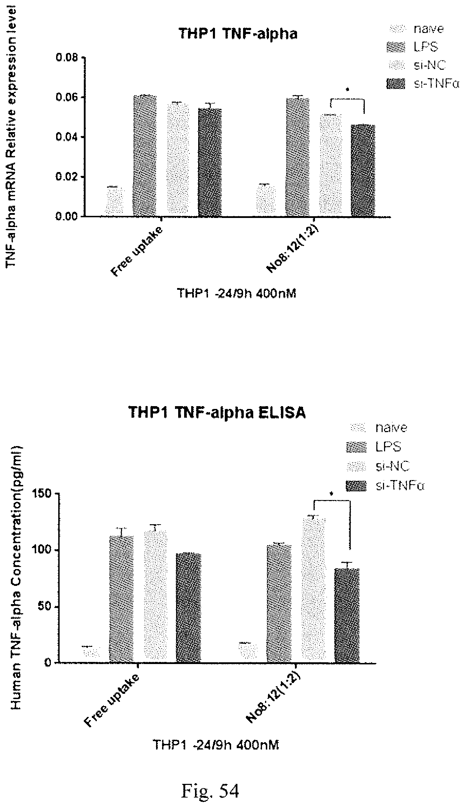

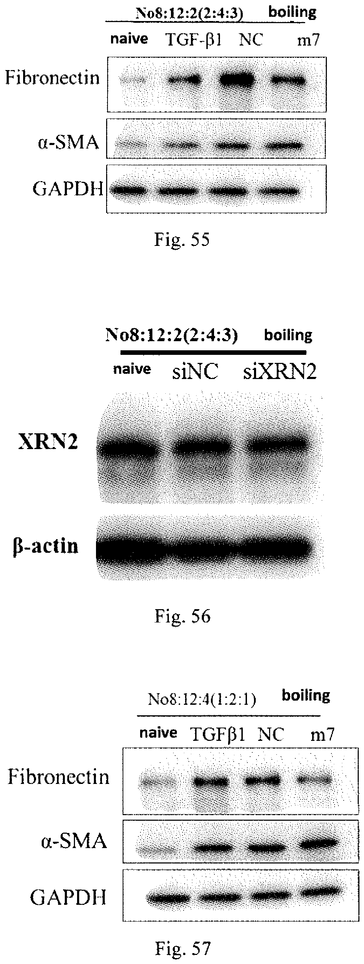

   As shown in Fig.54, by boiling method, No. 8 (PE):No. 12 (PC) (v:v=1:2) lipid mixture could effectively deliver nucleic acid into cells to function.
   Naive group: untreated cells, i.e., a blank control group;
   LPS group: no siRNA, but only LPS was added for stimulation, and the final concentration was 1 µg/mL. The RNA samples and cell supernatants were harvested after 9 hours;
   si-NC group: the mixture of lipid combination of No.8 (PE):No. 12 (PC) (v:v=1:2) and si-NC was added to THP-1 cells and mixed, and the final concentration was 400 nM; LPS was added after 24 hours at a final concentration of 1 µg/mL for stimulation, and the TRIzol lysate of the cells were collected 9 hours after the stimulation , and the supernatants were collected for ELISA detection.
   si-TNFα group: the mixture of lipid combination of No.8 (PE):No. 12 (PC) (v:v=1:2) and si-TNFα was added to THP-1 cells and mixed, and the final concentration was 400 nM; LPS was added after 24 hours at a final concentration of 1 µg/mL for stimulation, the TRIzol lysate of the cells were collected 9 hours after the stimulation, and the supernatants were collected for ELISA detection.
2. No. 8 (PE):No. 12 (PC):No. 2 (DG) (v:v:v=2:4:3) lipid mixture mediated entry of nucleic acids into cells to function.
   1) No. 8 (PE):No. 12 (PC):No. 2 (DG) (v:v:v=2:4:3) lipid mixture mediated anti-fibrotic HJT-sRNA-m7 entry into MRC-5 cells.
      As shown in Fig.55, by boiling method, No. 8 (PE):No. 12 (PC):No. 2 (DG) (v:v:v=2:4:3) lipid mixture could effectively deliver anti-fibrotic HJT-sRNA-m7 into MRC-5 cells to reduce fibronectin protein expression.
   2) No. 8 (PE):No. 12 (PC):No. 2 (DG) (v:v:v=2:4:3) lipid mixture mediated XRN2 siRNA entry into A549 cells to inhibit gene expression.

   As shown in Fig.56, by boiling method, the addition of No. 2 (DG) to the mixture of No. 8 (PE): No. 12(PC): No. 20 (DG), V:V:V=2:4:3 could effectively deliver nucleic acid into the cells to function.
   Naive group: untreated A549 cells;
   NC siRNA group: the mixture of lipid mixture of No. 8 (PE):No. 12 (PC):No. 2 (DG) (v:v:v=2:4:3) and si-NC that was prepared by boiling method was added to the cells and mixed, and the final concentration of the nucleic acid was 400 nM;
   XRN2 siRNA group: the mixture of lipid mixture of No. 8 (PE):No. 12 (PC):No. 2 (DG) (v: v: v=2:4:3) and XRN2 siRNA that was prepared by boiling method was added to the cells and mixed, and the final concentration of the nucleic acid was 400 nM.
3. No. 8 (PE):No. 12 (PC):No. 4 (Cer) (v:v:v=1:2:1) lipid mixture mediated entry of nucleic acids into cells to function.
   1) No. 8 (PE):No. 12 (PC): No. 4 (Cer) (v:v:v=1:2:1) lipid mixture mediated anti-fibrotic HJT-sRNA-m7 entry into MRC-5 cells (boiling method).
      As shown in Fig.57, by boiling method, the addition of No. 4 (Cer) to the lipid mixture of No. 8 (PE), No. 12 (PC) (V:V=1:2), v:v:v=1:2:1, could effectively deliver anti-fibrotic HJT-sRNA-m7 into MRC-5 cells to reduce fibronectin protein expression.
      Naive group: untreated cells;
      TGF-β1 group: TGF-β1 protein was added at a final concentration of 3 ng/mL for stimulation, and the samples were collected after 72 hours.
      NC group: lipid combination of No. 38 (PE):No. 37 (LPC):No. 32 (TG) (V:V:V=32:8:5) was used to deliver NC mimics. After 24 hours, TGF-β1 TGFb1 protein (final concentration of 3 ng/mL) was added for stimulation, and the samples were collected after 72 hours.
      m7 group: the mixture of lipid combination of No. 8 (PE):No. 12 (PC):No. 4 (Cer) (V:V:V=1:2:1) with double-stranded HJT-sRNA-m7 was added to the MRC-5 cells and mixed, and the final concentration of nucleic acid was 400 nM. After 24 hours, TGF-β1 protein (final concentration of 3 ng/mL) was added for stimulation, and the samples were collected after 72 hours.
   2) No. 8 (PE):No. 12 (PC):No. 4 (Cer) (v:v:v=1:2:1) lipid mixture mediated NF-κB siRNA entry into A549 cells to inhibit gene expression (boiling method).

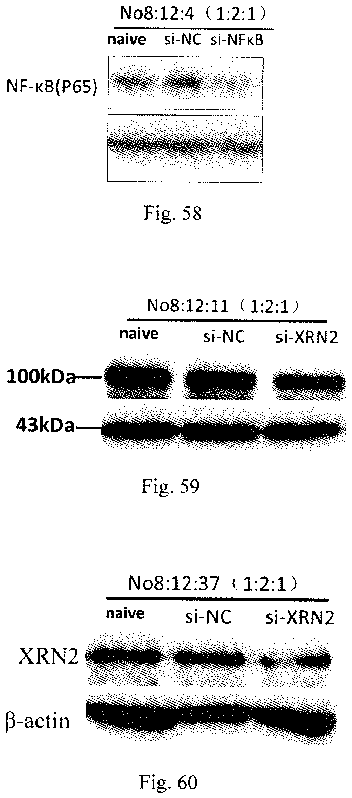

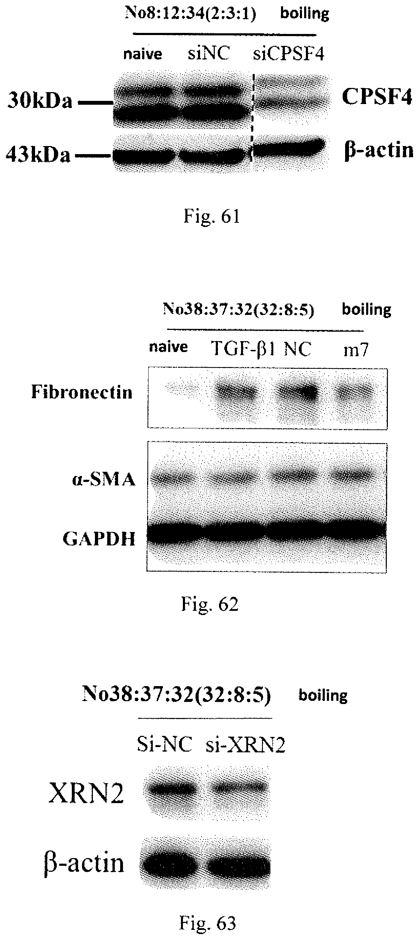

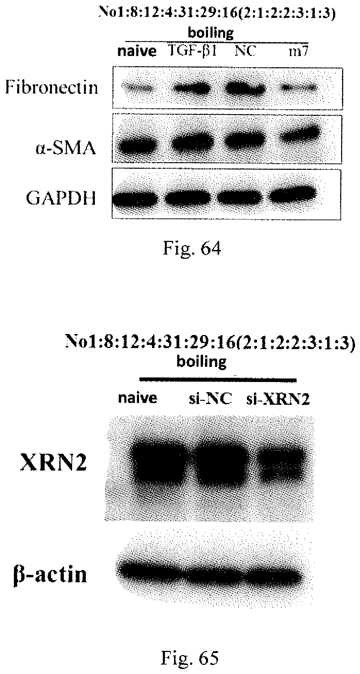

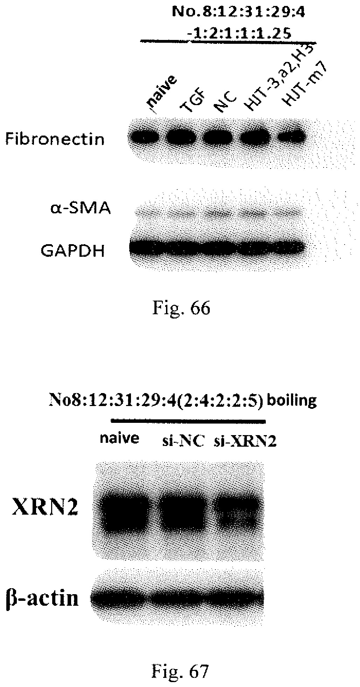

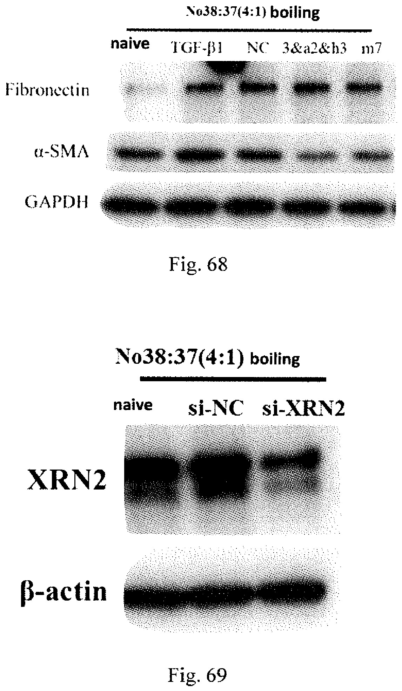

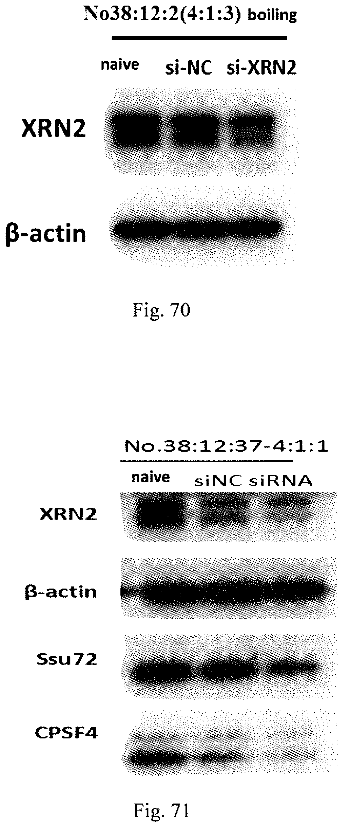

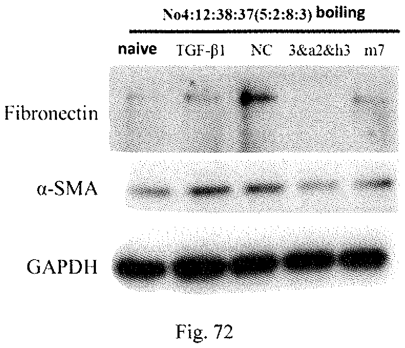

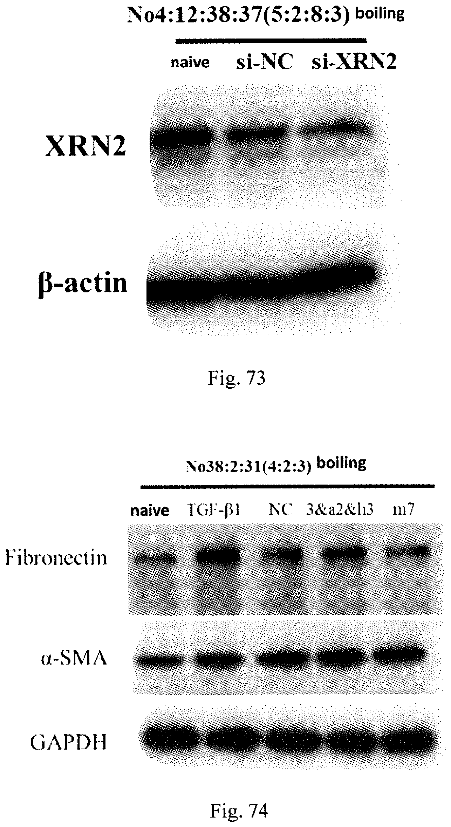

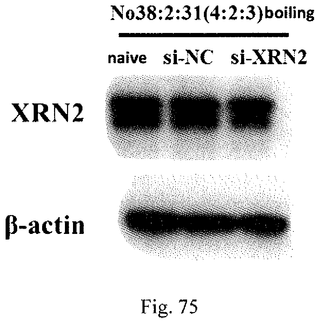

   As shown in Fig.58, the addition of No. 4 (Cer) to a lipid mixture of No. 8 (PE), No. 12 (PC) (V:V=1:2), v:v:v=1:2:1, could effectively deliver nucleic acids into cells to function.
   Naive group: untreated cells;
   si-NC group: the mixture of lipid mixture of No. 8 (PE):No. 12 (PC):No. 4 (Cer) (v:v:v=1:2:1) and si-NC siNC was added to cells and mixed, and the final concentration of the nucleic acid was 400 nM;
   si-NF-κB group: the mixture of lipid mixture of No.8 (PE):No. 12 (PC):No. 4 (Cer) (v:v:v=1:2: 1) and NF-κB siRNA was added to cells and mixed, the final concentration of the nucleic acid was 400 nM;
4. No. 8 (PE):No. 12 (PC):No. PC (11) (v:v:v=1:2:1) lipid mixture mediated entry of nucleic acids into cells to function.
   1) No. 8 (PE):No. 12 (PC):No. PC (11) (v:v:v=1:2:1) lipid mixture mediated XRN2 siRNA entry into A549 cells to inhibit gene expression.
      As shown in Fig.59, the addition of No. 11 (PC) to the mixture of No. 8 (PE), No. 12 (PC) (V:V=1:2), V:V:V=1:2:1, could effectively deliver nucleic acid into the cells to function.
      Naive group: untreated cells;
      si-NC siNC group: the mixture of lipid mixture of No. 8 (PE):No. 12 (PC):No. PC (11) (v:v:v=1:2:1) and si-NC was added to the cells and mixed, and the final concentration of the nucleic acid was 400 nM;
      si-XRN2 group: the mixture of lipid mixture of No. 8 (PE):No. 12 (PC): No. PC (11) (v:v:v=1:2:1) and XRN2 siRNA was added to the cells and mixed, and the final concentration of the nucleic acid was 400 nM.
5. No. 8 (PE):No. 12 (PC):No. LPC (37) (v:v:v=1:2:1) lipid mixture mediated entry of nucleic acids into cells to function.
   1) No. 8 (PE):No. 12 (PC):No. LPC (37) (v:v:v=1:2:1) lipid mixture mediated XRN2 siRNA entry into A549 cells to inhibit gene expression.
      As shown in Fig.60, based on the addition of No. 37 (LPC) to the lipid mixture of No. 8 (PE), No. 12 (PC) (V:V=1:2), V:V:V=1:2:1, could effectively deliver nucleic acid into the cells to function.
      Naive group: untreated cells;
      si-NC group: the mixture of lipid mixture of No. 8 (PE):No. 12 (PC):No. LPC (37) (v:v:v=1:2: 1) and si-NC was added to the cells and mixed, and the final concentration of the nucleic acid was 400 nM;
      si-XRN2 group: the mixture of lipid mixture of No. 8 (PE):No. 12 (PC): No. LPC (37) (v:v:v=1:2: 1) and XRN2 siRNA was added to the cells and mixed, and the final concentration of the nucleic acid was 400 nM.
6. No. 8 (PE):No. 12 (PC):No. MG (34) (v:v:v=2:3:1) lipid mixture mediated entry of nucleic acids into cells to function.
   1) No. 8 (PE):No. 12 (PC):No. MG (34) (v:v:v=2:3:1) lipid mixture mediated CPSF4 siRNA entry into A549 cells to inhibit gene expression.
      Naive group: untreated cells;
      si-NC siNC group: the mixture of lipid mixture of No. 8 (PE):No. 12 (PC):No. MG (34) (v:v:v=2:3:1) and si-NCsiNC was added to the cells and mixed, and the final concentration of the nucleic acid was 400 nM;
      si-CPSF4 group: the mixture of lipid mixture of No. 8 (PE):No. 12 (PC): No. MG (34) (v:v:v=2:3:1) and CPSF4 siRNA was added to the cells and mixed, and the final concentration of the nucleic acid was 400 nM.
         As shown in Fig.61, No. 8 (PE):No. 12 (PC):No. MG (34) (v:v:v=2:3:1) lipid mixture could effectively deliver nucleic acid into the cells to function.
7. No. 38 (PE):No. 37 (LPC):No. 32 (TG) (v:v:v=32:8:5) lipid mixture mediated entry of nucleic acids into cells to function.
   1) No. 38 (PE):No. 37 (LPC):No. 32 (TG) (v:v:v=32:8:5) lipid mixture mediated anti-fibrotic HJT-sRNA-m7 entry into MRC-5 cells (boiling method).
      As shown in Fig.62, the m7 band was lighter compared to control. The effect of M7 was not sufficient to restore cells to unstimulated levels.
      Naive group: untreated cells, i.e., a blank control group;
      TGF-β1 group: cells were stimulated with TGF-β1 protein (final concentration of 3 ng/mL), and the samples were collected after 72 hours.
      NC group: lipid combination of No. 38 (PE):No. 37 (LPC):No. 32 (TG) (V:V:V=32:8:5) was used to deliver NC mimics. After 24 hours, the cells were stimulated with TGF-β1 protein (final concentration of 3 ng/mL), and the samples were collected after 72 hours.
      M7 group: the mixture of lipid combination of No. 38 (PE):No. 37 (LPC):No. 32 (TG) (V:V:V=32:8:5) with double-stranded HJT-sRNA-m7 was added to the MRC-5 cells and mixed, and the final concentration of nucleic acid was 400 nM. After 24 hours, the cells were stimulated with TGF-β1 protein (final concentration of 3 ng/mL), and the samples were collected after 72 hours.
   2) No. 38 (PE):No. 37 (LPC):No. 32 (TG) (V:V:V=32:8:5) lipid mixture mediated XRN2 siRNA entry into A549 cells to inhibit gene expression.
      As shown in Fig.63, No. 38 (PE):No. 37 (LPC):No. 32 (TG) (V:V:V=32:8:5) lipid mixture could effectively deliver nucleic acid entering the cells to function.
      si-NC group: the mixture of lipid mixture of No. 38 (PE):No. 37 (LPC):No. 32 (TG) (V:V:V=32:8:5) and si-NC was added to the cells and mixed, and the final concentration of the nucleic acid was 400 nM;
      si-XRN2 group: the mixture of the lipid mixture of No. 38 (PE):No. 37 (LPC):No. 32 (TG) (V:V:V=32:8:5) and XRN2 siRNA was added to the cells and mixed, and the final concentration of the nucleic acid was 400 nM.
8. No. 1 (DG):No. 8 (PE):No. 12 (PC):No. 4 (Cer):No. 31 (So):No. 29 (FA):No. 16 (TG) (v:v:v:v:v:v:v=2:1:2:2:3:1:3) lipid mixture mediated entry of nucleic acids into cells to function.
   1) As shown in Fig.64, No. 1 (DG):No. 8 (PE):No. 12 (PC):No. 4 (Cer):No. 31 (So):No. 29 (FA):No. 16 (TG) (v:v:v:v:v:v:v=2:1:2:2:3:1:3) lipid mixture mediated anti-fibrotic HJT-sRNA-m7 entry into MRC-5 cells (boiling method).
      Naive group: untreated cells, i.e., a blank control group;
      TGF-β1 group: cells were stimulated with TGF-β1 protein (final concentration of 3 ng/mL), and the samples were collected after 72 hours.
      NC group: lipid combination of No. 1 (DG):No. 8 (PE):No. 12 (PC):No. 4 (Cer):No. 31 (So):No. 29 (FA):No. 16 (TG) (v:v:v:v:v:v:v=2:1:2:2:3:1:3) was used to was used to deliver NC mimics. After 24 hours, TGF-β1 protein (final concentration of 3 ng/mL) was added for stimulation, and the samples were collected after 72 hours.
      M7 group: the mixture of lipid combination of No. 1 (DG):No. 8 (PE):No. 12 (PC):No. 4 (Cer):No. 31 (So):No. 29 (FA):No. 16 (TG) (v:v:v:v:v:v:v=2:1:2:2:3:1:3) with single-stranded HJT-sRNA-m7 was added to the MRC-5 cells, and mixed, and the final concentration of nucleic acid was 400 nM. After 24 hours, TGF-β1 protein (final concentration of 3 ng/mL) was added for stimulation, and the samples were collected after 72 hours.
   2) As shown in Fig.65, No. 1 (DG):No. 8 (PE):No. 12 (PC):No. 4 (Cer):No. 31 (So):No. 29 (FA):No. 16 (TG) (v:v:v:v:v:v:v=2:1:2:2:3:1:3) lipid mixture mediated XRN2 siRNA entry into A549 cells to inhibit gene expression (boiling method).
      No. 1 (DG):No. 8 (PE):No. 12 (PC):No. 4 (Cer):No. 31 (So):No. 29 (FA):No. 16 (TG) (v:v:v:v:v:v:v=2:1:2:2:3:1:3) lipid mixture could effectively deliver nucleic acid entering the cells to function.
      si-NC group: the mixture of lipid mixture of No. 1 (DG):No. 8 (PE):No. 12 (PC):No. 4 (Cer):No. 31 (So):No. 29 (FA):No. 16 (TG) (v:v:v:v:v:v:v=2:1:2:2:3:1:3) and si-NC was added to the cells and mixed, and the final concentration of the nucleic acid was 400 nM;
      si-XRN2 group: the mixture of lipid mixture of No. 1 (DG):No. 8 (PE):No. 12 (PC):No. 4 (Cer):No. 31 (So):No. 29 (FA):No. 16 (TG) (v:v:v:v:v:v:v=2:1:2:2:3:1:3) and XRN2 siRNA was added to the cells and mixed, and the final concentration of the nucleic acid was 400 nM.
9. No. 8 (PE):No. 12 (PC):No. 31 (So):No. 29 (FA):No. 4 (Cer) (v:v:v:v:v=2:4:2:2:5) lipid mixture mediated entry of nucleic acids into cells to function.
   1) As shown in Fig.66, No. 8 (PE):No. 12 (PC):No. 31 (So):No. 29 (FA):No. 4 (Cer) (v:v:v:v:v=2:4:2:2:5) lipid mixture mediated anti-fibrotic HJT-sRNA, HJT-sRNA-3, HJT-sRNA-a2, HJT-sRNA-h3, HJT-sRNA-m7 entry into MRC-5 cells (boiling method).
      Naive group: untreated cells, i.e., a blank control group;
      TGF-β1 group: cells were stimulated with TGF-β1 protein (final concentration of 3 ng/mL), and the samples were collected after 72 hours.
      NC group: lipid combination of No. 8 (PE):No. 12 (PC):No. 31 (So):No. 29 (FA):No. 4 (Cer) (v:v:v:v:v=2:4:2:2:5) was used to deliver NC mimics. After 24 hours, the cells were stimulated with TGF-β1 protein (final concentration of 3 ng/mL), and the samples were collected after 72 hours.
      HJT-3, a2, H3 group: the mixture of lipid combination of No. 8 (PE):No. 12 (PC):No. 31 (So):No. 29 (FA):No. 4 (Cer) (v:v:v:v:v=2:4:2:2:5) with HJT-sRNA-3, HJT-sRNA-a2, HJT-sRNA-h3 was added into cells, mixed, and the final concentration of nucleic acid was 400nM. After 24 hours, the cells were stimulated with TGF-β1 protein (final concentration of 3 ng/mL), and the samples were collected after 72 hours.
      HJT-M7 group: the mixture of lipid combination of No. 8 (PE):No. 12 (PC):No. 31 (So):No. 29 (FA):No. 4 (Cer) (v:v:v:v:v=2:4:2:2:5) with HJT-sRNA-m7 single-stranded was added to the MRC-5 cells, mixed, and the final concentration of nucleic acid was 400 nM. After 24 hours, the cells were stimulated with TGF-β1 protein (final concentration of 3 ng/mL), and the samples were collected after 72 hours.
   2) As shown in Fig.67, No. 8 (PE):No. 12 (PC):No. 31 (So):No. 29 (FA):No. 4 (Cer) (v:v:v:v:v=2:4:2:2:5) lipid mixture mediated XRN2 siRNA entry into A549 cells to inhibit gene expression (boiling method).
      No. 8 (PE):No. 12 (PC):No. 31 (So):No. 29 (FA):No. 4 (Cer) (v:v:v:v:v=2:4:2:2:5) lipid mixture could effectively deliver nucleic acid into the cells to function.
      si-NC group: the mixture of lipid mixture of No. 8 (PE):No. 12 (PC):No. 31 (So):No. 29 (FA):No. 4 (Cer) (v:v:v:v:v=2:4:2:2:5) and si-NC was added to the cells and mixed, and the final concentration of the nucleic acid was 400 nM;
      si-XRN2 group: the mixture of lipid mixture of No. 8 (PE):No. 12 (PC):No. 31 (So):No. 29 (FA):No. 4 (Cer) (v:v:v:v:v=2:4:2:2:5) and XRN2 siRNA was added to the cells and mixed, and the final concentration of the nucleic acid was 400 nM.
10. No. 38 (PE):No. 37 (LPC) (v:v =4:1) lipid mixture mediated entry of nucleic acids into cells to function.
   1) As shown in Fig.68, No. 38 (PE):No. 37 (LPC) (v:v =4:1) lipid mixture mediated anti-fibrotic HJT-sRNA, HJT-sRNA-3, HJT-sRNA-a2, HJT-sRNA-h3, HJT-sRNA-m7 entry into MRC-5 cells (boiling method).
      Naive group: untreated cells, i.e., a blank control group;
      TGF-β1 group: cells were stimulated with TGF-β1 protein (final concentration of 3 ng/mL), and the samples were collected after 72 hours.
      NC group: lipid combination of No. 38 (PE):No. 37 (LPC) (v:v =4:1) was used to was used to deliver NC mimics. After 24 hours, the cells were stimulated with TGF-β1 protein (final concentration of 3 ng/mL), and the samples were collected after 72 hours.
      3&a2&H3 group: the mixture of lipid combination of No. 38 (PE):No. 37 (LPC) (v:v =4:1) with HJT-sRNA-3, HJT-sRNA-a2, HJT-sRNA-h3 double-stranded combination was added to cells, and mixed, and the final concentration of nucleic acid was 400 nM. After 24 hours, the cells were stimulated with TGF-β1 protein (final concentration of 3 ng/mL), and the samples were collected after 72 hours.
      M7 group: the mixture of lipid combination of No. 38 (PE):No. 37 (LPC) (v:v =4:1) with HJT-sRNA-3, HJT-sRNA-a2, HJT-sRNA-h3, HJT-sRNA-m7 was added to the MRC-5 cells, and mixed, and the final concentration of nucleic acid was 400 nM. After 24 hours, the cells were stimulated with TGF-β1 protein (final concentration of 3 ng/mL), and the samples were collected after 72 hours.
   2) As shown in Fig.69, No. 38 (PE):No. 37 (LPC) (v:v =4:1) lipid mixture mediated XRN2 siRNA entry into A549 cells to inhibit gene expression (boiling method).
      No. 38 (PE):No. 37 (LPC) (v:v =4:1) lipid mixture could effectively deliver nucleic acid entering the cells to function.
      si-NC group: the mixture of lipid mixture of No. 38 (PE):No. 37 (LPC) (v:v =4:1) and si-NC was added to the cells and mixed, and the final concentration of the nucleic acid was 400 nM;
      si-XRN2 group: the mixture of lipid mixture of No. 38 (PE):No. 37 (LPC) (v:v =4:1) and XRN2 siRNA was added to the cells and mixed, and the final concentration of the nucleic acid was 400 nM.
11. No. 38 (PE):No. 12 (PC):No. 2 (DG) (v:v:v =4:1:3) lipid mixture mediated entry of nucleic acids into cells to function.
   As shown in Fig.70, No. 38 (PE):No. 12 (PC):No. 2 (DG) (v:v:v =4:1:3) lipid mixture mediated XRN2 siRNA entry into A549 cells to inhibit gene expression.
   The lipid mixture of No. 38 (PE), in place of No. 8 (PE), with No. 12 (PC), No. 2 (DG) (v:v:v=4:1:3) could effectively deliver nucleic acid entering the cells to function.
   si-NC group: the mixture of lipid mixture of No. 38 (PE):No. 12 (PC):No. 2 (DG) (v:v:v =4:1:3) and si-NC was added to the cells and mixed, and the final concentration of the nucleic acid was 400 nM;
   si-XRN2 group: the mixture of lipid mixture of No. 38 (PE):No. 12 (PC):No. 2 (DG) (v:v:v =4:1:3) and XRN2 siRNA was added to the cells and mixed, and the final concentration of the nucleic acid was 400 nM.
12. No. 38 (PE):No. 37 (LPC):No. 12 (PC) (v:v:v =4:1:1) lipid mixture mediated entry of nucleic acids into cells to function.
   As shown in Fig.71, No. 38 (PE):No. 37 (LPC):No. 12 (PC) (v:v:v =4:1:1) lipid mixture mediated XRN2 siRNA entry into A549 cells to inhibit gene expression (reverse evaporation method).
   The addition of No. 12 (PC) (v:v:v =4:1:1) to the lipid mixture of No. 38 (PE):No. 37 (LPC) (v:v=4:1), could effectively deliver nucleic acid into cells to inhibit gene expression.
   si-NC group: the mixture of lipid mixture of No. 38 (PE):No. 37 (LPC):No. 12 (PC) (v:v:v =4:1:1) and si-NC was added to the cells and mixed, and the final concentration of the nucleic acid was 400 nM;
   si-RNA group: the mixture of lipid mixture of No. 38 (PE):No. 37 (LPC):No. 12 (PC) (v:v:v =4:1:1) and XRN2 siRNA, β-actin siRNA, Ssu 72 siRNA or CPSF4 siRNA was added to the cells and mixed, and the final concentration of the nucleic acid was 400 nM.
13. No. 4 (Cer):No. 12 (PC):No. 38 (PE):No. 37 (LPC) (v:v:v:v=5:2:8:3) lipid mixture mediated entry of nucleic acids into cells to function.
   1) As shown in Fig.72, the addition of No. 4 (Cer) to the lipid mixture of No. 38 (PE), No. 37 (LPC), No. 12 (PC) led to the lipid mixture of No. 4 (Cer):No. 12 (PC):No. 38 (PE):No. 37 (LPC) (v:v:v:v=5:2:8:3), which mediated anti-fibrotic HJT-sRNA, HJT-sRNA-3, HJT-sRNA-a2, HJT-sRNA-h3, HJT-sRNA-m7 double-stranded RNA entry into MRC-5 cells to reduce fibronectin expression levels (boiling method).
      Naive group: untreated cells, i.e., a blank control group;
      TGF-β1 group: cells were stimulated with TGF-β1 protein (final concentration of 3 ng/mL), and the samples were collected after 72 hours.
      NC group: lipid combination of No. 4 (Cer):No. 12 (PC):No. 38 (PE):No. 37 (LPC) (v:v:v:v=5:2:8:3) was used to deliver NC mimics, after 24 hours, TGF-β1 protein (final concentration of 3 ng/mL) was added for stimulation, and the samples were collected after 72 hours.
      HJT-3 & a2 & h3 group: the mixture of lipid mixture of No. 4 (Cer):No. 12 (PC):No. 38 (PE):No. 37 (LPC) (v:v:v:v=5:2:8:3) with HJT-sRNA-3, HJT-sRNA-a2, HJT-sRNA-h3 and HJT-sRNA-m7 double-strand, was added to the cells, and mixed and the final concentration of nucleic acid was 400 nM.
      m7 group: the mixture of lipid combination of No. 4 (Cer):No. 12 (PC):No. 38 (PE):No. 37 (LPC) (v:v:v:v=5:2:8:3) with HJT-sRNA-m7 was added to the cells, and mixed, and the final concentration of nucleic acid was 400 nM.
   2) As shown in Fig.73, No. 4 (Cer):No. 12 (PC):No. 38 (PE):No. 37 (LPC) (v:v:v:v=5:2:8:3) lipid mixture mediated XRN2 siRNA entry into cells to inhibit gene expression.
      si-NC group: the mixture of lipid mixture of No. 4 (Cer):No. 12 (PC):No. 38 (PE):No. 37 (LPC) (v:v:v:v=5:2:8:3) and si-NC was added to the cells and mixed, and the final concentration of the nucleic acid was 400 nM;
      si-XRN2 group: the mixture of lipid mixture of No. 4 (Cer):No. 12 (PC):No. 38 (PE):No. 37 (LPC) (v:v:v:v=5:2:8:3) and XRN2 siRNA was added to the cells and mixed, and the final concentration of the nucleic acid was 400 nM.
14. No. 38 (PE):No. 2 (DG):No. 31 (So) (v:v:v =4:2:3) lipid mixture mediated entry of nucleic acids into cells to function.
   1) As shown in Fig.74, No. 38 (PE):No. 2 (DG):No. 31 (So) (v:v:v =4:2:3) lipid mixture mediated anti-fibrotic HJT-sRNA, HJT-sRNA-3, HJT-sRNA-a2, HJT-sRNA-h3, HJT-sRNA-m7 double-stranded RNA entry into MRC-5 cells to reduce fibronectin expression levels (boiling method).
      Naive group: untreated cells, i.e., a blank control group;
      TGF-β1 group: cells were stimulated with TGF-β1 protein (final concentration of 3 ng/mL), and the samples were collected after 72 hours.
      NC group: lipid combination of No. 38 (PE):No. 2 (DG):No. 31 (So) (v:v:v =4:2:3) was used to deliver NC mimics. After 24 hours, TGF-β1 protein (final concentration of 3 ng/mL) was added for stimulation, and the samples were collected after 72 hours.
      HJT-3 & a2 & h3 group: the mixture of lipid mixture of No. 38 (PE):No. 37 (LPC) (v:v:v =4:1) with HJT-sRNA-3, HJT-sRNA-a2 and HJT-sRNA-h3, was added to the cells, and mixed and the final concentration of nucleic acid was 400 nM.
      M7 group: the mixture of lipid combination of No. 38 (PE):No. 37 (LPC) (v:v=4:1) with HJT-sRNA-m7 was added to the cells, and mixed, and the final concentration of nucleic acid was 400 nM.
   2) As shown in Fig.75, No. 38 (PE):No. 2 (DG):No. 31 (So) (v:v:v =4:2:3) lipid mixture mediated XRN2 siRNA entry into A549 cells to inhibit gene expression (boiling method).
      No. 38 (PE):No. 2 (DG):No. 31 (So) (v:v:v =4:2:3) lipid mixture effectively delivered XRN2 siRNA into A549 cells to function.
      si-NC group: the mixture of lipid mixture of No. 38 (PE):No. 2 (DG):No. 31 (So) (v:v:v =4:2:3) and si-NC was added to the cells and mixed, and the final concentration of the nucleic acid was 400 nM;
      si-XRN2 group: the mixture of lipid mixture of No. 38 (PE):No. 2 (DG):No. 31 (So) (v:v:v =4:2:3) and XRN2 siRNA was added to the cells and mixed, and the final concentration of the nucleic acid was 400 nM.

### Example 5: Validation of the effects of lipid No. 41 and its composition

### I. Single lipids delivered nucleic acids (double-stranded RNA and single-stranded RNA) into cells by different preparation methods (reverse evaporation and boiling method)

### Lipid No. 41. Sphinganine (d22:0)

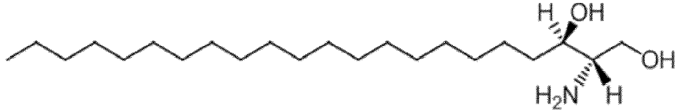

### 1. Quantitative real-time PCR (Real-Time PCR) detection of the efficiency of nucleic acid delivery by lipid.

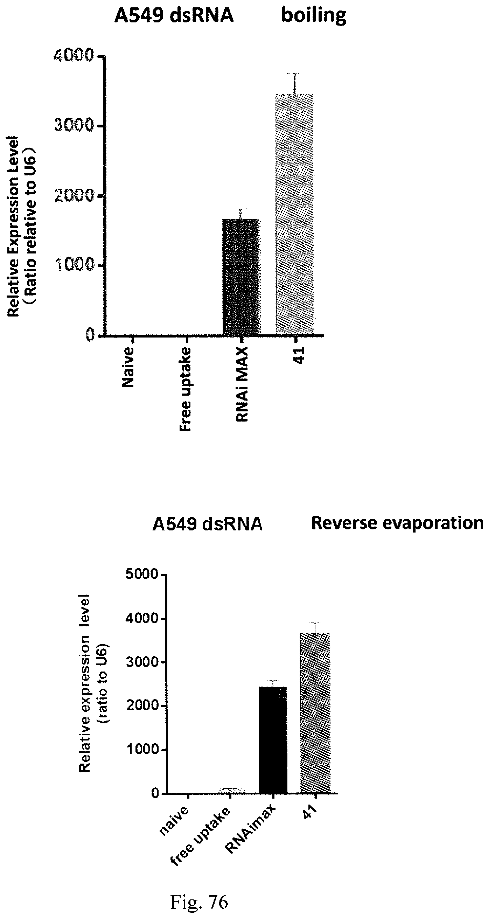

As shown in Fig.76, lipid No. 41 prepared by different methods (boiling or reverse evaporation method) delivered HJT-sRNA-m7 double-stranded RNA into A549 cells. For A549 cells, in the case of the boiling method, the delivery effect of lipid No. 41 was about twice that of RNAiMAX, and in the case of the reverse evaporation method, the delivery effect of lipid No. 41 was also significantly higher than that of RNAiMAX.

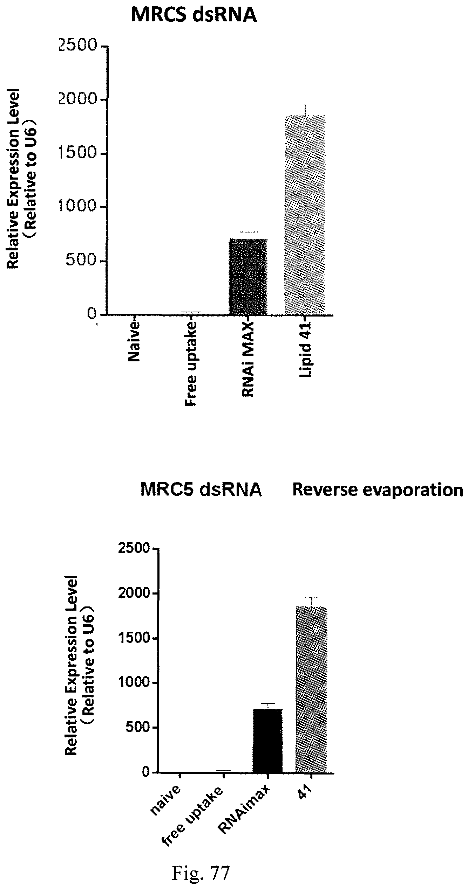

As shown in Fig.77, lipid No. 41 preprared by different methods (boiling or reverse evaporation method) delivered HJT-sRNA-m7 double-stranded RNA into MRC-5 cells. For MRC-5 cells, in the case of the boiling method, lipid No. 41 delivered double-stranded RNA into MRC-5 cells, and in the case of the reverse evaporation method, the delivery effect of lipid No. 41 was significantly higher than that of RNAiMAX.

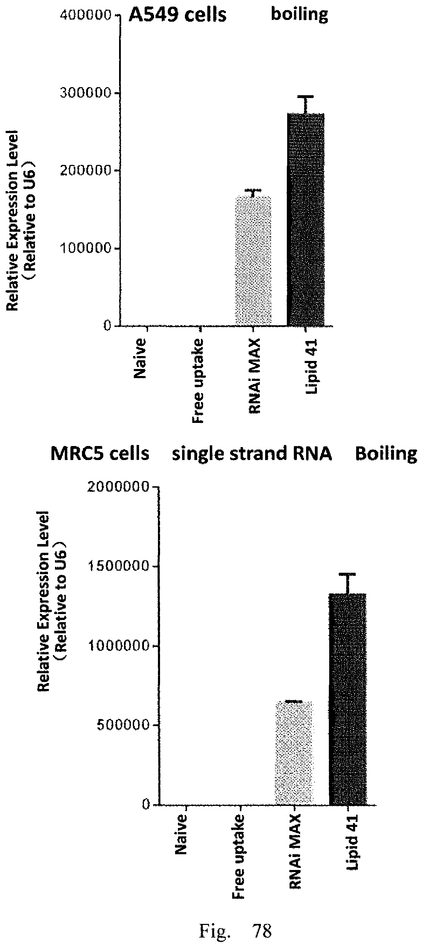

As shown in Fig.78, lipid No. 41 delivered HJT-sRNA-m7 single-stranded RNA into A549 and MRC-5 cells by the boiling method.

### 2. Digital PCR (ddPCR) detection of the efficiency of nucleic acid delivery by lipid

2.1 Experimental materials: A549 cells were purchased from the Cell Center of the Institute of Basic Medical Sciences, Chinese Academy of Medical Sciences, TRIzol lysis buffer was purchased from Sigma, High capacity cRNA Reverse Transcription Kit was purchased from ABI, USA, and the digital PCR related reagents were purchased from Bio-Rad USA.

2.2 Experimental method: the total cellular RNA was collected and extracted by TRIzol lysis buffer according to the above methods, and reverse transcribed to cDNA using High capacity cRNA Reverse Transcription Kit, and the cDNA from different groups was subject to digital PCR reaction. Refering to the QX200 Droplet Reader and QuantaSoft Software manual for the protocols, the results were analyzed using QuantaSoft software. The groups were as follows: (1) naive group: A549 cells without treatment; (2) free uptake group: the cells were directly incubated with HJT-sRNA-m7 dsRNA for 6 hours; (3) RNAiMAX group: A549 cells were transfected with the HJT -sRNA-m7 dsRNA by RNAiMAX, and the samples were collected for detection after 6 hours; (4) No. 41 group: lipid No. 41 prepared by different methods (boiling method or reverse evaporation method) delivered double-stranded RNA into A549 cells, and samples were collected for detection after 6 hours.

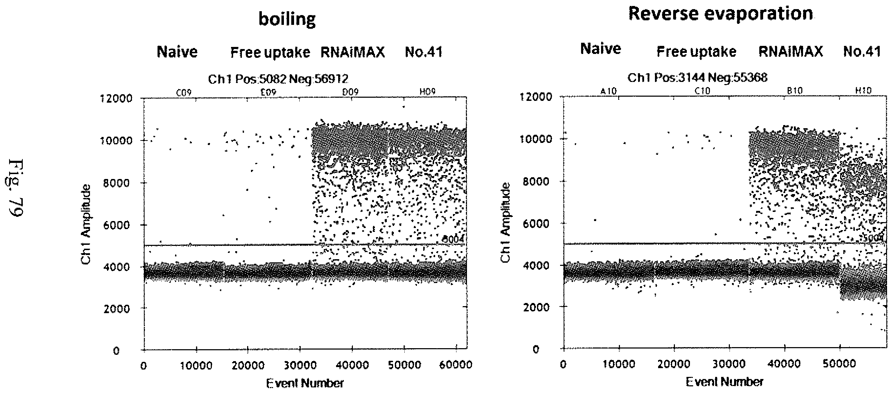

Experimental results and analysis: as shown in Fig.79, by both the boiling method and reverse evaporation method, lipid No. 41 could effectively deliver HJT-sRNA-m7 dsRNA into A549 cells, and the boiling method had better effects than reverse evaporation method.

### 3. Flow cytometry detection of the efficiency of nucleic acid delivery by lipid

Experimental materials: A549 cells (purchased from the Cell Center of the Chinese Academy of Medical Sciences), FAM-sRNA (purchased from Ribobio Biotechnology Co., Ltd.), lipid No. 41, Accuri® C6 instrument (purchased from BD, USA).

Experimental methods: PGY-sRNA-6-FAM was dissolved in 100 µl water, mixed with 4 µl lipid, and prepared by boiling method. Then the mixture was dropped into A549 cells, and after 6 hours of co-incubation, the samples were collected for detection as follows: firstly wash three times with PBS, then digest with trypsin for 3 minutes and remove trypsin, wash with PBS again and then blow down the cells. The detection was performed using Accuri® C6 instrument.

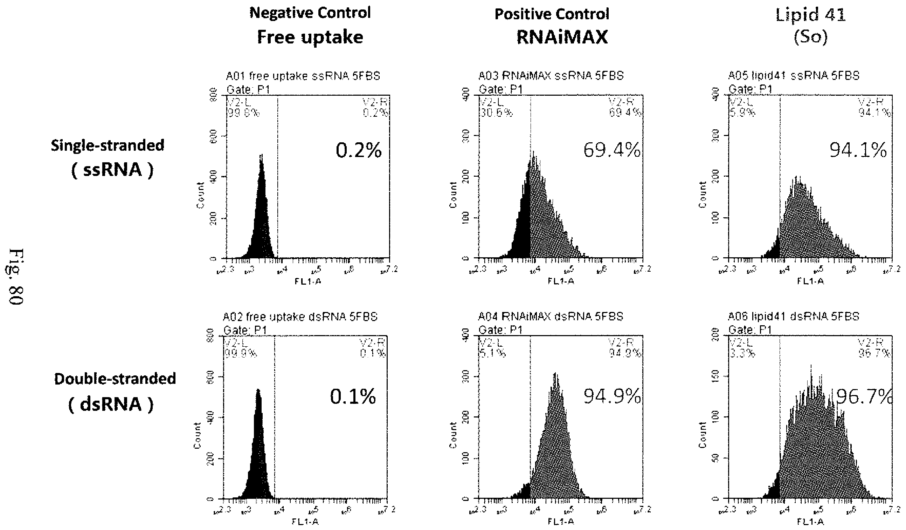

Experimental results as shown in Fig.80: lipid No. 41 had an efficiency of 94.1% in delivering PGY-sRNA-6 single-stranded RNA, which was higher than 69.4% of the positive control RNAiMAX. And lipid No. 41 had an efficiency of 96.7% in delivering PGY-sRNA-6 double-stranded RNA, which was also higher than 94.9% of the positive control RNAiMAX. Lipids 41 could efficiently deliver single-stranded and double-stranded nucleic acids into A549 cells.

### 4. Observe of the localization of the nucleic acid delivered by lipid in cells by confocal fluorescence microscopy

Experimental materials: A549 cells (purchased from the Cell Center of the Chinese Academy of Medical Sciences), PGY-sRNA-6-Cy3 (purchased from Ribobio Biotechnology Co., Ltd.), lipid No. 41, Zeiss LSM780 (purchased from Zeiss, Germany), Alexa Fluor® 488 phalloidin (purchased from Invitrogen, USA), DAPI (purchased from Invitrogen, USA), paraformaldehyde (purchased from sigma, USA).

Experimental methods: PGY-sRNA-6-FAM was dissolved in 100 µl water, mixed with 4 µl lipid, and prepared by the boiling method. Then the mixture was dropped into A549 cells, and after 6 hours of co-incubation, the samples were washed three times with PBS, fixed with 4% paraformaldehyde, washed three times with PBS, stained with Alexa Fluor® 488 phalloidin for 30 min, washed 3 times with PBS, and stained with DAPI for 5 min, washed with PBS, and then sealed.

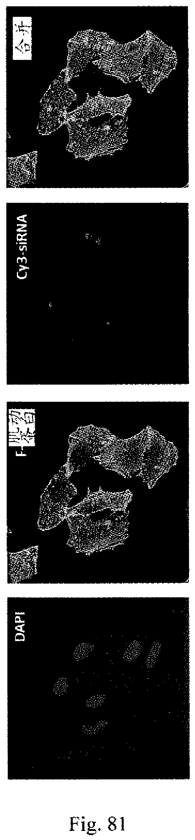

Experimental results as shown in Fig.81: the entry of red PGY-sRNA-6-Cy3 could be obviously observed under the confocal microscopy. Lipid No. 41 could effectively deliver double-stranded nucleic acid into A549 cells.

### 5. Western Blot detection of the efficiency of nucleic acid delivery by lipid

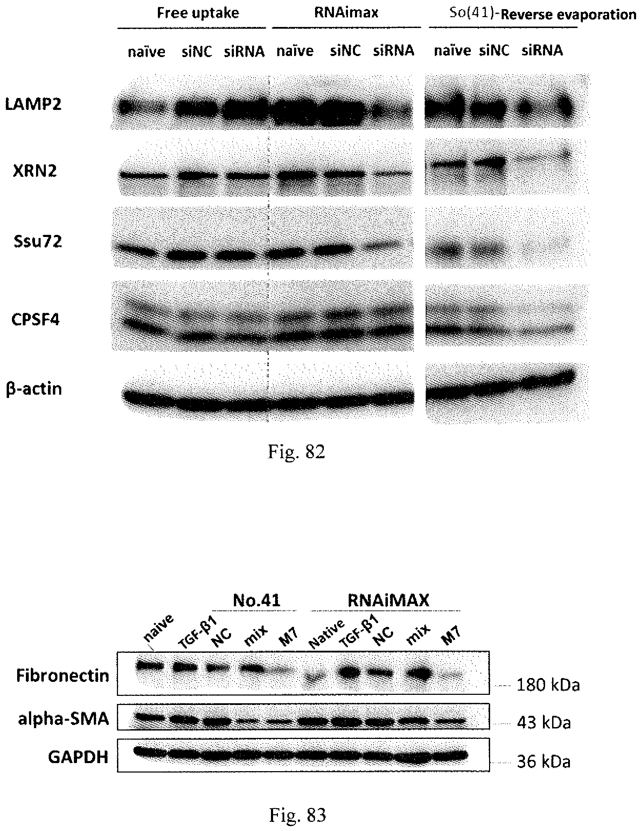

As shown in Fig.82, single lipid No. 41 mediated sRNAi entry into MRC-5A549 cells to knockdown protein expression (by reverse evaporation method). At protein level, the protein knockdown effect mediated by the single lipid No.41 was significantly higher than the inhibitory effect of HJT-sRNA-m7 mediated by RNAiMAX.
Naive group: untreated MRC-5A549 cells.
siNC group: the mixture of single lipid No. 41 and siNC was added to the cells and mixed, and the final concentration of the nucleic acid was 400 nM;
siRNA group: the mixture of single lipid No.41 and LAMP2, XPN2, Ssu72, CPSF4 or β-actin siRNA was added to the cells, mixed, and the final concentration of the nucleic acid was 400 nM;
Free uptake group: the test substance was directly added;
RNAiMAX group: 2ul RNAiMAX transfection reagent and nucleic acid solution were diluted with 100 ul opti-MEM medium, respectively, and the two were mixed, allowed to stay for 15min, added to the cells, and then mixed, and the final concentration of nucleic acid was 400nM;
So (41) group (reverse evaporation method): the mixture of lipid No. 41 and the nucleic acid was added to the cells and mixed, and the final concentration of the nucleic acid was 400 nM;

As shown in Fig.83, single lipid No. 41 mediated anti-fibrotic HJT-sRNA-m7 double-strand entry into MRC-5 cells (reverse evaporation method). At protein level, single lipid No. 41 mediated HJT-sRNA-m7 inhibition was higher than RNAiMAX mediated HJT-sRNA-m7 inhibition.
TGF β 1 group: TGF-β 1 protein (final concentration was 3 ng/mL)was added for stimulation, and the samples were collected after 72 hours;
NC group: single lipid No. 41 delivered NC mimics. After 24 hours, the cells were stimulated with TGF-β1 TGFb1 protein (final concentration was 3 ng/mL), and the samples were collected after 72 hours;
HJT-3 & a2 & H3 group: the mixture of single lipid No. 41 and HJT-sRNA-3, HJT-sRNA-a2 and HJT-sRNA-h3 were added to the cells and mixed, and the final concentration of the nucleic acid was 400 nM;
m7 group: the mixture of single lipid No. 41 and HJT-sRNA-m7 was added to the cells and mixed, and the final concentration of the nucleic acid was 400 nM;

### 6. Summary of in vivo results of lipid No. 41

[Experimental method] 6-8 weeks old mice, 22-24 g, were raised in SPF room of the Animal Center of the Institute of Basic Medical Sciences of Chinese Academy of Medical Sciences. The mice were fasted for 12 hours before intragastric administration. The mice were randomly divided into 3 groups: (1) control group, 400 µl DEPC-treated water, intragastric administration; (2) free uptake group, small RNA (PGY-sRNA-26, PGY-sRNA-32 and PGY-sRNA-23), each small RNA 1 nmol/animal, dissolved in 400 µl DEPC-treated water, intragastric administration; (3) lipid No. 41 group: a mixture of small RNA (PGY-sRNA-26 and PGY-sRNA-32) and lipid No. 41 prepared by heating method was intragastrically administered, each small RNA 1 nmol/animal, lipid No. 41 10 µl/animal, dissolved in 400 µl DEPC-treated water. All tissue and organ samples were collected after 6 hours of intragastric administration. All small RNAs were single-stranded RNA modified by 3p- terminal 2-O-methylation.

### [Experimental results]

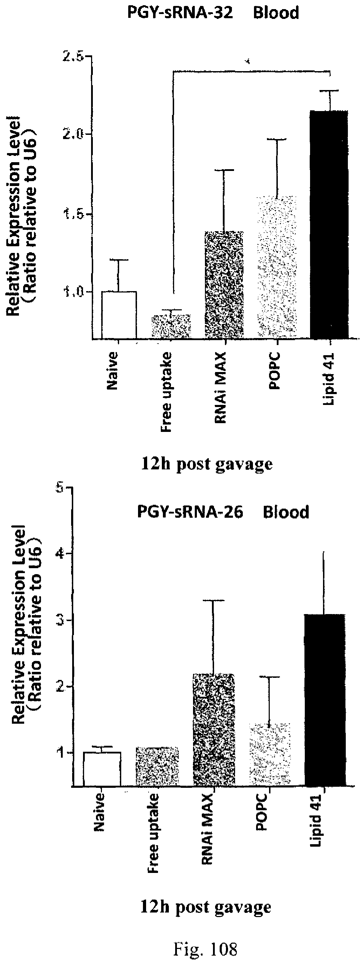

As shown in Fig. 108, lipid No. 41 could promote the entry of small RNA into the blood, protecting it from degradation in the blood.

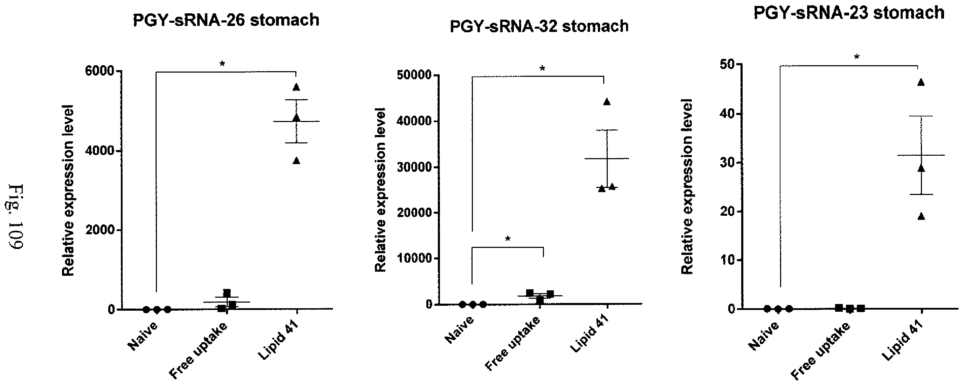

As shown in Fig. 109, lipid No. 41 could promote the entry of small RNA into the stomach cells, protecting it from degradation in the stomach.

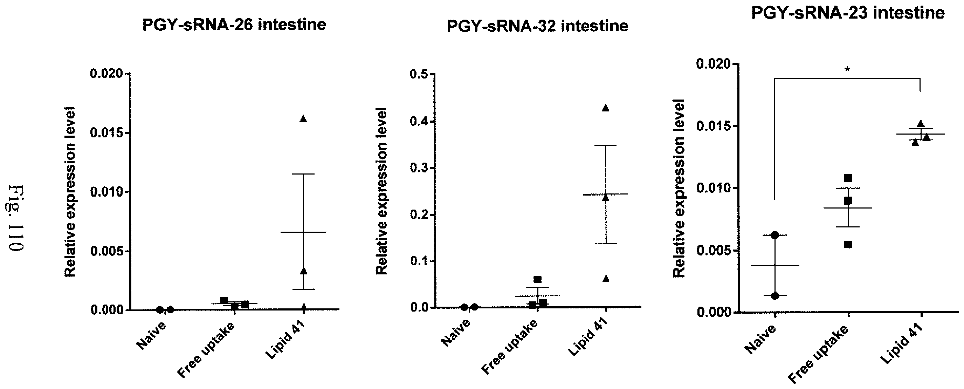

As shown in Fig.110, lipid No. 41 could promote the entry of small RNA into small intestinal cells, protecting it from degradation in the small intestine.

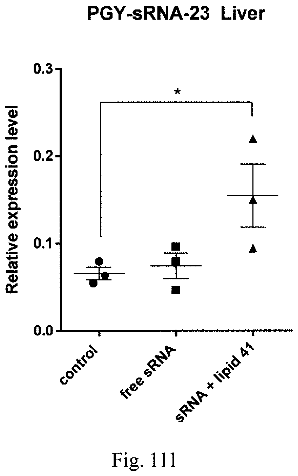

As shown in Fig.111, lipid No. 41 could promote the entry of small RNA into the liver, protecting it from degradation in the liver.

### 7. Effect of lipid combination containing lipid No. 41 on nucleic acid delivery

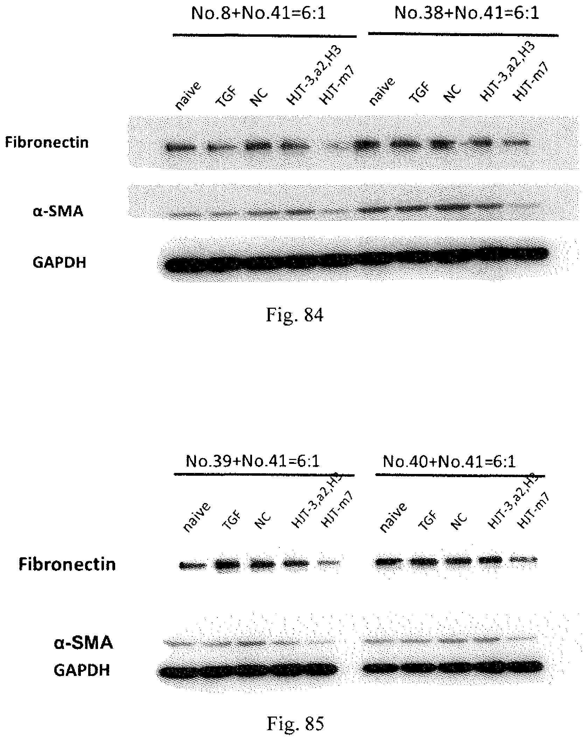

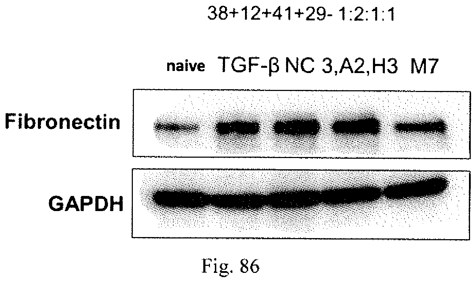

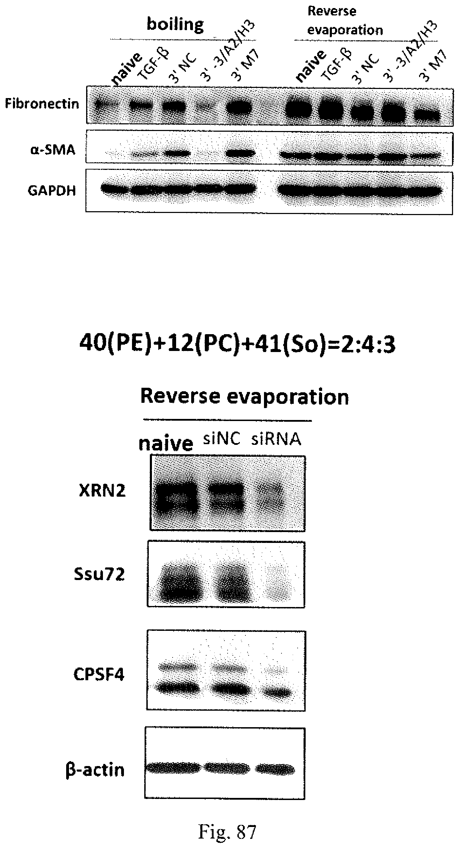

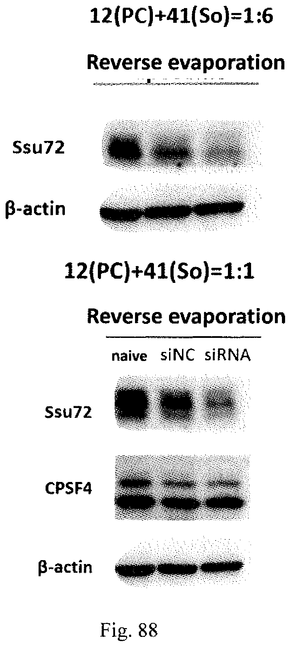

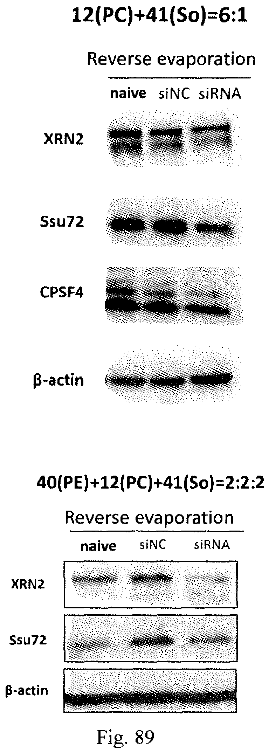

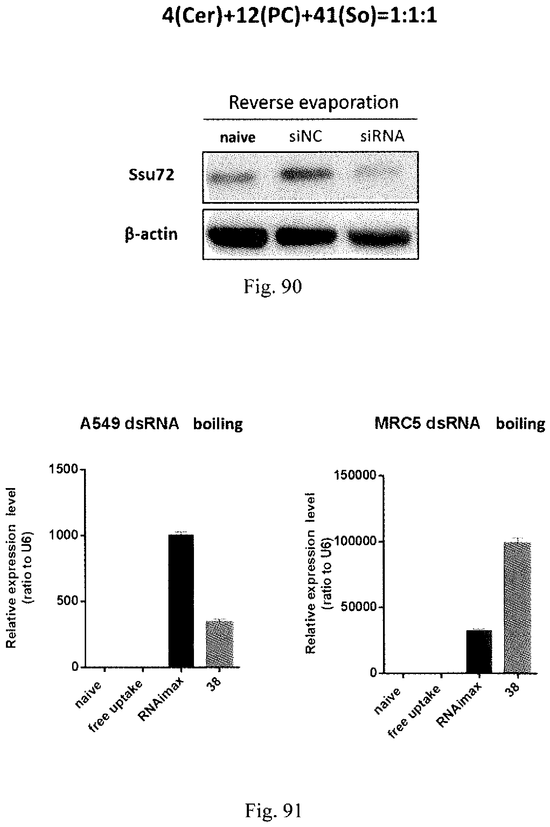

1) Effect of lipid combination 1 (No. 8+No. 41=6:1) and lipid combination 2 (No. 38+No. 41=6:1) on nucleic acid delivery.
   As shown in Fig.84, lipid combination 1 (No. 8+No. 41=6:1) and lipid combination 2 (No. 38+No. 41=6:1) mediated anti-fibrotic HJT-3 & a2 & H3, HJT-sRNA-m7 entry into MRC-5 cells (heating method), and mediated a significant inhibitory effect of the HJT-sRNA-m7 at protein level.
   TGF: TGF-β1 protein (final concentration was 3 ng/mL) was added for stimulation, and the samples were collected after 72 hours;
   NC group: single lipid No. 41 was used to deliver NC mimics. After 24 hours, TGF-β1 TGF- b 1 protein (final concentration was 3 ng/mL) was added for stimulation, and the samples were collected after 72 hours;
   HJT-3 & a2 & H3 group: the mixture of the lipid mixture with HJT-sRNA-3, HJT-sRNA-a2 and HJT-sRNA-h3 was added to the cells and mixed, and the final concentration of the nucleic acid was 400 nM;
   HJT-m7: the mixture of the lipid mixture and HJT-sRNA-m7 was added to the cells and mixed, and the final concentration of the nucleic acid was 400 nM;
2) Effects of lipid combination 3 (No. 39+No. 41=6:1) and lipid combination 4 (No. 40+No. 41=6:1) on nucleic acid delivery.
   As shown in Fig. 85, lipid combination 3 (No. 39+No. 41=6:1) and lipid combination 4 (No. 40+No. 41=6:1) mediated anti-fibrotic HJT-3 & a2 & H3, HJT-sRNA-m7 entering into MRC-5 cells (heating method), and mediated a significant inhibitory effect of HJT-sRNA-m7 at protein the level.
   TGF: TGF-β1 protein (final concentration was 3 ng/mL) was added for stimulation, and the samples were collected after 72 hours;
   NC group: lipid mix was used to deliver NC mimics. After 24 hours, the TGF-β1 protein (final concentration was 3 ng/mL) was added for stimulation, and the samples were collected after 72 hours;
   HJT-3 & a2 & H3 group: the mixture of the lipid mixture with HJT-sRNA-3, HJT-sRNA-a2 andHJT-sRNA-H3was added to the cells and mixed, and the final concentration of the nucleic acid was 400 nM;
   HJT-m7: the mixture of the lipid mixture and HJT-sRNA-m7 was added to the cells and mixed, and the final concentration of the nucleic acid was 400 nM;
3) Effect of lipid combination 5 (No. 38+12+41+29=1:2:1:1) on nucleic acid delivery.
   As shown in Fig.86, lipid combination 5 (No. 38+12+41+29=1:2:1:1) mediated anti-fibrotic HJT-3 & a2 & H3 and HJT-sRNA-m7 entering into MRC-5 cells (heating method), and mediated a significant inhibitory effect of HJT-sRNA-m7 at the protein level.
   TGF: TGF-β1 protein (final concentration was 3 ng/mL) was added for stimulation, and samples were collected after 72 hours;
   NC group: lipid mixture was used to deliver NC mimics. After 24 hours TGF-β1 protein (final concentration was 3 ng/mL) was added for stimulation, and the samples were collected after 72 hours;
   HJT-3 & a2 & H3 group: the mixture of the lipid mixture with HJT-sRNA-3, HJT-sRNA-a2 and HJT-sRNA-H3 mixture was added to the cells and mixed, and the final concentration of the nucleic acid was 400 nM;
   HJT-m7: a mixture of the lipid mixture and HJT-sRNA-m7 was added to the cells and mixed, and the final concentration of the nucleic acid was 400 nM;
4) Effect of lipid combination 6 (No. 40 (PE)+No. 12 (PC)+No. 41 (So)=2:4:3) on nucleic acid delivery.
   As shown in Fig.87, lipid combination 6 (No. 40 (PE)+No. 12 (PC)+No. 41 (So)=2:4:3) mediated anti-fibrotic HJT-3 & a2 & H3, HJT-sRNA-m7 entering into MRC-5 cells (boiling and reverse evaporation method), and mediated a significant inhibitory effect of the HJT-3 & a2 & H3, HJT-sRNA-m7 at the protein level.
   TGF: TGF-β1 protein (final concentration was 3 ng/mL) was added for stimulation, and samples were collected after 72 hours;
   3'-NC group: lipid mixture was used to deliver NC mimics, and after 24 hours TGF-β1 TGFb1 protein (final concentration was 3 ng/mL) was added for stimulation, and samples were collected after 72 hours;
   3' -3 & a2 & H3 group: the mixture of lipid mixture with HJT-sRNA-3, HJT-sRNA-a2, HJT-sRNA-H3 was added to the cells and mixed, and the final concentration of the nucleic acid was 400 nM;
   3'-m7: a mixture of lipid mixture and HJT-sRNA-m7 was added to the cells, mixed, and the final concentration of the nucleic acid was 400 nM;
   Right Figure: lipid-RNA mixture was prepared by reverse evaporation. Lipid combination 6 (No. 40 (PE)+No. 12 (PC)+No. 41 (So)=2:4:3) could effectively deliver XRN2, Ssu72, CPSF4 siRNA into A549 Cells, which significantly reduce expression levels at the protein level.
   siNC: the mixture of lipid mixture and siNC was added to the cells and mixed, and the final concentration of the nucleic acid was 400 nM;
   siRNA: the mixture of lipid mixture and XRN2, Ssu72, CPSF4 siRNA were added to the cells, mixed, and the final concentration of the nucleic acid was 400 nM;
   5) Effect of lipid combination 7 (No. 12 (PC)+No. 41 (So)=1:6) and lipid combination 8 (No. 12 (PC)+No. 41 (So)=1:1) on nucleic acid delivery.
      As shown in Fig.88, by the reverse evaporation method, lipid combination 7 (No. 12 (PC)+No. 41 (So)=1:6) and lipid combination 8 (No. 12 (PC)+No. 41 (So)=1: 1) could effectively deliver Ssu72, CPSF4 siRNA into A549 Cells, which significantly reduced the expression levels at the protein level.
      siNC: the mixture of lipid mixture and siNC was added to the cells and mixed, and the final concentration of the nucleic acid was 400 nM;
      siRNA: the mixture of lipid mixture and XRN2, Ssu72, CPSF4 siRNA was added to the cells, mixed, and the final concentration of the nucleic acid was 400 nM;
   6) Effect of lipid combination 9 (No. 12 (PC)+No. 41 (So)=6:1) and lipid combination 10 (No. 40 (PE)+No. 12 (PC)+No. 41 (So)=2:2:2) on nucleic acid delivery.
      As shown in Fig.89, by the reverse evaporation method, lipid combination 9 (No. 12 (PC)+No. 41 (So)=6:1) and lipid combination 10 (No. 40 (PE)+No. 12 (PC)+No. 41 (So)=2:2:2) could effectively deliver XRN2, Ssu72, CPSF4 siRNA into A549 Cells, which significantly reduced the expression levels at the protein level.
      siNC: the mixture of lipid mixture and siNC was added to the cells and mixed, and the final concentration of the nucleic acid was 400 nM;
      siRNA: the mixture of lipid mixture and XRN2, Ssu72, CPSF4 siRNA was added to the cells, mixed, and the final concentration of the nucleic acid was 400 nM;
   7) Effect of lipid combination 11 (No.4 (Cer)+No. 12 (PC)+No. 41 (So)=1:1:1) on nucleic acid delivery.
      As shown in Fig.90, by the reverse evaporation method, lipid combination 11 (No.4 (Cer)+No. 12 (PC)+No. 41 (So)=1:1:1) could effectively deliver Ssu72 siRNA into A549 Cells, which significantly reduced the expression levels at protein level.
      siNC: the mixture of lipid mixture and siNC was added to the cells and mixed, and the final concentration of the nucleic acid was 400 nM;
      siSsu72: the mixture of lipid mixture and Ssu72 siRNA was added to the cells, mixed, and the final concentration of the nucleic acid was 400 nM;

### Example 6: Validation of the effect of lipid No. 38 and its combination Lipid No. 38 PE (16:0/16:1)

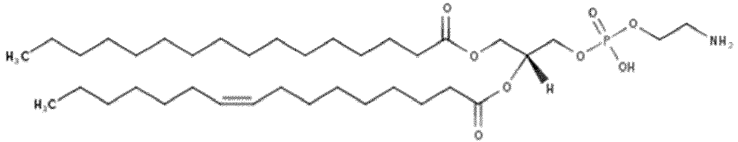

### 1. Quantitative real-time PCR (Real-Time PCR) detection of the efficiency of the nucleic acid delivery by lipid

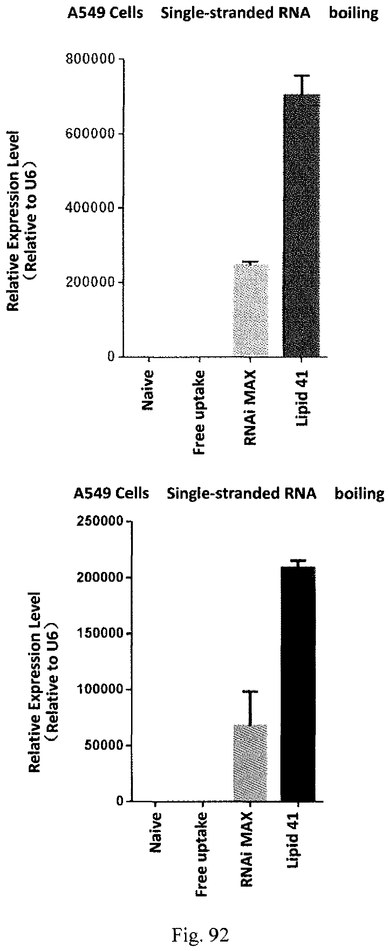

(1) Lipid No. 38 by boiling method delivered double-stranded RNA into A549 and MRC-5 cells.
   As shown in Fig.91, lipid No. 38 by heating method delivered double-stranded RNA into A549 and MRC-5 cells. For MRC-5 cells, in the case of the heating method, the delivery effect of lipid No. 38 on double-stranded RNA was about twice that of RNAiMAX.
   1) Naive group: untreated A549 cells;
   2) Free uptake group: HJT-sRNA-m7 dsRNA was directly incubated with cells for 12 hours; the final concentration of nucleic acid was 100 nM;
   3) RNAiMAX group: 2 µL RNAiMAX transfection reagent and double-stranded HJT-sRNA-m7 solution were diluted in 100 µL opti-MEM medium respectively, and then the two were mixed, allowed to stay for 15 min, added into the cells, and then mixed. The final concentration of HJT-sRNA-m7 double-strand was 100 nM;
   4) Treatment group of lipid and nucleic acid: a mixture of 2.5 µL single lipid No. 38 and HJT-sRNA-m7 double-stranded nucleic acid solution was prepared by boiling method or reverse evaporation method, and then added to A549 cells. The final concentration of RNA was 100 nM. After 12 hours, the sample was collected to detect the amount of entry.
(2) Lipid No. 38 by boiling method delivered HJT-sRNA-m7 single-stranded RNA into A549 and MRC-5 cells.
   As shown in Fig.92, lipid No. 38 by heating method delivered HJT-sRNA-m7 single-stranded RNA into A549 and MRC-5 cells, where the efficiency of delivery was much higher than that of RNAiMAX.
   1) Naive group: untreated A549 cells;
   2) Free uptake group: HJT-sRNA-m7 single stranded RNA was directly incubated with cells for 12 hours; the final concentration of nucleic acid was 100 nM;
   3) RNAiMAX group: 2 µL RNAiMAX transfection reagent and single-stranded HJT-sRNA-m7 solution were diluted in 100 µL opti-MEM medium respectively, and then the two were mixed, allowed to stay for 15 min, added into the cells, and then mixed, and the final concentration of single-stranded HJT-sRNA-m7 was 100 nM;
   4) Treatment group of lipid and nucleic acid: a mixture of 2.5 µL single lipid No. 64 and HJT-sRNA-m7 double-stranded nucleic acid solution was prepared by boiling method or reverse evaporation method, and added to A549 cells, the final concentration of RNA was 100 nM. After 12 hours, the sample was collected to detect the amount of entry.

### 2. Digital PCR (ddPCR) detection of the efficiency of nucleic acid delivery by lipid

2.1 Experimental materials: A549 cells were purchased from the Cell Center of the Institute of Basic Medical Sciences, Chinese Academy of Medical Sciences, TRIzol lysis buffer was purchased from Sigma, High capacity cRNA Reverse Transcription Kit was purchased from ABI, USA, and digital PCR related reagents were purchased from Bio-Rad.

2.2 Experimental method: total RNA was collected and extracted by TRIzol lysis buffer according to the above method, and reverse transcribed to cDNA using High capacity cRNA Reverse Transcription Kit, and the cDNA from different groups was subjected to digital PCR reaction. Refer to the QX200 Droplet Reader and QuantaSoft Software manual for the protocols; the results were analyzed using QuantaSoft software.
(1) Naive group: A549 cells without any treatment;
(2) Free uptake group: the cells were directly co-incubated with HJT-sRNA-m7 dsRNA for 6 hours;
(3) RNAiMAX group: the HJT -sRNA-m7 dsRNA was transfected into A549 cells by RNAiMAX, and the samples were collected for detection after 6 hours;
(4) No. 38 group: lipid No. 38 delivered double-stranded RNA into A549 cells by different prepration methods (boiling or evaporation method), and the samples were collected for detection after 6 hours;

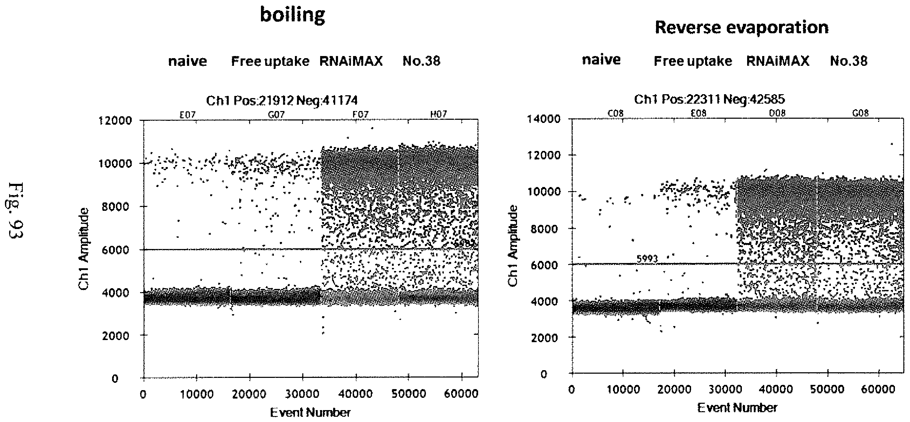

Experimental results and analysis: As shown in Fig.93, in the boiling or reverse evaporation method, lipid No. 38 could effectively deliver HJT-sRNA-m7 dsRNA into A549 cells.

### 3. Flow cytometry detection of the efficiency of nucleic acid delivery by lipid

Experimental materials: A549 cells (purchased from the Cell Center of the Chinese Academy of Medical Sciences), FAM-sRNA (purchased from Ribobio Biotechnology Co., Ltd.), lipid No. 38, Accuri® C6 instrument (purchased from BD, USA).

Experimental Method: PGY-sRNA-6-FAM was dissolved in 100 µl water, and mixed with 4 µl lipid, and prepared into lipid-sRNA mixture by boiling method. Then, the mixture was dropped into A549 cells, and after 6 hours of co-incubation, the samples were collected and washed three times with PBS, then digested with trypsin into single cells, washed with re-suspended with PBS and then blown down for Accuri® C6 instrument detection.

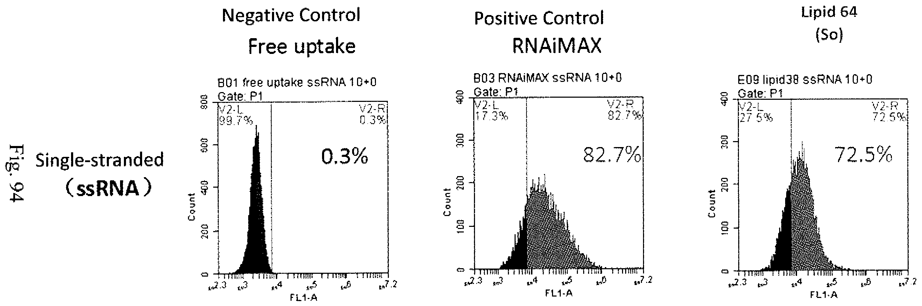

Experimental results (shown in Fig.94): lipid No. 38 delivered PGY-sRNA-6 single-stranded RNA at an efficiency of 72.5%, which was close to that of the positive control RNAiMAX.

### 4. Confocal fluorescence microscopy to observe the location of the nucleic acid delivered by lipids in cells

Experimental materials: A549 cells (purchased from the Cell Center of the Chinese Academy of Medical Sciences), PGY-sRNA-6-Cy3 (purchased from Ribobio Biotechnology Co., Ltd.), lipid No. 38, Zeiss LSM780 (purchased from Zeiss, Germany), Alexa Fluor® 488 phalloidin (purchased from Invitrogen, USA), DAPI (purchased from Invitrogen, USA), paraformaldehyde (purchased from sigma, USA).

Experimental method: PGY-sRNA-6-FAM was dissolved in 100 µl water, and mixed with 4 µl lipid, and prepared by boiling method. Then, the mixture was dropped into A549 cells, and after 6 hours of co-incubation, the samples were washed three times with PBS, fixed with 4% paraformaldehyde, washed three times with PBS, stained with Alexa Fluor® 488 phalloidin for 30 min, washed 3 times with PBS, and stained with DAPI for 5 min, PBS washed, and then sealed.

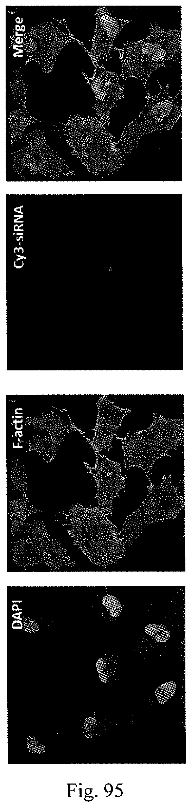

Experimental results (shown in Fig.95): the entry of red PGY-sRNA-6-Cy3 could be obviously observed under the confocal microscopy. Lipid No. 38-sRNA mixture prepared by boiling method could effectively deliver double-stranded nucleic acid into A549 cells.

### Example 7: Validation of the effect of lipid No. 64 and its composition

### Lipid No. 64 PE (15:0/24:1 (15Z))

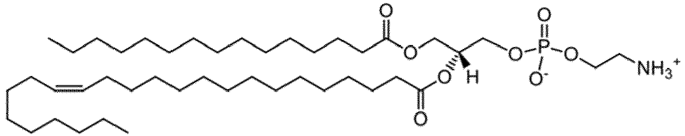

### 1. Quantitative real-time PCR (Real-Time PCR) detection of the efficiency of the nucleic acid delivery by lipid

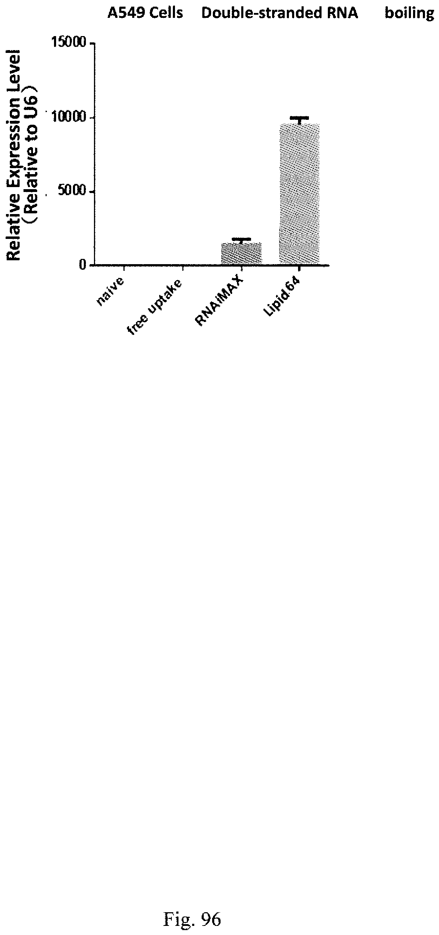

(1) Lipid No. 64 prepared by different methods (boiling or reverse evaporation method) delivered HJT-sRNA-m7 double-stranded RNA into A549 cells.
As shown in Fig.96, lipid No. 64 delivered HJT-sRNA-m7 double-stranded RNA into A549 cells by different prepration methods (boiling or reverse evaporation method). For A549 cells, in the case of the boiling method, the delivery effect of lipid No. 64 was about 3 times that of RNAiMAX.
1) Naive group: untreated A549 cells;
2) Free uptake group: HJT-sRNA-m7 dsRNA was directly incubated with cells for 12 hours; the final concentration of nucleic acid was 100 nM;
3) RNAiMAX group: 2 µL RNAiMAX transfection reagent and double-stranded HJT-sRNA-m7 solution were diluted in 100 µL opti-MEM medium respectively, mixed, and allowed to stay for 15 min, added into the cells and mixed, and the final concentration of HJT-sRNA-m7 double-strand was 100 nM;
4) Treatment group of lipid and nucleic acid: a mixture of 2.5 µL single lipid No. 64 and HJT-sRNA-m7 double-stranded nucleic acid solution was prepared by boiling method or reverse evaporation method and added to A549 cells, the final concentration of RNA was 100 nM. After 12 hours, the sample was collected to detect the amount of entry.

### 2. Flow cytometry detection of the efficiency of nucleic acid delivery by lipid

Experimental materials: A549 cells (purchased from the Cell Center of the Chinese Academy of Medical Sciences), FAM-sRNA (purchased from Ribobio Biotechnology Co., Ltd.), lipid No. 64, Accuri® C6 instrument (purchased from BD, USA).

Experimental Method: FAM-sRNA was dissolved in 100 µl water, and mixed with 4 µl lipid, prepared by boiling method. Then, the lipid-sRNA mixture was dropped into A549 cells, and after 6 hours of co-incubation, the samples were collected and washed three times with PBS, then digested into single cells with trypsin, re-suspended with PBS and then blown down for Accuri® C6 instrument detection.

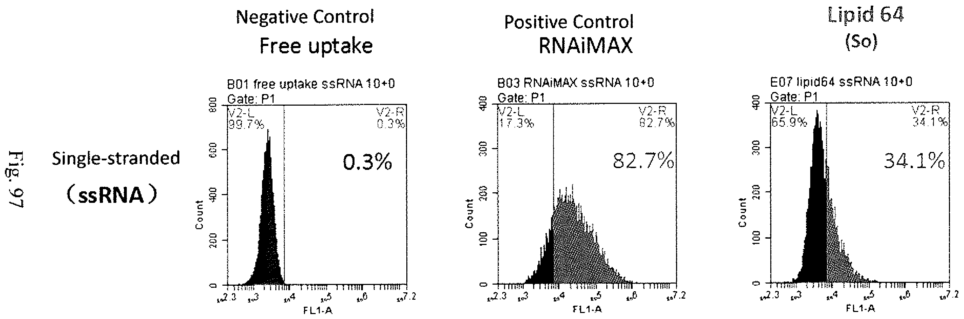

Experimental results (shown in Fig.97): lipid No. 64 deliverED PGY-sRNA-6 single-stranded RNA with an efficiency of about a half (1/2) of efficiency the positive control RNAiMAX.

### 3. Confocal fluorescence microscopy to observe the location of the nucleic acid delivered by lipids in cells

Experimental materials: A549 cells (purchased from the Cell Center of the Chinese Academy of Medical Sciences), PGY-sRNA-6-Cy3 (purchased from Ribobio Biotechnology Co., Ltd.), lipid No. 64, Zeiss LSM780 (purchased from Zeiss, Germany), Alexa Fluor® 488 phalloidin (purchased from Invitrogen, USA), DAPI (purchased from Invitrogen, USA), paraformaldehyde (purchased from sigma, USA).

Experimental method: PGY-sRNA-6-FAM was dissolved in 100 µl water, and mixed with 4 µl lipid, and prepared by boiling method. Then, the mixture was dropped into A549 cells, and after 6 hours of co-incubation, the samples were washed three times with PBS, fixed with 4% paraformaldehyde, washed three times with PBS, stained with Alexa Fluor® 488 phalloidin for 30 min, washed 3 times with PBS, and stained with DAPI for 5 min, PBS washed, and then sealed.

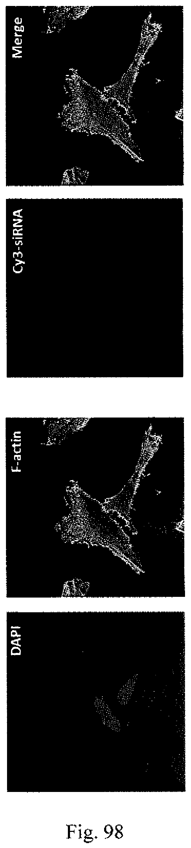

Experimental results (shown in Fig.98) the entry of red PGY-sRNA-6-Cy3 could be obviously observed under the confocal microscopy. Lipid No. 64 could effectively deliver single-stranded RNA into A549 cells.

### Example 8: Validation of the effect of lipid No. 40 and its composition Lipid No. 40 PE (16:0/22:1)

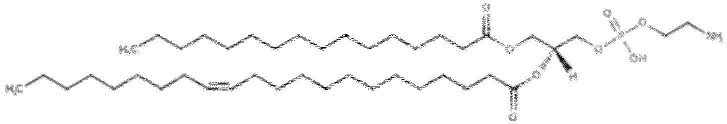

### 1. Quantitative real-time PCR (Real-Time PCR) detection of the efficiency of the nucleic acid delivery by lipid

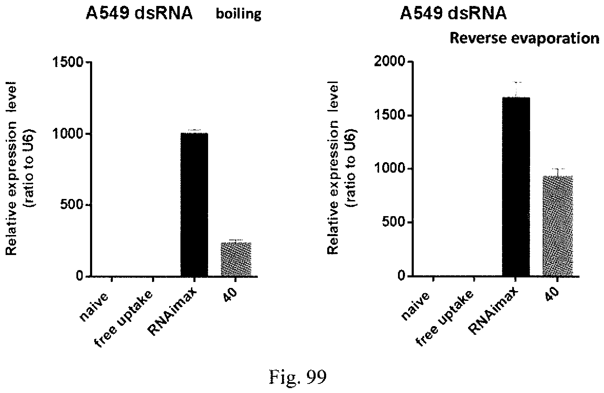

(1) Lipid No. 40 prepraed by different methods (boiling or reverse evaporation method) delivered double-stranded RNA into A549 cells.
As shown in Fig.99, lipid No. 40 by prepared by different methods (boiling or reverse evaporation method) delivered double-stranded RNA into A549 cells. For A549 cells, in the case of the reverse evaporation method, delivery effect of lipid No. 40 was about a half (1/2) of that of RNAiMAX.
1) Naive group: untreated A549 cells;
2) Free uptake group: HJT-sRNA-m7 dsRNA was directly incubated with cells for 12 hours; the final concentration of nucleic acid was 100 nM;
3) RNAiMAX group: 2 µL RNAiMAX transfection reagent and double-stranded HJT-sRNA-m7 solution were diluted in 100 µL opti-MEM medium respectively, and then the two were mixed, allowed to stay for 15 min, added into the cells, mixed, and the final concentration of HJT-sRNA-m7 double-strand was 100 nM;
4) Treatment group of lipid and nucleic acid: a mixture of 2.5 µL single lipid No. 40 and HJT-sRNA-m7 double-stranded nucleic acid solution was prepared by boiling method or reverse evaporation method, and added to A549 cells. The final concentration of RNA was 100 nM. After 12 hours, the sample was collected to detect the amount of entry.

### 2. Digital PCR (ddPCR) detection of the efficiency of nucleic acid delivery by lipid

2.1 Experimental materials: A549 cells were purchased from the Cell Center of the Institute of Basic Medical Sciences, Chinese Academy of Medical Sciences, TRIzol lysis buffer was purchased from Sigma, TaqMan™ MicroRNA Reverse Transcription KitHigh was purchased from Thermo Fisher Technology, and digital PCR related reagents were purchased from Bio-Rad.

2.2 Experimental method: Total RNA was collected and extracted by TRIzol lysis buffer according to the above method, and reverse transcribed to cDNA using TaqMan™ MicroRNA Reverse Transcription KitHigh, and the cDNA from different groups was subjected to digital PCR reaction. Refer to the QX200 Droplet Reader and QuantaSoft Software manual for the protocols; the results were analyzed using QuantaSoft software.
(1) Naive group: A549 cells without any treatment
(2) Free uptake group: the cells were directly co-incubated with HJT-sRNA-m7 dsRNA for 6 hours;
(3) RNAiMAX group: the HJT -sRNA-m7 dsRNA was transfected into A549 cells by RNAiMAX, and the samples were collected for detection after 6 hours;
(4) No. 40 group: lipid No. 40 prepared by different methods (boiling or evaporation method) delivered double-stranded RNA into A549 cells, and the samples were collected for detection after 6 hours;

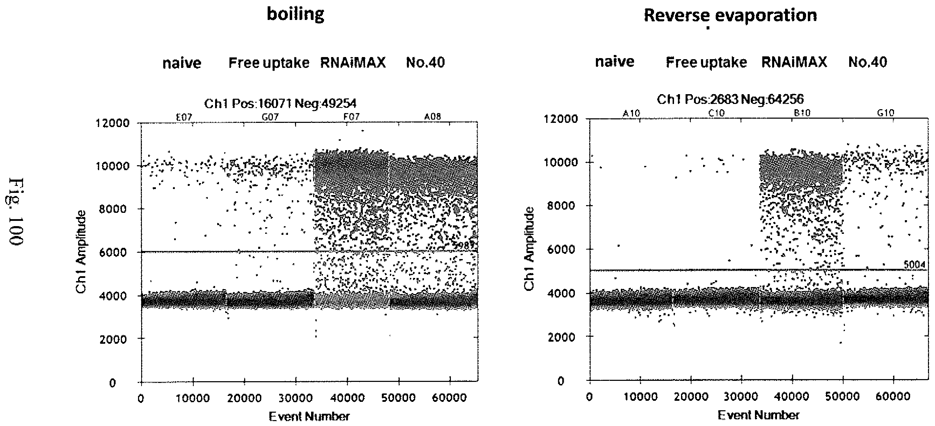

Experimental results and analysis: As shown in Fig.100, in the boiling or reverse evaporation method, lipid No. 40 could effectively deliver HJT-sRNA-m7 dsRNA into A549 cells.

### 3. Confocal fluorescence microscopy to observe the location of the nucleic acid delivered by lipids in cells

Experimental materials: A549 cells (purchased from the Cell Center of the Chinese Academy of Medical Sciences), PGY-sRNA-6-Cy3 (purchased from Ribobio Biotechnology Co., Ltd.), lipid No. 40, Zeiss LSM780 (purchased from Zeiss, Germany), Alexa Fluor® 488 phalloidin (purchased from Invitrogen, USA), DAPI (purchased from Invitrogen, USA), paraformaldehyde (purchased from sigma, USA).

Experimental method: PGY-sRNA-6-FAM was dissolved in 100 µl water, and mixed with 4 µl lipid, and prepared by boiling method. Then, the mixture was dropped into A549 cells, and after 6 hours of co-incubation, the samples were washed three times with PBS, fixed with 4% paraformaldehyde, washed three times with PBS, stained with Alexa Fluor® 488 phalloidin for 30 min, washed 3 times with PBS, and stained with DAPI for 5 min, PBS washed, and then sealed.

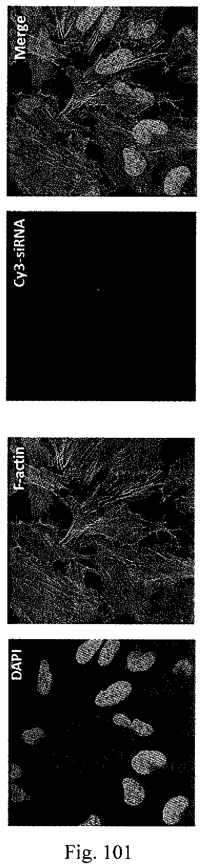

Experimental results (shown in Fig. 101): the entry of red PGY-sRNA-6-Cy3 could be obviously observed under the confocal microscopy. Lipid No. 40 could effectively deliver single-stranded RNA into A549 cells.

### 4. Western Blotting detection of the efficiency of nucleic acid delivery by lipid

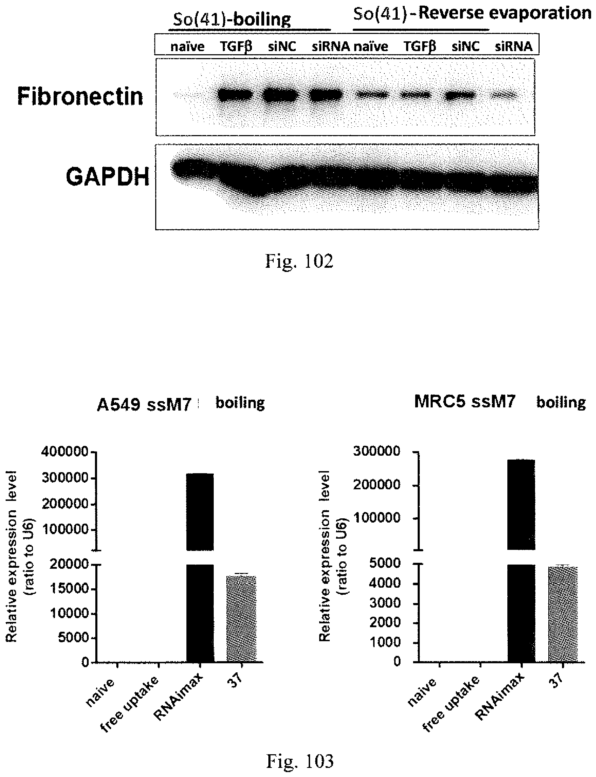

As shown in Fig.102, phosphatidylethanolamine single lipid No. 40 mediated anti-fibrotic double-stranded RNA HJT-sRNA-m7 entry into MRC-5 cells to down-regulate fibronectin protein expression.
TGF: TGF-β1 protein (final concentration was 3 ng/mL) was added for stimulation, and the samples were collected after 72 hours;
3'-NC group: lipid mixture was used to deliver NC mimics and after 24 hours, the cells were stimulated with TGF-β 1 protein (final concentration was 3 ng/mL), and the samples were collected after 72 hours;
3'-m7 group: a mixture of lipid mixture and HJT-sRNA-m7 double-stranded nucleic acid solution was added to the cells and mixed, and the final concentration of the nucleic acid was 400 nM;

### Example 8: Validation of the effect of lipid No. 37

### Lipid No. 37 LPC (18:3)

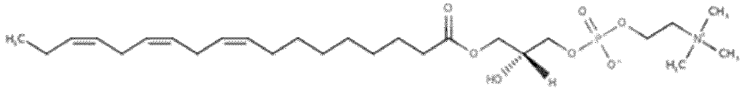

### 1. Quantitative real-time PCR (Real-Time PCR) detection of the efficiency of the nucleic acid delivery by lipid

(1) Lipid No. 37 delivered single-stranded RNA into A549 and MRC-5 cells by boiling method.
As shown in Fig.103, single-stranded RNA was delivered to A549 and MRC5 cells by boiling method.
1) Naive group: untreated A549 cells;
2) Free uptake group: HJT-sRNA-m7 dsRNA was directly incubated with cells for 3 hours; the final concentration of nucleic acid was 100 nM;
3) RNAiMAX group: 2 µL RNAiMAX transfection reagent and single-stranded HJT-sRNA-m7 solution were diluted in 100 µL opti-MEM medium respectively, mixed, and allowed to stay for 15 min, added into the cells, mixed, and the final concentration of HJT-sRNA-m7 single-strand was 100 nM;
4) Treatment group of lipid and nucleic acid: a mixture of 2.5 µL single lipid No. 39 and HJT-sRNA-m7 single-stranded nucleic acid solution was prepared by boiling method or reverse evaporation method and added to A549 cells, the final concentration of RNA was 100 nM. After 3 hours, the sample was collected to detect the amount of entry.

### Example 9: Validation of the effect of lipid No. 39

### Lipid No. 39 PE (16:1-18:1)

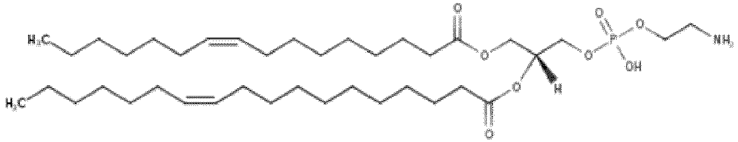

### 1. Quantitative real-time PCR (Real-Time PCR) detection of the efficiency of the nucleic acid delivery by lipid

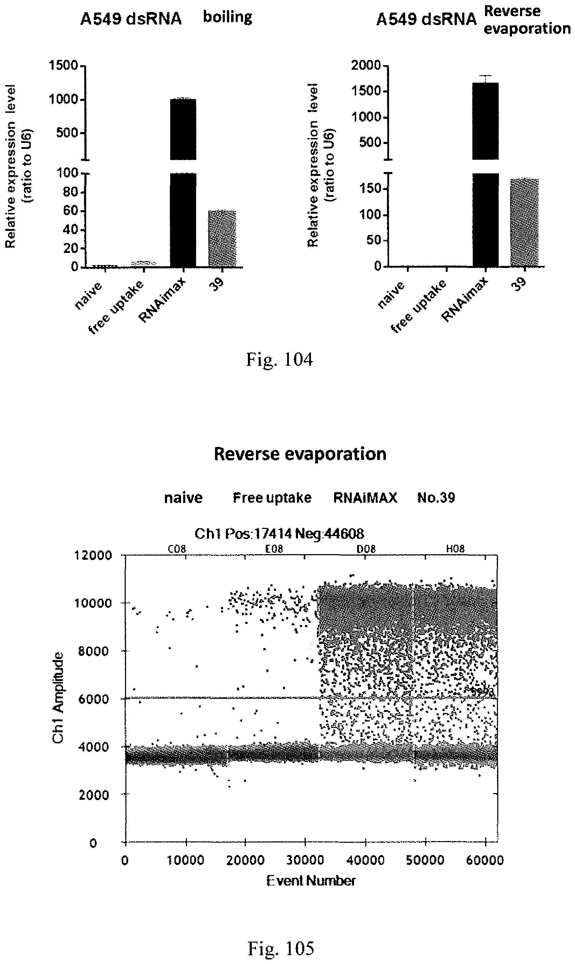

As shown in Fig.104, Lipid No. 39 prepared by different methods (boiling or reverse evaporation method) delivered double-stranded RNA into A549 cells
1) Naive group: untreated A549 cells;
2) Free uptake group: HJT-sRNA-m7 dsRNA was directly incubated with cells for 6 hours; the final concentration of nucleic acid was 100 nM;
3) RNAiMAX group: 2 µL RNAiMAX transfection reagent and double-stranded HJT-sRNA-m7 solution were diluted in 100 µL opti-MEM medium respectively, mixed, and allowed to stay for 15 min, added into the cells and mixed, and the final concentration of HJT-sRNA-m7 double-strand was 100 nM;
4) Treatment group of lipid and nucleic acid: a mixture of 2.5 µL single lipid No. 39 and HJT-sRNA-m7 double-stranded nucleic acid solution was prepared by boiling method or reverse evaporation method and added to A549 cells, the final concentration of RNA was 100 nM. After 12 hours, the sample was collected to detect the amount of entry.

### 2. Digital PCR (ddPCR) detection of the efficiency of nucleic acid delivery by lipid

2.1 Experimental materials: A549 cells were purchased from the Cell Center of the Institute of Basic Medical Sciences, Chinese Academy of Medical Sciences, TRIzol lysis buffer was purchased from Sigma, High capacity cRNA Reverse Transcription Kit was purchased from ABI, USA, and digital PCR related reagents were purchased from Bio-Rad.

2.2 Experimental method: Total RNA was collected and extracted by TRIzol lysis buffer according to the above method, and reversed to cDNA using High capacity cRNA Reverse Transcription Kit, and the cDNA from different groups was subjected to digital PCR reaction. Refer to the QX200 Droplet Reader and QuantaSoft Software manual for the protocols; the results were analyzed using QuantaSoft software.
(1) Naive group: A549 cells without any treatment;
(2) Free uptake group: the cells were directly co-incubated with HJT-sRNA-m7 dsRNA for 6 hours; 12 hours;
(3) RNAiMAX group: the HJT -sRNA-m7 dsRNA was transfected into A549 cells by RNAiMAX, and the samples were collected for detection after 6 hours, 12 hours;
(4) No. 39 group: lipid No. 39 delivered double-stranded RNA into A549 cells by reverse evaporation method, and the samples were collected for detection after 6 hours, 12 hours;

As shown in Fig.105, by the reverse evaporation method, lipid No. 39 could effectively deliver HJT-sRNA-m7 dsRNA into A549 cells.

### Example 10: Validation of the effect of lipid No. 60 and No. 612

### Lipid No. 60 dMePE (16:1/16:1)

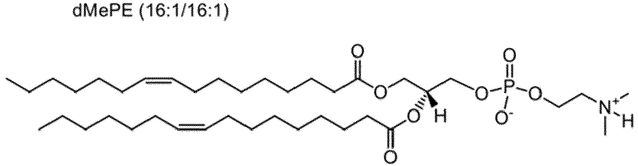

### 1. Quantitative real-time PCR (Real-Time PCR) detection of the efficiency of the nucleic acid delivery by lipid

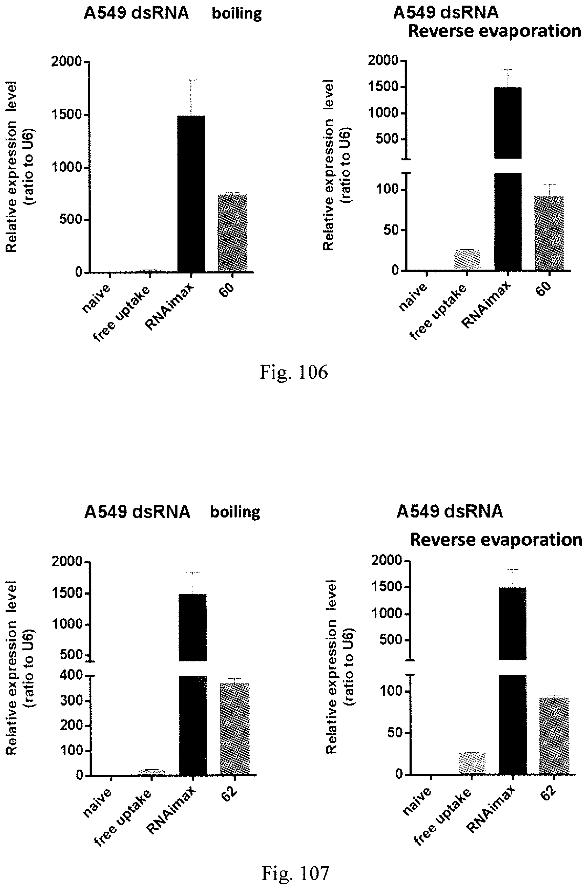

As shown in Fig.106, Lipid No. 60 prepared by different methods (boiling or reverse evaporation method) delivered double-stranded RNA into A549 cells
7) Naive group: untreated A549 cells;
8) Free uptake group: HJT-sRNA-m7 dsRNA was directly incubated with cells for 6 hours; the final concentration of nucleic acid was 100 nM;
   RNAiMAX group: 2 µL RNAiMAX transfection reagent and double-stranded HJT-sRNA-m7 solution were diluted in 100 µL opti-MEM medium respectively , and then the two were mixed, allowed to stay for 15 min, added into the cells, and then mixed, the the final concentration of double-stranded HJT-sRNA-m7 was 100 nM;
4) Lipid and nucleic acid: a mixture of 2.5 µL single lipid No. 60 and HJT-sRNA-m7 double-stranded nucleic acid solution was prepared by boiling method or reverse evaporation method and added to cells, the final concentration of RNA was 100 nM. After 12 hours, the sample was collected to detect the amount of entry.

### Lipid No. 62 dMePE (16:1/18:1)

### 1. Quantitative real-time PCR (Real-Time PCR) detection of the efficiency of the nucleic acid delivery by lipid

As shown in Fig.107, Lipid No. 62 prepared by different methods (boiling or reverse evaporation method) delivered double-stranded RNA into A549 cells
1) Naive group: untreated A549 cells;
2) Free uptake group: HJT-sRNA-m7 dsRNA was directly incubated with cells for 6 hours; the final concentration of nucleic acid was 100 nM;
3) RNAiMAX group: 2 µL RNAiMAX transfection reagent and double-stranded HJT-sRNA-m7 solution were diluted in 100 µL opti-MEM medium respectively, and then the two were mixed, allowed to stay for 15 min, added into the cells, and then mixed, and the final concentration of HJT-sRNA-m7 double-strand was 100 nM;
4) Treatment group of lipid and nucleic acid: a mixture of 2.5 µL single lipid No. 62 and HJT-sRNA-m7 double-stranded nucleic acid solution was prepared by boiling method or reverse evaporation method and added to cells, and the final concentration of RNA was 100 nM. After 12 hours, the sample was collected to detect the amount of entry.

### In vivo delivery experiment of lipid nucleic acid mixture

1. Experimental animals: C57 mice, male, approximately 6 weeks old.
2. Manufacture of lipid mixture: the prepration was conducted on the basis of a dose of 10 µl lipid-1 nmol sRNA per mouse as follows: dissolved 1 nmol of each sRNA in 500 µl DEPC water, added 10 µl of the corresponding lipid, pipette to mix thoroughly, and then naturally cooled down after water bath for 15 min at 90°C, and administered via gavage.
3. sRNA: PGY-sRNA-26, PGY-sRNA-32
4. Experimental groups:
   1) Naive group: intragastric administration of 500 µl saline;
   2) RNAiMAX treatment group: 10 µl RNAiMAX-1 nmol sRNA was mixed thoroughly and intragastrically administered to each mouse. This group served as a positive control group. RNAiMAX was purchased from Invitrogen.
   3) Free uptake group: sRNA solution (1 nmol/animal, 500 µL) was directly added, and the group served as a negative control;
   4) Treatment group of lipid nucleic acid mixture: the lipid-sRNA mixture prepared in the step 2 was intragastrically administrated.
5. Detection of the relative amont of entry:
   1) Tissue sampling and extraction of RNA: 6 hours after gavage in mice, took 500 µl of blood from the eyeball, added 1.5 ml Trizol Reagent LS to thoroughly mix and lyse, added 3 ml Trizol Reagent (purchased from Invitrogen) to the tissue samples and homogenized until complete lysis. Tissues sampled: live /stomach/small intestine.
   2) Reverse transcription of sRNA to cDNA: Reverse Transcription Kit (High-Capacity cDNA Reverse Transcription Kits, Applied Biosystems, cat. no. 4368813), was used to reverse transcribe the total RNA to cDNA, and the reverse system was as follows: template RNA (150 ng/µL) 10 µL, 10X RT buffer, 2.0 µL, 25X dNTP Mix (100 mM) 0.8 µL, random primers 2.0 µL, the MultiScribe™ reverse Transcriptase 1.0 µL, RNase inhibitor 1.0 µL, nuclease-free H₂O 3.2 µL. After a brief centrifugation, the reaction was loaded in a PCR reactor. The reaction conditions were as follows: (1) 25°C, 10 min; (2) 37°C, 120 min; (3) 85°C, 5 min; (4) 4°C, termination of the reaction. After the reaction, 20 µL RNase-free ddH₂O was added to make up the final volume to 40 µL.
   3) Quantitative PCR amplification reactions: the qPCR reaction system had a total volume of 10 µl, containing: 5 µl 2 × SYBR Green Master Mix, 0.5 µl forward primer (10 µM), 0.5 µL reverse primer, 1µl cDNA by reverse transcription, 3 µl RNase-free dH₂O. LightCycler 480 fluorescence quantitative PCR instrumentwas used, and the PCR reaction conditions were: 95 °C , 5 min for pre-denaturation, followed by the PCR amplification cycle: (1) 95°C, 10 s; (2) 55°C, 10 s; (3) 72°C, 20 s; a total of 40 cycles; 40°C for 10 s in the end to cool down. The forward primer and reverse primer of the amplification reaction were designed and synthesized by Beijing Qing Ke New Industrial Biotechnology Co., Ltd. (U6 F primer: GCGCGTCGTGAAGCGTTC, U6 R primer: GTGCAGGGTCCGAGGT).
   3) The relative expression amount was calculated by the 2-ΔCt method.

### Example 11-1: Delivery of single-stranded nucleic acids by single lipid No. 41 in vivo

1. Experimental animals: C57 mice, male, approximately 6 weeks old.
   1) Naive group: intragastric administration of 500 µl saline;
   2) RNAiMAX treatment group: 10 µl RNAiMAX-1 nmol sRNA was mixed and intragastrically administered to each mouse. This group served as a positive control group. RNAiMAX was purchased from Invitrogen.
   3) Free uptake group: single-stranded sRNA mixture solution (1nmol each) was directly added (1 nmol each);
   4) Treatment group of POPC (sigma, cat. No.: 42773) and nucleic acid mixture: a mixture of 10 µL of POPC with single-stranded sRNA mixture solution (1nmol) was treated by heating method and then given to mice via intragastric administration.
   5) Treatment group of single lipid and nucleic acid mixture: a mixture of 10 µL of single lipid (No. 41) with single-stranded sRNA mixure solution (PGY-sRNA-23, PGY-sRNA-26 and PGY-sRNA-32, 1 nmol each) was treated by heating method and then given to mice via intragastric administration.
2. 12 hours after intragastric administration, the blood was taken from the eyeball, and various tissues (liver/stomach/small intestine) was sampled. TRIzol was used for full lysis and the RNA was extracted to detect the amount of entry.

### Conclusion:

As shown in Fig.108, single PE (No. 41) could effectively deliver sRNA single-stranded nucleic acid into the mouse blood via oral administration to protect sRNA from degradation, and the delivery effect was better than POPC and Lipofectamine RNAiMAX.

As shown in Fig.109, single PE (No. 41) could effectively deliver sRNA single-stranded nucleic acid into the mouse stomach via oral administration to protect sRNA from degradation.

As shown in Fig.110, single PE (No. 41) could effectively deliver sRNA single-stranded nucleic acid into the mouse small intestine via oral administration to protect sRNA from degradation.

As shown in Fig.111, single PE (No. 41) could effectively deliver sRNA single-stranded nucleic acid into the mouse liver via oral administration to protect sRNA from degradation.

### Example 11-2: Delivery of single-stranded nucleic acids by single lipid No. 38 in vivo

1. Experimental animals: C57 mice, male, approximately 6 weeks old.
   1) Naive group: intragastric administration of 500 µl saline;
   2) RNAiMAX treatment group: 10 µl RNAiMAX-1 nmol sRNA was mixed and intragastrically administered to each mouse. This group served as a positive control group. RNAiMAX was purchased from Invitrogen.
   3) Free uptake group: single-stranded sRNA mixture solution (1nmol each) was directly added (each 1 nmol);
   4) Treatment group of POPC and nucleic acid: a mixture of 10 µL POPC and single-stranded PGY-sRNA-32 sRNA (each 1 nmol) mixture solution that was treated by heating method was given to mice by gavage.
   5) Treatment group of single lipid and nucleic acid mixture: a mixture of a 10 µL single lipid (No. 38) and single-stranded sRNA (PGY-sRNA-32) mixture solution (each 1 nmol) that was treated by heating method was given to mice by gavage.
2. 12 hours after gavage, the blood was taken from the eyeball and lysed by TRIzol to extract RNA for the dection of the amount of entry.

### Conclusion:

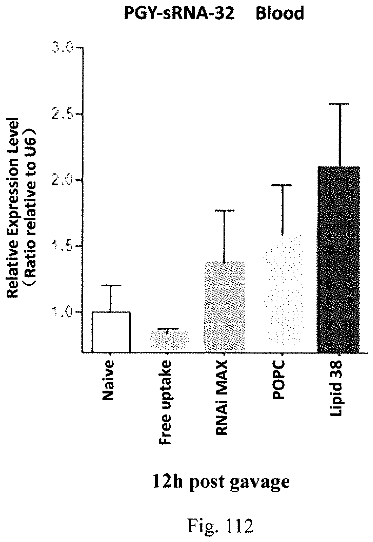

As shown in Fig.112, single PE (No. 38) could effectively deliver sRNA single-stranded nucleic acid into mouse blood via oral administration, and the delivery effect was better than POPC and Lipofectamine RNAiMAX.

### Example 11-3: Delivery of single-stranded nucleic acids by single lipid No. 40 in vivo

1. Experimental animals: C57 mice, male, approximately 6 weeks old.
   1) Naive group: intragastric administration of 500 µl saline;
   2) RNAiMAX treatment group: 10 µl RNAiMAX-1 nmol sRNA was mixed and intragastrically administered to each mouse. This group served as a positive control group. RNAiMAX was purchased from Invitrogen.
   3) Free uptake group: single-stranded sRNA mixture solution was directly added (each 1 nmol);
   4) Treatment group of POPC and nucleic acid: a mixture of 10 µL POPC and single-stranded sRNA (each 1 nmol) mixture solution that was treated by heating method was given to mice by gavage.
   5) Treatment group of single lipid and nucleic acid mixture: a mixture of a 10 µL single lipid (No. 40) and single-stranded sRNA (PGY-sRNA-32 and PGY-sRNA-26, 1nmol each) mixture solution that was treated by heating method was given to mice by gavage.
2. 12 hours after gavage, the blood was taken from the eyeball and lysed by TRIzol to extract RNA for the dection of the amount of entry.

### Conclusion:

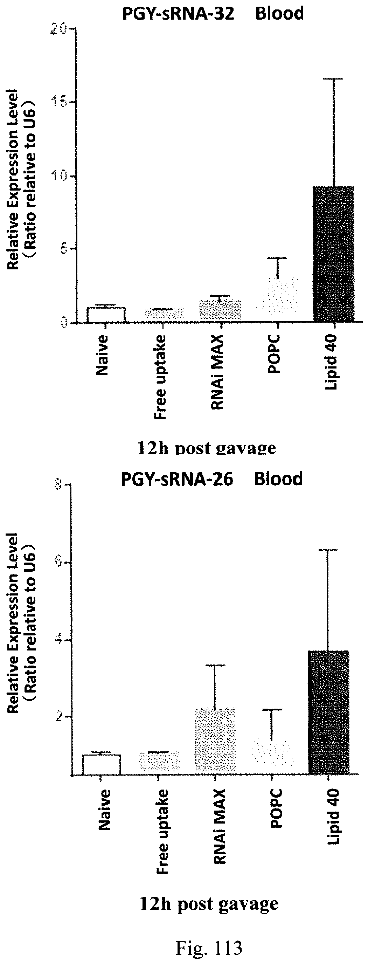

As shown in Fig.113, single PE (No. 40) could effectively deliver sRNA single-stranded nucleic acid into mouse blood via oral administration, and the delivery effect was better than POPC and Lipofectamine RNAiMAX.

### Example 11-4: Delivery of single-stranded nucleic acids by single lipid No. 64 in vivo

1. Experimental animals: C57 mice, male, approximately 6 weeks old.
   1) Naive group: intragastric administration of 500 µl saline;
   2) RNAiMAX treatment group: 10 µl RNAiMAX-1 nmol sRNA was mixed and intragastrically administered to each mouse. This group served as a positive control group. RNAiMAX was purchased from Invitrogen.
   3) Free uptake group: single-stranded sRNA mixture solution was directly added (each 1 nmol);
   4) Treatment group of POPC and nucleic acid: a mixture of 10 µL POPC and single-stranded sRNA (each 1 nmol) mixture solution that was treated by heating method was given to mice by gavage.
   5) Treatment group of single lipid and nucleic acid mixture: a mixture of a 10 µL single lipid (No. 64) and single-stranded sRNA (PGY-sRNA-32, 1nmol each) mixture solution that was treated by heating method was given to mice by gavage.
2. 12 hours after gavage, the blood was taken from the eyeball and lysed by TRIzol to extract RNA for the dection of the amount of entry.

### Conclusion:

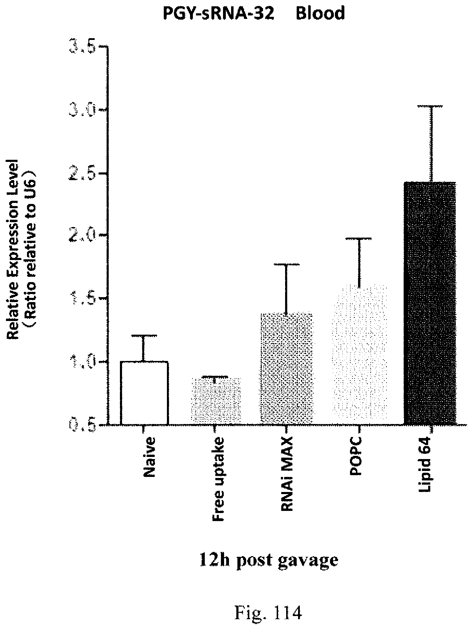

As shown in Fig.114, single PE (No. 64) could effectively deliver sRNA single-stranded nucleic acid into mouse blood via oral administration, and the delivery effect was better than POPC and Lipofectamine RNAiMAX.

### Example 11-5: Delivery of single-stranded nucleic acids by single lipid No. 71 in vivo

1. Experimental animals: C57 mice, male, approximately 6 weeks old.
   1) Naive group: intragastric administration of 500 µl saline;
   2) RNAiMAX treatment group: 10 µl RNAiMAX-1 nmol sRNA was mixed and intragastrically administered to each mouse. This group served as a positive control group. RNAiMAX was purchased from Invitrogen.
   3) Free uptake group: single-stranded sRNA mixture solution was directly added (each 1 nmol);
   4) Treatment group of POPC and nucleic acid: a mixture of 10 µL POPC and single-stranded sRNA (each 1 nmol) mixture solution that was treated by heating method was given to mice by gavage.
   5) Treatment group of single lipid and nucleic acid mixture: a mixture of a 10 µL single lipid (No. 71) and single-stranded sRNA mixture (PGY-sRNA-32, 1nmol each) solution that was treated by heating method was given to mice by gavage.
2. 12 hours after gavage, the blood was taken from the eyeball and lysed by TRIzol to extract RNA for the dection of the amount of entry.

### Conclusion:

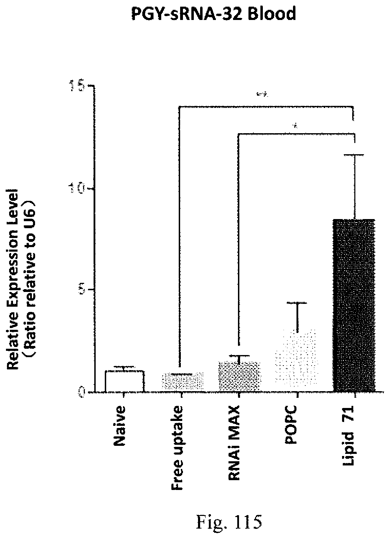

As shown in Fig.115, single PE (No. 71) could effectively deliver sRNA single-stranded nucleic acid into mouse blood via oral administration, and the delivery effect was better than POPC and Lipofectamine RNAiMAX.

### Example 12: Lipids effectively deliver single-stranded nucleic acids into MRC-5 cell at different temperature gradients

### 1. Experimental groups:

1) Naive group: untreated cells;
2) RNAiMAX treatment group: 2 µL RNAiMAX transfection reagent and single-stranded HJT-sRNA-m7 solution were diluted in 100 µL opti-MEM medium respectively, and then the two were mixed, allowed to stay for 15 min, added into the cells, and then mixed, and the final concentration of single-standed HJT-sRNA-m7 was 100 nM;
3) Treatment group of single lipid and nucleic acid mixture: mixtures of 2.5 µL single lipid (No. 38) and HJT-sRNA-m7 double-stranded nucleic acid solution that were treated by boiling method at different temperatures was added to the cells and then mixed, and the final concentration of RNA was 100 nM.

4°C: to 100 µL single-stranded HJT-sRNA-m7 solution was added 2.5 µL single lipid and placed at 4°C for 15 min; 6 hours after the addition of the cells, the expression level of HJT-sRNA-m7 in cells was detected by RT-qPCR.

37°C: to 100 µL single-stranded HJT-sRNA-m7 solution was added 2.5 µL single lipid and placed at 37°C for 15 min. 6 hours after the addition of the cells, the expression level of HJT-sRNA-m7 in cells was detected by RT-qPCR.

60°C: to 100 µL single-stranded HJT-sRNA-m7 solution was added 2.5 µL single lipid and heated at 60°C for 15 min. 6 hours after the addition of the cells, the expression level of HJT-sRNA-m7 in cells was detected by RT-qPCR.

80°C: to 100 µL single-stranded HJT-sRNA-m7 solution was added 2.5 µL single lipid and heated at 80°C for 15 min. 6 hours after the addition of the cells, the expression level of HJT-sRNA-m7 in cells was detected by RT-qPCR.

100 °C: to 100 µL HJT-sRNA-m7 single-stranded solution was added 2.5 µL single lipid and heated at 100°C for 15 min. 6 hours after the addition of the cells, the expression level of HJT-sRNA-m7 in cells was detected by RT-qPCR.

### Conclusion:

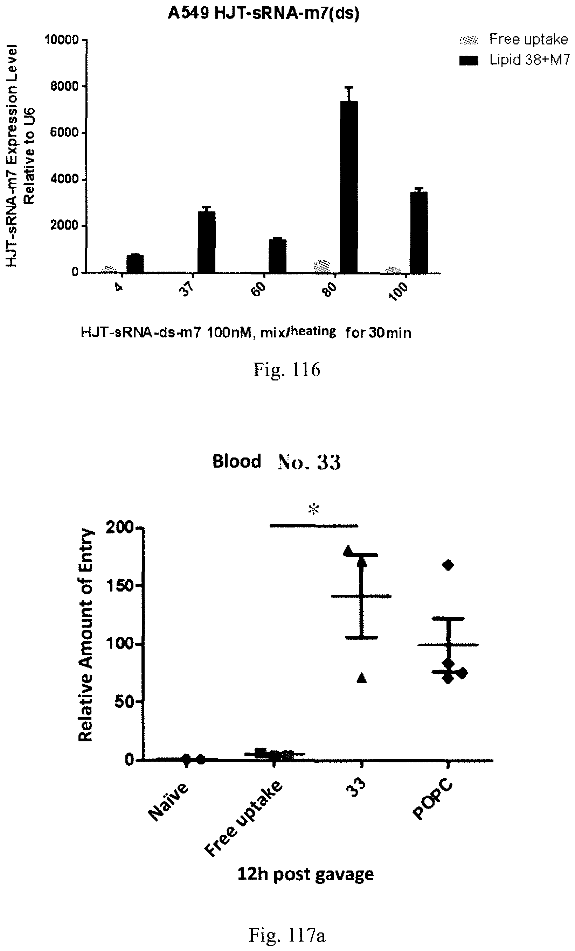

As shown in Fig.116, results showed that the lipids by the boiling method at different temperate conditions could effectively deliver nucleic acids into cells (statistically significant, p< 0.01), having the potential of improving the efficiency of the delivery of nucleic acid drug in clinical settings.

### EXAMPLE 13: In vivo delivery experiment of lipid nucleic acid mixture

### Experiment method:

1. Experiment animals: C57 mice, male, approximately 6 weeks old, 20-24g. Mice were raised in the SPF room of the Animal Center of the Institute of Basic Medical Sciences of Chinese Academy of Medical Sciences. The mice were fasted for 12 hours before intragastric administration.
2. Preparation of the mixture of lipid and small RNA: the prepration was conducted on the basis of a dose of 100µg lipid: 1nmol single stranded small RNA PGY-sRNA-26 per mouse as follows: 1nmol of each sRNA was dissolved with 500µl DEPC water in a glass tube, 100µg corresponding lipid solution No.33, No.35,No.37, No.42, No.43, No.44, No.45, No.46, No.47, No.48, No.49, No.50, No.51, No.52, No.53, No.54, No.55, No.56, No.57, No.58, No.59, No.60, No.61, No.62, No.65, No.66, No.67, No.68, No.69 or No.70 (lipid chloroform solution with a concentration as shown in Table 1-1) was added, pipetted to mix thoroughly. The solution was heated in water bath at 90 °C for 15 min, naturally cooled down, and was administered via gavage.

### 4. Experimental groups:

1) Naive group: intragastric administration of 500 µl saline;
2) Free takeup group: intragastric administration of sRNA solution directly (1nmol/animal, 500 µL). This group served as a negative control.
3) Lipid group: instragastric administration of the lipid-sRNA mixture prepared in step 2.
4) Treatment group of POPC (sigma, cat. No. 42773) and nucleic acid: a mixture of 10 µL POPC and single-stranded sRNA (each 1 nmol) mixture solution that was treated by heating method was given to mice by gavage.

### 5. Detection of the relative amont of entry:

1) Tissue sampling and extraction of RNA: 6 hours after gavage in mice, 500 µl of blood was taken from the eyeball, and was added 1.5 ml Trizol Reagent LS to thoroughly mix and lyse. Tissue sampling: the mouse was sarcrified by breaking its neck, and the thoracic and abdominal cavities of the mouse was exposed to remove the heart/whole spleen/ two kidneys/intestine (5 cm of upper end), which was immediately added 2 ml Trizol Reagent (purchased from Invitrogen) and homogenized until complete lysis with a tissue homogenizer.
2) Reverse transcription of sRNA to cDNA: Reverse Transcription Kit (High-Capacity cDNA Reverse Transcription Kits, Applied Biosystems, cat. no. 4368813), was used to reverse transcribe the total RNA to cDNA, and the reverse system was as follows: template RNA (150 ng/µL) 10 µL, 10X RT buffer, 2.0 µL, 25X dNTP Mix (100 mM) 0.8 µL, random primers 2.0 µL, the MultiScribe™ reverse Transcriptase 1.0 µL, RNase inhibitor 1.0 µL, nuclease-free H₂O 3.2 µL. After a brief centrifugation, the reaction was loaded in a PCR reactor. The reaction conditions were as follows: (1) 25 °C, 10 min; (2) 37°C, 120 min; (3) 85 °C, 5 min; (4) 4°C, termination of the reaction. After the reaction, 20 µL RNase-free ddH₂O was added to make up the final volume to 40 µL.
3) Quantitative PCR amplification reactions: the qPCR reaction system had a total volume of 10 µl, containing: 5 µl 2 × SYBR Green Master Mix, 0.5 µl forward primer (10 µM), 0.5 µL reverse primer, 1µl cDNA by reverse transcription, 3 µl RNase-free dH₂O. LightCycler 480 fluorescence quantitative PCR instrument was used, and the PCR reaction conditions were: 95 °C , 5 min for pre-denaturation, followed by the PCR amplification cycle: (1) 95°C, 10 s; (2) 55°C, 10 s; (3) 72°C, 20 s; a total of 40 cycles; 40°C for 10 s in the end to cool down. The forward primer and reverse primer of the amplification reaction were designed and synthesized by Beijing Qing Ke New Industrial Biotechnology Co., Ltd. (U6 F primer: GTGCAGGGTCCGAGGT, PGY-sRNA-26F primer: TCGCGCTCCGGAATGATTGGG, reverse primer miR all rev: GTGCACGCTCCGAGGT).
3) The relative expression amount was calculated by the 2-ΔCt method.

The data were represented in the form of mean±SEM. The parameter difference between the experimental group and the control group was evaluated by unpaired t test. The data were statistically analyzed by GraphPad Prism software, and P<0.05 was considered statistically significant.

### Conclusion

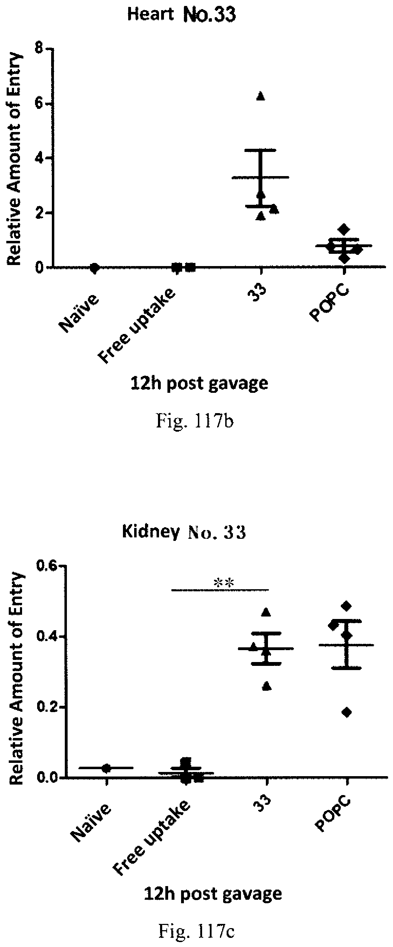

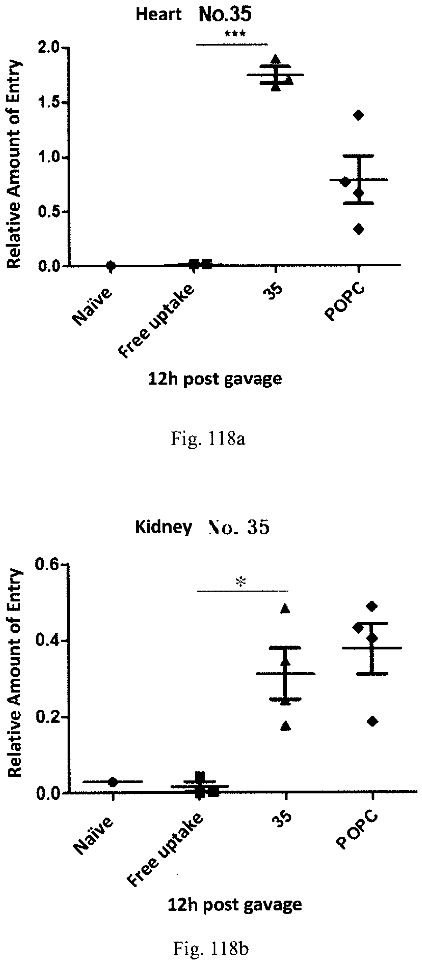

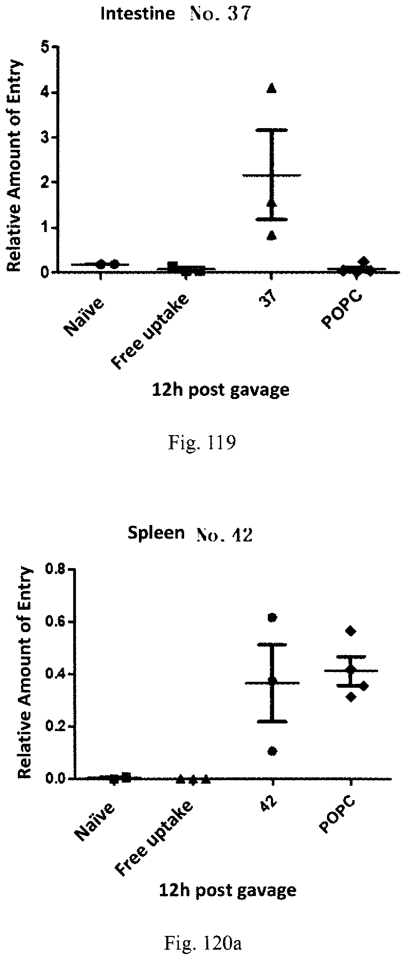

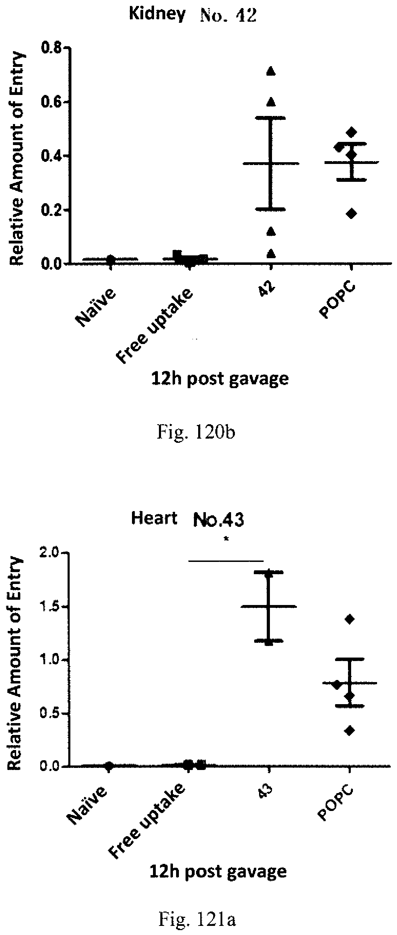

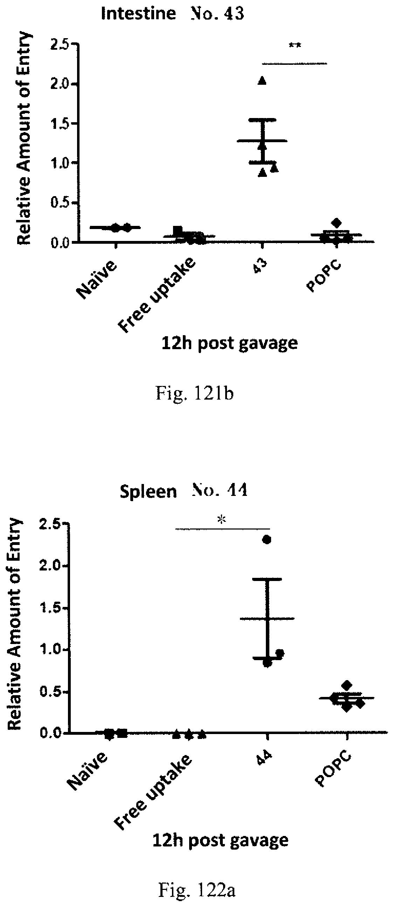

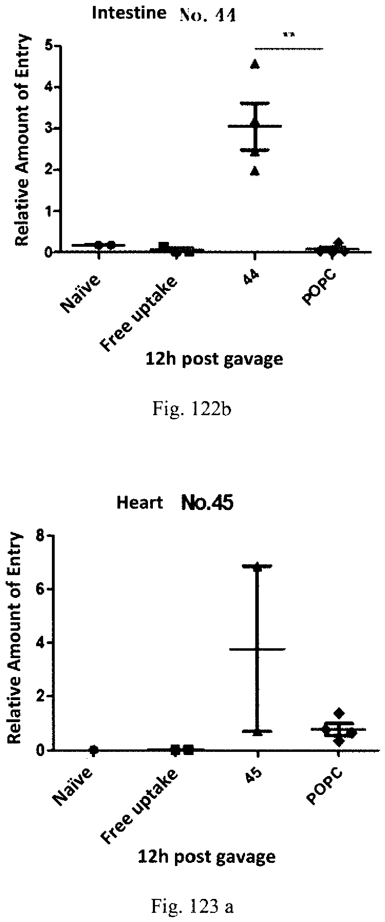

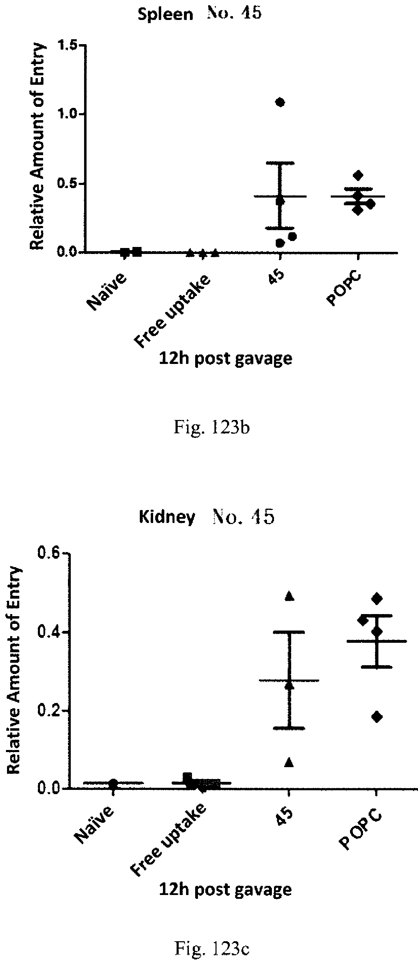

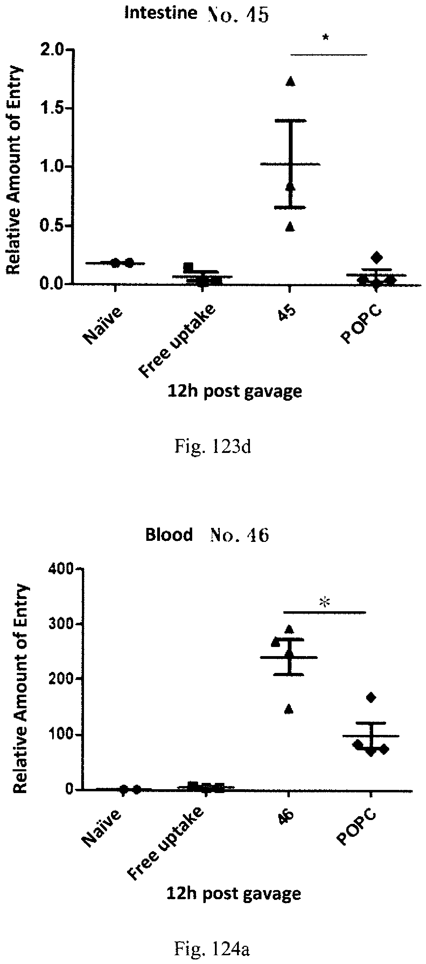

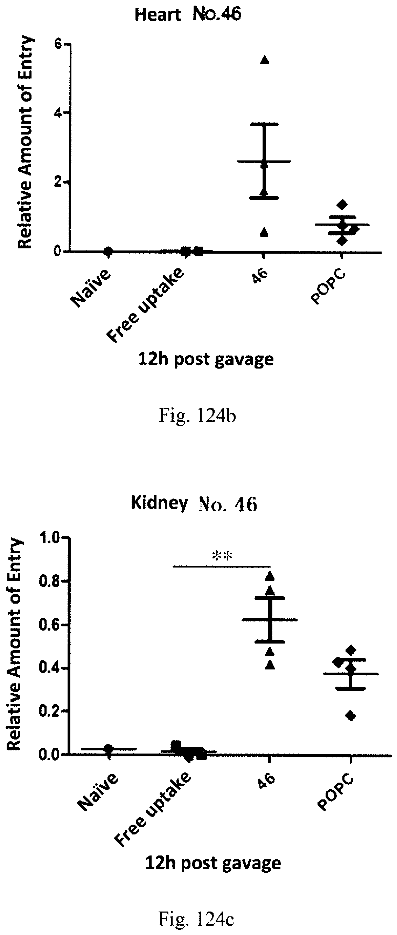

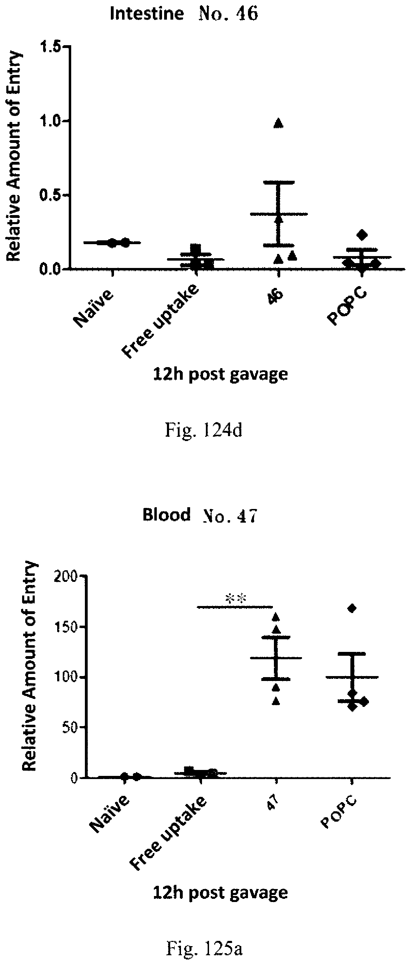

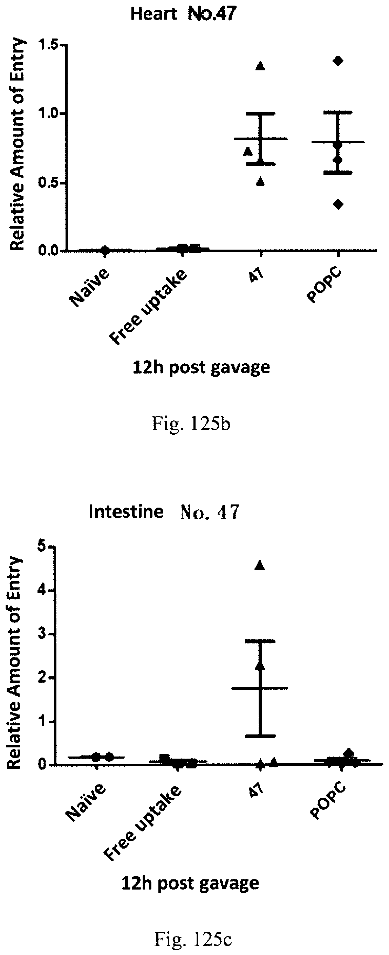

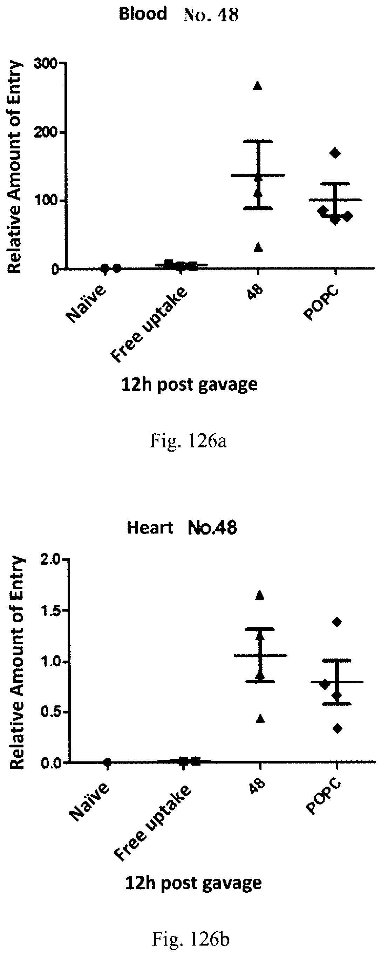

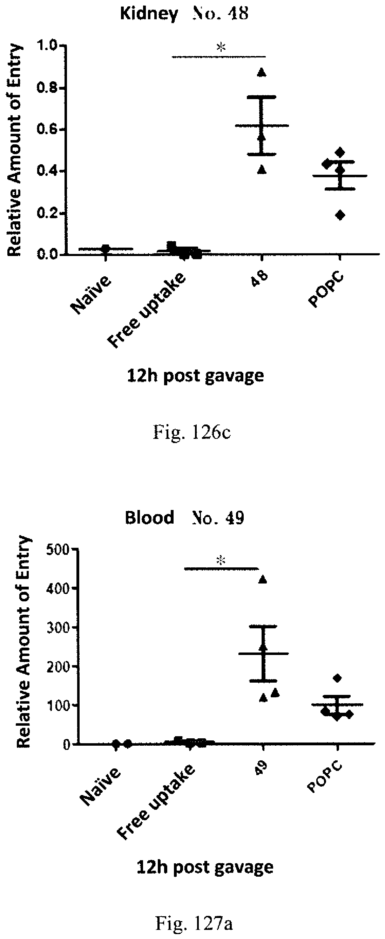

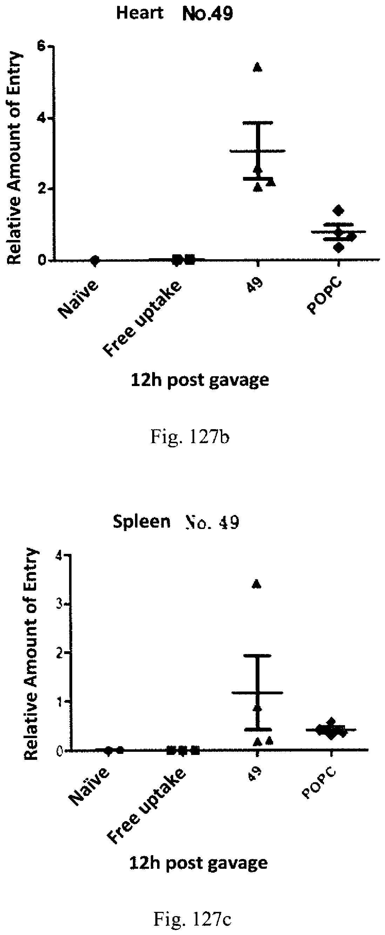

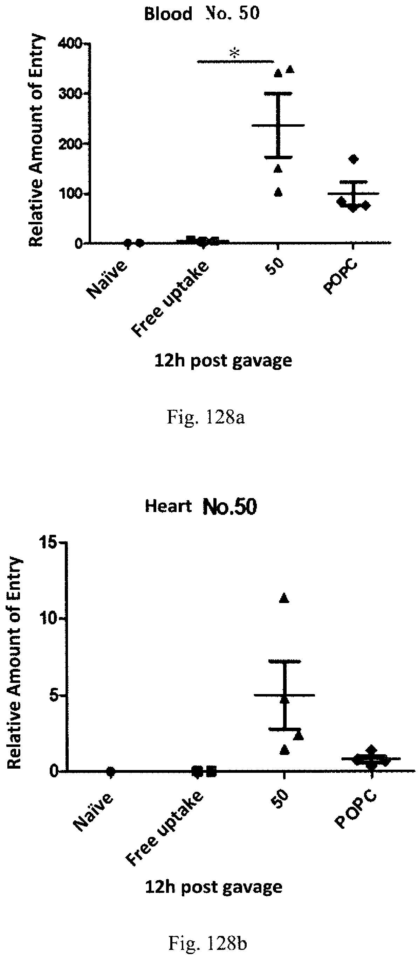

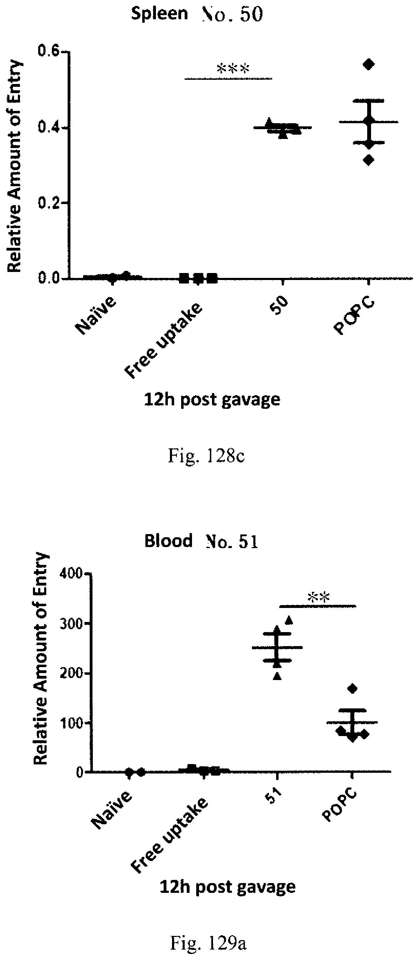

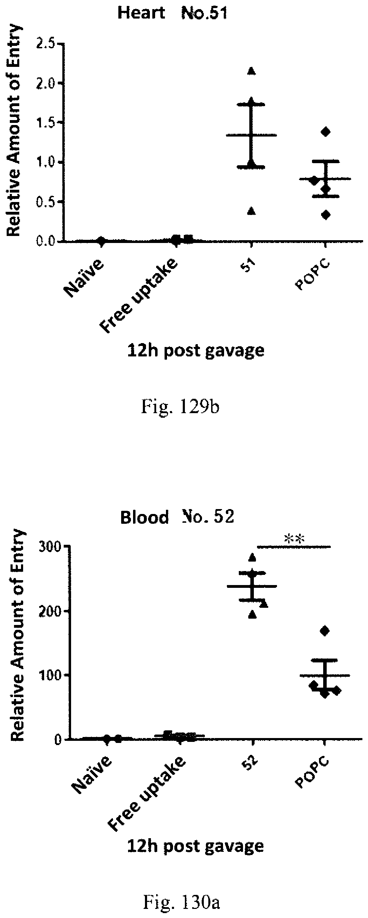

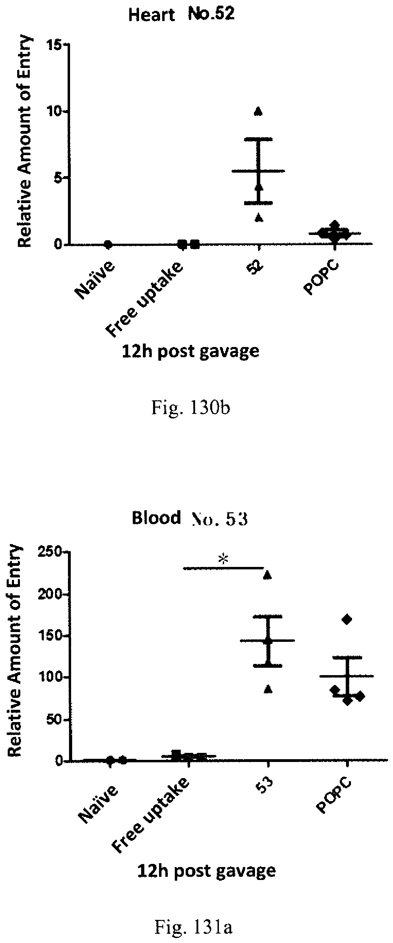

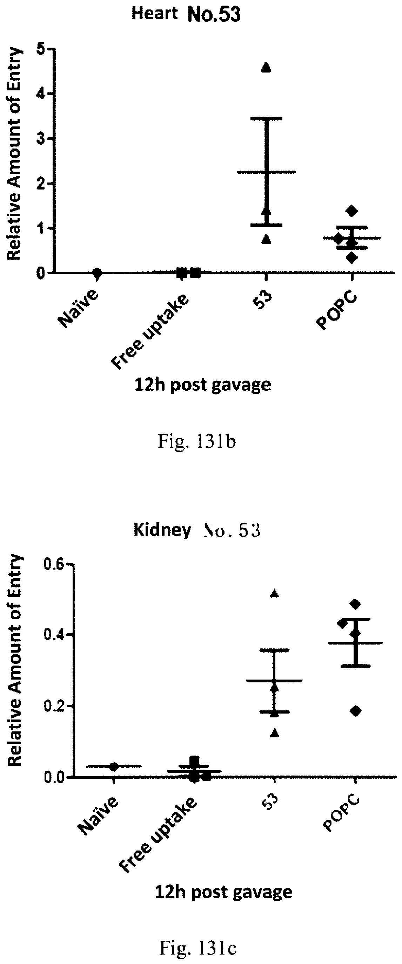

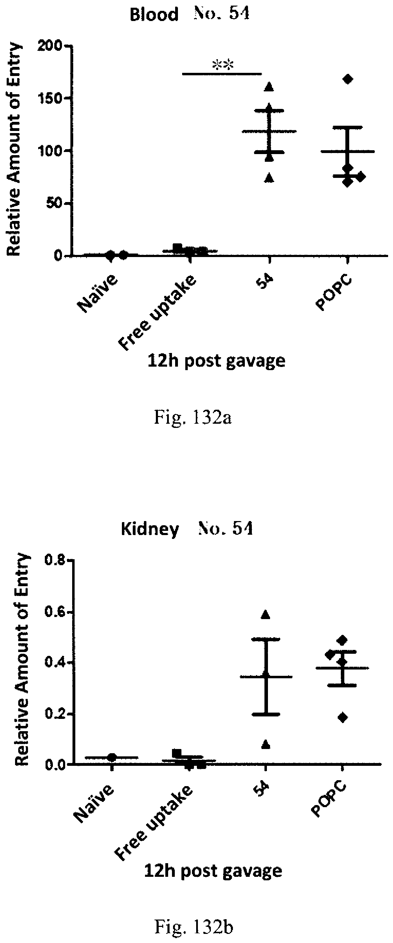

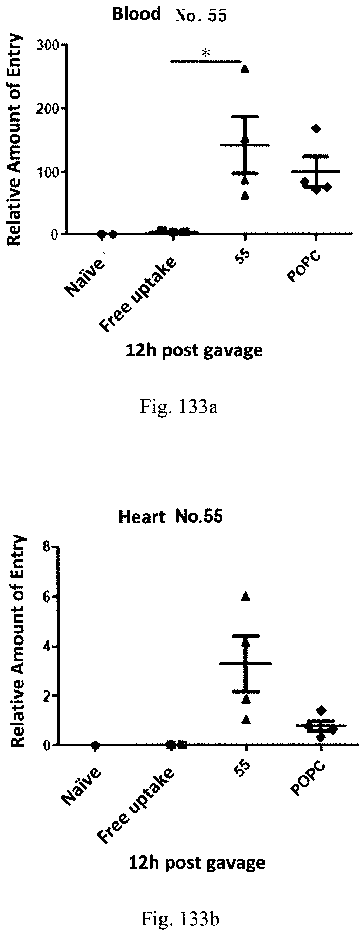

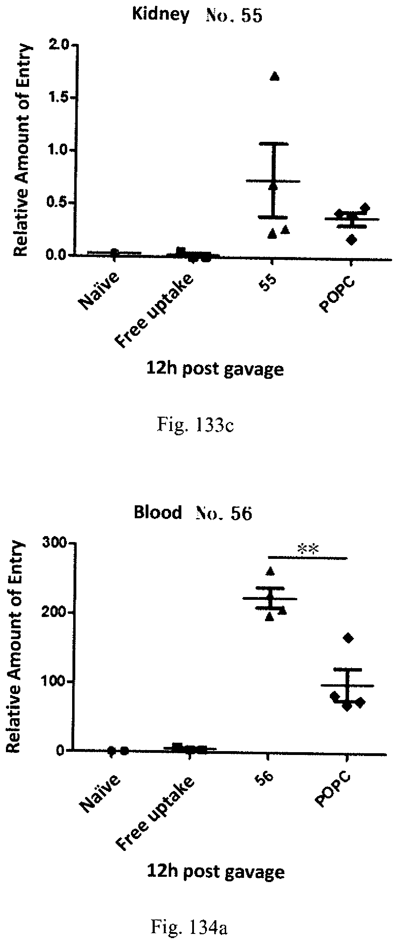

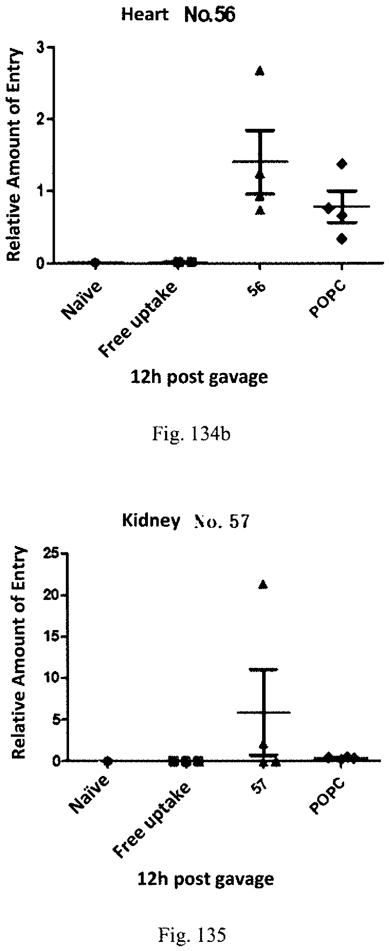

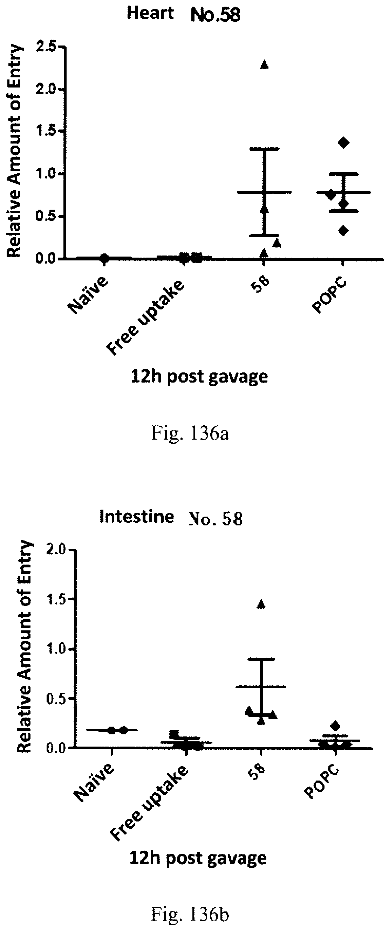

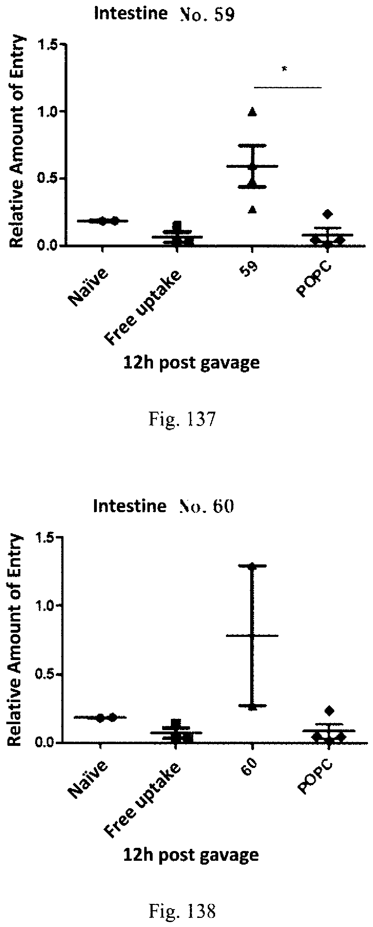

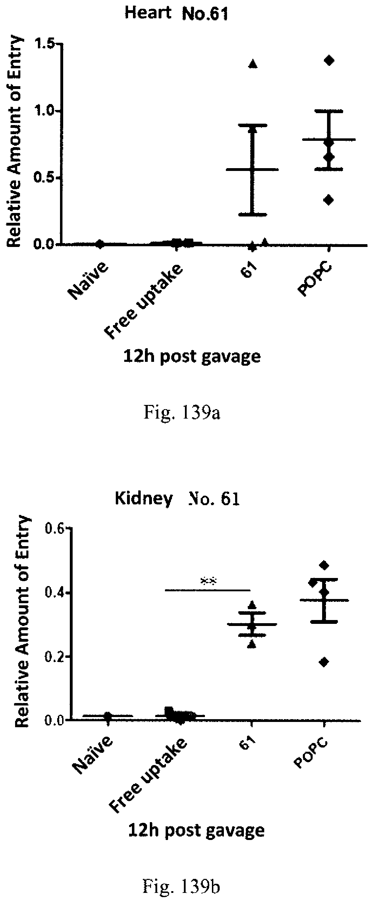

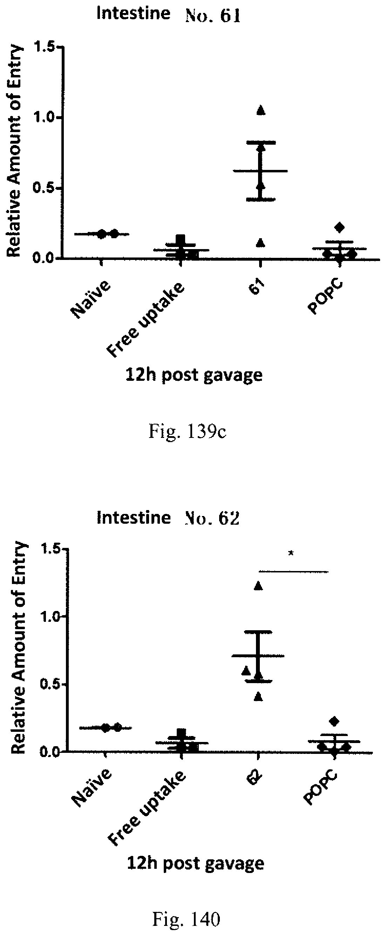

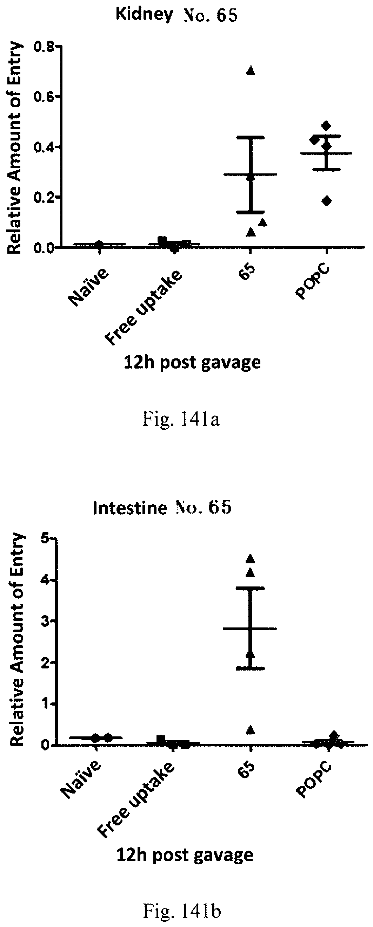

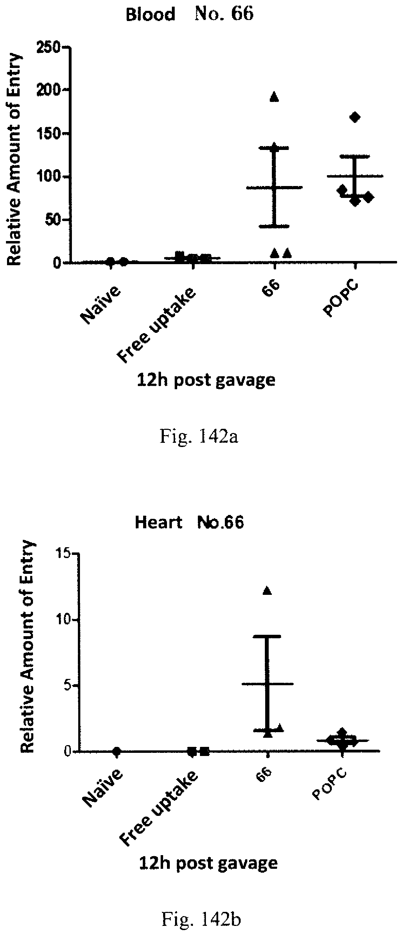

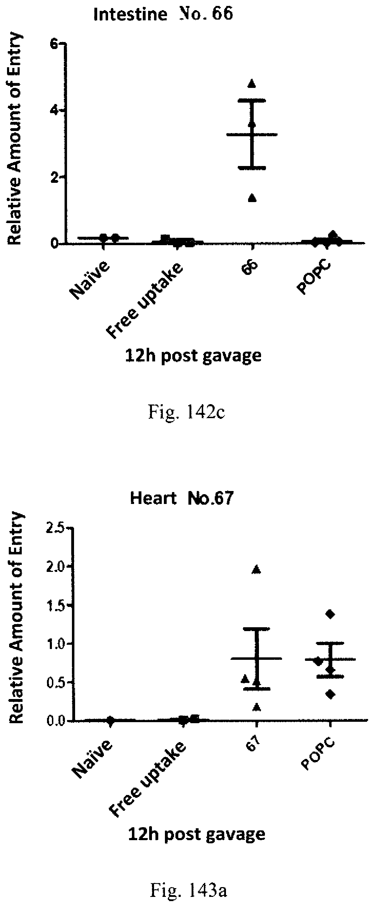

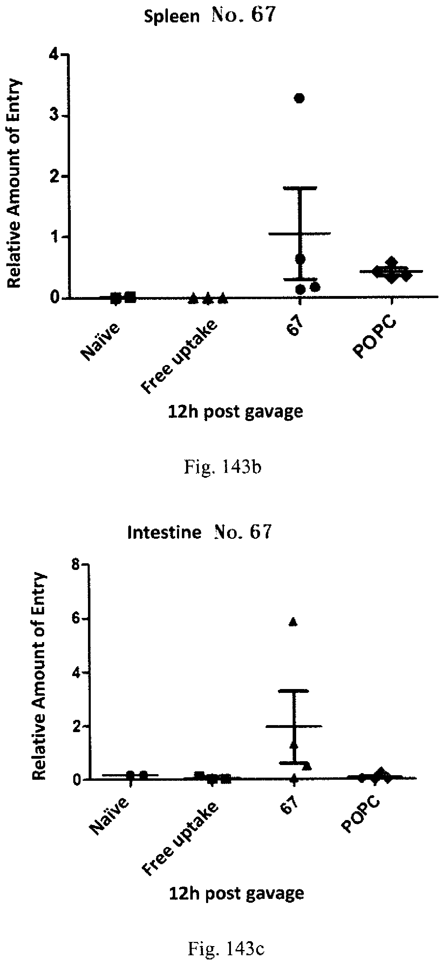

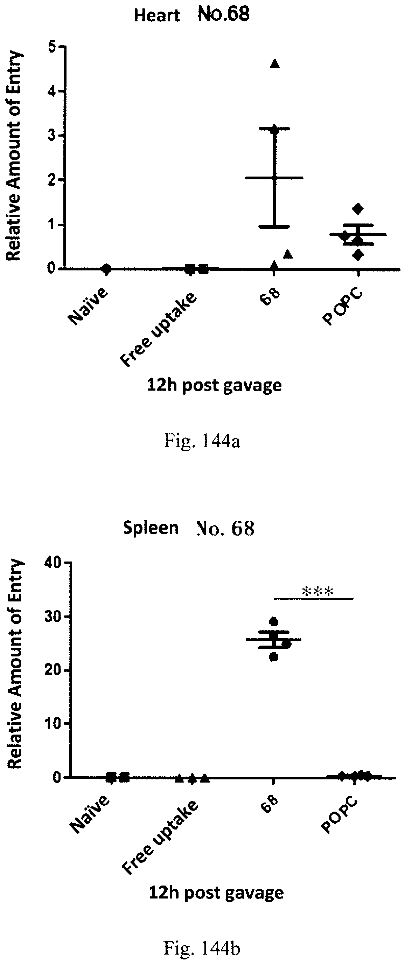

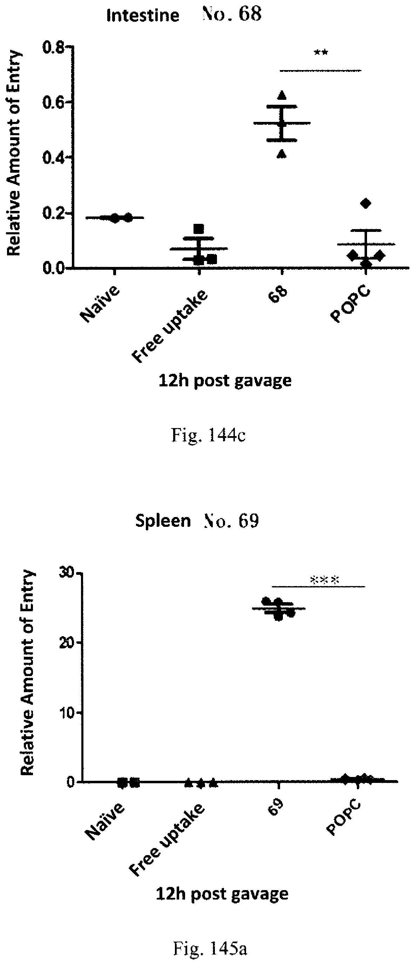

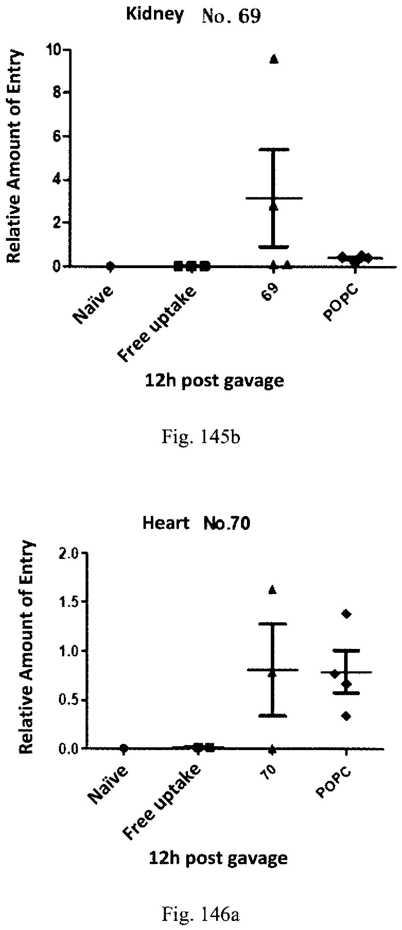

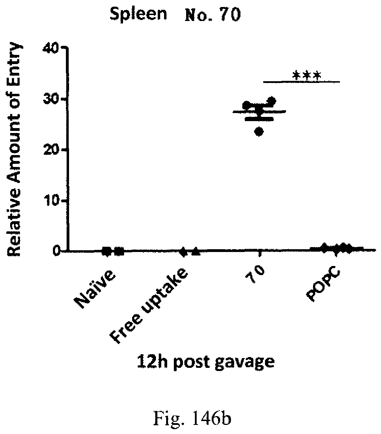

As shown in Fig.117a-146b, single lipid No.33, No.35,No.37, No.42, No.43, No.44, No.45, No.46, No.47, No.48, No.49, No.50, No.51, No.52, No.53, No.54, No.55, No.56, No.57, No.58, No.59, No.60, No.61, No.62, No.65, No.66, No.67, No.68, No.69 or No.70 could effectively deliver sRNA single-stranded nucleic acid into mouse blood and tissues such as heart, spleen, kidney or intestine and the like via oral administration, with the delivery effect significantly better than or equivalent to that of POPC.

## Claims

1. Use of a compound derived naturally (including a traditional Chinese medicine extract) or synthetically having the following formula for the manufacture of a nucleic acid delivery reagent, wherein the extract has the structure of the following formula or comprises a compound having the structure of the following formula: 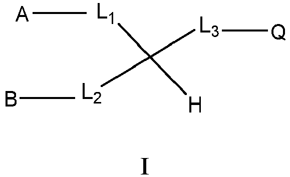
wherein L1, L2, or L3 is absent, or L1, L2, and L3 are each independently selected from the group consisting of -C(O)O-CH2-, -CH(OH)-, -C(O)-NH-CH2-, -CH2-OC(O)-, -CH2-NH-C(O)-, -C(O)O-, -C(O)NH-, -OC(O)-, -NH-C(O)-, -CH2-, 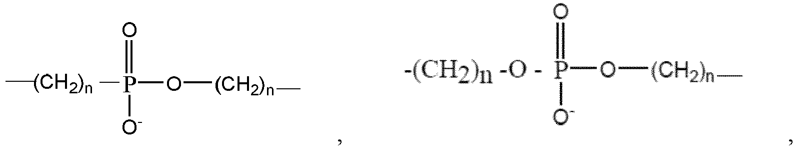 and 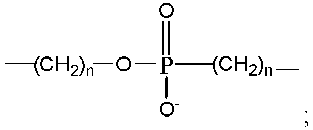 with the proviso that at most two of L₁, L₂ and L₃ are absent;
with respect to the divalent groups L₁ and L₂, the dash "-" on the left side is linked to the groups A and B, respectively, and the dash "-" on the right side is linked to the central carbon atom;
with respect to the divalent group L₃, the dash "-" on the left side is linked to the central carbon atom, and the dash "-" on the right side is linked to the group Q;
A, B and Q are each independently selected from the group consisting of H, -OH, C₁₋₂₀ alkyl, C₁₋₂₀ alkenyl, C₁₋₂₀ heteroalkyl, C₁₋₂₀ heteroalkenyl, -NH₂, and -NR₃⁺, R is H or C₁₋₆ alkyl; and
n is an integer 0, 1, 2, 3 or 4;
wherein preferably, the nucleic acid is a small nucleic acid, preferably is single stranded or double stranded, preferably the length of the small nucleic acid is 14-32 bp, 16-28 bp or 18-24 bp;
preferably, the traditional Chinese medicine is selected from the group consisting of decoction pieces of Rhodiola crenulata, Taraxacum mongolicum, Andrographis paniculata and Lonicera japonica, preferably the extract is obtained by extracting a lipid-soluble component by the Bligh & Dyer method, and more preferably by soaking the Chinese medicine decoction pieces in water, and then sequentially performing intense heating and slow heating, and the heated Chinese medicine soup is concentrated, and then is sequentially added with chloroform-methanol, chloroform and water for stirring, and the chloroform layer is obtained;
preferably, the reagent is an oral reagent; preferably, the nucleic acid is used for treating a disease, for example, heart, spleen, kidney, gastric, lung and/or intestinal diseases, such as cancer, e.g., gastric cancer or lung cancer;
preferably, the nucleic delivery is a nucleic delivery to cells or organs, preferably, the organs are selected from the group consisting of heart, spleen, kidney, stomach, lung and intestine.

2. The use of claim 1, wherein in said structure
L1 is absent, or L1 is selected from -C(O)O-CH2- and -CH(OH)-,
L2 is absent, or L2 is selected from -C(O)O- and -C(O)NH-,
L3 is absent, or L3 is selected from the group consisting of -C(O)O-, -CH2-OC(O)-, -CH₂- and 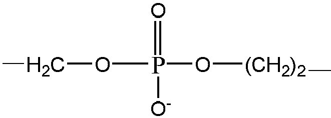 A is selected from the group consisting of H, C₁₋₂₀ alkyl and C₁₋₂₀ alkenyl;
B is selected from the group consisting of H, -NH₂, C₁₋₂₀ alkyl and C₁₋₂₀ alkenyl;
Q is selected from the group consisting of H, -OH, C₁₋₂₀ alkyl and C₁₋₂₀ alkenyl, and -NR₃⁺, wherein R is H or C₁₋₆ alkyl.

3. The use of claim 1 or 2, wherein the said compound has a structure of the following formula:

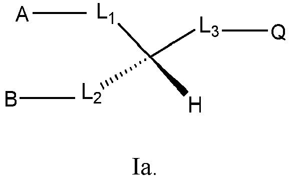

4. The use of any one of preceding claims, wherein in the structure
A is selected from the group consisting of H, C₁₀₋₂₀ alkyl and C₁₀₋₂₀ alkenyl;
B is selected from the group consisting of H, -NH₂, C₁₀₋₂₀ alkyl and C₁₀₋₂₀ alkenyl;
Q is selected from the group consisting of H, -OH, C₁₀₋₂₀ alkyl and C₁₀₋₂₀ alkenyl, and -NR₃⁺, wherein R is H or C₁₋₄ alkyl.

5. The use of claim 4, wherein in said structure:
A is selected from the group consisting of H, a straight-chain C₁₅₋₁₈ alkyl group and a straight-chain C₁₅₋₁₈ alkenyl group;
B is selected from the group consisting of H, -NH2, a straight-chain C₁₅₋₁₈ alkyl group and a straight-chain C₁₅₋₁₈ alkenyl group;
Q is selected from the group consisting of H, -OH, a straight-chain C₁₅₋₁₈ alkyl group and a straight-chain C₁₅₋₁₈ alkenyl group, and -NR₃⁺ wherein R is H or a C₁₋₄ alkyl group; and
the alkenyl group in the A, B, Q has 1-5 double bonds.

6. The use of claim 5, wherein in the A, B, Q of the said structure, the alkenyl group has 1-4 double bonds and is in a Z configuration.

7. The use of claim 6, wherein the alkenyl group in the A, B, Q has 1-3 double bonds and is in a Z configuration.

8. The use of any one of the preceding claims, wherein said extract is selected from the following formulas or comprises a compound selected from the following formulas: 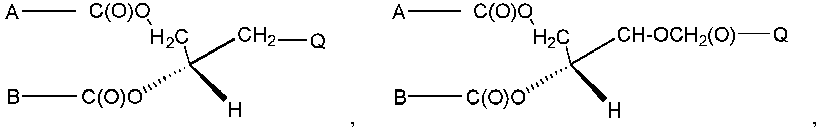 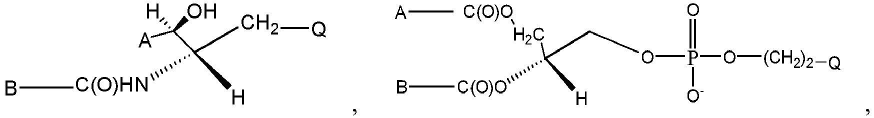 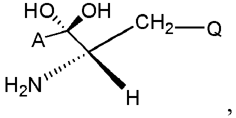 and A-L₃-Q, wherein
A is selected from a straight-chain C₁₅₋₁₈ alkyl group and a straight-chain C₁₅₋₁₈ alkenyl group;
B is selected from a straight-chain C15-18 alkyl group and a straight-chain C₁₅₋₁₈ alkenyl group;
Q is selected from the group consisting of H, -OH, a straight-chain C₁₅₋₁₈ alkyl group and a straight-chain C₁₅₋₁₈ alkenyl group, and -NR₃⁺ wherein R is H or methyl; and
L3 is -C(O)O-.

9. The use of any one of the preceding claims, wherein the extract is or comprises lysolecithin, ceramide, diglyceride, phosphatidylethanolamine, phosphatidylcholine, triglyceride, monogalactosyl diglyceride, (neuro) sphingosine, phosphatidyl ethanol, monoacylglycerol, fatty acid, platelet activating factor, or dimethyl phosphatidyl ethanolamine.

10. The use of any one of the preceding claims, wherein said extract is selected from lipids shown in Table 1 or comprises any one or more lipids selected from Table 1.

11. The use of any one of the preceding claims, wherein said extract comprises any one of the lipids shown in Table 1 as No. 41, No. 71, No. 11, No. 12, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, No. 70, or its combination with any one or more of the other lipids in Table 1, or its combination with any one or more lipids and other related chemicals.

12. Use of a combination comprising any one or more lipids shown in Table 1 in the manufacture of a nucleic acid delivery reagent, wherein preferably, the combination comprises any one of the lipids shown in Table 1 as No. 41, No. 71, No. 11, No. 12, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, No. 70, or its combination with any one or more of the other lipids in Table 1, or its combination with any one or more lipids and other related chemicals, preferably the said nucleic acid is a small nucleic acid, preferably is single or double stranded, preferably the small nucleic acid has a length of 14-32 bp, 16-28 bp or 18-24 bp; preferably, the reagent is an oral reagent; preferably, the nucleic acid is used for treating a disease, for example, heart, spleen, kidney, gastric, lung and/or intestinal diseases, such as cancer, e.g., gastric cancer or lung cancer;
preferably, the nucleic delivery is a nucleic delivery to cells or organs;
preferably, the organs are selected from the group consisting of heart, spleen, kidney, stomach, lung and intestine.

13. Use of a traditional Chinese medicine in the manufacture of a nucleic acid delivery reagent, wherein preferably the nucleic acid is a small nucleic acid, preferably is single or double stranded, preferably the small nucleic acid has a length of 14-32 bp, 16-28 bp or 18-24 bp, preferably the reagent is an oral reagent, preferably the nucleic acid is used for treating a disease, for example, heart, spleen, kidney, gastric, lung and/or intestinal diseases, such as cancer, e.g., gastric cancer or lung cancer;
preferably, the nucleic delivery is a nucleic delivery to cells or organs;
preferably, the organs are selected from the group consisting of heart, spleen, kidney, stomach, lung and intestine.

14. The use of claim 13, wherein said traditional Chinese medicine is selected from Rhodiola crenulata, Taraxacum mongolicum, Andrographis paniculata and Lonicera japonica Chinese medicine decoction pieces.

15. The use of claim 13 or 14, wherein the reagent comprises a compound extracted from a traditional Chinese medicine or artificially synthesized, and preferably, the compound is obtained by extracting a lipid-soluble component by the Bligh & Dyer method, or extracting by decoction of traditional Chinese medicine, more preferably, the Chinese medicine decoction pieces are soaked in water, and then performed to intense heating and slow heating, and the heated Chinese medicine soup is concentrated, and then is sequentially added with chloroform-methanol, chloroform and water for stirring, and the chloroform layer is obtained.

16. The use of claim 15, wherein the said compound has the structure shown in any one of claims 1 to 11, or the reagent comprises any one or more lipids shown in Table 1, preferably any one of lipids shown in Table 1 as No. 41, No. 71, No. 11, No. 12, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, No. 70, or its combination with any one or more of the other lipids in Table 1, or its combination with any one or more lipids and other related chemicals.

17. The use of claim 16, wherein the compound is selected from the group consisting of lysolecithin, ceramide, diglyceride, phosphatidylethanolamine, phosphatidylcholine, triglyceride, monogalactosyldiglyceride, (neural) sphingosine, phosphatidyl ethanol, monoacylglycerol, fatty acid, platelet activating factor, or dimethyl phosphatidyl ethanolamine.

18. The use of claim 17, wherein the compound is selected from Table 1.

19. The use of claim 18, wherein the said compound is selected from lipids shown in Table 1 as No. 41, No. 71, No. 11, No. 12, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, No. 70.

20. The use of any one of claims 13-18, wherein the delivery comprises in vitro cell delivery, or in vivo gastrointestinal delivery.

21. The use of any one of claims 13-20, wherein the use includes the manufacture of lipid nucleic acid mixture.

22. The use of claim 21, wherein the lipid nucleic acid mixture is manufactured by a boiling method, or by a reverse evaporation method, or by direct mixing.

23. The use of claim 22, wherein temperature in the boiling method is from about 4 °C to about 100 °C, from about 25 °C to about 100 °C, preferably from about 80°C to about 100 °C, i.e. 4°C, 37°C, 60 °C, 80°C or 100 °C; temperature in the reverse evaporation method is from about 25 °C to about 70 °C, preferably about 55 °C.

24. A pharmaceutical composition comprising one or more lipid extracts of any structure of claims 1-11 and a nucleic acid, preferably the lipid is selected from any one or more lipids in Table 1, preferably any one lipid shown in Table 1 as No. 41, No. 71, No. 11, No. 12, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, No. 70, or its combination with any one or more of the other lipids in Table 1, or its combination with any one or more lipids and other related chemicals, wherein preferably the nucleic acid is a small nucleic acid, preferably is single or double stranded, preferably the small nucleic acid has a length of 14-32 bp, 16-28 bp or 18-24 bp, preferably, the pharmaceutical composition is an oral pharmaceutical combination, preferably the pharmaceutical composition is used for treating a disease, for example, heart, spleen, kidney, gastric, lung and/or intestinal diseases, such as cancer, e.g., gastric cancer or lung cancer.

25. The pharmaceutical composition of claim 24, wherein at least part of or all of the lipids and the nucleic acids exist in the form of lipid nucleic acid mixture.

26. The pharmaceutical composition of claim 25, wherein the lipid nucleic acid mixture is prepared by a boiling method, or by a reverse evaporation method, or by direct mixing.

27. The pharmaceutical composition of claim 26, wherein temperature in the boiling method is from about 4 °C to about 100 °C, from 25 °C to about 100 °C, preferably from about 80 °C to 100 °C, i.e. 4 °C, 37 °C, 60 °C, 80 °C or 100 °C; temperature in the reverse evaporation method is from about 25 °C to about 70 °C, preferably about 55 °C.

28. A kit comprising one or more lipids having the structure of claims 1-11, preferably the lipid is selected from any one or more lipids in Table 1, preferably shown in Table 1 as No. 41, No. 71, No. 11, No. 12, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, No. 70 or its combination thereof with any one or more of other lipids in Table 1, or its combination thereof with any one or more lipids and other related chemicals, nucleic acids, wherein the lipid and nucleic acid are each independently provided in a first container and a second container, the first container and the second container are the same or different, wherein preferably the nucleic acid is a small nucleic acid, preferably is single or double stranded, preferably the small nucleic acid has a length of 14-32 bp, 16-28 bp or 18-24 bp; preferably, the kit is an oral kit, preferably the kit is used for treating a disease, for example, heart, spleen, kidney, gastric, lung and/or intestinal diseases, such as cancer, e.g., gastric cancer or lung cancer.

29. The kit of claim 28, wherein at least part of or all of said lipid and nucleic acid are prepared into lipid nucleic acid mixture immediately prior to use.

30. The kit of claim 29, wherein the preparation method of lipid nucleic acid mixture is a boiling method, or a reverse evaporation method, or direct mixing.

31. The kit of claim 30, wherein temperature in said boiling method is from about 4 °C to about 100 °C, from 25 °C to about 100 °C, preferably from about 80 °C to about 100 °C, i.e. 4 °C, 37 °C, 60 °C, 80 °C or 100 °C; temperature in the reverse evaporation method is from about 25 °C to about 70 °C, preferably about 55 °C.

32. A method of delivering a nucleic acid into a target cell, wherein the nucleic acid is provided in a form of a pharmaceutical composition of any one of claims 24-27 or the kit of any one of claims 28-31, preferably the nucleic acid is a small nucleic acid, preferably is single or double stranded, preferably the small nucleic acid has a length of 14-32 bp, 16-28 bp or 18-24 bp; preferably, the nucleic acid is used for treating a disease, for example, heart, spleen, kidney, gastric, lung and/or intestinal diseases, such as cancer, e.g., gastric cancer or lung cancer.

33. A method of delivering a nucleic acid into a subject in vivo in need thereof, wherein the nucleic acid provided from the pharmaceutical composition of any one of claims 24-27 or the kit of any one of claims 28-31, wherein preferably said nucleic acid is a small nucleic acid, preferably is single or double stranded, preferably said small nucleic acid has a length of 14-32 bp, 16-28 bp or 18-24 bp; preferably said nucleic acid is used for treating a disease, for example, heart, spleen, kidney, gastric, lung and/or intestinal diseases, such as cancer, e.g., gastric cancer or lung cancer.

34. The method of claim 33, wherein the subject is a human or an animal, such as a mammal.

35. The method of any one of claims 33-34, wherein the nucleic acid is delivered to blood circulation or a target tissue/cell of the subject in vivo, the said target tissue is selected from the group consisting of heart, spleen, kidney, stomach, lung and intestine.

36. The method of claim 35, wherein the method includes directly delivering the pharmaceutical composition of any one of claims 24-27 or the kit of any one of claims 28-31 to a subject in need by digestive tract.

37. The pharmaceutical composition of any one of claims 24-27, or the kit of any one of claims 28-31, wherein the nucleic acid and the lipid are prepared for administration and/or injection.

38. The pharmaceutical composition or the kit of claim 37, wherein the nucleic acid and lipid are prepared for digestive administration or respiratory administration.

39. The pharmaceutical composition or the kit of claim 37 or 38, wherein the nucleic acid and lipid are prepared for oral administration or inhalation administration.

40. The pharmaceutical composition or the kit of any one of claims 37-39, wherein the nucleic acid is a small RNA.

41. The pharmaceutical composition or the kit of any one of claims 37-40, wherein the nucleic acid has a stem-loop structure.

42. The pharmaceutical composition, or the kit of any one of claims 37-41, wherein the small RNA has a length of 14-32 bp, or 18-24 bp, for example, the length is of 14, 15 , 16, 17 , 18 , 19, 20, 21, 22, 23, 24, 25, 26, 27, 28, 29, 30, 31 and 32 bp.

43. A compound extracted from a traditional Chinese medicine or artificially synthesized can be used for nucleic acid delivery, having the following structure: 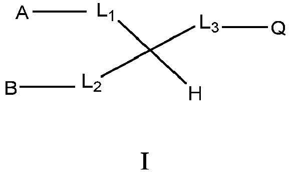
L1, L2 or L3 is absent, or L1, L2 and L3 are each independently selected from the group consisting of -C(O)O-CH₂-, -CH(OH)-, -C(O)-NH-CH₂-, -CH₂-OC(O)-, -CH₂-NH-C(O)-, -C(O)O-, -C(O)NH-, -OC(O)-, -NH-C(O)-, -CH₂-, 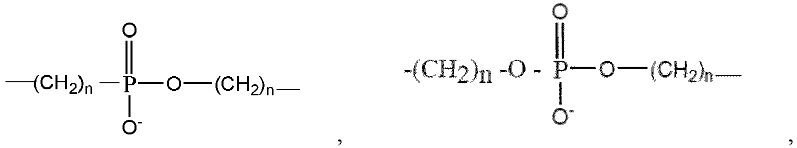 and 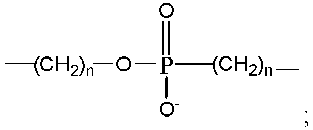 with the proviso that at most two of L1, L2 and L3 are absent;
with respect to the divalent groups L1, L2, the dash "-" on the left side is linked to the groups A and B, respectively, and the dash "-" on the right side is linked to the central carbon atom;
with respect to the divalent group L3, the dash "-" on the left side is linked to the central carbon atom, and the dash "-" on the right side is linked to the group Q;
A, B and Q are each independently selected from the group consisting of H, -OH, C₁₋₂₀ alkyl, C₁₋₂₀ alkenyl, C₁₋₂₀ heteroalkyl, C₁₋₂₀ heteroalkenyl, -NH₂, and -NR₃⁺, R is H or C₁₋₆ alkyl; and
n is an integer of 0, 1, 2, 3 or 4, preferably the compound is an oral compound; preferably, the nucleic acid is used for treating a disease, for example, heart, spleen, kidney, gastric, lung and/or intestinal diseases, such as cancer, e.g., gastric cancer or lung cancer;
preferably, the nucleic delivery is a nucleic delivery to cells or organs, preferably, the organs are selected from the group consisting of heart, spleen, kidney, stomach, lung and intestine.

44. The compound of claim 43 wherein
L1 is absent, or L1 is selected from the group consisting of -C(O)O-CH₂- and - CH(OH)-,
L2 is absent, or L2 is selected from the group consisting of -C(O)O- and -C(O)NH-,
L3 is absent, or L3 is selected from the group consisting of -C(O)O-, -CH₂-OC(O)-, -CH₂- and 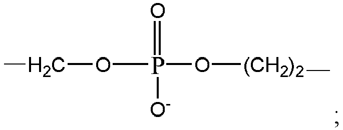 A is selected from the group consisting of H, C₁₋₂₀ alkyl and C₁₋₂₀ alkenyl;
B is selected from the group consisting of H, -NH₂, C₁₋₂₀ alkyl and C₁₋₂₀ alkenyl;
Q is selected from the group consisting of H, -OH, C₁₋₂₀ alkyl and C₁₋₂₀ alkenyl, and -NR₃⁺, wherein R is H or C₁₋₆ alkyl, wherein preferably the traditional Chinese medicine is selected from Rhodiola crenulata, Taraxacum mongolicum, Andrographis paniculata and Lonicera japonica Chinese medicine decoction pieces, preferably the compound is obtained by extracting a lipid-soluble component by the Bligh & Dyer method, more preferably by soaking Chinese medicine decoction pieces in water, and then sequentially performing intense heating and slow heating, and the heated Chinese medicine soup is concentrated, and then is added with chloroform-methanol, chloroform and water for stirring, and the chloroform layer is obtained; preferably, the nucleic acid is a small nucleic acid, preferably is single or double-stranded, preferably the small nucleic acid has a length of 14-32 bp, 16-28 bp or 18-24 bp.

45. The compound of claim 43 or 44, having the following formula:

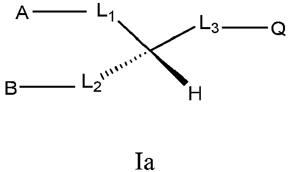

46. The compound of any one of claims 43-45, wherein
A is selected from the group consisting of H, C₁₀₋₂₀ alkyl and C₁₀₋₂₀ alkenyl;
B is selected from the group consisting of H, -NH₂, C₁₀₋₂₀ alkyl and C₁₀₋₂₀ alkenyl;
Q is selected from the group consisting of H, -OH, C₁₀₋₂₀ alkyl and C₁₀₋₂₀ alkenyl, and -NR₃⁺ wherein R is H or C₁₋₄ alkyl.

47. The compound of any one of claims 43-46, wherein
A is selected from the group consisting of H, a straight-chain C₁₅₋₁₈ alkyl group and a straight-chain C₁₅₋₁₈ alkenyl group;
B is selected from the group consisting of H, -NH₂, a straight-chain C₁₅₋₁₈ alkyl group and a straight-chain C₁₅₋₁₈ alkenyl group;
Q is selected from the group consisting of H, -OH, a straight-chain C₁₅₋₁₈ alkyl group and a straight-chain C₁₅₋₁₈ alkenyl group, and -NR₃⁺ wherein R is H or a C₁₋₄ alkyl group;
the alkenyl group in the A, B, Q has 1-5 double bonds.

48. The compound of any one of claims 43-47, wherein in the A, B, Q of the said structure, the alkenyl group has 1-4 double bonds, and is in a Z configuration.

49. The compound of any one of claims 43-48, wherein in the A, B, Q of the structure, the alkenyl group has 1-3 double bonds and is in a Z configuration.

50. The compound of any one of claims 43-49, wherein the compound is selected from the following formulas: 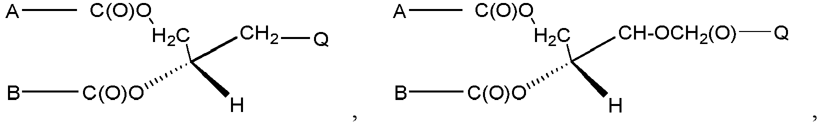 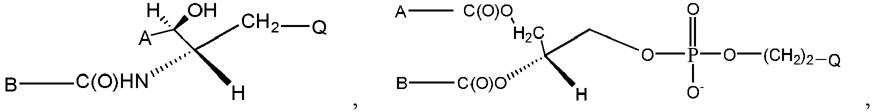 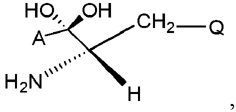 and A-L₃-Q,
wherein
A is selected from the group consisting of a straight-chain C₁₅₋₁₈ alkyl group and a straight-chain C₁₅₋₁₈ alkenyl group;
B is selected from the group consisting of a straight-chain C₁₅₋₁₈ alkyl group and a straight-chain C₁₅₋₁₈ alkenyl group;
Q is selected from the group consisting of H, -OH, a straight-chain C₁₅₋₁₈ alkyl group and a straight-chain C₁₅₋₁₈ alkenyl group, and -NR₃⁺ wherein R is H or methyl; and
L3 is -C(O)O-.

51. The compound of any one of claims 43-50, wherein the compound is selected from lipids shown in Table 1.

52. The compound of any one of claims 43-51, wherein the compound is selected from lipids shown in Table 1 as No. 41, No. 71, No. 11, No. 12, No. 38, No. 64, No. 40, No. 37, No. 39, No. 60 or No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, No. 70.

53. A method of facilitating nucleic acid delivery comprising heating or warming up a nucleic acid and a traditional Chinese medicine extract, any compound derived naturally or synthetically, preferably the lipid of any one of claims 1 to 11; temperature for heating or warming up is preferably from about 4 °C to about 100 °C, from about 25 °C to about 100 °C , preferably from about 50 °C to about 100 °C, more preferably from about 95 °C to about 100 °C, particularly preferably from about 80 °C to about 100 °C, i.e. 4 °C, 37 °C, 60 °C, 80 °C or 100 °C, wherein preferably the nucleic acid is a small nucleic acid, preferably is single or double stranded, preferably the small nucleic acid has a length of 14-32 bp, 16-28 bp or 18-24 bp; preferably, the nucleic acid delivery is by oral administration; preferably, the nucleic acid is used for treating a disease, for example, heart, spleen, kidney, gastric, lung and/or intestinal diseases, such as cancer, e.g., gastric cancer or lung cancer; preferably, the nucleic delivery is a nucleic delivery to cells or organs, preferably, the organs are selected from the group consisting of heart, spleen, kidney, stomach, lung and intestine.

54. The method of claim 53, wherein the traditional Chinese medicine extract comprises a compound of the structure as set forth in claims 1-9.

55. The method of claim 53, wherein the traditional Chinese medicine extract comprises any one or more lipids shown in Table 1.

56. The method of claim 53, wherein the tradition Chinese medicine extract comprises any one of lipids shown in Table 1 as No. 41, No. 71, No. 11, No. 12, No. 38, No. 64, No. 40, No. 37, No. 39, No. 60, No. 62, No. 33, No. 35, No. 42, No. 43, No. 44, No. 45, No. 46, No. 47, No. 48, No. 49, No. 50, No. 51, No. 52, No. 53, No. 54, No. 55, No. 56, No. 57, No. 58, No. 59, No. 61, No. 65, No. 66, No. 67, No. 68, No. 69, No. 70, or its combination with any one or more of the other lipids in Table 1, or its combination with any one or more lipids and other related chemicals.

57. The use of claim 11, 12 or 16, the pharmaceutical composition of claim 24, or the kit of claim 28, wherein the combination is any one of the following: a lipid combination of No. 8: No. 41=6:1; a lipid combination of No. 38:No. 41=6:1; a lipid combination of No. 39:No. 41=6:1; a lipid combination of No. 40:No. 41=6:1; a lipid combination of No. 38:No. 12:No. 41:No. 29=1:2:1:1; a lipid combination of No. 40:No. 12:No. 41=2:4:3; a lipid combination of No. 12:No. 41=1:6; a lipid combination of No. 12:No. 41=1:1; a lipid combination of No. 12:No. 41=6:1; a lipid combination of No. 40:No. 12:No. 41=2:2:2; a lipid combination of No. 4:No. 12:No. 41=1:1:1; DG combination of No. 1:No.2:No.3:No.19:No.35=1:1:1:1:1; TG combination of No. 6:No. 9:No. 10:No. 13:No. 15:No. 16:No. 18:No. 20:No. 21:No. 22:No. 23:No. 24:No. 25:No. 26:No. 27:No. 28:No. 32:No. 33 =1:1:1:1:1:1:1:1:1:1:1:1:1:1:1:1:1:1; LPC combination of No. 36:No. 37=1:1; PC combination of No. 11:No. 12=1:1 ; PE combination of No. 8:No. 38=1:1; Cer combination of No. 4:No. 14=1:1; So combination of No. 17:No. 30:No. 31=1:1:1; an equal volume combination of No. 1-36 without No. 5, No. 7 ; an equal volume combination of No. 1-36 without No. 5, No. 7, No. 34; an equal volume combination of No. 1-36 without No. 5, No. 7, No. 1, No. 2, No. 3, No. 19, No. 35; an equal volume combination of No. 1-36 without No. 5, No. 7, No. 6, No. 9, No. 10, No. 13, No. 15, No. 16, No. 18, No. 20, No. 21, No. 22, No. 23, No. 24, No. 25, No. 26, No. 27, No. 28, No. 32, No. 33; an equal volume combination of No. 1-36 without No. 5, No. 7, No. 36, No. 37; an equal volume combination of No. 1-36 without No. 5, No. 7, No. 11, No. 12; an equal volume combination of No. 1-36 without No. 5, No. 7, No. 8 in; an equal volume combination of No. 1-36 without No. 5, No. 7, No. 4, No. 14; an equal volume combination of No. 1-36 without No. 5, No. 7, No. 29; a lipid combination of No. 1:No. 34=2:1; a lipid combination of No. 1: said DG composition=2:1; a lipid combination of No. 1: said TG composition=2:1; a lipid combination of No. 1: said LPC composition=2:1; a lipid combination of No. 1:No. 8=2:1; a lipid combination of No. 1:No. 12=2:1; a lipid combination of No. 1: said Cer composition=2:1; a lipid combination of No. 1: said So composition=2:1; a lipid combination of No. 1:No. 29=2:1; a lipid combination of No. 1:No. 8:No. 12=1:1:1; a lipid combination of No. 8:No. 34=2:1; a lipid combination of No. 8: said DG composition=2:1; a lipid combination of No. 8: said TG composition=2:1; a lipid combination of No. 8: said LPC composition=2:1; a lipid combination of No. 8:No. 37=4:1; a lipid combination of No. 8:No. 12=2:1; a lipid combination of No. 8: said Cer composition=2: 1 ; a lipid combination of No. 8: said So composition=2:1; a lipid combination of No. 8:No. 31=6:1; a lipid combination of No. 8:No. 29=2:1; a lipid combination of No. 12:No. 34=2:1; a lipid combination of No. 12: said DG composition=2:1; a lipid combination of No. 12: said TG composition=2:1; a lipid combination of No. 12: said LPC composition=2:1; a lipid combination of No. 12:No. 8=2:1; a lipid combination of No. 12: said Cer composition=2:1; a lipid combination of No. 12: said So composition=2:1; a lipid combination of No. 12:No. 29=2:1; a lipid combination of No. 12:No. 8:No. 1&2=2:1:1; a lipid combination of No. 12:No. 8:No. 15=2:1:1; a lipid combination of No. 12:No. 8:No. 36&37=2:1:1; a lipid combination of No. 12:No. 8:No. 11=2:1:1; a lipid combination of No. 12:No. 8:No. 12=2:1:1; a lipid combination of No. 12:No. 8:No. 4=2:1:1; a lipid combination of No. 12:No. 8:No. 31=2:1:1; a lipid combination of No. 12:No. 8:No. 29=2:1:1; a lipid combination of No. 12:No. 8:No. 34=3:2:1; a lipid combination of No. 12:No. 8:No. 34=4:2:3; a lipid combination of No. 12:No. 8:No. 2=4:2:3; a lipid combination of No. 12:No. 8:No. 2=16:8:3; a lipid combination of No. 12:No. 8:No. 32=4:2:3; a lipid combination of No. 12:No. 8:No. 37=4:2:3; a lipid combination of No. 12:No. 8:No. 11=4:2:3; a lipid combination of No. 12:No. 8:No. 38=4:2:3; a lipid combination of No. 12:No. 8:No. 4=4:2:3; a lipid combination of No. 12:No. 8:No. 31=4:2:3; a lipid combination of No. 12:No. 8:No. 29=4:2:3; a lipid combination of No. 12:No. 8:No. 29:No. 31=2:1:1:1; a lipid combination of No. 12:No. 8:No. 29:No. 31:No. 34=4:2:2:2:5; a lipid combination of No. 12:No. 8:No. 29:No. 31:No. 2=4:2:2:2:5; a lipid combination of No. 12:No. 8:No. 29:No. 31:No. 32=4:2:2:2:5; a lipid combination of No. 12:No. 8:No. 29:No. 31:No. 11=4:2:2:2:5; a lipid combination of No. 12:No. 8:No. 29:No. 31:No. 37=4:2:2:2:5; a lipid combination of No. 12:No. 8:No. 29:No. 31:No. 38=4:2:2:2:5; a lipid combination of No. 12:No. 8:No. 29:No. 31:No. 4=4:2:2:2:5; a lipid combination of No. 12:No. 8:No. 29:No. 31:No. 4:No. 1:No. 16=2:1:1:3:2:2:3; a lipid combination of No. 1:No. 8:No. 12:No. 1&2=2:2:2:3; a lipid combination of No. 1:No. 8:No. 12:No. 15=2:2:2:3; a lipid combination of No. 1:No. 8:No. 12:No. 36&37=2:2:2:3; a lipid combination of No. 1:No. 8:No. 12:No. 12=2:2:2:3; a lipid combination of No. 1:No. 8:No. 12:No. 4=2:2:2:3; a lipid combination of No. 1:No. 8:No. 12:No. 31=2:2:2:3; a lipid combination of No. 1:No. 8:No. 12:No. 29=2:2:2:3; a lipid combination of No. 8:No. 34:No. 1&2=2:1:1; a lipid combination of No. 8:No. 34:No. 15=2:1:1; a lipid combination of No. 8:No. 34:No. 36&37=2:1:1; a lipid combination of No. 8:No. 34:No. 12=2:1:1; a lipid combination of No. 8:No. 34:No. 4=2:1:1; a lipid combination of No. 8:No. 34:No. 31=2:1:1; a lipid combination of No. 8:No. 34:No. 29=2:1:1; a lipid combination of No. 8:No. 3 1:No. 34=12:3:5; a lipid combination of No. 8:No. 31:No. 2=12:3:5; a lipid combination of No. 8:No. 31:No. 37=12:3:5; a lipid combination of No. 8:No. 31:No. 11=12:3:5; a lipid combination of No. 8:No. 31:No. 12=12:3:5; a lipid combination of No. 8:No. 31:No. 4=12:3:5; a lipid combination of No. 8:No. 31:No. 29=12:3:5; a lipid combination of No. 8:No. 31:No. 32=12:3:5; a lipid combination of No. 8:No. 4:No. 34=12:3:5; a lipid combination of No. 8:No. 4:No. 2=12:3:5; a lipid combination of No .8:No. 4:No. 37=12:3:5; a lipid combination of No. 8:No. 4:No. 12=12:3:5; a lipid combination of No. 8:No. 4:No. 31=12:3:5; a lipid combination of No. 8:No. 4:No. 29=12:3:5; a lipid combination of No. 8:No. 4:No. 32=12:3:5; a lipid combination of No. 38:No. 34=2:1; a lipid combination of No. 38:No. 1=2:1; a lipid combination of No. 38:No. 2=2:1; a lipid combination of No. 38:No. 1&2=2:1; a lipid combination of No. 38:No. 15=2:1; a lipid combination of No. 38:No. 32=2:1; a lipid combination of No. 38:No. 37=2:1; a lipid combination of No. 38:No. 37=4:1; a lipid combination of No. 38:No. 11=2:1; a lipid combination of No. 38:No. 12=2:1; a lipid combination of No. 38:No. 11&12=2:1; a lipid combination of No. 38:No. 12=4:1; a lipid combination of No. 38:No. 8=2:1; a lipid combination of No. 38:No. 4=2:1; a lipid combination of No.38: So (30)=2:1; a lipid combination of No. 38:No. 31=2:1; a lipid combination of No. 38:No. 29=2:1; a lipid combination of No. 1:No. 38:No. 12:No. 34=2:2:2:3; a lipid combination of No. 1:No. 38:No. 12:No. 15=2:2:2:3; a lipid combination of No. 1:No. 38:No. 12:No. 37=2:2:2:3; a lipid combination of No. 1:No. 38:No. 12:No. 8=2:2:2:3; a lipid combination of No. 1:No. 38:No. 12:No. 4=2:2:2:3; a lipid combination of No. 1:No. 38:No. 12:No. 31=2:2:2:3; a lipid combination of No. 1:No. 38:No. 12:No. 29=2:2:2:3; a lipid combination of No. 38:No. 34:No. 1=2:1:3; a lipid combination of No. 38:No. 34:No. 15=2:1:3; a lipid combination of No. 38:No. 34:No. 37=2:1:3; a lipid combination of No. 38:No. 34:No. 12=2:1:3; a lipid combination of No. 38:No. 34:No. 8=2:1:3; a lipid combination of No. 38:No. 34:No. 4=2:1:3; a lipid combination of No. 38:No. 34:No. 31=2:1:3; a lipid combination of No. 38:No. 34:No. 29=2:1:3; a lipid combination of No. 38:No. 12:No. 1=2:1:3; a lipid combination of No. 38:No. 12:No. 2=4:1:3; a lipid combination of No. 38:No. 12:No. 15=2:1:3; a lipid combination of No. 38:No. 12:No. 37=2:1:3; a lipid combination of No. 38:No. 12:No. 8=2:1:3; a lipid combination of No. 38:No. 12:No. 4=2:1:3; a lipid combination of No. 38:No. 12:No. 31=2:1:3; a lipid combination of No. 38:No. 12:No. 29=2:1:3; a lipid combination of No. 38:No. 12:No. 1:No. 15:No. 34=22:22:22:33:36; a lipid combination of No. 38:No. 12:No. 1:No. 15:No. 37=22:22:22:33:36; a lipid combination of No. 38:No. 12:No. 1:No. 15:No. 4=22:22:22:33:36; a lipid combination of No. 38:No. 12:No. 1:No. 15:No. 31=22:22:22:33:36; a lipid combination of No. 38:No. 12:No. 1:No. 15:No. 29=22:22:22:33:36; a lipid combination of No. 38:No. 34:No. 37:No. 1=44:22:33:36; a lipid combination of No. 38:No. 34:No. 37:No. 15=44:22:33:36; a lipid combination ofNo. 38:No. 34:No. 37:No. 12=44:22:33:36; a lipid combination of No. 38:No. 34:No. 37:No. 4=44:22:33:36; a lipid combination of No. 38:No. 34:No. 37:No. 31=44:22:33:36; a lipid combination of No. 38:No. 12:No. 4:No. 34=44:22:33:36; a lipid combination of No. 38:No. 12:No. 4:No. 1=44:22:33:36; a lipid combination of No. 38:No. 12:No. 4:No. 15=44:22:33:36; a lipid combination of No. 38:No. 12:No. 4:No. 37=44:22:33:36; a lipid combination of No. 38:No. 12:No. 4:No. 37=8:2:5:3; a lipid combination of No. 38:No. 12:No. 4:No. 31=44:22:33:36; a lipid combination of No. 38:No. 12:No. 4:No. 29=44:22:33:36; a lipid combination of No. 38:No. 12:No. 4:No. 29:No. 34=88:44:66:72:135; a lipid combination of No. 38:No. 12:No. 4:No. 29:No. 1=88:44:66:72:135; a lipid combination of No. 38:No. 12:No. 4:No. 29:No. 15=88:44:66:72:135; a lipid combination of No. 38:No. 12:No. 4:No. 29:No. 37=88:44:66:72:135; a lipid combination of No. 38:No. 12:No. 4:No. 29:No. 31=88:44:66:72:135; a lipid combination of No. 38:No. 12:No. 4:No. 2=20:10:15:9; a lipid combination of No. 38:No. 12:No. 4:No. 6=20:10:15:9; a lipid combination of No. 38:No. 12:No. 4:No. 17=20:10:15:9; a lipid combination of No. 38:No. 12:No. 4:No. 29=20:10:15:9; a lipid combination of No. 38:No. 12:No. 4:No. 34=20:10:15:9; a lipid combination of No. 38:No. 12:No. 4:No. 37=20:10:15:9; a lipid combination of No. 38:No. 12:No. 31:No. 34=2:1:3:3; a lipid combination of No. 38:No. 12:No. 31:No. 1=2:1:3:3; a lipid combination of No. 38:No. 12:No. 31:No. 15=2:1:3:3; a lipid combination of No. 38:No. 12:No. 31:No. 37=2:1:3:3; a lipid combination of No. 38:No. 12:No. 31:No. 4=2:1:3:3; a lipid combination of No. 38:No. 12:No. 31:No. 29=2:1:3:3; a lipid combination of No. 38:No. 34:No. 37:No. 31:No. 1=88:44:66:72:135; a lipid combination of No. 38:No. 34:No. 37:No. 31:No. 15=88:44:66:72:135; a lipid combination of No. 38:No. 34:No. 37:No. 31:No. 12=88:44:66:72:135; a lipid combination of No. 38:No. 34:No. 37:No. 31:No. 4=88:44:66:72:135; a lipid combination of No. 38:No. 34:No. 37:No. 31:No. 29=88:44:66:72:135; a lipid combination of No. 38:No. 37:No. 34=4:2:3; a lipid combination of No. 38:No. 37:No. 1=4:2:3; a lipid combination of No. 38:No. 37:No. 2=4:2:3; a lipid combination of No. 38:No. 37:No. 1&2=4:2:3; a lipid combination of No. 38:No. 37:No. 2=32:8:5; a lipid combination of No. 38:No. 37:No. 32=32:8:5; a lipid combination of No. 38:No. 37:No. 15=4:2:3; a lipid combination of No. 38:No. 37:No. 32=4:2:3; a lipid combination of No. 38:No. 37:No. 8=4:2:3; a lipid combination of No. 38:No. 37:No. 11=4:2:3; a lipid combination of No. 38:No. 37:No. 12=4:2:3; a lipid combination of No. 38:No. 37:No. 11&12=4:2:3; a lipid combination of No. 38:No. 37:No. 12=4:1:1; a lipid combination of No. 38:No. 37:No. 4=4:2:3; a lipid combination of No. 38:No. 37:No. 30=4:2:3; a lipid combination of No. 38:No. 37:No. 31=4:2:3; a lipid combination of No. 38:No. 37:No. 29=4:2:3; a lipid combination of No. 8:No. 37:No. 32=4:1:2; a lipid combination of No. 8:No. 37:No. 2=4:1:2; a lipid combination of No. 38:No. 37:No. 15:No. 34=64:16:10:45; a lipid combination of No. 38:No. 37:No. 15:No. 1=64:16:10:45; a lipid combination of No. 38:No. 37:No. 15:No. 12=64:16:10:45; a lipid combination of No. 38:No. 37:No. 15:No. 4=64:16:10:45; a lipid combination of No. 38:No. 37:No. 15:No. 31=64:16:10:45; a lipid combination of No. 38:No. 37:No. 15:No. 29=64:16:10:45; a lipid combination of No. 38:No. 2:No. 37=4:2:3; a lipid combination of No. 38:No. 2:No. 31=4:2:3; a lipid combination of No. 38:No. 2:No. 29=4:2:3; a lipid combination of No. 38:No. 2:No. 34=4:2:3; a lipid combination of No. 38:No. 2:No. 32=4:2:3; a lipid combination of No. 38:No. 2:No. 12=4:2:3; a lipid combination of No. 38:No. 2:No. 12=4:5:1; a lipid combination of No. 38:No. 2:No. 4=4:2:3; No. 1&2, No. 11&12 and No. 36&37 represent lipids No. 1 and No. 2 in any ratio, lipids No. 11 and No. 12 in any ratio, lipids No. 36 and No. 37 in any ratio, respectively.

58. Use of a compound having the following formula for the manufacture of nucleic acid delivery reagent: 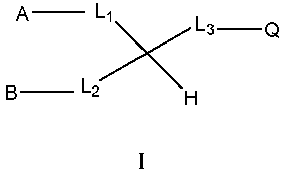 wherein
L1, L2 or L3 is absent, or L1, L2 and L3 are each independently selected from the group consisting of -C(O)O-CH2-, -CH(OH)-, -CH2-OC(O), -C(O)O-, -C(O)NH-;
with the proviso that at most two of L1, L2 and L3 are absent;
with respect to the divalent groups L1, L2, the dash "-" on the left side is linked to the groups A and B, respectively, and the dash "-" on the right side is linked to the central carbon atom;
with respect to the divalent group L3, the dash "-" on the left side is linked to the central carbon atom, and the dash "-" on the right side is linked to the group Q;
A, B and Q are independently selected from the group consisting of H, -OH, C₁₋₂₀ alkyl, C₁₋₂₀ alkenyl, -NH₂, and -NR₃⁺, R is H or C₁₋₆ alkyl; preferably the reagent is an oral reagent; preferably, the nucleic acid is used for treating a disease, for example, heart, spleen, kidney, gastric, lung and/or intestinal diseases, such as cancer, e.g., gastric cancer or lung cancer; preferably, the nucleic delivery is a nucleic delivery to cells or organs, preferably, the organs are selected from the group consisting of heart, spleen, kidney, stomach, lung and intestine.

59. The use of claim 58, wherein the compound has the following structure: 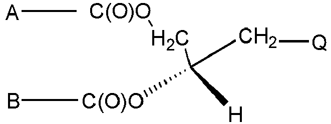 wherein
A is selected from the group consisting of a straight-chain C₁₀₋₂₀ alkyl group and a straight-chain C₁₀₋₂₀ alkenyl group;
B is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₅₋₂₀ alkyl group and a straight-chain C₁₅₋₂₀ alkenyl group;
B is selected from the group consisting of a straight-chain C₁₅₋₂₀ alkyl group and a straight-chain C₁₅₋₂₀ alkenyl group;
Q is -OH;
preferably,
A is selected from the group consisting of a straight-chain C₁₅₋₁₈ alkyl group and a straight-chain C₁₅₋₁₈ alkenyl group;
B is selected from the group consisting of a straight-chain C₁₅₋₁₈ alkyl group and a straight-chain C₁₅₋₁₈ alkenyl group;
Q is -OH.

60. The use of claim 58, wherein the compound has the following structure: 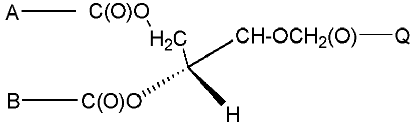 wherein
A is selected from the group consisting of a straight-chain C₁₀₋₂₀ alkyl group and a straight-chain C₁₀₋₂₂ alkenyl group;
B is selected from the group consisting of a straight-chain C₁₀₋₂₀ alkyl group and a straight-chain C₁₀₋₂₂ alkenyl group;
Q is selected from the group consisting of a straight-chain C₁₀₋₂₀ alkyl group and a straight-chain C₁₀₋₂₂ alkenyl group;
preferably,
A is selected from the group consisting of a straight-chain C₁₅₋₁₈ alkyl group and a straight-chain C₁₅₋₂₂ alkenyl group;
B is selected from the group consisting of a straight-chain C₁₅₋₁₈ alkyl group and a straight-chain C₁₅₋₂₂ alkenyl group;
Q is selected from the group consisting of a straight-chain C₁₅₋₁₈ alkyl group and a straight-chain C₁₅₋₂₂ alkenyl group;
preferably,
A is selected from the group consisting of a straight-chain C₁₅₋₁₈ alkyl group and a straight-chain C₁₅₋₂₀ alkenyl group;
B is selected from the group consisting of a straight-chain C₁₅₋₁₈ alkyl group and a straight-chain C₁₅₋₂₀ alkenyl group;
Q is selected from the group consisting of a straight-chain C₁₅₋₁₈ alkyl group and a straight-chain C₁₅₋₂₀ alkenyl group.

61. The use of claim 58, wherein the compound has the following structure: 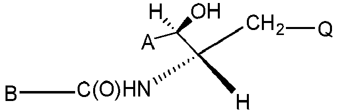 wherein
A is selected from the group consisting of a straight-chain C₁₀₋₂₀ alkyl group and a straight-chain C₁₀₋₂₀ alkenyl group;
B is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₅₋₂₀ alkyl group and a straight-chain C₁₅₋₁₈ alkenyl group;
B is selected from the group consisting of a straight-chain C₁₅₋₁₈ alkyl group and a straight-chain C₁₅₋₁₈ alkenyl group;
Q is -OH;
preferably,
A is a straight-chain C₁₅₋₂₀ alkyl group;
B is a straight-chain C₁₅₋₁₈ alkyl group;
Q is -OH.

62. The use of claim 58, wherein the compound has the following structure: 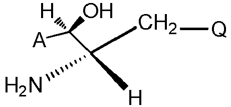 wherein
A is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₀₋₂₀ alkyl group and a straight-chain C₁₅₋₁₈ alkenyl group;
Q is -OH;
preferably,
A is a straight-chain C₁₅₋₂₀ alkyl group;
Q is -OH.

63. The use of any one of claims 1-23, the pharmaceutical composition of any one of claims 24-27, the kit of any one of claims 28-31, the method of any one of claims 32-36 and 53-56, or the method of claim 43, wherein the lipid or compound has the following structure: 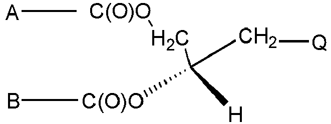 wherein
A is selected from the group consisting of a straight-chain C₁₀₋₂₀ alkyl group and a straight-chain C₁₀₋₂₀ alkenyl group;
B is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₅₋₂₀ alkyl group and a straight-chain C₁₅₋₂₀ alkenyl group;
B is selected from the group consisting of a straight-chain C₁₅₋₂₀ alkyl group and a straight-chain C₁₅₋₂₀ alkenyl group;
Q is -OH;
preferably,
A is selected from the group consisting of a straight-chain C₁₅₋₁₈ alkyl group and a straight-chain C₁₅₋₁₈ alkenyl group;
B is selected from the group consisting of a straight-chain C₁₅₋₁₈ alkyl group and a straight-chain C₁₅₋₁₈ alkenyl group;
Q is -OH.

64. The use of claim 63, the pharmaceutical composition, the kit, or the method, wherein said compound has the following structure: 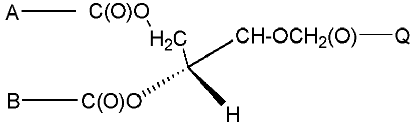 wherein
A is selected from the group consisting of a straight-chain C₁₀₋₂₀ alkyl group and a straight-chain C₁₀₋₂₂ alkenyl group;
B is selected from the group consisting of a straight-chain C₁₀₋₂₀ alkyl group and a straight-chain C₁₀₋₂₂ alkenyl group;
Q is selected from the group consisting of a straight-chain C₁₀₋₂₀ alkyl group and a straight-chain C₁₀₋₂₂ alkenyl group;
preferably,
A is selected from the group consisting of a straight-chain C₁₅₋₁₈ alkyl group and a straight-chain C₁₅₋₂₂ alkenyl group;
B is selected from the group consisting of a straight-chain C₁₅₋₁₈ alkyl group and a straight-chain C₁₅₋₂₂ alkenyl group;
Q is selected from the group consisting of a straight-chain C₁₅₋₁₈ alkyl group and a straight-chain C₁₅₋₂₂ alkenyl group;
preferably,
A is selected from the group consisting of a straight-chain C₁₅₋₁₈ alkyl group and a straight-chain C₁₅₋₂₀ alkenyl group;
B is selected from the group consisting of a straight-chain C₁₅₋₁₈ alkyl group and a straight-chain C₁₅₋₂₀ alkenyl group;
Q is selected from the group consisting of a straight-chain C₁₅₋₁₈ alkyl group and a straight-chain C₁₅₋₂₀ alkenyl group.

65. The use of claim 63, the pharmaceutical composition, the kit, or the method, wherein the compound has the following structure: 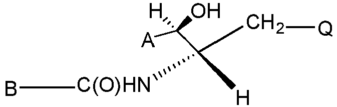 wherein
A is selected from the group consisting of a straight-chain C₁₀₋₂₀ alkyl group and a straight-chain C₁₀₋₂₀ alkenyl group;
B is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₅₋₂₀ alkyl group and a straight-chain C₁₅₋₁₈ alkenyl group;
B is selected from the group consisting of a straight-chain C₁₅₋₁₈ alkyl group and a straight-chain C₁₅₋₁₈ alkenyl group;
Q is -OH;
preferably,
A is a straight-chain C₁₅₋₂₀ alkyl group;
B is a straight-chain C₁₅₋₁₈ alkyl group;
Q is -OH.

66. The use of claim 63, the pharmaceutical composition, the kit, or the method, wherein the compound has the following structure: 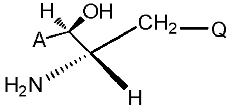 wherein
A is selected from the group consisting of a straight-chain C₁₀₋₂₀ alkyl group and a straight-chain C₁₀₋₂₀ alkenyl group;
Q is -OH;
preferably,
A is selected from the group consisting of a straight-chain C₁₀₋₂₀ alkyl group and a straight-chain C₁₅₋₁₈ alkenyl group;
Q is -OH;
preferably,
A is a straight-chain C₁₅₋₂₀ alkyl group;
Q is -OH.
